# EUROPEAN PATENT APPLICATION

(11) **EP 2 157 183 A1**
(43) Date of publication of application: **24.02.2010**
(21) Application number: 09177897.7
(22) Date of filing: 27.08.2001
(51) Int. Cl.: C12N 15/82, C12N 15/55, C12N 9/50, C12N 5/04, A01H 5/00, A01H 5/10

(54) **Plant polynucleotides encoding prenyl proteases**

(30) Priority: 25.08.2000 US 227794 P
(62) Divisional of application: 01968216.0
(71) Applicant: BASF Plant Science GmbH, 67056 Ludwigshafen (DE)
(72) Inventor: Da Costa e Silva, Oswaldo, Apex, NC 27502 (US); Henkes, Stefan, 14482, Potsdam (DE); Mittendorf, Volker, Hillsborough, NC 27278 (US); Sarria-Millan, Rodrigo, Cary, NC 27519 (US); Chen, Ruoying, Apex, NC 27502 (US); Allen, Damian, Champaign, IL 61822 (US); Härtel, Heiko A., 13088, Berlin (DE)
(74) Representative: Krieger, Stephan Gerhard

(57) **Abstract**

The present invention provides novel polynucleotides encoding plant prenyl protease polypeptides, fragments and homologs thereof. Also provided arc vectors, host cells, antibodies, and recombinant methods for producing said polypeptides. The invention further provides novel polynucleotides encoding plant promoters, polypeptides, fragments and homologs thereof. The invention further relates to methods of applying these novel plant polypeptides to the identification, prevention, and/or conferment of resistence to various plant diseases and/or disorders, particularly drought resistence.

SEQUENCE LISTING

<110> BASF PLANT SCIENCE GMBH

<120> PLANT POLYNUCLEOTIDES ENCODING NOVEL PRENYL PROTEASES

<130> PF ACY015018-2

<140> PCT/US01/26854
<141> 2001-08-27

<150> 60/227,794
<151> 2000-08-25

<160> 27

<170> PatentIn Ver. 2.1

<210> 1
<211> 1398
<212> DNA
<213> Physcomitrella patens

<400> 1
ggatcccccg ggctgcagga attcggcacg agctcaagct gtccaatctg ccagcgcctc 60
tcaagggaat agttagtcaa gagaaatttg agaaagcgca ggcgtacagc ttagacaaga 120
gccgattcca ttttgtgcac gcggctgtga atatcgtgga ggaatcggca attcttctgc 180
tggggttgtt gccgtgggcg tgggataaga gtggatcgtt agtagggaag ctagggtttg 240
atgagaagag cgaaattttg cagacgcttt cttttcttgc ggtgaccacg ttgtggtcgc 300
agatacttga gcttccattc tcgctctact ccacgtttgt catcgaggcc cgccatggct 360
tcaacaagca aaccatatgg ttgtttttac gggatatgat catggggctg gctctcatga 420
tggtggttgg cccacccata gtgtcggcaa ttatctatat tgtgcagaac ggtgggccat 480
atcttgccct ctatctgtgg gcctttatgt tgctgttatc cctcgtgttg atggccctat 540
atcccgttct catcgcgcct cttttcaaca cattcacacc cttgccagaa gggcagcttc 600
gtgccaagat cgagaagctg gcatcctcct tggacttccc attgaagaaa ttgtttgtaa 660
ttgacggttc tactcggtca agccatagca acgcctacat gtatggattt tacaacagca 720
agcgcatcgt tctgtacgac actctaatat cgcaatgtaa gaatgaggaa gaagtagtgg 780
cagttatagc tcatgagctt ggccattgga agctgagcca cactatgtac tcgttcctgg 840
ccatgcaggt gcttacactg ttgcaattcg gaggctatac gcttgttcgg aactctagtg 900
gcctgttttt gagcttcggt ttctccacac agccagtgct tatcgggctg atcctattcc 960
agcacactat tatgcccttc catcatcttg taagctttgc tctcaacctg ttgagccgag 1020
ccttcgaatt tcaggcggat gcgttcgccc gctcattagg gtacagagag ccattgagag 1080
ctggcctgat caagctgcag gaggagaatc tgtctgccat gaacacggat ccgtggtatt 1140
cagcgtatca tcattcacac cccccgcttg ttgagcgatt gcaagctctt gatgaaacgt 1200
ccaagaaaac ggattagaac ttaccccctt cggaccgtag ttgagatttg taggaatata 1260
gcttcttcag gagaaagaaa caaaatgagc tatgtcctag cacatccact gtagaattca 1320
ctgatgaatg acgaatagta catgaacact cattctttaa aaaaaaaaaa aaaaaactcg 1380
aggggggccc cggtaccc 1398

<210> 2
<211> 394
<212> PRT
<213> Physcomitrella patens

<400> 2
Leu Lys Leu Ser Asn Leu Pro Ala Pro Leu Lys Gly Ile Val Ser Gln
1               5                   10                  15

Glu Lys Phe Glu Lys Ala Gln Ala Tyr Ser Leu Asp Lys Ser Arg Phe
            20                  25                  30

His Phe Val His Ala Ala Val Asn Ile Val Glu Glu Ser Ala Ile Leu
        35                  40                  45

Leu Leu Gly Leu Leu Pro Trp Ala Trp Asp Lys Ser Gly Ser Leu Val
    50                  55                  60

Gly Lys Leu Gly Phe Asp Glu Lys Ser Glu Ile Leu Gln Thr Leu Ser
65                  70                  75                  80

Phe Leu Ala Val Thr Thr Leu Trp Ser Gln Ile Leu Glu Leu Pro Phe
                85                  90                  95

Ser Leu Tyr Ser Thr Phe Val Ile Glu Ala Arg His Gly Phe Asn Lys
            100                 105                 110

Gln Thr Ile Trp Leu Phe Leu Arg Asp Met Ile Met Gly Leu Ala Leu
        115                 120                 125

Met Met Val Val Gly Pro Pro Ile Val Ser Ala Ile Ile Tyr Ile Val
    130                 135                 140

Gln Asn Gly Gly Pro Tyr Leu Ala Leu Tyr Leu Trp Ala Phe Met Leu
145                 150                 155                 160

Leu Leu Ser Leu Val Leu Met Ala Leu Tyr Pro Val Leu Ile Ala Pro
                165                 170                 175

Leu Phe Asn Thr Phe Thr Pro Leu Pro Glu Gly Gln Leu Arg Ala Lys
            180                 185                 190

Ile Glu Lys Leu Ala Ser Ser Leu Asp Phe Pro Leu Lys Lys Leu Phe
        195                 200                 205

Val Ile Asp Gly Ser Thr Arg Ser Ser His Ser Asn Ala Tyr Met Tyr
    210                 215                 220

Gly Phe Tyr Asn Ser Lys Arg Ile Val Leu Tyr Asp Thr Leu Ile Ser
225                 230                 235                 240

Gln Cys Lys Asn Glu Glu Glu Val Val Ala Val Ile Ala His Glu Leu
                245                 250                 255

Gly His Trp Lys Leu Ser His Thr Met Tyr Ser Phe Leu Ala Met Gln
            260                 265                 270

Val Leu Thr Leu Leu Gln Phe Gly Gly Tyr Thr Leu Val Arg Asn Ser
        275                 280                 285

Ser Gly Leu Phe Leu Ser Phe Gly Phe Ser Thr Gln Pro Val Leu Ile
    290                 295                 300

Gly Leu Ile Leu Phe Gln His Thr Ile Met Pro Phe His His Leu Val
305                 310                 315                 320

Ser Phe Ala Leu Asn Leu Leu Ser Arg Ala Phe Glu Phe Gln Ala Asp
                325                 330                 335

Ala Phe Ala Arg Ser Leu Gly Tyr Arg Glu Pro Leu Arg Ala Gly Leu
            340                 345                 350

Ile Lys Leu Gln Glu Glu Asn Leu Ser Ala Met Asn Thr Asp Pro Trp
        355                 360                 365

Tyr Ser Ala Tyr His His Ser His Pro Pro Leu Val Glu Arg Leu Gln
    370                 375                 380

Ala Leu Asp Glu Thr Ser Lys Lys Thr Asp
385                 390

<210> 3
<211> 1275
<212> DNA
<213> Arabidopsis thaliana

<400> 3
atggcgattc ctttcatgga aaccgtcgtg ggttttatga tagtgatgta catttttgag 60
acgtatttgg atctgaggca actcactgct ctcaagcttc caactctccc gaaaaccttg 120
gttggtgtaa ttagccaaga gaagtttgag aaatcacgag catacagtct tgacaaaagc 180
tattttcact ttgttcatga gtttgtaact atacttatgg actctgcaat tttgttcttt 240
gggatcttgc cttggttttg gaagatgtct ggagctgttt taccgaggtt gggccttgat 300
ccagagaatg aaatactgca tactctttca ttcttggctg gtgttatgac atggtcacag 360
atcactgatt tgccattttc tttgtactca actttcgtga tcgagtctcg gcatgggttc 420
aacaaacaaa caatatggat gttcattagg gacatgatca aaggaacatt cctctctgtc 480
atactaggcc cacccattgt tgccgcgata attttcatag tccagaaagg aggtccttat 540
cttgccatct atctgtgggc attcatgttt atcctgtctc tagtgatgat gactatatac 600
ccggtcttga tagcaccgct cttcaacaag ttcactcctc ttccagatgg agacctccgg 660
gagaagattg agaaacttgc ttcttctcta aagtttcctt tgaagaagct gtttgttgtc 720
gatggatcta caaggtcaag ccatagcaat gcttacatgt atggtttctt taagaacaaa 780
aggattgttc tttatgatac gttgattcag cagtgcaaga atgaggatga aattgtggcg 840
gttattgcac acgagcttgg acattggaaa ctgaatcaca ctacatactc gttcattgca 900
gttcaaatcc ttgccttctt acaatttgga ggatacactc ttgtcagaaa ctccactgat 960
ctcttcagga gtttcggatt tgatacacag cctgttctca ttggtttgat catatttcag 1020
cacactgtaa taccactgca acatccagta agctttggcc tcaaccttgt tagtcgagcg 1080
tttgagtttc aggctgatgc ttttgctgtg aagcttggct atgcaaaaga tcttcgtcct 1140
actctagtga aactacagga agagaactta tcagcaatga atactgatcc attgtactca 1200
gcttatcact actcacatcc tcctcttgtt gaaaggcttc gagccattga tggagaagac 1260
aagaagacag attaa 1275

<210> 4
<211> 424
<212> PRT
<213> Arabidopsis thaliana

<400> 4
Met Ala Ile Pro Phe Met Glu Thr Val Val Gly Phe Met Ile Val Met
1               5                   10                  15

Tyr Ile Phe Glu Thr Tyr Leu Asp Leu Arg Gln Leu Thr Ala Leu Lys
            20                  25                  30

Leu Pro Thr Leu Pro Lys Thr Leu Val Gly Val Ile Ser Gln Glu Lys
        35                  40                  45

Phe Glu Lys Ser Arg Ala Tyr Ser Leu Asp Lys Ser Tyr Phe His Phe
    50                  55                  60

Val His Glu Phe Val Thr Ile Leu Met Asp Ser Ala Ile Leu Phe Phe
65                  70                  75                  80

Gly Ile Leu Pro Trp Phe Trp Lys Met Ser Gly Ala Val Leu Pro Arg
                85                  90                  95

Leu Gly Leu Asp Pro Glu Asn Glu Ile Leu His Thr Leu Ser Phe Leu
            100                 105                 110

Ala Gly Val Met Thr Trp Ser Gln Ile Thr Asp Leu Pro Phe Ser Leu
        115                 120                 125

Tyr Ser Thr Phe Val Ile Glu Ser Arg His Gly Phe Asn Lys Gln Thr
    130                 135                 140

Ile Trp Met Phe Ile Arg Asp Met Ile Lys Gly Thr Phe Leu Ser Val
145                 150                 155                 160

Ile Leu Gly Pro Pro Ile Val Ala Ala Ile Ile Phe Ile Val Gln Lys
                165                 170                 175

Gly Gly Pro Tyr Leu Ala Ile Tyr Leu Trp Ala Phe Met Phe Ile Leu
            180                 185                 190

Ser Leu Val Met Met Thr Ile Tyr Pro Val Leu Ile Ala Pro Leu Phe
        195                 200                 205

Asn Lys Phe Thr Pro Leu Pro Asp Gly Asp Leu Arg Glu Lys Ile Glu
    210                 215                 220

Lys Leu Ala Ser Ser Leu Lys Phe Pro Leu Lys Lys Leu Phe Val Val
225                 230                 235                 240

Asp Gly Ser Thr Arg Ser Ser His Ser Asn Ala Tyr Met Tyr Gly Phe
                245                 250                 255

Phe Lys Asn Lys Arg Ile Val Leu Tyr Asp Thr Leu Ile Gln Gln Cys
            260                 265                 270

Lys Asn Glu Asp Glu Ile Val Ala Val Ile Ala His Glu Leu Gly His
        275                 280                 285

Trp Lys Leu Asn His Thr Thr Tyr Ser Phe Ile Ala Val Gln Ile Leu
    290                 295                 300

Ala Phe Leu Gln Phe Gly Gly Tyr Thr Leu Val Arg Asn Ser Thr Asp
305                 310                 315                 320

Leu Phe Arg Ser Phe Gly Phe Asp Thr Gln Pro Val Leu Ile Gly Leu
                325                 330                 335

Ile Ile Phe Gln His Thr Val Ile Pro Leu Gln His Pro Val Ser Phe
            340                 345                 350

Gly Leu Asn Leu Val Ser Arg Ala Phe Glu Phe Gln Ala Asp Ala Phe
        355                 360                 365

Ala Val Lys Leu Gly Tyr Ala Lys Asp Leu Arg Pro Thr Leu Val Lys
    370                 375                 380

Leu Gln Glu Glu Asn Leu Ser Ala Met Asn Thr Asp Pro Leu Tyr Ser
385                 390                 395                 400

Ala Tyr His Tyr Ser His Pro Pro Leu Val Glu Arg Leu Arg Ala Ile
                405                 410                 415

Asp Gly Glu Asp Lys Lys Thr Asp
            420

<210> 5
<211> 1275
<212> DNA
<213> Arabidopsis thaliana

<400> 5
atggcgattc ctttcatgga aaccgtcgtg ggttttatga tagtgatgta catttttgag 60
acgtatttgg atctgaggca actcactgct ctcaagcttc caactctccc gaaaaccttg 120
gttggtgtaa ttagccaaga gaagtttgag aaatcacgag catacagtct tgacaaaagc 180
tattttcact ttgttcatga gtttgtaact atacttatgg actctgcaat tttgttcttt 240
gggatcttgc cttggttttg gaagatgtct ggagcagttt taccgaggtt gggccttgat 300
ccagagaatg aaatactgca tactctttca ttcttggctg gtgttatgac atggtcacag 360
atcactgatt tgccattttc tttgtactca actttcgtga tcgagtctcg gcatgggttc 420

aacaaacaaa caatatggat gttcattagg gacatgatca aaggaacatt cctctctgtc 480
atactaggcc cacccattgt tgctgcgata attttcatag tccagaaagg aggtccttat 540
cttgccatct atctgtgggc attcatgttt atcctgtctc tagtgatgat gactatatac 600
ccggtcttga tagcaccgct cttcaacaag ttcactcctc ttccagatgg agacctccgg 660
gagaagattg agaaacttgc ttcttctcta aagtttcctt tgaagaagct gtttgttgtc 720
gatggatcta caaggtcaag ccatagcaat gcttacatgt atggtttctt taagaacaaa 780
aggattgttc tttatgatac gttgattcag cagtgcaaga atgaggatga aattgtggcg 840
gttattgcac acgagcttgg acattggaaa ctgaatcaca ctacatactc gttcattgca 900
gttcaaatcc ttgccttctt acaatttgga ggatacactc ttgtcagaaa ctccactgat 960
ctcttcagga gtttcggatt tgatacacag cctgttctca ttggtttgat catatttcag 1020
cacactgtaa taccactgca acatctagta agctttggcc tgaacctcgt tagtcgagcg 1080
tttgagtttc aggctgatgc ttttgctgtg aagcttggct atgcaaaaga tcttcgtcct 1140
gctctagtga aactacagga agagaactta tcagcaatga acactgatct attgtactca 1200
gcttatcact actcacatcc tcctcttgtt gaaaggcttc gagccattga tggagaagac 1260
aagaagacag attaa 1275

<210> 6
<211> 424
<212> PRT
<213> Arabidopsis thaliana

<400> 6
Met Ala Ile Pro Phe Met Glu Thr Val Val Gly Phe Met Ile Val Met
1               5                   10                  15

Tyr Ile Phe Glu Thr Tyr Leu Asp Leu Arg Gln Leu Thr Ala Leu Lys
            20                  25                  30

Leu Pro Thr Leu Pro Lys Thr Leu Val Gly Val Ile Ser Gln Glu Lys
        35                  40                  45

Phe Glu Lys Ser Arg Ala Tyr Ser Leu Asp Lys Ser Tyr Phe His Phe
    50                  55                  60

Val His Glu Phe Val Thr Ile Leu Met Asp Ser Ala Ile Leu Phe Phe
65                  70                  75                  80

Gly Ile Leu Pro Trp Phe Trp Lys Met Ser Gly Ala Val Leu Pro Arg
                85                  90                  95

Leu Gly Leu Asp Pro Glu Asn Glu Ile Leu His Thr Leu Ser Phe Leu
            100                 105                 110

Ala Gly Val Met Thr Trp Ser Gln Ile Thr Asp Leu Pro Phe Ser Leu
        115                 120                 125

Tyr Ser Thr Phe Val Ile Glu Ser Arg His Gly Phe Asn Lys Gln Thr
    130                 135                 140

Ile Trp Met Phe Ile Arg Asp Met Ile Lys Gly Thr Phe Leu Ser Val
145                 150                 155                 160

Ile Leu Gly Pro Pro Ile Val Ala Ala Ile Ile Phe Ile Val Gln Lys
                165                 170                 175

Gly Gly Pro Tyr Leu Ala Ile Tyr Leu Trp Ala Phe Met Phe Ile Leu
            180                 185                 190

Ser Leu Val Met Met Thr Ile Tyr Pro Val Leu Ile Ala Pro Leu Phe
        195                 200                 205

Asn Lys Phe Thr Pro Leu Pro Asp Gly Asp Leu Arg Glu Lys Ile Glu
    210                 215                 220

Lys Leu Ala Ser Ser Leu Lys Phe Pro Leu Lys Lys Leu Phe Val Val

225                 230                 235                 240

Asp Gly Ser Thr Arg Ser Ser His Ser Asn Ala Tyr Met Tyr Gly Phe
                245                 250                 255

Phe Lys Asn Lys Arg Ile Val Leu Tyr Asp Thr Leu Ile Gln Gln Cys
            260                 265                 270

Lys Asn Glu Asp Glu Ile Val Ala Val Ile Ala His Glu Leu Gly His
        275                 280                 285

Trp Lys Leu Asn His Thr Thr Tyr Ser Phe Ile Ala Val Gln Ile Leu
    290                 295                 300

Ala Phe Leu Gln Phe Gly Gly Tyr Thr Leu Val Arg Asn Ser Thr Asp
305                 310                 315                 320

Leu Phe Arg Ser Phe Gly Phe Asp Thr Gln Pro Val Leu Ile Gly Leu
                325                 330                 335

Ile Ile Phe Gln His Thr Val Ile Pro Leu Gln His Leu Val Ser Phe
            340                 345                 350

Gly Leu Asn Leu Val Ser Arg Ala Phe Glu Phe Gln Ala Asp Ala Phe
        355                 360                 365

Ala Val Lys Leu Gly Tyr Ala Lys Asp Leu Arg Pro Ala Leu Val Lys
    370                 375                 380

Leu Gln Glu Glu Asn Leu Ser Ala Met Asn Thr Asp Leu Leu Tyr Ser
385                 390                 395                 400

Ala Tyr His Tyr Ser His Pro Pro Leu Val Glu Arg Leu Arg Ala Ile
                405                 410                 415

Asp Gly Glu Asp Lys Lys Thr Asp
            420


<210> 7
<211> 1434
<212> DNA
<213> Glycine max

<400> 7
ctaatacgac tcactatagg gcaagcagtg gtaacaacgc agagtacgcg gggggagacg 60
catggttctg aactaattgt tataaataat acctaaaatt ttgagttgtc ctaaacattg 120
gggtttaaac aaatccaatc tctcaatata aaacccaatg atctcaccct cactccgttt 180
ctgatttctc actcttcgtt tctcgttcgg ttcatcagcg tgtgtctcag ccatggcgtt 240
tccctacatg gaagccgttg tcggatttat gatattaatg tacatttttg aaacttactt 300
ggatgtgcga caacataggg ccctcaaact tcctactctt ccaaagactt tagaaggtgt 360
tatcagccaa gagaaatttg agaaatctag agcctatagt cttgataaaa gccacttcca 420
ttttgttcac gagtttgtga caatagtgac agactctaca attttgtact ttggggtatt 480
gccctggttt tggaagaaat caggagattt tatgacaata gctggtttca atgctgagaa 540
tgaaatactg catacccttg ccttcttagc agggctgatg atttggtcac agataacaga 600
tttgcccttt tctctgtact caacttttgt gattgaggcc cgtcatggtt ttaataagca 660
aacaccatgg ttattcttta gggacatgct taaaggaatt ttcctttccg taataattgg 720
tccacctatt gtggctgcaa tcattgtaat agtacagaaa ggaggtccat acttggccat 780
ctatctttgg gtttttacgt ttggtctttc tattgtgatg atgacccttt atccagtact 840
aatagctcca ctcttcaata agttcactcc acttccagat ggtcaactca gggagaaaat 900
cgagaaactt gcttcctccc tcaactatcc gttaaagaaa ctatttgttg tcgatggatc 960
cacaagatca agtcacagca atgcctatat gtatggattc ttcaagaaca agaggattgt 1020
cctttatgac acattaattc aacagtgcaa agacgatgag gaaattgttg ctgttattgc 1080
ccatgagttg ggacactgga agctcaacca tactgtgtac acatttgttg ctatgcagat 1140
tcttacactt ctacaatttg gaggatatac actagtgcga aattcagctg atctgtatcg 1200
aagctttggg tttgatacgc agccagtcct cattgggctc atcatatttc agcatactgt 1260

aatcccactt cagcaattgg tcagctttgg tctgaaccta gtcagccgat catttgaatt 1320
tcaggctgat ggctttgcca agaagcttgg atatgcatct ggattacgcg gtggtcttgt 1380
gaaactacag gaggagaatc tgtcagctat gaatacagat ccttgctcgt gccg 1434

<210> 8
<211> 400
<212> PRT
<213> Glycine max

<400> 8
Met Ala Phe Pro Tyr Met Glu Ala Val Val Gly Phe Met Ile Leu Met
1               5                   10                  15

Tyr Ile Phe Glu Thr Tyr Leu Asp Val Arg Gln His Arg Ala Leu Lys
            20                  25                  30

Leu Pro Thr Leu Pro Lys Thr Leu Glu Gly Val Ile Ser Gln Glu Lys
        35                  40                  45

Phe Glu Lys Ser Arg Ala Tyr Ser Leu Asp Lys Ser His Phe His Phe
    50                  55                  60

Val His Glu Phe Val Thr Ile Val Thr Asp Ser Thr Ile Leu Tyr Phe
65                  70                  75                  80

Gly Val Leu Pro Trp Phe Trp Lys Lys Ser Gly Asp Phe Met Thr Ile
                85                  90                  95

Ala Gly Phe Asn Ala Glu Asn Glu Ile Leu His Thr Leu Ala Phe Leu
            100                 105                 110

Ala Gly Leu Met Ile Trp Ser Gln Ile Thr Asp Leu Pro Phe Ser Leu
        115                 120                 125

Tyr Ser Thr Phe Val Ile Glu Ala Arg His Gly Phe Asn Lys Gln Thr
    130                 135                 140

Pro Trp Leu Phe Phe Arg Asp Met Leu Lys Gly Ile Phe Leu Ser Val
145                 150                 155                 160

Ile Ile Gly Pro Pro Ile Val Ala Ala Ile Ile Val Ile Val Gln Lys
                165                 170                 175

Gly Gly Pro Tyr Leu Ala Ile Tyr Leu Trp Val Phe Thr Phe Gly Leu
            180                 185                 190

Ser Ile Val Met Met Thr Leu Tyr Pro Val Leu Ile Ala Pro Leu Phe
        195                 200                 205

Asn Lys Phe Thr Pro Leu Pro Asp Gly Gln Leu Arg Glu Lys Ile Glu
    210                 215                 220

Lys Leu Ala Ser Ser Leu Asn Tyr Pro Leu Lys Lys Leu Phe Val Val
225                 230                 235                 240

Asp Gly Ser Thr Arg Ser Ser His Ser Asn Ala Tyr Met Tyr Gly Phe
                245                 250                 255

Phe Lys Asn Lys Arg Ile Val Leu Tyr Asp Thr Leu Ile Gln Gln Cys
            260                 265                 270

Lys Asp Asp Glu Glu Ile Val Ala Val Ile Ala His Glu Leu Gly His
        275                 280                 285

Trp Lys Leu Asn His Thr Val Tyr Thr Phe Val Ala Met Gln Ile Leu

290                 295                 300

Thr Leu Leu Gln Phe Gly Gly Tyr Thr Leu Val Arg Asn Ser Ala Asp
305                 310                 315                 320

Leu Tyr Arg Ser Phe Gly Phe Asp Thr Gln Pro Val Leu Ile Gly Leu
                325                 330                 335

Ile Ile Phe Gln His Thr Val Ile Pro Leu Gln Gln Leu Val Ser Phe
            340                 345                 350

Gly Leu Asn Leu Val Ser Arg Ser Phe Glu Phe Gln Ala Asp Gly Phe
        355                 360                 365

Ala Lys Lys Leu Gly Tyr Ala Ser Gly Leu Arg Gly Gly Leu Val Lys
    370                 375                 380

Leu Gln Glu Glu Asn Leu Ser Ala Met Asn Thr Asp Pro Cys Ser Cys
385                 390                 395                 400


<210> 9
<211> 1301
<212> DNA
<213> Zea mays

<400> 9
acgaggctga gtgctgagaa tgagataata cacacccttg ctttcttagc tggttccatg 60
gtttggtcgc agattacaga cttgccgttc tctctctatt caacttttgt tatagaggct 120
cgacatggtt ttaacaagca aactatatgg ctcttcatta gggatatgat caaaggaatt 180
ttactatcca tgatattggg gccaccaatc gtggctgcta tcatctacat agtacagatt 240
ggaggacctt acctggctat atatctctgg ggttttatgt ttgtattagc tctactgatg 300
atgacaatat accccattgt gatagctcct ctgttcaaca agttcactcc tcttcctgaa 360
ggagtcctca gggaaaaaat agagaagctg gcagcttccc tcaagtttcc tttgaaaaag 420
cttttcgtgg tagatgggtc taccagatca agccacagta atgcctacat gtatggtttt 480
ttcaagaaca agcgcatagt actctatgac acattgattc agcagtgtag caatgaggat 540
gagatagttt ctgttatagc acatgaactt ggacactgga aactcaatca tactgtctat 600
tcctttgtag ctgtccagct gcttatgttt cttcaatttg gaggatatac tctagtaagg 660
agctccaaag atctatttgg aagttttggc ttcaaggacc agccagtaat aattggattg 720
atcattttcc cgcacaccat aatacccatc caacaccttc tgagctttcg cctgaacctt 780
gtcagcagag catttgaatt tcaggctgat gcctttgcca agaaccttgg atatgcccct 840
cagctccgag cagcccttgt taaactacag gaggagaact tgtctgcgat gaacaccgat 900
ccttggtatt cggcatatca ctactcccac ccaccactcg tcgagaggct gcaagctttg 960
gaagattcag acgacaaaaa agaagattag tcgatccttg tatgaggttt acatatggat 1020
ttttccctgc cacatgcaca ccgattcagt gcttggatgg tgagggtttt gacataggag 1080
tgttgtcaaa gctttagagt gcatctttcg gtcaggtgca acagcctttc ggtcattgag 1140
acatataagc gaattagcta ttaaaaaaaa cagaactgtt gcatcaaaaa aaaaaaaaaa 1200
aaagaaacaa aaaaaaaaaa aaaaaaaaaa aagaaaaaaa aaaaaaaaaa aaaagtgctc 1260
tgcgttgtta ccactgcttg ccctatagtg atcgtatcag a                     1301


<210> 10
<211> 329
<212> PRT
<213> Zea mays

<400> 10
Thr Arg Leu Ser Ala Glu Asn Glu Ile Ile His Thr Leu Ala Phe Leu
1               5                   10                  15

Ala Gly Ser Met Val Trp Ser Gln Ile Thr Asp Leu Pro Phe Ser Leu
            20                  25                  30

Tyr Ser Thr Phe Val Ile Glu Ala Arg His Gly Phe Asn Lys Gln Thr
        35                  40                  45

Ile Trp Leu Phe Ile Arg Asp Met Ile Lys Gly Ile Leu Leu Ser Met
     50                  55                  60

Ile Leu Gly Pro Pro Ile Val Ala Ala Ile Ile Tyr Ile Val Gln Ile
 65                  70                  75                  80

Gly Gly Pro Tyr Leu Ala Ile Tyr Leu Trp Gly Phe Met Phe Val Leu
                 85                  90                  95

Ala Leu Leu Met Met Thr Ile Tyr Pro Ile Val Ile Ala Pro Leu Phe
            100                 105                 110

Asn Lys Phe Thr Pro Leu Pro Glu Gly Val Leu Arg Glu Lys Ile Glu
        115                 120                 125

Lys Leu Ala Ala Ser Leu Lys Phe Pro Leu Lys Lys Leu Phe Val Val
    130                 135                 140

Asp Gly Ser Thr Arg Ser Ser His Ser Asn Ala Tyr Met Tyr Gly Phe
145                 150                 155                 160

Phe Lys Asn Lys Arg Ile Val Leu Tyr Asp Thr Leu Ile Gln Gln Cys
                165                 170                 175

Ser Asn Glu Asp Glu Ile Val Ser Val Ile Ala His Glu Leu Gly His
            180                 185                 190

Trp Lys Leu Asn His Thr Val Tyr Ser Phe Val Ala Val Gln Leu Leu
        195                 200                 205

Met Phe Leu Gln Phe Gly Gly Tyr Thr Leu Val Arg Ser Ser Lys Asp
    210                 215                 220

Leu Phe Gly Ser Phe Gly Phe Lys Asp Gln Pro Val Ile Ile Gly Leu
225                 230                 235                 240

Ile Ile Phe Pro His Thr Ile Ile Pro Ile Gln His Leu Leu Ser Phe
                245                 250                 255

Arg Leu Asn Leu Val Ser Arg Ala Phe Glu Phe Gln Ala Asp Ala Phe
            260                 265                 270

Ala Lys Asn Leu Gly Tyr Ala Pro Gln Leu Arg Ala Ala Leu Val Lys
        275                 280                 285

Leu Gln Glu Glu Asn Leu Ser Ala Met Asn Thr Asp Pro Trp Tyr Ser
    290                 295                 300

Ala Tyr His Tyr Ser His Pro Pro Leu Val Glu Arg Leu Gln Ala Leu
305                 310                 315                 320

Glu Asp Ser Asp Asp Lys Lys Glu Asp
                325

<210> 11
<211> 2047
<212> DNA
<213> Arabidopsis thaliana

<400> 11
   ttgatgatga aaaagagaag atcgaaacca agtcttacaa agaagagcga tttctctcaa 60
   aggggaaggt aaaattgaca aatccacgcg ctagcttttc acgttcactc acgcgctaca 120
   ttttgtataa tccacaaaac tttcaataaa ttacagaaaa accttgataa atttttacca 180
   taacaacaag atccctgata ttattttcaa attgactcat aaagcattac aaaaggagat 240
   ggtttttctg aaacatgaaa tggttggtta cagaagacga tacatacaat aggcagctat 300
   gttcatcatc tctttccttt tcctttagca tcaaagtgat gagactttag tttcttcttc 360
   cgcactatcg cgcctgtgct gccaccacct ccttccctga aaggcattcc cattagagcc 420
   aatagtttct gtccttcttg atcgctttta gccgttgtgc tgatgcatac atccattcct 480
   ctcgtttttc caacggcatc aaacctagag tgtaataaac caaccaacca acatagagtt 540
   tagttgctcg gttgtaaaca aagagtggga ctaaagagaa accatgcttg atcttgttga 600
   accagcggat aacaagttag tagttttgat tttgagggta gatacaatag aactaacctg 660
   atttcaggga atacaccttg gtctttcaca ccaatactgt agtttccgtt cccatcaaag 720
   ctactgggac tcacaccttg gaaatctcga gttctcggaa gggctaagtt gataagacga 780
   tccaagaagg agtacattac ctgtcacaac agtcaaaatc acaaaatgtt aatacataaa 840
   taactcaaaa ggaaagtttg attagtcaag agaaatatag ctcacatctc ctctgagagt 900
   gacagcaatc ccaagaggtt gatcttccct gatcttgaaa gtagcaatgg aagctctagc 960
   tcgtgtctta ataggtttct gccctgtgat aagcgcgata tccttcatcg cagcctccaa 1020
   acccttgtcg ttctgcgccg catctccaat accacaattc actacaatct tctgtacctt 1080
   tggaacctgg tagagaagat gtccaagcag atatacaaca atgatgagat tgacaaattc 1140
   acattcacat ttgtaaatgg cacatcccaa cagcacgatt ttatcaacac aacaatctta 1200
   aatctaggct tcttcactac attttctaga agaaatcaat tcaaacttat cattaaaagc 1260
   aaattaggtt taacattcgc tcaaaaaatt cgaattcact gagaattaga cttcaatcaa 1320
   tcgcaacaga acaaaaacta ggtttgagct cagaggggaa ggatttgggg ttacctggtg 1380
   aatattaacg tacttgaact cttctttgag cgcagggata atcctctcga ggtaagcggt 1440
   tttgaggcgt tgagtttctt cggcttcaga tttctcgacc agtacagttc cagacgccga 1500
   gactttcacc acgtttctga gcggcggaga gagcattcgt gcggaggatg gagccgctaa 1560
   tggtgagaaa cgtccgtgaa acgaagaagc ggaagactgc agaagcgaag gagacgccat 1620
   tgtcgaagct ccaagtggat aaagtgtgaa gtgagagctc tcggcgttcg ttgttgatta 1680
   aacccaatgg caccttcgta atttgttgac agtttgagga taaggagttt tccgtttagc 1740
   ccctttaaaa cataatattt caactaaggc cccaatattt gataatatac tattatactt 1800
   agagatttta gataaaatat aggggttgtt tcatatgggc ctaaatctca gcccgtttac 1860
   tatttgggct tctaaggtat aacccgtacc cgtgtttttg ttgttttaca tatccacacc 1920
   gacctgagaa gagtcaaaaa cgaaaaacct ctcttttgtc gttcctctgc tttcttcgat 1980
   ttgcttctgc tttcttcgat ttgcttctgc tttcatcgcg gttcaggtca ctcttttctc 2040
   agccatg 2047

<210> 12
<211> 18
<212> DNA
<213> Artificial Sequence

<220>
<223> Description of Artificial Sequence: Primer

<400> 12
caggaaacag ctatgacc 18

<210> 13
<211> 19
<212> DNA
<213> Artificial Sequence

<220>
<223> Description of Artificial Sequence: Primer

<400> 13
ctaaagggaa caaaagctg 19

<210> 14
<211> 18
<212> DNA
<213> Artificial Sequence

<220>
<223> Description of Artificial Sequence: Primer

<400> 14
tgtaaaacga cggccagt 18

<210> 15
<211> 33
<212> DNA
<213> Artificial Sequence

<220>
<223> Description of Artificial Sequence: Primer

<400> 15
ccgttaacag ccatggcgat tcctttcatg gaa                                33


<210> 16
<211> 30
<212> DNA
<213> Artificial Sequence

<220>
<223> Description of Artificial Sequence: Primer

<400> 16
gtcccgggac ttaatctgtc ttcttgtctt                                    30


<210> 17
<211> 8
<212> PRT
<213> Artificial Sequence

<220>
<223> Description of Artificial Sequence: Synthetic Flag
      peptide

<400> 17
Asp Tyr Lys Asp Asp Asp Asp Lys
  1               5


<210> 18
<211> 733
<212> DNA
<213> Homo sapiens

<400> 18
gggatccgga gcccaaatct tctgacaaaa ctcacacatg cccaccgtgc ccagcacctg 60
aattcgaggg tgcaccgtca gtcttcctct tccccccaaa acccaaggac accctcatga 120
tctcccggac tcctgaggtc acatgcgtgg tggtggacgt aagccacgaa gaccctgagg 180
tcaagttcaa ctggtacgtg gacggcgtgg aggtgcataa tgccaagaca aagccgcggg 240
aggagcagta caacagcacg taccgtgtgg tcagcgtcct caccgtcctg caccaggact 300
ggctgaatgg caaggagtac aagtgcaagg tctccaacaa agccctccca acccccatcg 360
agaaaaccat ctccaaagcc aaagggcagc cccgagaacc acaggtgtac accctgcccc 420
catcccggga tgagctgacc aagaaccagg tcagcctgac ctgcctggtc aaaggcttct 480
atccaagcga catcgccgtg gagtgggaga gcaatgggca gccggagaac aactacaaga 540
ccacgcctcc cgtgctggac tccgacggct ccttcttcct ctacagcaag ctcaccgtgg 600
acaagagcag gtggcagcag gggaacgtct tctcatgctc cgtgatgcat gaggctctgc 660
acaaccacta cacgcagaag agcctctccc tgtctccggg taaatgagtg cgacggccgc 720
gactctagag gat                                                      733


<210> 19
<211> 62
<212> DNA
<213> Artificial Sequence

<220>
<223> Description of Artificial Sequence: Primer

<400> 19
ggccagtgaa ttgtaatacg ctcactatag ggaggcggtt tttttttttt tttttttttt 60
tt                                                                62


<210> 20
<211> 25
<212> PRT
<213> Artificial Sequence

<220>
<223> Description of Artificial Sequence: Signal peptide
      sequence

<400> 20
Met Lys Asn Thr Ser Ser Leu Cys Leu Leu Leu Leu Val Val Leu Cys
  1               5                  10                  15

Ser Leu Thr Cys Asn Ser Gly Gln Ala
             20                  25


<210> 21
<211> 25
<212> DNA
<213> Artificial Sequence

<220>
<223> Description of Artificial Sequence: Synthetic
      oligonucleotide

<400> 21
agcagccacg attggtggcc ccaat                                       25


<210> 22
<211> 25
<212> DNA
<213> Artificial Sequence

<220>
<223> Description of Artificial Sequence: Synthetic
      oligonucleotide

<400> 22
gggccaccaa tcgtggctgc tatca                                       25


<210> 23
<211> 26
<212> DNA
<213> Artificial Sequence

<220>
<223> Description of Artificial Sequence: Synthetic
      oligonucleotide

<400> 23
cgcagccagt cctcattggg ctcatc                                      26


<210> 24
<211> 24

<212> DNA
<213> Artificial Sequence

<220>
<223> Description of Artificial Sequence: Synthetic
      oligonucleotide

<400> 24
cggatagttg agggaggaag caag                                              24


<210> 25
<211> 475
<212> PRT
<213> Homo sapiens

<400> 25


<210> 26
<211> 6
<212> PRT
<213> Artificial Sequence

<220>
<223> Description of Artificial Sequence: 6X His tag

<400> 26
His His His His His His
  1               5


<210> 27
<211> 403
<212> PRT
<213> Saccharomyces cerevisiae

<400> 27
Met Phe Asp Leu Lys Thr Ile Leu Asp His Pro Asn Ile Pro Trp Lys
  1               5                  10                  15

Leu Ile Ile Ser Gly Phe Ser Ile Ala Gln Phe Ser Phe Glu Ser Tyr
             20                  25                  30

Leu Thr Tyr Arg Gln Tyr Gln Lys Leu Ser Glu Thr Lys Leu Pro Pro
        35                  40                  45

Val Leu Glu Asp Glu Ile Asp Asp Glu Thr Phe His Lys Ser Arg Asn
    50                  55                  60

Tyr Ser Arg Ala Lys Ala Lys Phe Ser Ile Phe Gly Asp Val Tyr Asn
65                  70                  75                  80

Leu Ala Gln Lys Leu Val Phe Ile Lys Tyr Asp Leu Phe Pro Lys Ile
                85                  90                  95

Trp His Met Ala Val Ser Leu Leu Asn Ala Val Leu Pro Val Arg Phe
            100                 105                 110

His Met Val Ser Thr Val Ala Gln Ser Leu Cys Phe Leu Gly Leu Leu
        115                 120                 125

Ser Ser Leu Ser Thr Leu Val Asp Leu Pro Leu Ser Tyr Tyr Ser His
    130                 135                 140

Phe Val Leu Glu Glu Lys Phe Gly Phe Asn Lys Leu Thr Val Gln Leu
145                 150                 155                 160

Trp Ile Thr Asp Met Ile Lys Ser Leu Thr Leu Ala Tyr Ala Ile Gly
                165                 170                 175

Gly Pro Ile Leu Tyr Leu Phe Leu Lys Ile Phe Asp Lys Phe Pro Thr
            180                 185                 190

Asp Phe Leu Trp Tyr Ile Met Val Phe Leu Phe Val Val Gln Ile Leu
        195                 200                 205

Ala Met Thr Ile Ile Pro Val Phe Ile Met Pro Met Phe Asn Lys Phe
    210                 215                 220

Thr Pro Leu Glu Asp Gly Glu Leu Lys Lys Ser Ile Glu Ser Leu Ala
225                 230                 235                 240

Asp Arg Val Gly Phe Pro Leu Asp Lys Ile Phe Val Ile Asp Gly Ser
                245                 250                 255

Lys Arg Ser Ser His Ser Asn Ala Tyr Phe Thr Gly Leu Pro Phe Thr
            260                 265                 270

Ser Lys Arg Ile Val Leu Phe Asp Thr Leu Val Asn Ser Asn Ser Thr
        275                 280                 285

Asp Glu Ile Thr Ala Val Leu Ala His Glu Ile Gly His Trp Gln Lys
    290                 295                 300

Asn His Ile Val Asn Met Val Ile Phe Ser Gln Leu His Thr Phe Leu
305                 310                 315                 320

Ile Phe Ser Leu Phe Thr Ser Ile Tyr Arg Asn Thr Ser Phe Tyr Asn
                325                 330                 335

Thr Phe Gly Phe Phe Leu Glu Lys Ser Thr Gly Ser Phe Val Asp Pro
            340                 345                 350

Val Ile Thr Lys Glu Phe Pro Ile Ile Ile Gly Phe Met Leu Phe Asn
        355                 360                 365

Asp Leu Leu Thr Pro Leu Glu Cys Ala Met Gln Phe Val Met Ser Leu
    370                 375                 380

Ile Ser Arg Thr His Glu Tyr Gln Ala Asp Ala Tyr Ala Lys Lys Leu
385                 390                 395                 400

Gly Tyr Lys

## Description

### FIELD OF THE INVENTION

The present invention provides novel polynucleotides encoding plant prenyl protease polypeptides, fragments and homologs thereof. Also provided are vectors, host cells, antibodies, and recombinant methods for producing said polypeptides. The invention further relates to methods of applying these novel plant polypeptides to the identification, prevention, and/or conferment of resistence to various plant diseases and/or disorders, particularly drought resistence, and/or to manipulate the amount of seed storage compounds, particularly oils, sugars and protein.

### BACKGROUND OF THE INVENTION

Drought is one of the most limiting factors in plant growth and productivity. Crop and yield losses due to drought spells in crops such as soybeans, corn, rice and cotton represent a significant economic factor. Moreover, drought is also responsible for food shortages in many countries worldwide. Developing crops tolerant to drought is a strategy that has potential to alleviate some of these adverse situations.

Traditional plant breeding strategies to develop new lines of plants that exhibit tolerance to drought are relatively slow and require specific tolerant lines for crossing with the desired commercial lines. Limited germplasm resources for drought tolerance and incompatibility in crosses between distantly related plant species therefore represent significant problems encountered in conventional breeding. In contrast, plant genetic transformation and availability of useful genes subjected to specific expression patterns allow one to generate drought-tolerant plants using transgenic approaches.

Plants are exposed during their entire life cycle to conditions of reduced environmental water content. Most plants have evolved strategies to protect themselves against these conditions of desiccation. However, if the severity and duration of the drought conditions are extensive, the effects on plant development, growth and yield of most crop plants are profound.

The physiology of a drought-stress plant is dramatically altered as compared with a plant grown under normal conditions. Most of the changes and their causes remain uncharacterized. Abscisic acid (ABA) plays a central role in mediating the processes between desiccation perception and cellular changes. ABA increases readily upon the onset of cell desiccation. This increase causes the closure of stomata, thereby decreasing water loss through transpiration.

Storage lipids in seeds are synthesized from carbohydrate derived precursors. Plants do have a complete glycolytic pathway in the cytosol (Plaxton 1996, Annu. Rev. Plant Physiol. Plant Mol. Biol. 47: 185-214) and it has been shown that a complete pathway also exists in the plastids of rapeseeds (Kang & Rawsthorne 1994, Plant J. 6: 795-805). Sucrose is the primary source of carbon and energy, transported from the leaves into the developing seeds. During the storage phase of seeds sucrose is converted in the cytosol to provide the metabolic precursors glucose-6-phosphate and pyruvate. These are transported into the plastids and converted into acetyl-CoA that serves as the primary precursor for the synthesis of fatty acids. Although several nucleic acids that are involved in enzymatic steps of the metabolism of lipids, fatty acids and starch have been cloned and identified, there are likely a multitude of such plant nucleic acids that have yet to be identified. Phenotypic analysis of several oilseed plants and other mutated plants has revealed other putative proteins involved in plant lipid metabolism, but the prior art has yet to describe the genomic location of these proteins or the sequence of the nucleic acids that encode them.

The regulation of protein phosphorylation by kinases and phosphatases is accepted as a universal mechanism of cellular control (Cohen 1992, Trends Biochem. Sci. 17: 408-413), and Ca²⁺ and calmodulin signals are frequently transduced via Ca²⁺ and calmodulin-dependent kinases and phosphatases (Roberts & Harmon 1992, Annu. Rev. Plant Physiol. Plant Mol. Biol. 43: 375-414.). Okadaic acid, a protein phosphatase inhibitor, has been found to affect both gibberellic (GA) and absisic acid (ABA) pathways (Kuo et al. 1996, Plant Cell. 8: 259-269). Although the molecular basis of GA and ABA signal transduction remains poorly understood, it seems well established that the two phytohormones are involved in overall regulatory processes in seed development (e.g. Ritchie & Gilroy 1998, Plant Physiol. 116: 765-776; Arenas-Huertero et al. 2000, Genes Dev. 14: 2085-2096). Likewise, the plant hormones ethylene (e.g. Zhou et al. 1998, Proc. Natl. Acad. Sci. USA 95: 10294-10299; Beaudoin et al. 2000, Plant Cell 2000: 1103-1115) and auxin (e.g. Colon-Carmona et al. 2000, Plant Physiol. 124:1728-1738) are involved in controlling plant development as well.

Protein farnesylation, the addition of a C-terminal, 15-carbon chain to proteins and subsequent processing, has been identified as being crucial for the mediating role of ABA in the desiccation-signal transduction chain (1). In short, protein farnesylation is required for ABA-induced stomata closure, thus for control of water loss.

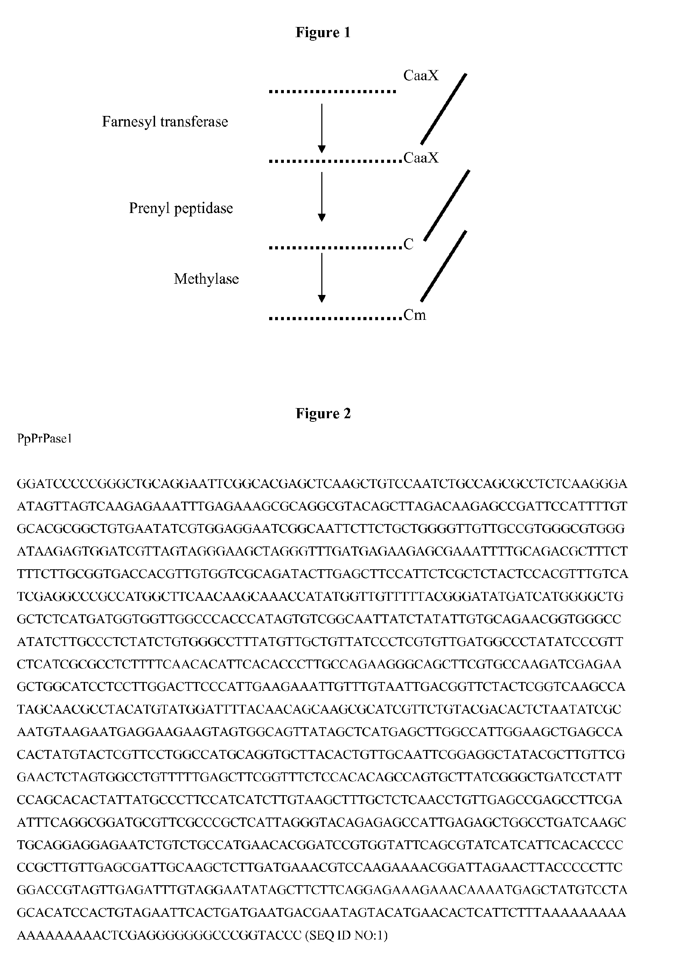

Protein farnesylation is a three-step enzymatic reaction as shown in Fig 1. The drought-tolerant phenotype of the eral Arabidopsis mutant is due to a null mutation in the beta-subunit of the enzyme farnesyl transferase (FTase), the first enzyme in the protein farnesylation pathway.

Currently, sequences corresponding to the clones of the other enzymes involved in protein farnesylation, namely prenyl protease (PrPase) and Methylase, have not been described in the plant literature. Therefore, there is a need in the art to identify plant genes encoding these protein farnesylation enzymes as another opportunity to generate plants tolerant to desicating conditions (e.g., drought).

### BRIEF SUMMARY OF THE INVENTION

The present invention provides novel polynucleotides encoding either fully active prenyl peptidase polypetides or fragments thereof from Physcomitrella patens, Arabidopsis thaliana, soybeans (Glycine max) and corn (Zea mays).

The present invention further provides a general method for engineering drought-tolerant plants. Said method being generally applicable to all plants.

Further provided is the promoter of the Arabidopsis FTase gene. This promoter is expressed most strongly in guard-cells, i.e. it is a guard-cell specific promoter.

Another aspect of this invention provides yeast expression vectors used to produce large quantities of the Arabidopsis PrPase in yeast.

Further described in this invention are transformation vectors used to transform Arabidopsis, Rapeseed, soybeans and corn plants.

Also encompassed by the present invention are deposits with the ATCC of the polynucleotides encoding the polypeptides of the present invention.

Moreover, the invention provides methods of applying the polynucleotides and polypeptides of the invention for creating transgenic plants with desirable traits, which include, but are not limited, to enhanced plant defense, drought tolerance, salt tolerance, ultraviolet(uv) tolerance, enhanced flower development, terpene synthesis and increased formation of seed storage compounds, like oil, sugars and proteins.

Further provided is the promoter of the Arabidopsis USP gene. This promoter is expressed most strongly during seed developmental stages, i.e. it is a seed-specific promoter. The invention provides antibodies, specific to polypeptides of the present invention. Further, the invention provides methods of using antibodies of the present invention to modulate plant phenotype, both functionally and morphologically. Finally, the invention provides methods for more particularly refining the function of the polynucleotides and/or polypeptides of the present invention.

The present invention fulfills a need in the art, in part, by providing the isolated polynucleotide and polypeptide sequences of five plant-derived PrPases. Namely, we describe the PrPase sequences from moss (Physcomitrella patens), two PrPase sequences from Arabidopsis thaliana, one PrPase from soybeans (Glycine max), and one PrPase from corn (Zea mays).

Moreover, the present invention provides the first results suggesting reduced PrPase mRNA quantity (i.e., reduced PrPase gene expression) in an Arabidopsis plant directly correlates with increased drought tolerance as compared to untransformed control plants. The present invention also describes methods of engineering drought-tolerant Rapeseed, soybeans, and corn plants strains generated through decreased PrPase expression.

The conservation of the PrPase activity in eukaryotes, ranging from humans to lower plants, strongly suggests the PrPase enzyme is an essential enzyme and is present in all eukaryotes. Therefore, the described method of engineering drought-tolerant plants should be generally applicable to all plants.

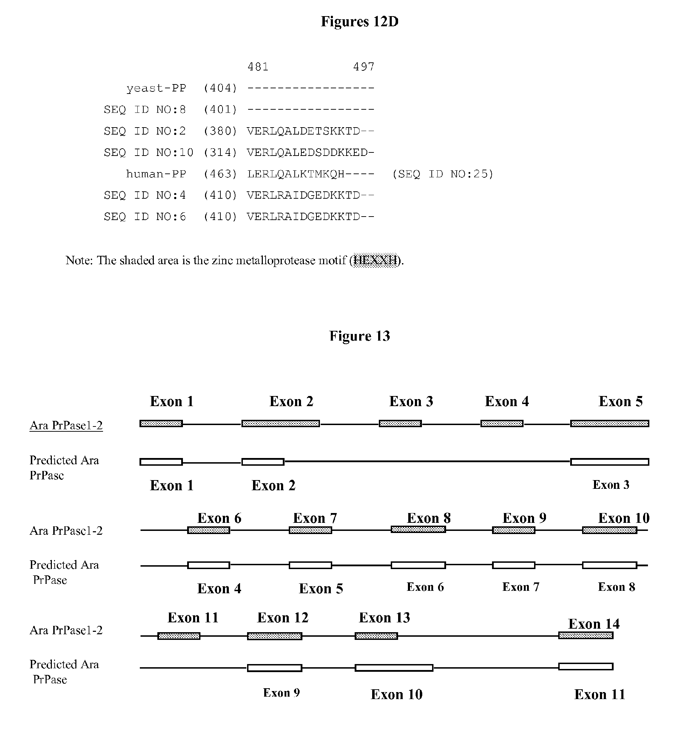

The previously described ORF for the Arabidopsis PrPase (AF007269 (GenBank accession number, gene= "A_IG002N01.21) from 24979 to 28076) was predicted by the computer program (Genefinder (P.Green and L.Hillier, www.ncbi.nlm.nih.gov)) and deposited in the GenBank database. It does not reflect the real ORF for this gene as shown in Figure 13. This clearly demonstrates that both AtPrPase1-2 are novel sequences.

Farnesylation of plant chaperones has been positively implicated with the biological function of these proteins in stress responses. Over-expression of the farnesylation pathway results in increased tolerance to heat, drought and salt stress. In addition, protein farnesylation is also involved in positive regulation of cell cycle control. An increase of protein farnesylation results in increased cell proliferation, ultimately leading to both increased growth and increased accumulation of seed storage compounds.

Moreover, the present invention provides novel polynucleotides encoding plant PrPase polypeptides, fragments and homologs thereof. Also provided are vectors, host cells, antibodies, and recombinant methods for producing said polypeptides. The invention further relates to methods of applying these novel plant polypeptides to the identification, prevention, and/or conferment of resistence to various plant diseases and/or disorders, and/or to the increase in the amount of seed storage compounds.

### BRIEF DESCRIPTION OF THE FIGURES/DRAWINGS

**Figure 1** **-** Schematic representation of the protein farnesylation pathway. This figure identifies the known enzymes involved in the protein farnesylation pathway, in addition to their functional relationship. For illustration purposes, a prospective target protein is represented by a dotted line; while the solid line represents the 15-carbon atom chain added to the C-terminus of the target protein at the "CaaX" site. The 15-carbon chain is added to a conserved Cysteine (C) by the enzyme farnesyl transferase (Ftase). The last three amino acid residues (aaX) are cleaved by the enzyme prenyl peptidase (PrPase). Lastly, the modified Cysteine is methylated by a methylase to create the final active product of the protein farnesylation pathway.
**Figure 2** shows the polynucleotide sequence of the partial PrPase from Physcomitrella patens (SEQ ID NO: 1) (Clone ID No: PpPrPase1) of the present invention. The polynucleotide sequence contains a sequence of 1398 nucleotides.
**Figure 3** shows the deduced amino acid sequence of SEQ ID NO: 1 (SEQ ID NO: 2). The polypeptide sequence contains a sequence of 394 amino acids. The standard one-letter abbreviation for amino acids is used to illustrate the deduced amino acid sequence.
**Figure 4** **-** Nucleotide sequence of the full-length PrPase (AtPrPase1) from Arabidopsis thaliana (SEQ ID NO: 3) (Clone ID No: AtPrPase1) of the present invention. The polynucleotide sequence contains a sequence of 1275 nucleotides.
**Figure 5** **-** Deduced amino acid sequence of SEQ ID NO: 3 (SEQ ID NO: 4). The polypeptide sequence contains a sequence of 424 amino acids. The standard one-letter abbreviation for amino acids is used to illustrate the deduced amino acid sequence.
**Figure 6** **-** Nucleotide sequence of a full-length PrPase (AtPrPase2) from Arabidopsis thaliana (SEQ ID NO: 5) (Clone ID No: AtPrPase2) of the present invention. The polynucleotide sequence contains a sequence of 1275 nucleotides. SEQ ID NO:3 and SEQ ID NO:5 are full-length Arabidopsis AtPrPases with 8 nucleotide differences at nucleotide positions: 276, 504, 1046, 1062, 1068, 1141, 1182, and 1190.
**Figure 7** **-** Deduced amino acid sequence of SEQ ID NO: 5 (SEQ ID NO: 6) The polypeptide sequence contains a sequence of 424 amino acids. The standard one-letter abbreviation for amino acids is used to illustrate the deduced amino acid sequence. SEQ ID NO:4 and SEQ ID NO:6 are full-length Arabidopsis AtPrPases with 3 amino acid differences at amino acid positions: 349, 381, and 397.
**Figure 8** **-** Nucleotide sequence of the full-length PrPase (GmPrPasel) from soybeans (SEQ ID NO: 7) (Clone ID No: GmPrPase1) of the present invention. The polynucleotide sequence contains a sequence of 1434 nucleotides.
**Figure 9** **-** Deduced amino acid sequence of SEQ ID NO: 7 (SEQ ID NO: 8). The polypeptide sequence contains a sequence of 400 amino acids. The standard one-letter abbreviation for amino acids is used to illustrate the deduced amino acid sequence.
**Figure 10** **-** Nucleotide sequence of a partial PrPase from (ZmPrPasel) corn (SEQ ID NO: 9). (Clone ID No: ZmPrPase1) of the present invention. The polynucleotide sequence contains a sequence of 1301 nucleotides.
**Figure 11** **-** Deduced amino acid sequence of SEQ ID NO: 9 (SEQ ID NO: 10). The polypeptide sequence contains a sequence of 329 amino acids. The standard one-letter abbreviation for amino acids is used to illustrate the deduced amino acid sequence.
**Figures 12A-D** **-** Amino acid multiple sequence alignment (CLUSTAL W algorithm, blosum62 scoring matrix) of SEQ ID NOs: 2, 4, 6, 8, 10 and sequences of the Saccharomyces cerevisies PrPase(Swiss-Prot accession #P47154) and human PrPase (Swiss-Prot accession #O75844). The active site is shaded.
**Figure 13** **-** Comparison of the Exon/Intron structure of the computer-predicted (Genefinder (P.Green and L.Hillier, www.ncbi.nlm.nih.gov)) and experimentally demonstrated (SEQ ID NO: 4 and 6) ORFs of the Arabidopsis PrPases. The exons are numbered sequentially in both clones. Corresponding exons are placed below each other. The lines connecting the introns have no biological significance. Breaks were introduced in the sequences to make this figure.

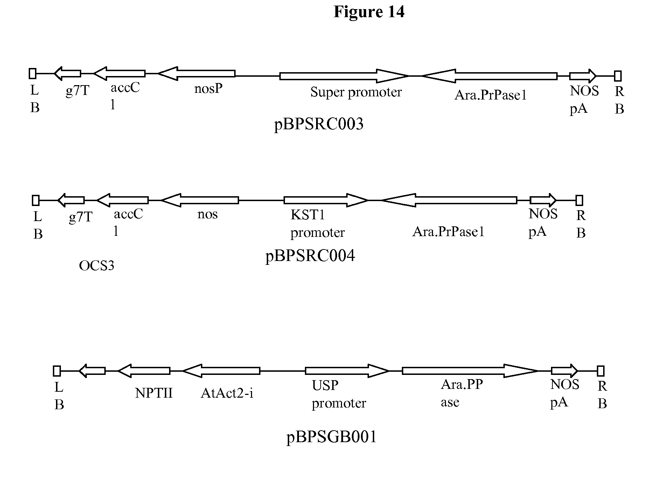

**Figure 14** **-** Diagram of the plant expression vectors pBPSRC003, pBPSRC005 and pBPSGB01 containing different plant promoters controlling the expression of an Arabidopsis PrPase antisense gene. In pBPSRC003 the expression of the antisense PrPase gene is under control of the constitutive superpromoter (Ni et al., The Plant Journal 7: 661-76 (1995)). Whereas in pBPSRC005, a guard-cell specific promoter KST1 (G. Plesch et al, unpublished results) and in pBPSGB01 a seed-specific promoter USP (unknown seed protein, Bäumlein et al., Mol Gen Genet 225: 459-467, 1991) drives the expression of the same gene. The components are: aacCI gentamycin resistance gene (Hajdukiewicz et al., 1994 Plant Molecular Biology 25: 989-94), NOS promoter (Becker et al., 1992 Plant Molecular Biology 20: 1195-7), g7T terminator (Becker et al., 1992), NOSpA terminator (Jefferson et al., EMBO J 6:3901-7 1987) and nptII (neomycine phosphotransferase II) kanamycin resistance gene, AtAct2-i actin promoter, OCS3 octopine synthase (MacDonald et al., Nucleic Acids Res. 19: 5575-5581) terminator, seed-specific USP promoter, NOSpA terminator (Jefferson et al., EMBO J 6:3901-7 1987).
**Figures 15A-B** - Nucleotide sequence of an Arabidopsis AtPrPase1 promoter (Promoter/AtPrPase1) SEQ ID NO: 11 (Clone ID No: Promoter/AtPrPase1) of the present invention. The polynucleotide sequence contains a sequence of 2047 nucleotides.
**Figure 16** **-** Amino acid sequence comparison (fasta: Pairewise alignment, blosum 62 scoring matrix, gap opening penalty: 10, gap extension penalty: 0.1) of the PrPase polypeptides of the present invention (SEQ ID NOS: 2, 4, 6, 8, 10) with the yeast PrPase (Swiss-Prot accession #P47154) and human PrPase (Swiss-Prot accession #O75844). The percent identity and percent similarity values are shown in parenthesis.

### DETAILED DESCRIPTION OF THE INVENTION

The present invention may be understood more readily by reference to the following detailed description of the preferred embodiments of the invention and the Examples included herein.

One aspect of this invention pertains to isolated nucleic acid molecules that encode fully active polypeptides or portions of the enzymes PrPase from *Physcomitrella patens, Arabidopsis thaliana,* soybeans and corn. Moreover, this invention pertains to nucleic acid fragments originated from the clones mentioned above. As well as other nucleic acid fragments from the mentioned as well as other organisms that can be isolated using the described nucleic acid fragments as probes in hybridization experiments.

This invention describes the principle of using reduction of PrPase gene activity to engineer drought-tolerant plants, and/or to increase the amount of storage compounds in seeds of plants. This strategy has herein been demonstrated for *Arabidopsis thaliana,* Rapeseed, soybeans and corn but its application is not restricted to these plants. The only condition to realize this is the isolation of the corresponding PrPase genes from the target plants. The use of the described clones to isolate corresponding PrPase genes form other plants is something appreciated by some one skilled in the art.

The reduction in PrPase can be accomplished by, but is not limited to, one of the following examples: (a) antisense gene-expression repression, (b) targeted antibodies to PrPase, and (c) targeted, engineered promoter repression with for example zinc-finger derived transcription factors.

In another aspect of this invention, the promoter of an Arabidopsis PrPase is described. This promoter is guard-cell specific and can be used to engineer traits such as drought tolerance and regulation of gas exchange in the plant.

The present invention can make a significant contribution to the art by providing new strategies to engineer drought-tolerance in crop plants, especially the use of the previously unknown PrPase clones from plant origin. This is achieved by reducing the expression of the referred genes in transformed plant cells, preferably but not restricted to guard cells.

Moreover, farnesylation of the bacterial chaperone DnaJ is essential for the bacterial growth at high temperatures (Wickner, S. et al 1991 Nature 350:165-7). Farnesylation of this chaperone is required for its full activity. In the plant Atriplex nummularia, ANJ1 is a homologue of DnaJ and is induced by heat and salt treatments (Zhu, J.-K. et al 1993 The Plant Cell 5:341-9). This enzyme is a target of farnesylation. Increase in the farnesylation of plant chaperones has been shown to result in higher biological activity of these enzymes and consequently lead to increased plant stress tolerance. Therefore, the polynucleotides and polypeptides of the present invention, including agonists and/or fragments thereof, have uses which include conferring resistence to heat, drought, and salt stress in plants. Alternatively, antagonists of the present invention may have uses that include modulating a plants susceptibility to biotic and/or abiotic stresses which include, but are not limited to heat, drought, and salt stress in plants. Preferably antagonists of the present invention increase a plants resistence to heat, drought, and salt stress. In one embodiment, over-expression of a PrPase polypeptide of the present invention within a plant using a constitutive promoter (e.g., 35S, or other promoters disclosed herein), though not in the guard-cell, would be useful for improving drought and salt tolerance in a plant.

Protein farnesylation is positively involved with control of cell cycle (Ziegelhoffer et al 2000 PNAS 97:7633-8). Mutations in genes involved in protein farnesylation, namely farnesyl transferase, results in inhibition of cell proliferation in Arabidopsis plants (Bonetta et al. 2000 Planta 211:182-90). Constitutive over-expression in plants of the farnesylation pathway, namely of the prenyl peptidase, results in increased cell proliferation, increased plant growth. The polynucleotides and polypeptides of the present invention, including agonists and/or fragments thereof, have uses that include modulating plant growth, and potentially plant yield, preferably increasing plant growth. In addition, antagonists of the present invention may have uses that include modulating plant growth and/or yield, through preferably increasing plant growth and yield. In yet another embodiment, over-expression of a PrPase polypeptide of the present invention using a constitutive promoter (e.g., 35S, or other promoters disclosed herein) may be useful for increasing plant growth by accelerating cell division.

The polynucleotides can also be used to express recombinant proteins for analysis, characterization and agronomic use, to express recombinant proteins to raise antibodies directed against polypeptides of the present invention, as markers for tissues in which the corresponding protein is expressed (e.g., preferentially, or non-preferentially), as hybridization markers on Southern gels, as genetic markers for breeding assistance, as RFLP markers, as markers for genotyping (varieties, etc,), and the encoded protein, can, at the very least, be used as a molecular weight marker.

The polynucleotides of the present invention are also useful as chromosome markers or tags (when labeled) to identify chromosomes, to map related gene positions within a chromosome, or as a comparative reference to endogenous DNA sequences of mutant plants to identify allelic varients, and/or spontaneous or biotic mutations.

The polynucleotides of the present invention are also useful for genetic fingerprinting, for selecting and making oligomers for attachment to a "gene chip" or other support, including for examination of expression patterns for particular genes, to differentiate intron and/or exon boundaries, to identify splice and/or allelic variants, and as diagnostic tools for identification of developmental stages, disease states, and/or nutrient levels.

The present invention encompasses polynucleotides that hybridize to the polynucleotides of the present invention under either stringent or non-stringent conditions. Such hybidization may be used to identigy orthologs, homologs, alleleic variants, variants, and/or mutants of the polynucleotides of the present invention. Additionally, the polynucleotides of the present invention may be used to clone orthologs, homologs, alleleic variants, variants, and/or mutants of the polynucleotides of the present by using oligonucleotides directed to polynucleotide sequences of the present invention, and performing PCR on plant cell or tissue samples.

The present invention encompasses the identification of proteins, nucleic acids, or other molecules, that bind to polypeptides and polynucleotides of the present invention (for example, in a receptor-ligand interaction). The polynucleotides of the present invention can also be used in interaction trap assays (such as, for example, that discribed by Ozenberger and Young (Mol Endocrinol., 9(10):1321-9, (1995); and Ann N Y Acad Sci., 7;766:279-81, (1995)).

Potential uses of polynucleotides and polypeptides of the present invention include nutritional (e.g., as an amino acid supplement), as a carbon source, as nitrogen source, as a carbohydrate source, modulating plant defense activity, modulating signal transduction, modulating metabolite transport (e.g., carbon, nitrogen fluxes, etc.), conferring abiotic stress tolerance and/or resistance (water, drought, cold, salt, etc.), conferring xenobiotic stress tolerance and/or resistance, and development control (for example, yield, flowering time, etc.).

The polynucleotide and polypeptides of the present invention are useful as probes for the identification and isolation of full-length cDNAs and/or genomic DNA which correspond to the polynucleotides of the present invention, as probes to hybridize and discover novel, related DNA sequences, as probes for positional cloning of this or a related sequence, as probe to "subtract-out" known sequences in the process of discovering other novel polynucleotides, as probes to quantify gene expression, and as probes for microarays.

In addition, polynucleotides and polypeptides of the present invention may comprise one, two, three, four, five, six, seven, eight, or more membrane domains.

Also, in preferred embodiments the present invention provides methods for further refining the biological fuction of the polynucleotides and/or polypeptides of the present invention.

Specifically, the invention provides methods for using the polynucleotides and polypeptides of the invention to identify orthologs, homologs, variants, and/or allelic variants of the invention. Also provided are methods of using the polynucleotides and polypeptides of the invention to identify the entire coding region of the invention, non-coding regions of the invention, regulatory sequences of the invention, and secreted, mature, pro-, prepro-, forms of the invention.

In preferred embodiments, the invention provides methods for identifying the glycosylation sites inherent in the polynucleotides and polypeptides of the invention, and the subsequent alteration, deletion, and/or addition of said sites for a number of desirable characteristics which include, but are not limited to, augmentation of protein folding, inhibition of protein aggregation, regulation of intracellular trafficking to organelles, increasing resistance to proteolysis, modulation of protein antigenicity, and mediation of intercellular adhesion

In further preferred embodiments, methods are provided for evolving the polynucleotides and polypeptides of the present invention using molecular evolution in an effort to create and identify novel variants with desired structural, functional, and/or physical characteristics.

The present invention in further preferred embodiments provides for other experimental methods and procedures currently available to derive functional assignments. These procedures include but are not limited to spotting of clones on arrays, micro-array technology, PCR based methods and other procedures that could use sequence information from our clones to build a primer or a hybrid partner.

As used herein, the term "environmental stress" refers to any sub-optimal growing condition and includes, but is not limited to, sub-optimal conditions associated with salinity, drought, temperature, metal, chemical, pathogenic and oxidative stresses, or combinations thereof. In preferred embodiments, the environmental stress can be salinity, drought, or temperature, or combinations thereof, and in particular, can be high salinity, low water content or low temperature. It is also to be understood that as used in the specification and in the claims, "a" or "an" can mean one or more, depending upon the context in which it is used. Thus, for example, reference to "a cell" can mean that at least one cell can be utilized.

As also used herein, the term "nucleic acid" and "polynucleotide" refer to RNA or DNA that is linear or branched, single or double stranded, or a hybrid thereof. The term also encompasses RNA/DNA hybrids. These terms also encompass untranslated sequence located at both the 3' and 5' ends of the coding region of the gene: at least about 1000 nucleotides of sequence upstream from the 5' end of the coding region and at least about 200 nucleotides of sequence downstream from the 3' end of the coding region of the gene. Less common bases, such as inosine, 5-methylcytosine, 6-methyladenine, hypoxanthine and others can also be used for antisense, dsRNA and ribozyme pairing. For example, polynucleotides that contain C-5 propyne analogues of uridine and cytidine have been shown to bind RNA with high affinity and to be potent antisense inhibitors of gene expression. Other modifications, such as modification to the phosphodiester backbone, or the 2'-hydroxy in the ribose sugar group of the RNA can also be made. The antisense polynucleotides and ribozymes can consist entirely of ribonucleotides, or can contain mixed ribonucleotides and deoxyribonucleotides. The polynucleotides of the invention may be produced by any means, including genomic preparations, cDNA preparations, in vitro synthesis, RT-PCR and *in vitro* or *in vivo* transcription.

An "isolated" nucleic acid molecule is one that is substantially separated from other nucleic acid molecules which are present in the natural source of the nucleic acid (i.e., sequences encoding other polypeptides). Preferably, an "isolated" nucleic acid is free of some of the sequences which naturally flank the nucleic acid (i.e., sequences located at the 5' and 3' ends of the nucleic acid) in its naturally occurring replicon. For example, a cloned nucleic acid is considered isolated. In various embodiments, the isolated PPSRP nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. A nucleic acid is also considered isolated if it has been altered by human intervention, or placed in a locus or location that is not its natural site, or if it is introduced into a cell by agroinfection. Moreover, an "isolated" nucleic acid molecule, such as a cDNA molecule, can be free from some of the other cellular material with which it is naturally associated, or culture medium when produced by recombinant techniques, or chemical precursors or other chemicals when chemically synthesized.

Specifically excluded from the definition of "isolated nucleic acids" are: naturally-occurring chromosomes (such as chromosome spreads), artificial chromosome libraries, genomic libraries, and cDNA libraries that exist either as an in vitro nucleic acid preparations or as a transfected/transformed host cell preparation, wherein the host cells are either an in vitro heterogeneous preparation or plated as a heterogeneous population of single colonies. Also specifically excluded are the above libraries wherein a specified nucleic acid makes up less than 5% of the number of nucleic acid inserts in the vector molecules. Further specifically excluded are whole cell genomic DNA or whole cell RNA preparations (including whole cell preparations that are mechanically sheared or enzymatically digested). Even further specifically excluded are the whole cell preparations found as either an in vitro preparation or as a heterogeneous mixture separated by electrophoresis wherein the nucleic acid of the invention has not further been separated from the heterologous nucleic acids in the electrophoresis medium (e.g., further separating by excising a single band from a heterogeneous band population in an agarose gel or nylon blot).

### Polynucleotides and Polypeptides of the Invention

### Features of the Polypeptide Encoded by Gene No:1

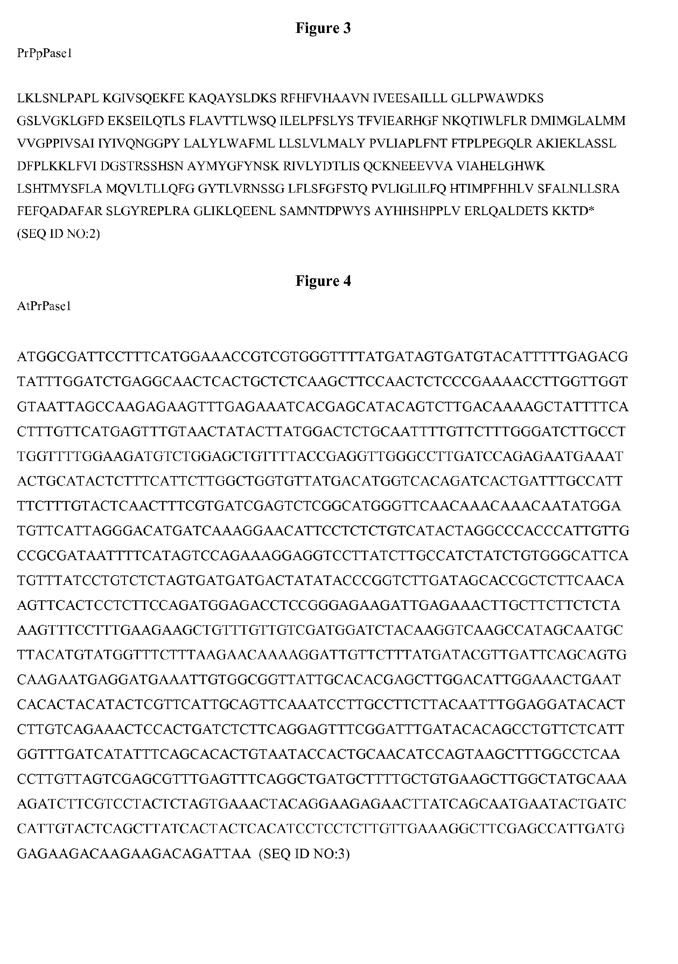

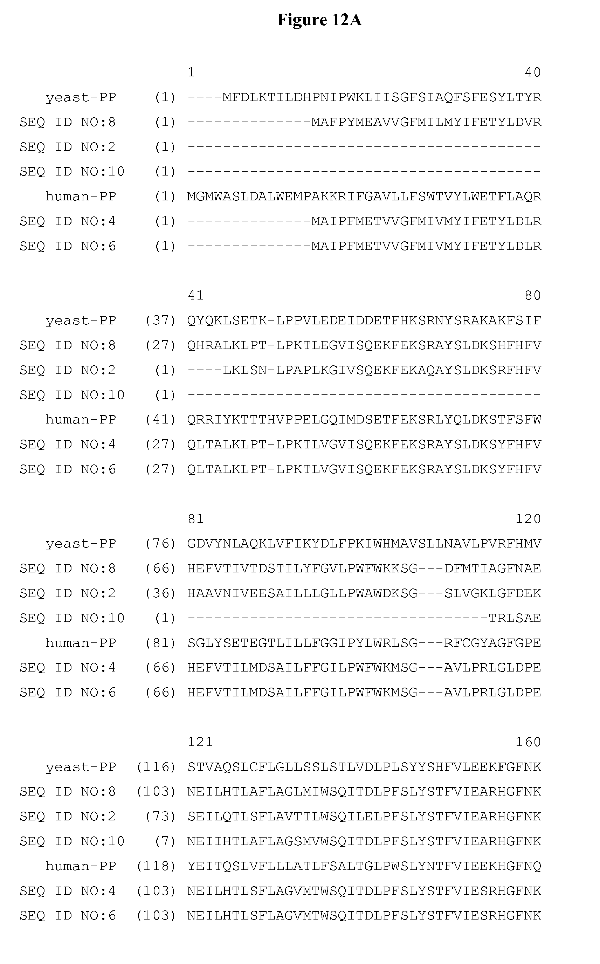

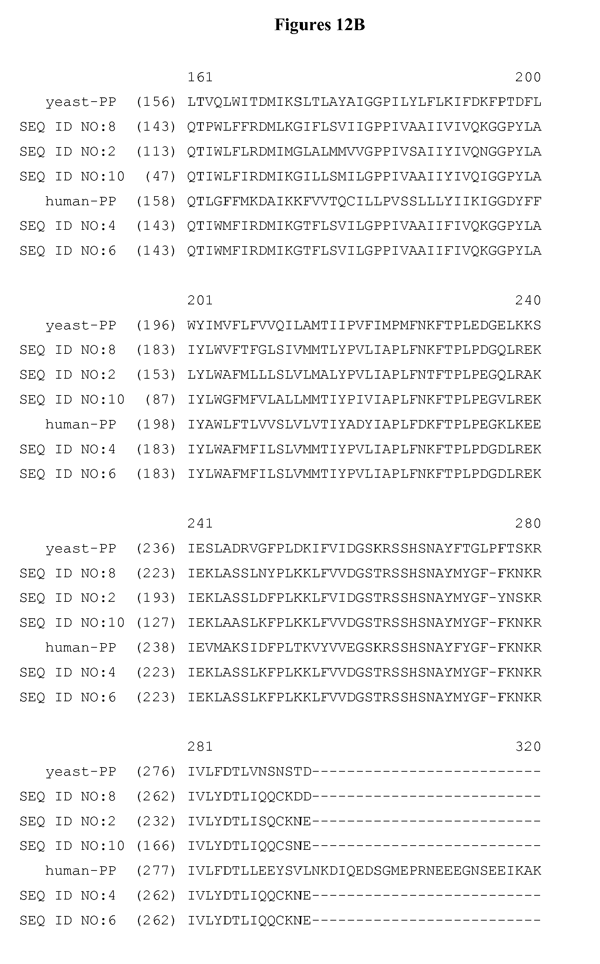

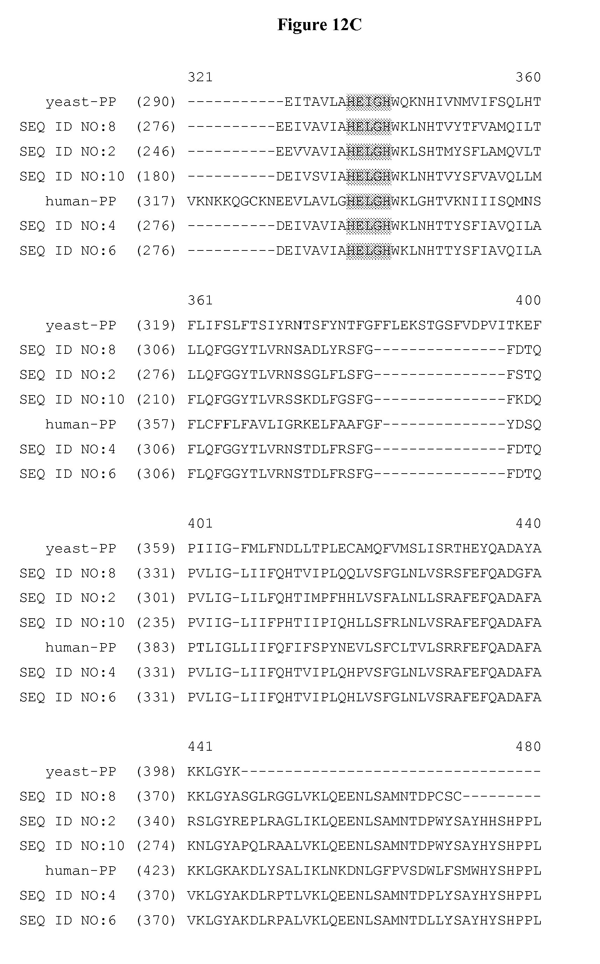

The polypeptide of this gene provided as SEQ ID NO:2 (Figure 3), encoded by the polynucleotide sequence according to SEQ ID NO:1 (Figure 2), and/or encoded by the polynucleotide contained within the deposited clone, PpPrPase1, has significant homology at the nucleotide and amino acid level to both human and yeast prenyl peptidases (see Figures 12A-D). Based upon the homology, the polypeptide of the present invention may share at least some biological activity with prenyl peptidases. The polynucleotide and polypeptide of the present invention has uses that include, but are not limited to conferring drought tolerance and/or salt tolerance to plants. The polynucleotide also has uses which includes identifying the full-length PpPrPase1. Moreover, antagonists directed against the polypeptide of the present invention may be useful in fungicidal applications.

Farnesylation of the bacterial chaperone DnaJ is essential for the bacterial growth at high temperatures (Wickner, S. et al 1991 Nature 350:165-7). Farnesylation of this chaperone is required for its full activity. In the plant Atriplex nummularia, ANJ1 is a homologue of DnaJ and is induced by heat and salt treatments (Zhu, J.-K. et al 1993 The Plant Cell 5:341-9). This enzyme is a target of farnesylation. Increase in the farnesylation of plant chaperones has been shown to result in higher biological activity of these enzymes and consequently lead to increased plant stress tolerance. Therefore, the polynucleotides and polypeptides of the present invention, including agonists and/or fragments thereof, have uses which include conferring resistence to heat, drought, and salt stress in plants. Alternatively, antagonists of the present invention may have uses that include modulating a plants susceptibility to biotic and/or abiotic stresses which include, but are not limited to heat, drought, and salt stress in plants. Preferably antagonists of the present invention increase a plants resistence to heat, drought, and salt stress. In one embodiment, over-expression of a PrPase polypeptide of the present invention within a plant using a constitutive promoter (e.g., 35S, or other promoters disclosed herein), though not in the guard-cell, would be useful for improving drought and salt tolerance in a plant.

Protein farnesylation is positively involved with control of cell cycle (Ziegelhoffer et al 2000 PNAS 97:7633-8). Mutations in genes involved in protein farnesylation, namely farnesyl transferase, results in inhibition of cell proliferation in Arabidopsis plants (Bonetta et al. 2000 Planta 211:182-90). Constitutive over-expression in plants of the farnesylation pathway, namely of the prenyl peptidase, results in increased cell proliferation, increased plant growth. The polynucleotides and polypeptides of the present invention, including agonists and/or fragments thereof, have uses that include modulating plant growth, and potentially plant yield, preferably increasing plant growth. In addition, antagonists of the present invention may have uses that include modulating plant growth and/or yield, through preferably increasing plant growth and yield. In yet another embodiment, over-expression of the PpPrPase1 polypeptide of the present invention using a constitutive promoter (e.g., 35S, or other promoters disclosed herein) may be useful for increasing plant growth by accelerating cell division.

Although it is believed the encoded polypeptide may share at least some biological activities with prenyl proteases, a number of methods of determining the exact biological function of this clone are either known in the art or are described elsewhere herein. Briefly, the function of this clone may be determined by applying microarray methodology. The PpPrPase1 clone in addition to other clones of the present invention, may be arrayed on microchips for expression profiling. Depending on which polynucleotide probe is used to hybridize to the slides, a change in expression of a specific gene may provide additional insight into the function of this gene based upon the conditions being studied. For example, an observed increase or decrease in expression levels when the polynucleotide probe used comes from tissue that has been cold treated might indicate a function in modulating cold tolerance, for example. In the case of PpPrPase1, tissue deprived of water or stressed by other biotic or abiotic stresses (heat, drought, high light, high salt, etc.) should be used to extract RNA to prepare the probe.

In addition, the function of the protein may be assessed by applying quantitative PCR methodology, for example. Real time quantitative PCR would provide the capability of following the expression of specific genes throughout the plant development cycle, for example. Quantitative PCR methodology requires only a nominal amount of tissue from each developmentally important step (3 days germinated seedlings, 1 week old seedlings [roots, shoots, and stems]; roots, leaves and stems before the onset of flowering, flowers [different parts]; and/or developing embryos) is needed to perform such experiements. Therefore, the application of quantitative PCR methodology to refining the biological function of this polypeptide is encompassed by the present invention. Also encompassed by the present invention are quantitative PCR probes corresponding to the polynucleotide sequence provided as SEQ ID NO:1 (Figure 2).

The function of the protein may also be assessed through complementation assays in yeast. For example, in the case of the PpPrPase1 clone, transforming yeast deficient in prenyl protease activity and assessing their ability to grow would provide convincing evidence the PpPrPase1 clone has prenyl protease activity. Additional assay conditions and methods that may be used in assessing the function of the polynucletides and polypeptides of the present invention are known in the art, some of which are disclosed elsewhere herein.

Alternatively, the biological function of the encoded polypeptide may be determined by disrupting a homologue of this polypeptide in *Synecosystis.* Cyanobacteria (blue-green algae) is considered a precursor to the plant chloroplast. It possesses both photosynthetic systems and many other metabolic processes reminiscent to those of plants. These processes are often targets for many commercial herbicides, and this organism has been widely used in the study of the mode of action of many classes of herbicides. *Synechocystis* is one of the best-studied cyanobacteria. In addition to most of the features common to cyanobacteria, it offers many other added advantages. *Synechocystis* has a naturally occurring genetic transformation system, thus entailing vigorous and sophisticated genetic and molecular manipulation (e.g. targeted-gene disruption, gene replacement, etc.) applicable to some of the well-characterized systems (*S. cerevisiae, E. coli).* Most importantly, the availability of the complete genomic sequence information of the *Synechocystis* affords an avenue for the rapid identification and cloning of gene(s) of interest, and elucidation of gene function through genetic and molecular means.

Moreover, the biological function of this polypeptide may be determined by the application of antisense and/or sense methodology and the resulting generation of transgenic plants. Expressing a particular gene in either sense or antisense orientation in a transgenic plant can lead to respectively higher or lower expression levels of that particular gene. Altering the endogenous expression levels of a gene can lead to the obervation of a particular phenotype that can then be used to derive indications on the function of the gene. The gene can be either over-expressed or under expressed in every cell of the plant at all times using a strong ubiquitous promoter, or it can be expressed in one or more discrete parts of the plant using a well characterized tissue-specific promoter (i.e., a root promoter or a flower specific promoter), or it can be expressed at a specified time of development using an inducible and/or a developmentally regulated promoter.

In the case of PpPrPase1 transgenic plants, if no phenotype is apparent in normal growth conditions, observing the plants under stress conditions (deprivation of water, presence of high salt, or other biotic or abiotic stresses, such as cold, heat, drought, high light, etc.) may lead to understanding the function of the gene. In the case of seed-storage compounds biochemical analysis of oil, sugars and protein (see Example 34) may lead to understanding the function of the gene. Therefore, the application of antisense and/or sense methodology to the creation of transgenic plants to refine the biological function of the polypeptide is encompassed by the present invention.

In preferred embodiments, the following N-terminal deletion mutants are encompassed by the present invention: L1-D394, K2-D394, L3-D394, S4-D394, N5-D394, L6-D394, P7-D394, A8-D394, P9-D394, L10-D394, K11-D394, G12-D394, I13-D394, V14-D394, S15-D394, Q16-D394, E17-D394, K18-D394, F19-D394, E20-D394, K21-D394, A22-D394, Q23-D394, A24-D394, Y25-D394, S26-D394, L27-D394, D28-D394, K29-D394, S30-D394, R31-D394, F32-D394, H33-D394, F34-D394, V35-D394, H36-D394, A37-D394, A38-D394, V39-D394, N40-D394, I41-D394, V42-D394, E43-D394, E44-D394, S45-D394, A46-D394, I47-D394, L48-D394, L49-D394, L50-D394, G51-D394, L52-D394, L53-D394, P54-D394, W55-D394, A56-D394, W57-D394, D58-D394, K59-D394, S60-D394, G61-D394, S62-D394, L63-D394, V64-D394, G65-D394, K66-D394, L67-D394, G68-D394, F69-D394, D70-D394, E71-D394, K72-D394, S73-D394, E74-D394, I75-D394, L76-D394, Q77-D394, T78-D394, L79-D394, S80-D394, F81-D394, L82-D394, A83-D394, V84-D394, T85-D394, T86-D394, L87-D394, W88-D394, S89-D394, Q90-D394, I91-D394, L92-D394, E93-D394, L94-D394, P95-D394, F96-D394, S97-D394, L98-D394, Y99-D394, S100-D394, T101-D394, F102-D394, V103-D394, I104-D394, E105-D394, A106-D394, R107-D394, H108-D394, G109-D394, F110-D394, N111-D394, K112-D394, Q113-D394, T114-D394, I115-D394, W116-D394, L117-D394, F118-D394, L119-D394, R120-D394, D121-D394, M122-D394, I123-D394, M124-D394, G125-D394, L126-D394, A127-D394, L128-D394, M129-D394, M130-D394, V131-D394, V132-D394, G133-D394, P134-D394, P135-D394, I136-D394, V137-D394, S138-D394, A139-D394, I140-D394, I141-D394, Y142-D394, I143-D394, V144-D394, Q145-D394, N146-D394, G147-D394, G148-D394, P149-D394, Y150-D394, L151-D394, A152-D394, L153-D394, Y154-D394, L155-D394, W156-D394, A157-D394, F158-D394, M159-D394, L160-D394, L161-D394, L162-D394, S163-D394, L164-D394, V165-D394, L166-D394, M167-D394, A168-D394, L169-D394, Y170-D394, P171-D394, V172-D394, L173-D394, I174-D394, A175-D394, P176-D394, L177-D394, F178-D394, N179-D394, T180-D394, F181-D394, T182-D394, P183-D394, L184-D394, P185-D394, E186-D394, G187-D394, Q188-D394, L189-D394, R190-D394, A191-D394, K192-D394, I193-D394, E194-D394, K195-D394, L196-D394, A197-D394, S198-D394, S199-D394, L200-D394, D201-D394, F202-D394, P203-D394, L204-D394, K205-D394, K206-D394, L207-D394, F208-D394, V209-D394, I210-D394, D211-D394, G212-D394, S213-D394, T214-D394, R215-D394, S216-D394, S217-D394, H218-D394, S219-D394, N220-D394, A221-D394, Y222-D394, M223-D394, Y224-D394, G225-D394, F226-D394, Y227-D394, N228-D394, S229-D394, K230-D394, R231-D394, I232-D394, V233-D394, L234-D394, Y235-D394, D236-D394, T237-D394, L238-D394, I239-D394, S240-D394, Q241-D394, C242-D394, K243-D394, N244-D394, E245-D394, E246-D394, E247-D394, V248-D394, V249-D394, A250-D394, V251-D394, I252-D394, A253-D394, H254-D394, E255-D394, L256-D394, G257-D394, H258-D394, W259-D394, K260-D394, L261-D394, S262-D394, H263-D394, T264-D394, M265-D394, Y266-D394, S267-D394, F268-D394, L269-D394, A270-D394, M271-D394, Q272-D394, V273-D394, L274-D394, T275-D394, L276-D394, L277-D394, Q278-D394, F279-D394, G280-D394, G281-D394, Y282-D394, T283-D394, L284-D394, V285-D394, R286-D394, N287-D394, S288-D394, S289-D394, G290-D394, L291-D394, F292-D394, L293-D394, S294-D394, F295-D394, G296-D394, F297-D394, S298-D394, T299-D394, Q300-D394, P301-D394, V302-D394, L303-D394, I304-D394, G305-D394, L306-D394, I307-D394, L308-D394, F309-D394, Q310-D394, H311-D394, T312-D394, I313-D394, M314-D394, P315-D394, F316-D394, H317-D394, H318-D394, L319-D394, V320-D394, S321-D394, F322-D394, A323-D394, L324-D394, N325-D394, L326-D394, L327-D394, S328-D394, R329-D394, A330-D394, F331-D394, E332-D394, F333-D394, Q334-D394, A335-D394, D336-D394, A337-D394, F338-D394, A339-D394, R340-D394, S341-D394, L342-D394, G343-D394, Y344-D394, R345-D394, E346-D394, P347-D394, L348-D394, R349-D394, A350-D394, G351-D394, L352-D394, I353-D394, K354-D394, L355-D394, Q356-D394, E357-D394, E358-D394, N359-D394, L360-D394, S361-D394, A362-D394, M363-D394, N364-D394, T365-D394, D366-D394, P367-D394, W368-D394, Y369-D394, S370-D394, A371-D394, Y372-D394, H373-D394, H374-D394, S375-D394, H376-D394, P377-D394, P378-D394, L379-D394, V380-D394, E381-D394, R382-D394, L383-D394, Q384-D394, A385-D394, L386-D394, D387-D394, E388-D394, of SEQ ID NO:2. Polynucleotide sequences encoding these polypeptides are also provided.

In preferred embodiments, the following C-terminal deletion mutants are encompassed by the present invention: L1-D394, L1-T393, L1-K392, L1-K391, L1-S390, L1-T389, L1-E388, L1-D387, L1-L386, L1-A385, L1-Q384, L1-L383, L1-R382, L1-E381, L1-V380, L1-L379, L1-P378, L1-P377, L1-H376, L1-5375, L1-H374, L1-H373, L1-Y372, L1-A371, L1-S370, L1-Y369, L1-W368, L1-P367, L1-D366, L1-T365, L1-N364, L1-M363, L1-A362, L1-S361, L1-L360, L1-N359, L1-E358, L1-E357, L1-Q356, L1-L355, L1-K354, L1-I353, L1-L352, L1-G351, L1-A350, L1-R349, L1-L348, L1-P347, L1-E346, L1-R345, L1-Y344, L1-G343, L1-L342, L1-5341, L1-R340, L1-A339, L1-F338, L1-A337, L1-D336, L1-A335, L1-Q334, L1-F333, L1-E332, L1-F331, L1-A330, L1-R329, L1-S328, L1-L327, L1-L326, L1-N325, L1-L324, L1-A323, L1-F322, L1-S321, L1-V320, L1-L319, L1-H318, L1-H317, L1-F316, L1-P315, L1-M314, L1-I313, L1-T312, L1-H311, L1-Q310, L1-F309, L1-L308, L1-I307, L1-L306, L1-G305, L1-I304, L1-L303, L1-V302, L1-P301, L1-Q300, L1-T299, L1-S298, L1-F297, L1-G296, L1-F295, L1-S294, L1-L293, L1-F292, L1-L291, L1-G290, L1-S289, L1-S288, L1-N287, L1-R286, L1-V285, L1-L284, L1-T283, L1-Y282, L1-G281, L1-G280, L1-F279, L1-Q278, L1-L277, L1-L276, L1-T275, L1-L274, L1-V273, L1-Q272, L1-M271, L1-A270, L1-L269, L1-F268, L1-S267, L1-Y266, L1-M265, L1-T264, L1-H263, L1-5262, L1-L261, L1-K260, L1-W259, L1-H258, L1-G257, L1-L256, L1-E255, L1-H254, L1-A253, L1-I252, L1-V251, L1-A250, L1-V249, L1-V248, L1-E247, L1-E246, L1-E245, L1-N244, L1-K243, L1-C242, L1-Q241, L1-S240, L1-I239, L1-L238, L1-T237, L1-D236, L1-Y235, L1-L234, L1-V233, L1-I232, L1-R231, L1-K230, L1-5229, L1-N228, L1-Y227, L1-F226, L1-G225, L1-Y224, L1-M223, L1-Y222, L1-A221, L1-N220, L1-S219, L1-H218, L1-5217, L1-S216, L1-R215, L1-T214, L1-S213, L1-G212, L1-D211, L1-I210, L1-V209, L1-F208, L1-L207, L1-K206, L1-K205, L1-L204, L1-P203, L1-F202, L1-D201, L1-L200, L1-5199, L1-5198, L1-A197, L1-L196, L1-K195, L1-E194, L1-I193, L1-K192, L1-A191, L1-R190, L1-L189, L1-Q188, L1-G187, L1-E186, L1-P185, L1-L184, L1-P183, L1-T182, L1-F181, L1-T180, L1-N179, L1-F178, L1-L177, L1-P176, L1-A175, L1-I174, L1-L173, L1-V172, L1-P171, L1-Y170, L1-L169, L1-A168, L1-M167, L1-L166, L1-V165, L1-L164, L1-S163, L1-L162, L1-L161, L1-L160, L1-M159, L1-F158, L1-A157, L1-W156, L1-L155, L1-Y154, L1-L153, L1-A152, L1-L151, L1-Y150, L1-P149, L1-G148, L1-G147, L1-N146, L1-Q145, L1-V144, L1-I143, L1-Y142, L1-I141, L1-I140, L1-A139, L1-5138, L1-V137, L1-I136, L1-P135, L1-P134, L1-G133, L1-V132, L1-V131, L1-M130, L1-M129, L1-L128, L1-A127, L1-L126, L1-G125, L1-M124, L1-I123, L1-M122, L1-D121, L1-R120, L1-L119, L1-F118, L1-L117, L1-W116, L1-I115, L1-T114, L1-Q113, L1-K112, L1-N111, L1-F110, L1-G109, L1-H108, L1-R107, L1-A106, L1-E105, L1-I104, L1-V103, L1-F102, L1-T101, L1-5100, L1-Y99, L1-L98, L1-S97, L1-F96, L1-P95, L1-L94, L1-E93, L1-L92, L1-I91, L1-Q90, L1-S89, L1-W88, L1-L87, L1-T86, L1-T85, L1-V84, L1-A83, L1-L82, L1-F81, L1-S80, L1-L79, L1-T78, L1-Q77, L1-L76, L1-I75, L1-E74, L1-573, L1-K72, L1-E71, L1-D70, L1-F69, L1-G68, L1-L67, L1-K66, L1-G65, L1-V64, L1-L63, L1-S62, L1-G61, L1-560, L1-K59, L1-D58, L1-W57, L1-A56, L1-W55, L1-P54, L1-L53, L1-L52, L1-G51, L1-L50, L1-L49, L1-L48, L1-I47, L1-A46, L1-S45, L1-E44, L1-E43, L1-V42, L1-I41, L1-N40, L1-V39, L1-A38, L1-A37, L1-H36, L1-V35, L1-F34, L1-H33, L1-F32, L1-R31, L1-S30, L1-K29, L1-D28, L1-L27, L1-S26, L1-Y25, L1-A24, L1-Q23, L1-A22, L1-K21, L1-E20, L1-F19, L1-K18, L1-E17, L1-Q16, L1-S15, L1-V14, L1-I13, L1-G12, L1-K11, L1-L10, L1-P9, L1-A8, L1-P7, of SEQ ID NO:2. Polynucleotide sequences encoding these polypeptides are also provided.

Many polynucleotide sequences, such as EST sequences, are publicly available and accessible through sequence databases. Some of these sequences are related to SEQ ID NO: 1 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1398 of SEQ ID NO:1, b is an integer between 15 to 1398, where both a and b correspond to the positions of nucleotide residues shown in SEQ ID NO:1, and where b is greater than or equal to a+14.

### Features of the Polypeptide Encoded by Gene No:2

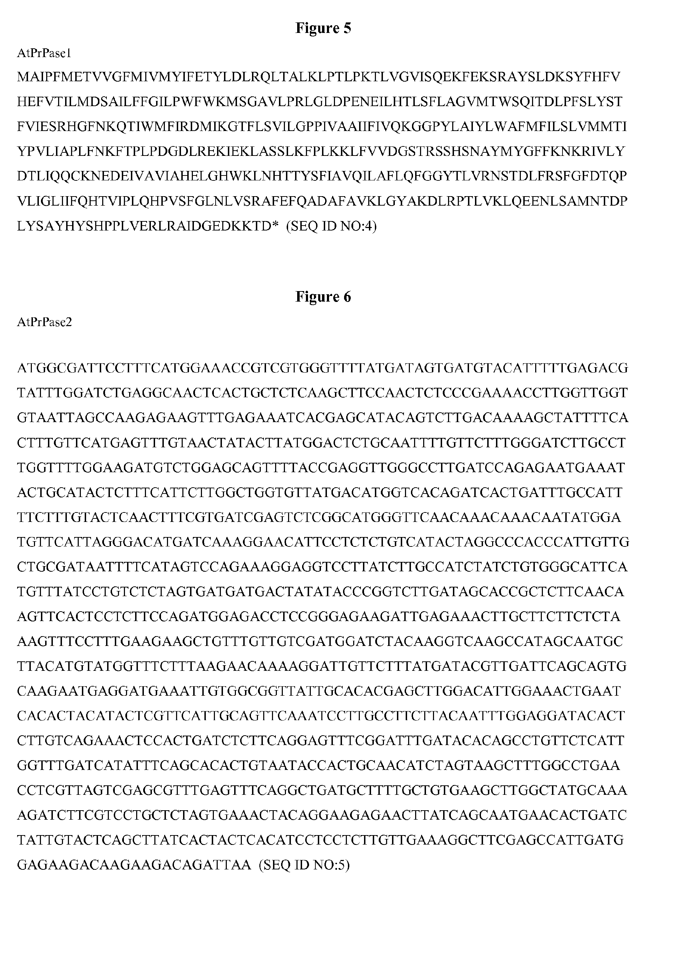

The polypeptide of this gene provided as SEQ ID NO:4 (Figure 5), encoded by the polynucleotide sequence according to SEQ ID NO:3 (Figure 4), and/or encoded by the polynucleotide contained within the deposited clone, AtPrPase1, has significant homology at the nucleotide and amino acid level to both human and yeast prenyl peptidases (see Figures 12A-D). Based upon the homology, the polypeptide of the present invention may share at least some biological activity with prenyl peptidases. The polynucleotide and polypeptide of the present invention, including agonists has uses that include, but are not limited to conferring drought tolerance and/or salt tolerance to plants, particular Arabidopsis. Alternatively, antagonists directed against the polypeptide of the present invention may also be useful in conferring drought tolerance and/or salt tolerance to plants, particular Arabidopsis.

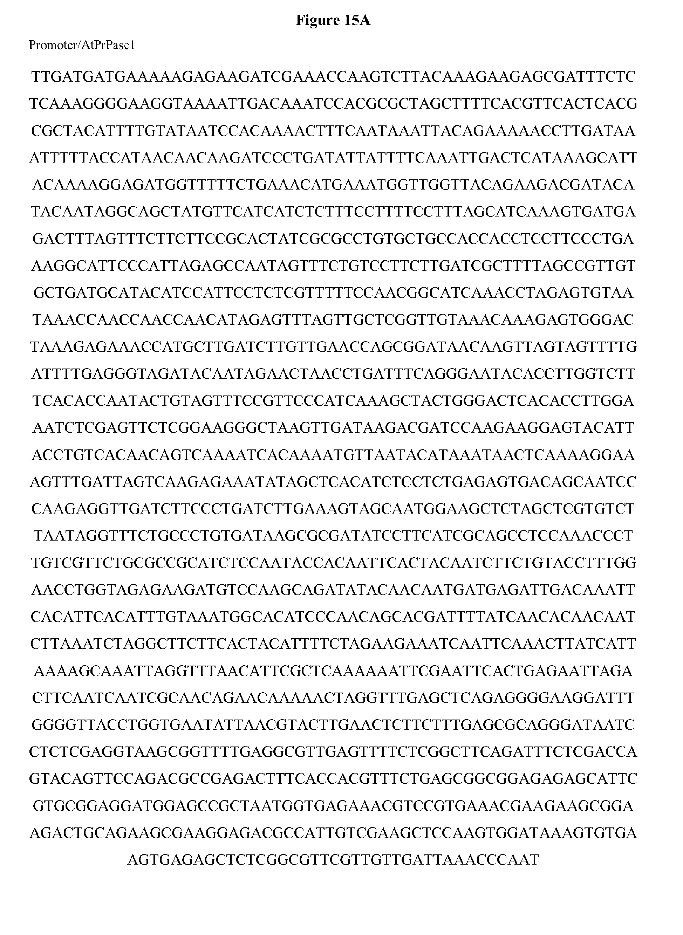

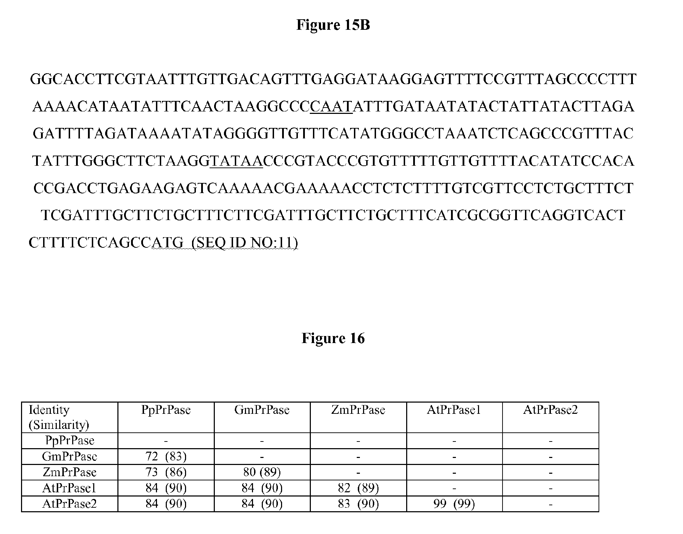

In an a preferred embodiment, the invention also encompasses the promoter of the AtPrPase1 gene (SEQ ID NO:11, Figures 15A-B). The promoter has uses including, but not limited to, directing expression of a gene of interest to plant guard-cells. The gene of interest may be any gene endogenous to a plant, a non-plant derived gene (e.g., viral, mammalian, human, synthetic, molecularly evolved, bacterial, funga1, etc.,), a reporter gene, a marker gene, a desired input trait, a desired output trait, a gene capable of conferring a specific phenotype in a plant, one or more genes of the present invention, antibody genes, antibody genes directed against the polypeptides of the present invention, antisense genes, in addition to other genes known in the art and/or disclosed herein.

Farnesylation of the bacterial chaperone DnaJ is essential for the bacterial growth at high temperatures (Wickner, S. et al 1991 Nature 350:165-7). Farnesylation of this chaperone is required for its full activity. In the plant Atriplex nummularia, ANJ1 is a homologue of DnaJ and is induced by heat and salt treatments (Zhu, J.-K. et al 1993 The Plant Cell 5:341-9). This enzyme is a target of farnesylation. Increase in the farnesylation of plant chaperones has been shown to result in higher biological activity of these enzymes and consequently lead to increased plant stress tolerance. Therefore, the polynucleotides and polypeptides of the present invention, including agonists and/or fragments thereof, have uses which include conferring resistence to heat, drought, and salt stress in plants. Alternatively, antagonists of the present invention may have uses that include modulating a plants susceptibility to biotic and/or abiotic stresses which include, but are not limited to heat, drought, and salt stress in plants. Preferably antagonists of the present invention increase a plants resistence to heat, drought, and salt stress. In one embodiment, over-expression of the AtPrPase1 polypeptide of the present invention within a plant using a constitutive promoter (e.g., 35S, or other promoters disclosed herein), though not in the guard-cell, would be useful for improving drought and salt tolerance in a plant.

Protein farnesylation is positively involved with control of cell cycle (Ziegelhoffer et al 2000 PNAS 97:7633-8). Mutations in genes involved in protein farnesylation, namely farnesy1 transferase, results in inhibition of cell proliferation in Arabidopsis plants (Bonetta et al. 2000 Planta 211:182-90). Constitutive over-expression in plants of the farnesylation pathway, namely of the prenyl peptidase, results in increased cell proliferation, increased plant growth. The polynucleotides and polypeptides of the present invention, including agonists and/or fragments thereof, have uses that include modulating plant growth, and potentially plant yield, preferably increasing plant growth. In addition, antagonists of the present invention may have uses that include modulating plant growth and/or yield, through preferably increasing plant growth and yield. In yet another embodiment, over-expression of a PrPase polypeptide of the present invention using a constitutive promoter (e.g., 35S, or other promoters disclosed herein) may be useful for increasing plant growth by accelerating cell division.

Although it is believed the encoded polypeptide may share at least some biological activities with prenyl proteases, a number of methods of determining the exact biological function of this clone are either known in the art or are described elsewhere herein. Briefly, the function of this clone may be determined by applying microarray methodology. The AtPrPase1 clone, in addition to, other clones of the present invention, may be arrayed on microchips for expression profiling. Depending on which polynucleotide probe is used to hybridize to the slides, a change in expression of a specific gene may provide additional insight into the function of this gene based upon the conditions being studied. For example, an observed increase or decrease in expression levels when the polynucleotide probe used comes from tissue that has been cold treated might indicate a function in modulating cold tolerance, for example. In the case of AtPrPase1, tissue deprived of water or stressed by other biotic or abiotic stresses (heat, drought, high light, high salt, etc.) should be used to extract RNA to prepare the probe.

In addition, the function of the protein may be assessed by applying quantitative PCR methodology, for example. Real time quantitative PCR would provide the capability of following the expression of specific genes throughout the plant development cycle, for example. Quantitative PCR methodology requires only a nominal amount of tissue from each developmentally important step (3 days germinated seedlings, 1 week old seedlings [roots, shoots, and stems]; roots, leaves and stems before the onset of flowering, flowers [different parts]; and/or developing embryos) is needed to perform such experiements. Therefore, the application of quantitative PCR methodology to refining the biological function of this polypeptide is encompassed by the present invention. Also encompassed by the present invention are quantitative PCR probes corresponding to the polynucleotide sequence provided as SEQ ID NO:3 (Figure 4).

The function of the protein may also be assessed through complementation assays in yeast. For example, in the case of the AtPrPase1 clone, transforming yeast deficient in prenyl protease activity and assessing their ability to grow would provide convincing evidence the AtPrPase1 clone has prenyl protease activity. Additional assay conditions and methods that may be used in assessing the function of the polynucletides and polypeptides of the present invention are known in the art, some of which are disclosed elsewhere herein.

Alternatively, the biological function of the encoded polypeptide may be determined by disrupting a homologue of this polypeptide in *Synecosystis.* Cyanobacteria (blue-green algae) is considered a precursor to the plant chloroplast. It possesses both photosynthetic systems and many other metabolic processes reminiscent to those of plants. These processes are often targets for many commercial herbicides, and this organism has been widely used in the study of the mode of action of many classes of herbicides. *Synechocystis* is one of the best-studied cyanobacteria. In addition to most of the features common to cyanobacteria, it offers many other added advantages. *Synechocystis* has a naturally occurring genetic transformation system, thus entailing vigorous and sophisticated genetic and molecular manipulation (e.g. targeted-gene disruption, gene replacement, etc.) applicable to some of the well-characterized systems (*S. cerevisiae, E. coli).* Most importantly, the availability of the complete genomic sequence information of the *Synechocystis* affords an avenue for the rapid identification and cloning of gene(s) of interest, and elucidation of gene function through genetic and molecular means.

Moreover, the biological function of this polypeptide may be determined by the application of antisense and/or sense methodology and the resulting generation of transgenic plants. Expressing a particular gene in either sense or antisense orientation in a transgenic plant can lead to respectively higher or lower expression levels of that particular gene. Altering the endogenous expression levels of a gene can lead to the obervation of a particular phenotype that can then be used to derive indications on the function of the gene. The gene can be either over-expressed or under expressed in every cell of the plant at all times using a strong ubiquitous promoter, or it can be expressed in one or more discrete parts of the plant using a well characterized tissue-specific promoter (i.e., a root promoter or a flower specific promoter or a seed-specific promoter), or it can be expressed at a specified time of development using an inducible and/or a developmentally regulated promoter.

In the case of AtPrPase transgenic plants, if no phenotype is apparent in normal growth conditions, observing the plants under stress conditions (deprivation of water, presence of high salt, or other biotic or abiotic stresses, such as cold, heat, drought, high light, etc.) or biochemical analysis of seed-storage compounds may lead to understanding the function of the gene. Therefore, the application of antisense and/or sense methodology to the creation of transgenic plants to refine the biological function of the polypeptide is encompassed by the present invention.

In preferred embodiments, the following N-terminal deletion mutants are encompassed by the present invention: M1-D424, A2-D424, I3-D424, P4-D424, F5-D424, M6-D424, E7-D424, T8-D424, V9-D424, V10-D424, G11-D424, F12-D424, M13-D424, I14-D424, V15-D424, M16-D424, Y17-D424, I18-D424, F19-D424, E20-D424, T21-D424, Y22-D424, L23-D424, D24-D424, L25-D424, R26-D424, Q27-D424, L28-D424, T29-D424, A30-D424, L31-D424, K32-D424, L33-D424, P34-D424, T35-D424, L36-D424, P37-D424, K38-D424, T39-D424, L40-D424, V41-D424, G42-D424, V43-D424, I44-D424, S45-D424, Q46-D424, E47-D424, K48-D424, F49-D424, E50-D424, K51-D424, S52-D424, R53-D424, A54-D424, Y55-D424, S56-D424, L57-D424, D58-D424, K59-D424, S60-D424, Y61-D424, F62-D424, H63-D424, F64-D424, V65-D424, H66-D424, E67-D424, F68-D424, V69-D424, T70-D424, I71-D424, L72-D424, M73-D424, D74-D424, S75-D424, A76-D424, I77-D424, L78-D424, F79-D424, F80-D424, G81-D424, I82-D424, L83-D424, P84-D424, W85-D424, F86-D424, W87-D424, K88-D424, M89-D424, S90-D424, G91-D424, A92-D424, V93-D424, L94-D424, P95-D424, R96-D424, L97-D424, G98-D424, L99-D424, D100-D424, P101-D424, E102-D424, N103-D424, E104-D424, I105-D424, L106-D424, H107-D424, T108-D424, L109-D424, S110-D424, F111-D424, L112-D424, A113-D424, G114-D424, V115-D424, M116-D424, T117-D424, W118-D424, S119-D424, Q120-D424, I121-D424, T122-D424, D123-D424, L124-D424, P125-D424, F126-D424, S127-D424, L128-D424, Y129-D424, S130-D424, T131-D424, F132-D424, V133-D424, I134-D424, E135-D424, S136-D424, R137-D424, H138-D424, G139-D424, F140-D424, N141-D424, K142-D424, Q143-D424, T144-D424, I145-D424, W146-D424, M147-D424, F148-D424, I149-D424, R150-D424, D151-D424, M152-D424, I153-D424, K154-D424, G155-D424, T156-D424, F157-D424, L158-D424, S159-D424, V160-D424, I161-D424, L162-D424, G163-D424, P164-D424, P165-D424, I166-D424, V167-D424, A168-D424, A169-D424, I170-D424, I171-D424, F172-D424, I173-D424, V174-D424, Q175-D424, K176-D424, G177-D424, G178-D424, P179-D424, Y180-D424, L181-D424, A182-D424, I183-D424, Y184-D424, L185-D424, W186-D424, A187-D424, F188-D424, M189-D424, F190-D424, I191-D424, L192-D424, S193-D424, L194-D424, V195-D424, M196-D424, M197-D424, T198-D424, I199-D424, Y200-D424, P201-D424, V202-D424, L203-D424, I204-D424, A205-D424, P206-D424, L207-D424, F208-D424, N209-D424, K210-D424, F211-D424, T212-D424, P213-D424, L214-D424, P215-D424, D216-D424, G217-D424, D218-D424, L219-D424, R220-D424, E221-D424, K222-D424, I223-D424, E224-D424, K225-D424, L226-D424, A227-D424, S228-D424, S229-D424, L230-D424, K231-D424, F232-D424, P233-D424, L234-D424, K235-D424, K236-D424, L237-D424, F238-D424, V239-D424, V240-D424, D241-D424, G242-D424, S243-D424, T244-D424, R245-D424, S246-D424, S247-D424, H248-D424, S249-D424, N250-D424, A251-D424, Y252-D424, M253-D424, Y254-D424, G255-D424, F256-D424, F257-D424, K258-D424, N259-D424, K260-D424, R261-D424, I262-D424, V263-D424, L264-D424, Y265-D424, D266-D424, T267-D424, L268-D424, I269-D424, Q270-D424, Q271-D424, C272-D424, K273-D424, N274-D424, E275-D424, D276-D424, E277-D424, I278-D424, V279-D424, A280-D424, V281-D424, I282-D424, A283-D424, H284-D424, E285-D424, L286-D424, G287-D424, H288-D424, W289-D424, K290-D424, L291-D424, N292-D424, H293-D424, T294-D424, T295-D424, Y296-D424, S297-D424, F298-D424, I299-D424, A300-D424, V301-D424, Q302-D424, I303-D424, L304-D424, A305-D424, F306-D424, L307-D424, Q308-D424, F309-D424, G310-D424, G311-D424, Y312-D424, T313-D424, L314-D424, V315-D424, R316-D424, N317-D424, S318-D424, T319-D424, D320-D424, L321-D424, F322-D424, R323-D424, S324-D424, F325-D424, G326-D424, F327-D424, D328-D424, T329-D424, Q330-D424, P331-D424, V332-D424, L333-D424, I334-D424, G335-D424, L336-D424, I337-D424, I338-D424, F339-D424, Q340-D424, H341-D424, T342-D424, V343-D424, I344-D424, P345-D424, L346-D424, Q347-D424, H348-D424, P349-D424, V350-D424, S351-D424, F352-D424, G353-D424, L354-D424, N355-D424, L356-D424, V357-D424, S358-D424, R359-D424, A360-D424, F361-D424, E362-D424, F363-D424, Q364-D424, A365-D424, D366-D424, A367-D424, F368-D424, A369-D424, V370-D424, K371-D424, L372-D424, G373-D424, Y374-D424, A375-D424, K376-D424, D377-D424, L378-D424, R379-D424, P380-D424, T381-D424, L382-D424, V383-D424, K384-D424, L385-D424, Q386-D424, E387-D424, E388-D424, N389-D424, L390-D424, S391-D424, A392-D424, M393-D424, N394-D424, T395-D424, D396-D424, P397-D424, L398-D424, Y399-D424, S400-D424, A401-D424, Y402-D424, H403-D424, Y404-D424, S405-D424, H406-D424, P407-D424, P408-D424, L409-D424, V410-D424, E411-D424, R412-D424, L413-D424, R414-D424, A415-D424, I416-D424, D417-D424, G418-D424, of SEQ ID NO:4. Polynucleotide sequences encoding these polypeptides are also provided.

In preferred embodiments, the following C-terminal deletion mutants are encompassed by the present invention: M1-D424, M1-T423, M1-K422, M1-K421, M1-D420, M1-E419, M1-G418, M1-D417, M1-I416, M1-A415, M1-R414, M1-L413, M1-R412, M1-E411, M1-V410, M1-L409, M1-P408, M1-P407, M1-H406, M1-S405, M1-Y404, M1-H403, M1-Y402, M1-A401, M1-S400, M1-Y399, M1-L398, M1-P397, M1-D396, M1-T395, M1-N394, M1-M393, M1-A392, M1-S391, M1-L390, M1-N389, M1-E388, M1-E387, M1-Q386, M1-L385, M1-K384, M1-V383, M1-L382, M1-T381, M1-P380, M1-R379, M1-L378, M1-D377, M1-K376, M1-A375, M1-Y374, M1-G373, M1-L372, M1-K371, M1-V370, M1-A369, M1-F368, M1-A367, M1-D366, M1-A365, M1-Q364, M1-F363, M1-E362, M1-F361, M1-A360, M1-R359, M1-S358, M1-V357, M1-L356, M1-N355, M1-L354, M1-G353, M1-F352, M1-S351, M1-V350, M1-P349, M1-H348, M1-Q347, M1-L346, M1-P345, M1-I344, M1-V343, M1-T342, M1-H341, M1-Q340, M1-F339, M1-I338, M1-I337, M1-L336, M1-G335, M1-I334, M1-L333, M1-V332, M1-P331, M1-Q330, M1-T329, M1-D328, M1-F327, M1-G326, M1-F325, M1-S324, M1-R323, M1-F322, M1-L321, M1-D320, M1-T319, M1-5318, M1-N317, M1-R316, M1-V315, M1-L314, M1-T313, M1-Y312, M1-G311, M1-G310, M1-F309, M1-Q308, M1-L307, M1-F306, M1-A305, M1-L304, M1-I303, M1-Q302, M1-V301, M1-A300, M1-I299, M1-F298, M1-S297, M1-Y296, M1-T295, M1-T294, M1-H293, M1-N292, M1-L291, M1-K290, M1-W289, M1-H288, M1-G287, M1-L286, M1-E285, M1-H284, M1-A283, M1-I282, M1-V281, M1-A280, M1-V279, M1-I278, M1-E277, M1-D276, M1-E275, M1-N274, M1-K273, M1-C272, M1-Q271, M1-Q270, M1-I269, M1-L268, M1-T267, M1-D266, M1-Y265, M1-L264, M1-V263, M1-I262, M1-R261, M1-K260, M1-N259, M1-K258, M1-F257, M1-F256, M1-G255, M1-Y254, M1-M253, M1-Y252, M1-A251, M1-N250, M1-S249, M1-H248, M1-S247, M1-S246, M1-R245, M1-T244, M1-5243, M1-G242, M1-D241, M1-V240, M1-V239, M1-F238, M1-L237, M1-K236, M1-K235, M1-L234, M1-P233, M1-F232, M1-K231, M1-L230, M1-S229, M1-S228, M1-A227, M1-L226, M1-K225, M1-E224, M1-I223, M1-K222, M1-E221, M1-R220, M1-L219, M1-D218, M1-G217, M1-D216, M1-P215, M1-L214, M1-P213, M1-T212, M1-F211, M1-K210, M1-N209, M1-F208, M1-L207, M1-P206, M1-A205, M1-I204, M1-L203, M1-V202, M1-P201, M1-Y200, M1-I199, M1-T198, M1-M197, M1-M196, M1-V195, M1-L194, M1-S193, M1-L192, M1-I191, M1-F190, M1-M189, M1-F188, M1-A187, M1-W186, M1-L185, M1-Y184, M1-I183, M1-A182, M1-L181, M1-Y180, M1-P179, M1-G178, M1-G177, M1-K176, M1-Q175, M1-V174, M1-I173, M1-F172, M1-I171, M1-I170, M1-A169, M1-A168, M1-V167, M1-I166, M1-P165, M1-P164, M1-G163, M1-L162, M1-I161, M1-V160, M1-S159, M1-L158, M1-F157, M1-T156, M1-G155, M1-K154, M1-I153, M1-M152, M1-D151, M1-R150, M1-I149, M1-F148, M1-M147, M1-W146, M1-I145, M1-T144, M1-Q143, M1-K142, M1-N141, M1-F140, M1-G139, M1-H138, M1-R137, M1-S136, M1-E135, M1-I134, M1-V133, M1-F132, M1-T131, M1-S130, M1-Y129, M1-L128, M1-S127, M1-F126, M1-P125, M1-L124, M1-D123, M1-T122, M1-I121, M1-Q120, M1-S119, M1-W118, M1-T117, M1-M116, M1-V115, M1-G114, M1-A113, M1-L112, M1-F111, M1-S110, M1-L109, M1-T108, M1-H107, M1-L106, M1-I105, M1-E104, M1-N103, M1-E102, M1-P101, M1-D100, M1-L99, M1-G98, M1-L97, M1-R96, M1-P95, M1-L94, M1-V93, M1-A92, M1-G91, M1-S90, M1-M89, M1-K88, M1-W87, M1-F86, M1-W85, M1-P84, M1-L83, M1-I82, M1-G81, M1-F80, M1-F79, M1-L78, M1-I77, M1-A76, M1-S75, M1-D74, M1-M73, M1-L72, M1-I71, M1-T70, M1-V69, M1-F68, M1-E67, M1-H66, M1-V65, M1-F64, M1-H63, M1-F62, M1-Y61, M1-S60, M1-K59, M1-D58, M1-L57, M1-S56, M1-Y55, M1-A54, M1-R53, M1-S52, M1-K51, M1-E50, M1-F49, M1-K48, M1-E47, M1-Q46, M1-S45, M1-I44, M1-V43, M1-G42, M1-V41, M1-L40, M1-T39, M1-K38, M1-P37, M1-L36, M1-T35, M1-P34, M1-L33, M1-K32, M1-L31, M1-A30, M1-T29, M1-L28, M1-Q27, M1-R26, M1-L25, M1-D24, M1-L23, M1-Y22, M1-T21, M1-E20, M1-F19, M1-I18, M1-Y17, M1-M16, M1-V15, M1-I14, M1-M13, M1-F12, M1-G11, M1-V10, M1-V9, M1-T8, M1-E7, of SEQ ID NO:4. Polynucleotide sequences encoding these polypeptides are also provided.

Many polynucleotide sequences, such as EST sequences, are publicly available and accessible through sequence databases. Some of these sequences are related to SEQ ID NO: 3 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1275 of SEQ ID NO:3, b is an integer between 15 to 1275, where both a and b correspond to the positions of nucleotide residues shown in SEQ ID NO:3, and where b is greater than or equal to a+14.

### Features of the Polypeptide Encoded by Gene No:3

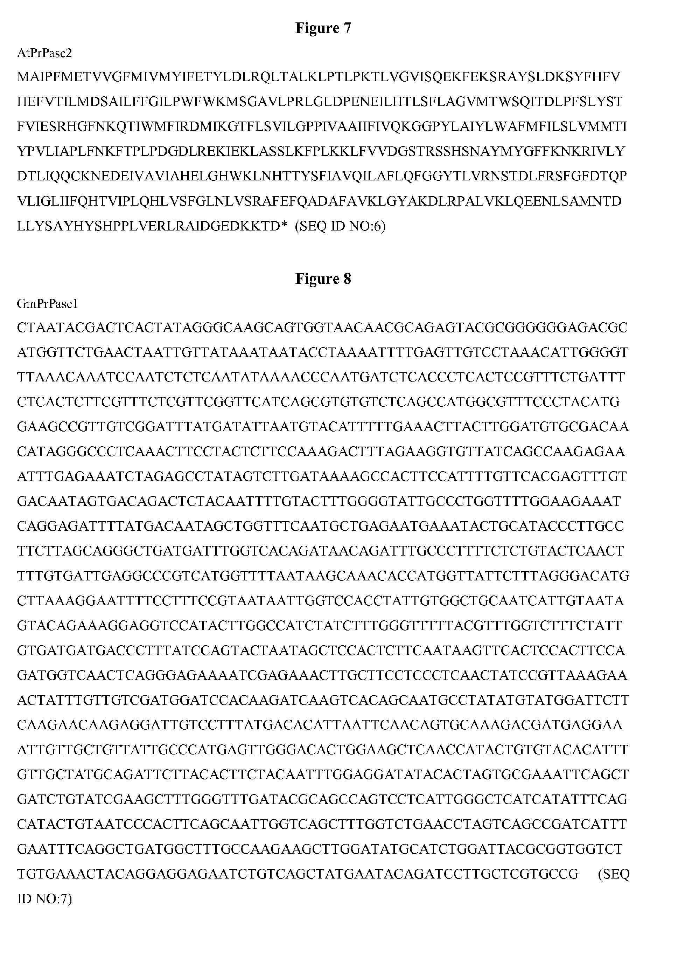

The polypeptide of this gene provided as SEQ ID NO:6 (Figure 7), encoded by the polynucleotide sequence according to SEQ ID NO:5 (Figure 6), and/or encoded by the polynucleotide contained within the deposited clone, AtPrPase2, has significant homology at the nucleotide and amino acid level to both human and yeast prenyl peptidases (see Figures 12A-D). Based upon the homology, the polypeptide of the present invention may share at least some biological activity with prenyl peptidases. The polynucleotide and polypeptide of the present invention, including agonists has uses that include, but are not limited to conferring drought tolerance and/or salt tolerance to plants, particular Arabidopsis. Alternatively, antagonists directed against the polypeptide of the present invention may also be useful in conferring drought tolerance and/or salt tolerance to plants, particular Arabidopsis, and/or to increase the amount of seed storage compounds.

Farnesylation of the bacterial chaperone DnaJ is essential for the bacterial growth at high temperatures (Wickner, S. et al 1991 Nature 350:165-7). Farnesylation of this chaperone is required for its full activity. In the plant Atriplex nummularia, ANJ1 is a homologue of DnaJ and is induced by heat and salt treatments (Zhu, J.-K. et al 1993 The Plant Cell 5:341-9). This enzyme is a target of farnesylation. Increase in the farnesylation of plant chaperones has been shown to result in higher biological activity of these enzymes and consequently lead to increased plant stress tolerance. Therefore, the polynucleotides and polypeptides of the present invention, including agonists and/or fragments thereof, have uses which include conferring resistence to heat, drought, and salt stress in plants. Alternatively, antagonists of the present invention may have uses that include modulating a plants susceptibility to biotic and/or abiotic stresses which include, but are not limited to heat, drought, and salt stress in plants. Preferably antagonists of the present invention increase a plants resistence to heat, drought, and salt stress. In one embodiment, over-expression of a PrPase polypeptide of the present invention within a plant using a constitutive promoter (e.g., 35S, or other promoters disclosed herein), though not in the guard-cell, would be useful for improving drought and salt tolerance in a plant.

Protein farnesylation is positively involved with control of cell cycle (Ziegelhoffer et al 2000 PNAS 97:7633-8). Mutations in genes involved in protein farnesylation, namely farnesyl transferase, results in inhibition of cell proliferation in Arabidopsis plants (Bonetta et al. 2000 Planta 211:182-90). Constitutive over-expression in plants of the farnesylation pathway, namely of the prenyl peptidase, results in increased cell proliferation, increased plant growth. The polynucleotides and polypeptides of the present invention, including agonists and/or fragments thereof, have uses that include modulating plant growth, and potentially plant yield, preferably increasing plant growth. In addition, antagonists of the present invention may have uses that include modulating plant growth and/or yield, through preferably increasing plant growth and yield. In yet another embodiment, over-expression of the AtPrPase2 polypeptide of the present invention using a constitutive promoter (e.g., 35S, or other promoters disclosed herein) may be useful for increasing plant growth by accelerating cell division.

Although it is believed the encoded polypeptide may share at least some biological activities with prenyl proteases, a number of methods of determining the exact biological function of this clone are either known in the art or are described elsewhere herein. Briefly, the function of this clone may be determined by applying microarray methodology. The AtPrPase2 clone, in addition to, other clones of the present invention, may be arrayed on microchips for expression profiling. Depending on which polynucleotide probe is used to hybridize to the slides, a change in expression of a specific gene may provide additional insight into the function of this gene based upon the conditions being studied. For example, an observed increase or decrease in expression levels when the polynucleotide probe used comes from tissue that has been cold treated might indicate a function in modulating cold tolerance, for example. In the case of AtPrPase2, tissue deprived of water or stressed by other biotic or abiotic stresses (heat, drought, high light, high salt, etc.) should be used to extract RNA to prepare the probe.

In addition, the function of the protein may be assessed by applying quantitative PCR methodology, for example. Real time quantitative PCR would provide the capability of following the expression of specific genes throughout the plant development cycle, for example. Quantitative PCR methodology requires only a nominal amount of tissue from each developmentally important step (3 days germinated seedlings, 1 week old seedlings [roots, shoots, and stems]; roots, leaves and stems before the onset of flowering, flowers [different parts]; and/or developing embryos) is needed to perform such experiements. Therefore, the application of quantitative PCR methodology to refining the biological function of this polypeptide is encompassed by the present invention. Also encompassed by the present invention are quantitative PCR probes corresponding to the polynucleotide sequence provided as SEQ ID NO:5 (Figure 6).

The function of the protein may also be assessed through complementation assays in yeast. For example, in the case of the AtPrPase2 clone, transforming yeast deficient in prenyl protease activity and assessing their ability to grow would provide convincing evidence the AtPrPase2 clone has prenyl protease activity. Additional assay conditions and methods that may be used in assessing the function of the polynucletides and polypeptides of the present invention are known in the art, some of which are disclosed elsewhere herein.

Alternatively, the biological function of the encoded polypeptide may be determined by disrupting a homologue of this polypeptide in *Synecosystis.* Cyanobacteria (blue-green algae) is considered a precursor to the plant chloroplast. It possesses both photosynthetic systems and many other metabolic processes reminiscent to those of plants. These processes are often targets for many commercial herbicides, and this organism has been widely used in the study of the mode of action of many classes of herbicides. *Synechocystis* is one of the best-studied cyanobacteria. In addition to most of the features common to cyanobacteria, it offers many other added advantages. *Synechocystis* has a naturally occurring genetic transformation system, thus entailing vigorous and sophisticated genetic and molecular manipulation (e.g. targeted-gene disruption, gene replacement, etc.) applicable to some of the well-characterized systems (*S. cerevisiae, E. coli*). Most importantly, the availability of the complete genomic sequence information of the *Synechocystis* affords an avenue for the rapid identification and cloning of gene(s) of interest, and elucidation of gene function through genetic and molecular means.

Moreover, the biological function of this polypeptide may be determined by the application of antisense and/or sense methodology and the resulting generation of transgenic plants. Expressing a particular gene in either sense or antisense orientation in a transgenic plant can lead to respectively higher or lower expression levels of that particular gene. Altering the endogenous expression levels of a gene can lead to the obervation of a particular phenotype that can then be used to derive indications on the function of the gene. The gene can be either over-expressed or under expressed in every cell of the plant at all times using a strong ubiquitous promoter, or it can be expressed in one or more discrete parts of the plant using a well characterized tissue-specific promoter (i.e., a root promoter or a flower specific promoter), or it can be expressed at a specified time of development using an inducible and/or a developmentally regulated promoter.

In the case of AtPrPase2 transgenic plants, if no phenotype is apparent in normal growth conditions, observing the plants under stress conditions (deprivation of water, presence of high salt, or other biotic or abiotic stresses, such as cold, heat, drought, high light, etc.) or biochemical analysis of seed-storage compounds (oil, sugar, proteins etc.) may lead to understanding the function of the gene. Therefore, the application of antisense and/or sense methodology to the creation of transgenic plants to refine the biological function of the polypeptide is encompassed by the present invention.

In preferred embodiments, the following N-terminal deletion mutants are encompassed by the present invention: M1-D424, A2-D424, I3-D424, P4-D424, F5-D424, M6-D424, E7-D424, T8-D424, V9-D424, V10-D424, G11-D424, F12-D424, M13-D424, I14-D424, V15-D424, M16-D424, Y17-D424, I18-D424, F19-D424, E20-D424, T21-D424, Y22-D424, L23-D424, D24-D424, L25-D424, R26-D424, Q27-D424, L28-D424, T29-D424, A30-D424, L31-D424, K32-D424, L33-D424, P34-D424, T35-D424, L36-D424, P37-D424, K38-D424, T39-D424, L40-D424, V41-D424, G42-D424, V43-D424, I44-D424, S45-D424, Q46-D424, E47-D424, K48-D424, F49-D424, E50-D424, K51-D424, S52-D424, R53-D424, A54-D424, Y55-D424, S56-D424, L57-D424, D58-D424, K59-D424, S60-D424, Y61-D424, F62-D424, H63-D424, F64-D424, V65-D424, H66-D424, E67-D424, F68-D424, V69-D424, T70-D424, I71-D424, L72-D424, M73-D424, D74-D424, S75-D424, A76-D424, I77-D424, L78-D424, F79-D424, F80-D424, G81-D424, I82-D424, L83-D424, P84-D424, W85-D424, F86-D424, W87-D424, K88-D424, M89-D424, S90-D424, G91-D424, A92-D424, V93-D424, L94-D424, P95-D424, R96-D424, L97-D424, G98-D424, L99-D424, D100-D424, P101-D424, E102-D424, N103-D424, E104-D424, I105-D424, L106-D424, H107-D424, T108-D424, L109-D424, S110-D424, F111-D424, L112-D424, A113-D424, G114-D424, V115-D424, M116-D424, T117-D424, W118-D424, S119-D424, Q120-D424, I121-D424, T122-D424, D123-D424, L124-D424, P125-D424, F126-D424, S127-D424, L128-D424, Y129-D424, S130-D424, T131-D424, F132-D424, V133-D424, I134-D424, E135-D424, S136-D424, R137-D424, H138-D424, G139-D424, F140-D424, N141-D424, K142-D424, Q143-D424, T144-D424, I145-D424, W146-D424, M147-D424, F148-D424, I149-D424, R150-D424, D151-D424, M152-D424, I153-D424, K154-D424, G155-D424, T156-D424, F157-D424, L158-D424, S159-D424, V160-D424, I161-D424, L162-D424, G163-D424, P164-D424, P165-D424, I166-D424, V167-D424, A168-D424, A169-D424, I170-D424, I171-D424, F172-D424, I173-D424, V174-D424, Q175-D424, K176-D424, G177-D424, G178-D424, P179-D424, Y180-D424, L181-D424, A182-D424, I183-D424, Y184-D424, L185-D424, W186-D424, A187-D424, F188-D424, M189-D424, F190-D424, I191-D424, L192-D424, S193-D424, L194-D424, V195-D424, M196-D424, M197-D424, T198-D424, I199-D424, Y200-D424, P201-D424, V202-D424, L203-D424, I204-D424, A205-D424, P206-D424, L207-D424, F208-D424, N209-D424, K210-D424, F211-D424, T212-D424, P213-D424, L214-D424, P215-D424, D216-D424, G217-D424, D218-D424, L219-D424, R220-D424, E221-D424, K222-D424, I223-D424, E224-D424, K225-D424, L226-D424, A227-D424, S228-D424, S229-D424, L230-D424, K231-D424, F232-D424, P233-D424, L234-D424, K235-D424, K236-D424, L237-D424, F238-D424, V239-D424, V240-D424, D241-D424, G242-D424, S243-D424, T244-D424, R245-D424, S246-D424, S247-D424, H248-D424, S249-D424, N250-D424, A251-D424, Y252-D424, M253-D424, Y254-D424, G255-D424, F256-D424, F257-D424, K258-D424, N259-D424, K260-D424, R261-D424, I262-D424, V263-D424, L264-D424, Y265-D424, D266-D424, T267-D424, L268-D424, I269-D424, Q270-D424, Q271-D424, C272-D424, K273-D424, N274-D424, E275-D424, D276-D424, E277-D424, I278-D424, V279-D424, A280-D424, V281-D424, I282-D424, A283-D424, H284-D424, E285-D424, L286-D424, G287-D424, H288-D424, W289-D424, K290-D424, L291-D424, N292-D424, H293-D424, T294-D424, T295-D424, Y296-D424, S297-D424, F298-D424, I299-D424, A300-D424, V301-D424, Q302-D424, I303-D424, L304-D424, A305-D424, F306-D424, L307-D424, Q308-D424, F309-D424, G310-D424, G311-D424, Y312-D424, T313-D424, L314-D424, V315-D424, R316-D424, N317-D424, S318-D424, T319-D424, D320-D424, L321-D424, F322-D424, R323-D424, S324-D424, F325-D424, G326-D424, F327-D424, D328-D424, T329-D424, Q330-D424, P331-D424, V332-D424, L333-D424, I334-D424, G335-D424, L336-D424, I337-D424, I338-D424, F339-D424, Q340-D424, H341-D424, T342-D424, V343-D424, I344-D424, P345-D424, L346-D424, Q347-D424, H348-D424, L349-D424, V350-D424, S351-D424, F352-D424, G353-D424, L354-D424, N355-D424, L356-D424, V357-D424, S358-D424, R359-D424, A360-D424, F361-D424, E362-D424, F363-D424, Q364-D424, A365-D424, D366-D424, A367-D424, F368-D424, A369-D424, V370-D424, K371-D424, L372-D424, G373-D424, Y374-D424, A375-D424, K376-D424, D377-D424, L378-D424, R379-D424, P380-D424, A381-D424, L382-D424, V383-D424, K384-D424, L385-D424, Q386-D424, E387-D424, E388-D424, N389-D424, L390-D424, S391-D424, A392-D424, M393-D424, N394-D424, T395-D424, D396-D424, L397-D424, L398-D424, Y399-D424, S400-D424, A401-D424, Y402-D424, H403-D424, Y404-D424, S405-D424, H406-D424, P407-D424, P408-D424, L409-D424, V410-D424, E411-D424, R412-D424, L413-D424, R414-D424, A415-D424, I416-D424, D417-D424, G418-D424, of SEQ ID NO:6. Polynucleotide sequences encoding these polypeptides are also provided.

In preferred embodiments, the following C-terminal deletion mutants are encompassed by the present invention: M1-D424, M1-T423, M1-K422, M1-K421, M1-D420, M1-E419, M1-G418, M1-D417, M1-I416, M1-A415, M1-R414, M1-L413, M1-R412, M1-E411, M1-V410, M1-L409, M1-P408, M1-P407, M1-H406, M1-S405, M1-Y404, M1-H403, M1-Y402, M1-A401, M1-S400, M1-Y399, M1-L398, M1-L397, M1-D396, M1-T395, M1-N394, M1-M393, M1-A392, M1-S391, M1-L390, M1-N389, M1-E388, M1-E387, M1-Q386, M1-L385, M1-K384, M1-V383, M1-L382, M1-A381, M1-P380, M1-R379, M1-L378, M1-D377, M1-K376, M1-A375, M1-Y374, M1-G373, M1-L372, M1-K371, M1-V370, M1-A369, M1-F368, M1-A367, M1-D366, M1-A365, M1-Q364, M1-F363, M1-E362, M1-F361, M1-A360, M1-R359, M1-S358, M1-V357, M1-L356, M1-N355, M1-L354, M1-G353, M1-F352, M1-S351, M1-V350, M1-L349, M1-H348, M1-Q347, M1-L346, M1-P345, M1-I344, M1-V343, M1-T342, M1-H341, M1-Q340, M1-F339, M1-I338, M1-I337, M1-L336, M1-G335, M1-I334, M1-L333, M1-V332, M1-P331, M1-Q330, M1-T329, M1-D328, M1-F327, M1-G326, M1-F325, M1-S324, M1-R323, M1-F322, M1-L321, M1-D320, M1-T319, M1-5318, M1-N317, M1-R316, M1-V315, M1-L314, M1-T313, M1-Y312, M1-G311, M1-G310, M1-F309, M1-Q308, M1-L307, M1-F306, M1-A305, M1-L304, M1-I303, M1-Q302, M1-V301, M1-A300, M1-I299, M1-F298, M1-S297, M1-Y296, M1-T295, M1-T294, M1-H293, M1-N292, M1-L291, M1-K290, M1-W289, M1-H288, M1-G287, M1-L286, M1-E285, M1-H284, M1-A283, M1-I282, M1-V281, M1-A280, M1-V279, M1-I278, M1-E277, M1-D276, M1-E275, M1-N274, M1-K273, M1-C272, M1-Q271, M1-Q270, M1-I269, M1-L268, M1-T267, M1-D266, M1-Y265, M1-L264, M1-V263, M1-I262, M1-R261, M1-K260, M1-N259, M1-K258, M1-F257, M1-F256, M1-G255, M1-Y254, M1-M253, M1-Y252, M1-A251, M1-N250, M1-S249, M1-H248, M1-S247, M1-S246, M1-R245, M1-T244, M1-S243, M1-G242, M1-D241, M1-V240, M1-V239, M1-F238, M1-L237, M1-K236, M1-K235, M1-L234, M1-P233, M1-F232, M1-K231, M1-L230, M1-S229, M1-S228, M1-A227, M1-L226, M1-K225, M1-E224, M1-I223, M1-K222, M1-E221, M1-R220, M1-L219, M1-D218, M1-G217, M1-D216, M1-P215, M1-L214, M1-P213, M1-T212, M1-F211, M1-K210, M1-N209, M1-F208, M1-L207, M1-P206, M1-A205, M1-I204, M1-L203, M1-V202, M1-P201, M1-Y200, M1-I199, M1-T198, M1-M197, M1-M196, M1-V195, M1-L194, M1-S193, M1-L192, M1-I191, M1-F190, M1-M189, M1-F188, M1-A187, M1-W186, M1-L185, M1-Y184, M1-I183, M1-A182, M1-L181, M1-Y180, M1-P179, M1-G178, M1-G177, M1-K176, M1-Q175, M1-V174, M1-I173, M1-F172, M1-I171, M1-I170, M1-A169, M1-A168, M1-V167, M1-I166, M1-P165, M1-P164, M1-G163, M1-L162, M1-I161, M1-V160, M1-S159, M1-L158, M1-F157, M1-T156, M1-G155, M1-K154, M1-I153, M1-M152, M1-D151, M1-R150, M1-I149, M1-F148, M1-M147, M1-W146, M1-I145, M1-T144, M1-Q143, M1-K142, M1-N141, M1-F140, M1-G139, M1-H138, M1-R137, M1-5136, M1-E135, M1-I134, M1-V133, M1-F132, M1-T131, M1-S130, M1-Y129, M1-L128, M1-S127, M1-F126, M1-P125, M1-L124, M1-D123, M1-T122, M1-I121, M1-Q120, M1-S119, M1-W118, M1-T117, M1-M116, M1-V115, M1-G114, M1-A113, M1-L112, M1-F111, M1-S110, M1-L109, M1-T108, M1-H107, M1-L106, M1-I105, M1-E104, M1-N103, M1-E102, M1-P101, M1-D100, M1-L99, M1-G98, M1-L97, M1-R96, M1-P95, M1-L94, M1-V93, M1-A92, M1-G91, M1-S90, M1-M89, M1-K88, M1-W87, M1-F86, M1-W85, M1-P84, M1-L83, M1-I82, M1-G81, M1-F80, M1-F79, M1-L78, M1-I77, M1-A76, M1-S75, M1-D74, M1-M73, M1-L72, M1-I71, M1-T70, M1-V69, M1-F68, M1-E67, M1-H66, M1-V65, M1-F64, M1-H63, M1-F62, M1-Y61, M1-S60, M1-K59, M1-D58, M1-L57, M1-S56, M1-Y55, M1-A54, M1-R53, M1-S52, M1-K51, M1-E50, M1-F49, M1-K48, M1-E47, M1-Q46, M1-S45, M1-I44, M1-V43, M1-G42, M1-V41, M1-L40, M1-T39, M1-K38, M1-P37, M1-L36, M1-T35, M1-P34, M1-L33, M1-K32, M1-L31, M1-A30, M1-T29, M1-L28, M1-Q27, M1-R26, M1-L25, M1-D24, M1-L23, M1-Y22, M1-T21, M1-E20, M1-F19, M1-I18, M1-Y17, M1-M16, M1-V15, M1-I14, M1-M13, M1-F12, M1-G11, M1-V10, M1-V9, M1-T8, M1-E7, of SEQ ID NO:6. Polynucleotide sequences encoding these polypeptides are also provided.

Many polynucleotide sequences, such as EST sequences, are publicly available and accessible through sequence databases. Some of these sequences are related to SEQ ID NO: 5 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1275 of SEQ ID NO:5, b is an integer between 15 to 1275, where both a and b correspond to the positions of nucleotide residues shown in SEQ ID NO:5, and where b is greater than or equal to a+14.

### Features of the Polypeptide Encoded by Gene No:4

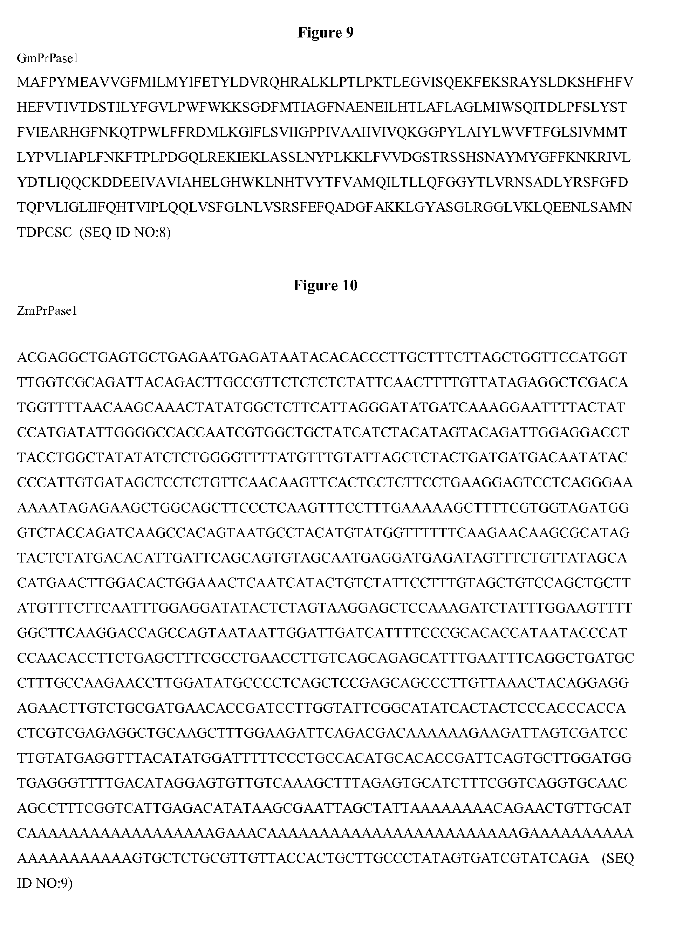

The polypeptide of this gene provided as SEQ ID NO:8 (Figure 9), encoded by the polynucleotide sequence according to SEQ ID NO:7 (Figure 8), and/or encoded by the polynucleotide contained within the deposited clone, GmPrPasel, has significant homology at the nucleotide and amino acid level to both human and yeast prenyl peptidases (see Figures 12A-D). Based upon the homology, the polypeptide of the present invention may share at least some biological activity with prenyl peptidases. The polynucleotide and polypeptide of the present invention, including agonists has uses that include, but are not limited to conferring drought tolerance and/or salt tolerance to plants, particular Arabidopsis. Alternatively, antagonists directed against the polypeptide of the present invention may also be useful in conferring drought tolerance and/or salt tolerance to plants, particular Arabidopsis.

Farnesylation of the bacterial chaperone DnaJ is essential for the bacterial growth at high temperatures (Wickner, S. et al 1991 Nature 350:165-7). Farnesylation of this chaperone is required for its full activity. In the plant Atriplex nummularia, ANJ1 is a homologue of DnaJ and is induced by heat and salt treatments (Zhu, J.-K. et al 1993 The Plant Cell 5:341-9). This enzyme is a target of farnesylation. Increase in the farnesylation of plant chaperones has been shown to result in higher biological activity of these enzymes and consequently lead to increased plant stress tolerance. Therefore, the polynucleotides and polypeptides of the present invention, including agonists and/or fragments thereof, have uses which include conferring resistence to heat, drought, and salt stress in plants. Alternatively, antagonists of the present invention may have uses that include modulating a plants susceptibility to biotic and/or abiotic stresses which include, but are not limited to heat, drought, and salt stress in plants. Preferably antagonists of the present invention increase a plants resistence to heat, drought, and salt stress. In one embodiment, over-expression of a PrPase polypeptide of the present invention within a plant using a constitutive promoter (e.g., 35S, or other promoters disclosed herein), though not in the guard-cell, would be useful for improving drought and salt tolerance in a plant.

In another embodiment, overexpression of a PrPase polypeptide of the present invention within a plant using a seed-specific promoter (e.g. unknown seed protein, USP, promoter) would be useful for increasing the amount of seed storage compounds. Furthermore, antagonists of the present intention may have uses that include modulating the amount of a plant seed-storage compound.

Protein farnesylation is positively involved with control of cell cycle (Ziegelhoffer et al 2000 PNAS 97:7633-8). Mutations in genes involved in protein farnesylation, namely farnesyl transferase, results in inhibition of cell proliferation in Arabidopsis plants (Bonetta et al. 2000 Planta 211:182-90). Constitutive over-expression in plants of the farnesylation pathway, namely of the prenyl peptidase, results in increased cell proliferation, increased plant growth. The polynucleotides and polypeptides of the present invention, including agonists and/or fragments thereof, have uses that include modulating plant growth, and potentially plant yield, preferably increasing plant growth. In addition, antagonists of the present invention may have uses that include modulating plant growth and/or yield, through preferably increasing plant growth and yield. In yet another embodiment, over-expression of the GmPrPase1 polypeptide of the present invention using a constitutive promoter (e.g., 35S, or other promoters disclosed herein) may be useful for increasing plant growth by accelerating cell division.

Although it is believed the encoded polypeptide may share at least some biological activities with prenyl proteases, a number of methods of determining the exact biological function of this clone are either known in the art or are described elsewhere herein. Briefly, the function of this clone may be determined by applying microarray methodology. The GmPrPasel clone, in addition to, other clones of the present invention, may be arrayed on microchips for expression profiling. Depending on which polynucleotide probe is used to hybridize to the slides, a change in expression of a specific gene may provide additional insight into the function of this gene based upon the conditions being studied. For example, an observed increase or decrease in expression levels when the polynucleotide probe used comes from tissue that has been cold treated might indicate a function in modulating cold tolerance, for example. In the case of GmPrPase1, tissue deprived of water or stressed by other biotic or abiotic stresses (heat, drought, high light, high salt, etc.) should be used to extract RNA to prepare the probe. Moreover, different stages of seed development (early, middle, late) should be used to extract RNA to prepare the probe.

In addition, the function of the protein may be assessed by applying quantitative PCR methodology, for example. Real time quantitative PCR would provide the capability of following the expression of specific genes throughout the plant development cycle, for example. Quantitative PCR methodology requires only a nominal amount of tissue from each developmentally important step (3 days germinated seedlings, 1 week old seedlings [roots, shoots, and stems]; roots, leaves and stems before the onset of flowering, flowers [different parts]; and/or developing embryos) is needed to perform such experiements. Therefore, the application of quantitative PCR methodology to refining the biological function of this polypeptide is encompassed by the present invention. Also encompassed by the present invention are quantitative PCR probes corresponding to the polynucleotide sequence provided as SEQ ID NO:7 (Figure 8).

The function of the protein may also be assessed through complementation assays in yeast. For example, in the case of the GmPrPasel clone, transforming yeast deficient in prenyl protease activity and assessing their ability to grow would provide convincing evidence the GmPrPase1 clone has prenyl protease activity. Additional assay conditions and methods that may be used in assessing the function of the polynucletides and polypeptides of the present invention are known in the art, some of which are disclosed elsewhere herein.

Alternatively, the biological function of the encoded polypeptide may be determined by disrupting a homologue of this polypeptide in *Synecosystis.* Cyanobacteria (blue-green algae) an ancestor to the plant chloroplast. It possesses both photosystems and many other metabolic processes reminiscent to those of plants. These processes are often targets for many commercial herbicides, and this organism has been widely used in the study of the mode of action of many classes of herbicides. *Synechocystis* is one of the best-studied cyanobacteria. In addition to most of the features common to cyanobacteria, it offers many other added advantages. *Synechocystis* has a naturally occurring genetic transformation system, thus entailing vigorous and sophisticated genetic and molecular manipulation (e.g. targeted-gene disruption, gene replacement, etc.) applicable to some of the well-characterized systems (*S. cerevisiae, E. coli*). Most importantly, the availability of the complete genomic sequence information of *Synechocystis* affords an avenue for the rapid identification and cloning of gene(s) of interest, and elucidation of gene function through genetic and molecular means.

Moreover, the biological function of this polypeptide may be determined by the application of antisense and/or sense methodology and the resulting generation of transgenic plants. Expressing a particular gene in either sense or antisense orientation in a transgenic plant can lead to respectively higher or lower expression levels of that particular gene. Altering the endogenous expression levels of a gene can lead to the obervation of a particular phenotype that can then be used to derive indications on the function of the gene. The gene can be either over-expressed or under expressed in every cell of the plant at all times using a strong ubiquitous promoter, or it can be expressed in one or more discrete parts of the plant using a well characterized tissue-specific promoter (i.e., a root promoter or a flower specific promoter or a seed-specific promoter), or it can be expressed at a specified time of development using an inducible and/or a developmentally regulated promoter.

In the case of GmPrPase1 transgenic plants, if no phenotype is apparent in normal growth conditions, observing the plants under stress conditions (deprivation of water, presence of high salt, or other biotic or abiotic stresses, such as cold, heat, drought, high light, etc.) and/or biochemical analyses of the amount of seed storage compounds (oils, sugars, proteins etc.) may lead to understanding the function of the gene. Therefore, the application of antisense and/or sense methodology to the creation of transgenic plants to refine the biological function of the polypeptide is encompassed by the present invention.

In preferred embodiments, the following N-terminal deletion mutants are encompassed by the present invention: M1-C400, A2-C400, F3-C400, P4-C400, Y5-C400, M6-C400, E7-C400, A8-C400, V9-C400, V10-C400, G11-C400, F12-C400, M13-C400, I14-C400, L15-C400, M16-C400, Y17-C400, I18-C400, F19-C400, E20-C400, T21-C400, Y22-C400, L23-C400, D24-C400, V25-C400, R26-C400, Q27-C400, H28-C400, R29-C400, A30-C400, L31-C400, K32-C400, L33-C400, P34-C400, T35-C400, L36-C400, P37-C400, K38-C400, T39-C400, L40-C400, E41-C400, G42-C400, V43-C400, I44-C400, S45-C400, Q46-C400, E47-C400, K48-C400, F49-C400, E50-C400, K51-C400, S52-C400, R53-C400, A54-C400, Y55-C400, S56-C400, L57-C400, D58-C400, K59-C400, S60-C400, H61-C400, F62-C400, H63-C400, F64-C400, V65-C400, H66-C400, E67-C400, F68-C400, V69-C400, T70-C400, I71-C400, V72-C400, T73-C400, D74-C400, S75-C400, T76-C400, I77-C400, L78-C400, Y79-C400, F80-C400, G81-C400, V82-C400, L83-C400, P84-C400, W85-C400, F86-C400, W87-C400, K88-C400, K89-C400, S90-C400, G91-C400, D92-C400, F93-C400, M94-C400, T95-C400, I96-C400, A97-C400, G98-C400, F99-C400, N100-C400, A101-C400, E102-C400, N103-C400, E104-C400, I105-C400, L106-C400, H107-C400, T108-C400, L109-C400, A110-C400, F111-C400, L112-C400, A113-C400, G114-C400, L115-C400, M116-C400, I117-C400, W118-C400, S119-C400, Q120-C400, I121-C400, T122-C400, D123-C400, L124-C400, P125-C400, F126-C400, S127-C400, L128-C400, Y129-C400, S130-C400, T131-C400, F132-C400, V133-C400, I134-C400, E135-C400, A136-C400, R137-C400, H138-C400, G139-C400, F140-C400, N141-C400, K142-C400, Q143-C400, T144-C400, P145-C400, W146-C400, L147-C400, F148-C400, F149-C400, R150-C400, D151-C400, M152-C400, L153-C400, K154-C400, G155-C400, I156-C400, F157-C400, L158-C400, S159-C400, V160-C400, I161-C400, I162-C400, G163-C400, P164-C400, P165-C400, I166-C400, V167-C400, A168-C400, A169-C400, I170-C400, I171-C400, V172-C400, I173-C400, V174-C400, Q175-C400, K176-C400, G177-C400, G178-C400, P179-C400, Y180-C400, L181-C400, A182-C400, I183-C400, Y184-C400, L185-C400, W186-C400, V187-C400, F188-C400, T189-C400, F190-C400, G191-C400, L192-C400, S193-C400, I194-C400, V195-C400, M196-C400, M197-C400, T198-C400, L199-C400, Y200-C400, P201-C400, V202-C400, L203-C400, I204-C400, A205-C400, P206-C400, L207-C400, F208-C400, N209-C400, K210-C400, F211-C400, T212-C400, P213-C400, L214-C400, P215-C400, D216-C400, G217-C400, Q218-C400, L219-C400, R220-C400, E221-C400, K222-C400, I223-C400, E224-C400, K225-C400, L226-C400, A227-C400, S228-C400, S229-C400, L230-C400, N231-C400, Y232-C400, P233-C400, L234-C400, K235-C400, K236-C400, L237-C400, F238-C400, V239-C400, V240-C400, D241-C400, G242-C400, S243-C400, T244-C400, R245-C400, S246-C400, S247-C400, H248-C400, S249-C400, N250-C400, A251-C400, Y252-C400, M253-C400, Y254-C400, G255-C400, F256-C400, F257-C400, K258-C400, N259-C400, K260-C400, R261-C400, I262-C400, V263-C400, L264-C400, Y265-C400, D266-C400, T267-C400, L268-C400, I269-C400, Q270-C400, Q271-C400, C272-C400, K273-C400, D274-C400, D275-C400, E276-C400, E277-C400, I278-C400, V279-C400, A280-C400, V281-C400, I282-C400, A283-C400, H284-C400, E285-C400, L286-C400, G287-C400, H288-C400, W289-C400, K290-C400, L291-C400, N292-C400, H293-C400, T294-C400, V295-C400, Y296-C400, T297-C400, F298-C400, V299-C400, A300-C400, M301-C400, Q302-C400, I303-C400, L304-C400, T305-C400, L306-C400, L307-C400, Q308-C400, F309-C400, G310-C400, G311-C400, Y312-C400, T313-C400, L314-C400, V315-C400, R316-C400, N317-C400, S318-C400, A319-C400, D320-C400, L321-C400, Y322-C400, R323-C400, S324-C400, F325-C400, G326-C400, F327-C400, D328-C400, T329-C400, Q330-C400, P331-C400, V332-C400, L333-C400, I334-C400, G335-C400, L336-C400, I337-C400, I338-C400, F339-C400, Q340-C400, H341-C400, T342-C400, V343-C400, I344-C400, P345-C400, L346-C400, Q347-C400, Q348-C400, L349-C400, V350-C400, S351-C400, F352-C400, G353-C400, L354-C400, N355-C400, L356-C400, V357-C400, S358-C400, R359-C400, S360-C400, F361-C400, E362-C400, F363-C400, Q364-C400, A365-C400, D366-C400, G367-C400, F368-C400, A369-C400, K370-C400, K371-C400, L372-C400, G373-C400, Y374-C400, A375-C400, S376-C400, G377-C400, L378-C400, R379-C400, G380-C400, G381-C400, L382-C400, V383-C400, K384-C400, L385-C400, Q386-C400, E387-C400, E388-C400, N389-C400, L390-C400, S391-C400, A392-C400, M393-C400, N394-C400, of SEQ ID NO:8. Polynucleotide sequences encoding these polypeptides are also provided.

In preferred embodiments, the following C-terminal deletion mutants are encompassed by the present invention: M1-C400, M1-S399, M1-C398, M1-P397, M1-D396, M1-T395, M1-N394, M1-M393, M1-A392, M1-S391, M1-L390, M1-N389, M1-E388, M1-E387, M1-Q386, M1-L385, M1-K384, M1-V383, M1-L382, M1-G381, M1-G380, M1-R379, M1-L378, M1-G377, M1-S376, M1-A375, M1-Y374, M1-G373, M1-L372, M1-K371, M1-K370, M1-A369, M1-F368, M1-G367, M1-D366, M1-A365, M1-Q364, M1-F363, M1-E362, M1-F361, M1-S360, M1-R359, M1-S358, M1-V357, M1-L356, M1-N355, M1-L354, M1-G353, M1-F352, M1-S351, M1-V350, M1-L349, M1-Q348, M1-Q347, M1-L346, M1-P345, M1-I344, M1-V343, M1-T342, M1-H341, M1-Q340, M1-F339, M1-I338, M1-I337, M1-L336, M1-G335, M1-I334, M1-L333, M1-V332, M1-P331, M1-Q330, M1-T329, M1-D328, M1-F327, M1-G326, M1-F325, M1-S324, M1-R323, M1-Y322, M1-L321, M1-D320, M1-A319, M1-S318, M1-N317, M1-R316, M1-V315, M1-L314, M1-T313, M1-Y312, M1-G311, M1-G310, M1-F309, M1-Q308, M1-L307, M1-L306, M1-T305, M1-L304, M1-I303, M1-Q302, M1-M301, M1-A300, M1-V299, M1-F298, M1-T297, M1-Y296, M1-V295, M1-T294, M1-H293, M1-N292, M1-L291, M1-K290, M1-W289, M1-H288, M1-G287, M1-L286, M1-E285, M1-H284, M1-A283, M1-I282, M1-V281, M1-A280, M1-V279, M1-I278, M1-E277, M1-E276, M1-D275, M1-D274, M1-K273, M1-C272, M1-Q271, M1-Q270, M1-I269, M1-L268, M1-T267, M1-D266, M1-Y265, M1-L264, M1-V263, M1-I262, M1-R261, M1-K260, M1-N259, M1-K258, M1-F257, M1-F256, M1-G255, M1-Y254, M1-M253, M1-Y252, M1-A251, M1-N250, M1-S249, M1-H248, M1-S247, M1-S246, M1-R245, M1-T244, M1-S243, M1-G242, M1-D241, M1-V240, M1-V239, M1-F238, M1-L237, M1-K236, M1-K235, M1-L234, M1-P233, M1-Y232, M1-N231, M1-L230, M1-S229, M1-S228, M1-A227, M1-L226, M1-K225, M1-E224, M1-I223, M1-K222, M1-E221, M1-R220, M1-L219, M1-Q218, M1-G217, M1-D216, M1-P215, M1-L214, M1-P213, M1-T212, M1-F211, M1-K210, M1-N209, M1-F208, M1-L207, M1-P206, M1-A205, M1-I204, M1-L203, M1-V202, M1-P201, M1-Y200, M1-L199, M1-T198, M1-M197, M1-M196, M1-V195, M1-I194, M1-S193, M1-L192, M1-G191, M1-F190, M1-T189, M1-F188, M1-V187, M1-W186, M1-L185, M1-Y184, M1-I183, M1-A182, M1-L181, M1-Y180, M1-P179, M1-G178, M1-G177, M1-K176, M1-Q175, M1-V174, M1-I173, M1-V172, M1-I171, M1-I170, M1-A169, M1-A168, M1-V167, M1-I166, M1-P165, M1-P164, M1-G163, M1-I162, M1-I161, M1-V160, M1-S159, M1-L158, M1-F157, M1-I156, M1-G155, M1-K154, M1-L153, M1-M152, M1-D151, M1-R150, M1-F149, M1-F148, M1-L147, M1-W146, M1-P145, M1-T144, M1-Q143, M1-K142, M1-N141, M1-F140, M1-G139, M1-H138, M1-R137, M1-A136, M1-E135, M1-I134, M1-V133, M1-F132, M1-T131, M1-S130, M1-Y129, M1-L128, M1-S127, M1-F126, M1-P125, M1-L124, M1-D123, M1-T122, M1-I121, M1-Q120, M1-S119, M1-W118, M1-I117, M1-M116, M1-L115, M1-G114, M1-A113, M1-L112, M1-F111, M1-A110, M1-L109, M1-T108, M1-H107, M1-L106, M1-I105, M1-E104, M1-N103, M1-E102, M1-A101, M1-N100, M1-F99, M1-G98, M1-A97, M1-I96, M1-T95, M1-M94, M1-F93, M1-D92, M1-G91, M1-S90, M1-K89, M1-K88, M1-W87, M1-F86, M1-W85, M1-P84, M1-L83, M1-V82, M1-G81, M1-F80, M1-Y79, M1-L78, MI-177, M1-T76, M1-575, M1-D74, M1-T73, M1-V72, M1-I71, M1-T70, M1-V69, M1-F68, M1-E67, M1-H66, M1-V65, M1-F64, M1-H63, M1-F62, M1-H61, M1-S60, M1-K59, M1-D58, M1-L57, M1-S56, M1-Y55, M1-A54, M1-R53, M1-S52, M1-K51, M1-E50, M1-F49, M1-K48, M1-E47, M1-Q46, M1-S45, M1-I44, M1-V43, M1-G42, M1-E41, M1-L40, M1-T39, M1-K38, M1-P37, M1-L36, M1-T35, M1-P34, M1-L33, M1-K32, M1-L31, M1-A30, M1-R29, M1-H28, M1-Q27, M1-R26, M1-V25, M1-D24, M1-L23, M1-Y22, M1-T21, M1-E20, M1-F19, M1-I18, M1-Y17, M1-M16, M1-L15, M1-I14, M1-M13, M1-F12, M1-G11, M1-V10, M1-V9, M1-A8, M1-E7, of SEQ ID NO:8. Polynucleotide sequences encoding these polypeptides are also provided.

Many polynucleotide sequences, such as EST sequences, are publicly available and accessible through sequence databases. Some of these sequences are related to SEQ ID NO: 7 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1434 of SEQ ID NO:7, b is an integer between 15 to 1434, where both a and b correspond to the positions of nucleotide residues shown in SEQ ID NO:7, and where b is greater than or equal to a+14.

### Features of the Polypeptide Encoded by Gene No:5

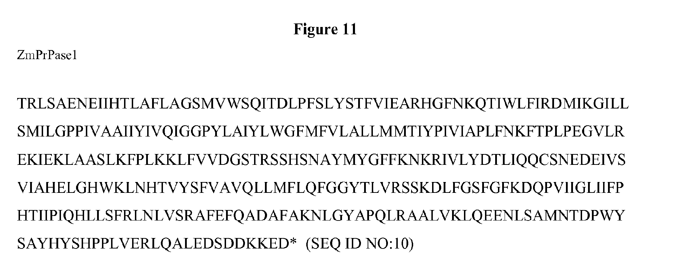

The polypeptide of this gene provided as SEQ ID NO:10 (Figure 11), encoded by the polynucleotide sequence according to SEQ ID NO:9 (Figure 10), and/or encoded by the polynucleotide contained within the deposited clone, ZmPrPase1, has significant homology at the nucleotide and amino acid level to both human and yeast prenyl peptidases (see Figures 12A-D). Based upon the homology, the polypeptide of the present invention may share at least some biological activity with prenyl peptidases. The polynucleotide and polypeptide of the present invention, in addition to agonists and/or fragments thereof, has uses that include, but are not limited to conferring drought tolerance and/or salt tolerance to plants. The polynucleotide also has uses which includes identifying the full-length ZmPrPase1. Alternatively, antagonists directed against the polypeptide of the present invention may also be useful in conferring drought tolerance and/or salt tolerance to plants.

Farnesylation of the bacterial chaperone DnaJ is essential for the bacterial growth at high temperatures (Wickner, S. et al 1991, Nature 350:165-7). Farnesylation of this chaperone is required for its full activity. In the plant Atriplex nummularia, ANJ1 is a homologue of DnaJ and is induced by heat and salt treatments (Zhu, J.-K. et al 1993 The Plant Cell 5:341-9). This enzyme is a target of farnesylation. Increase in the farnesylation of plant chaperones has been shown to result in higher biological activity of these enzymes and consequently lead to increased plant stress tolerance. Therefore, the polynucleotides and polypeptides of the present invention, including agonists and/or fragments thereof, have uses which include conferring resistence to heat, drought, and salt stress in plants. Alternatively, antagonists of the present invention may have uses that include modulating a plants susceptibility to biotic and/or abiotic stresses which include, but are not limited to heat, drought, and salt stress in plants. Preferably antagonists of the present invention increase a plants resistence to heat, drought, and salt stress. In one embodiment, over-expression of a PrPase polypeptide of the present invention within a plant using a constitutive promoter (e.g., 35S, or other promoters disclosed herein), though not in the guard-cell, would be useful for improving drought and salt tolerance in a plant.

Protein farnesylation is positively involved with control of cell cycle (Ziegelhoffer et al 2000 PNAS 97:7633-8). Mutations in genes involved in protein farnesylation, namely farnesyl transferase, results in inhibition of cell proliferation in Arabidopsis plants (Bonetta et al. 2000 Planta 211:182-90). Constitutive over-expression in plants of the farnesylation pathway, namely of the prenyl peptidase, results in increased cell proliferation, increased plant growth. The polynucleotides and polypeptides of the present invention, including agonists and/or fragments thereof, have uses that include modulating plant growth, and potentially plant yield, preferably increasing plant growth. In addition, antagonists of the present invention may have uses that include modulating plant growth and/or yield, through preferably increasing plant growth and yield. In yet another embodiment, over-expression of the ZmPrPase1 polypeptide of the present invention using a constitutive promoter (e.g., 35S, or other promoters disclosed herein) may be useful for increasing plant growth by accelerating cell division.

Although it is believed the encoded polypeptide may share at least some biological activities with prenyl proteases, a number of methods of determining the exact biological function of this clone are either known in the art or are described elsewhere herein. Briefly, the function of this clone may be determined by applying microarray methodology. The ZmPrPase1 clone, in addition to, other clones of the present invention, may be arrayed on microchips for expression profiling. Depending on which polynucleotide probe is used to hybridize to the slides, a change in expression of a specific gene may provide additional insight into the function of this gene based upon the conditions being studied. For example, an observed increase or decrease in expression levels when the polynucleotide probe used comes from tissue that has been cold treated might indicate a function in modulating cold tolerance, for example. In the case of ZmPrPase1, tissue deprived of water or stressed by other biotic or abiotic stresses (heat, drought, high light, high salt, etc.) should be used to extract RNA to prepare the probe.

In addition, the function of the protein may be assessed by applying quantitative PCR methodology, for example. Real time quantitative PCR would provide the capability of following the expression of specific genes throughout the plant development cycle, for example. Quantitative PCR methodology requires only a nominal amount of tissue from each developmentally important step (3 days germinated seedlings, 1 week old seedlings [roots, shoots, and stems]; roots, leaves and stems before the onset of flowering, flowers [different parts]; and/or developing embryos) is needed to perform such experiements. Therefore, the application of quantitative PCR methodology to refining the biological function of this polypeptide is encompassed by the present invention. Also encompassed by the present invention are quantitative PCR probes corresponding to the polynucleotide sequence provided as SEQ ID NO:9 (Figure 10).

The function of the protein may also be assessed through complementation assays in yeast. For example, in the case of the ZmPrPase1 clone, transforming yeast deficient in prenyl protease activity and assessing their ability to grow would provide convincing evidence the ZmPrPase1 clone has prenyl protease activity. Additional assay conditions and methods that may be used in assessing the function of the polynucletides and polypeptides of the present invention are known in the art, some of which are disclosed elsewhere herein.

Alternatively, the biological function of the encoded polypeptide may be determined by disrupting a homologue of this polypeptide in *Synecosystis*. Cyanobacteria (blue-green algae) is considered a precursor to the plant chloroplast. It possesses both photosynthetic systems and many other metabolic processes reminiscent to those of plants. These processes are often targets for many commercial herbicides, and this organism has been widely used in the study of the mode of action of many classes of herbicides. *Synechocystis* is one of the best-studied cyanobacteria. In addition to most of the features common to cyanobacteria, it offers many other added advantages. *Synechocystis* has a naturally occurring genetic transformation system, thus entailing vigorous and sophisticated genetic and molecular manipulation (e.g. targeted-gene disruption, gene replacement, etc.) applicable to some of the well-characterized systems (*S. cerevisiae, E. coli*). Most importantly, the availability of the complete genomic sequence information of the *Synechocystis* affords an avenue for the rapid identification and cloning of gene(s) of interest, and elucidation of gene function through genetic and molecular means.

Moreover, the biological function of this polypeptide may be determined by the application of antisense and/or sense methodology and the resulting generation of transgenic plants. Expressing a particular gene in either sense or antisense orientation in a transgenic plant can lead to respectively higher or lower expression levels of that particular gene. Altering the endogenous expression levels of a gene can lead to the obervation of a particular phenotype that can then be used to derive indications on the function of the gene. The gene can be either over-expressed or under expressed in every cell of the plant at all times using a strong ubiquitous promoter, or it can be expressed in one or more discrete parts of the plant using a well characterized tissue-specific promoter (i.e., a root promoter or a flower specific promoter), or it can be expressed at a specified time of development using an inducible and/or a developmentally regulated promoter.

In the case of ZmPrPase1 transgenic plants, if no phenotype is apparent in normal growth conditions, observing the plants under stress conditions (deprivation of water, presence of high salt, or other biotic or abiotic stresses, such as cold, heat, drought, high light, etc.) may lead to understanding the function of the gene. Therefore, the application of antisense and/or sense methodology to the creation of transgenic plants to refine the biological function of the polypeptide is encompassed by the present invention.

In preferred embodiments, the following N-terminal deletion mutants are encompassed by the present invention: T1-D329, R2-D329, L3-D329, S4-D329, A5-D329, E6-D329, N7-D329, E8-D329,19-D329, I10-D329, H11-D329, T12-D329, L13-D329, A14-D329, F15-D329, L16-D329, A17-D329, G18-D329, S19-D329, M20-D329, V21-D329, W22-D329, S23-D329, Q24-D329, 125-D329, T26-D329, D27-D329, L28-D329, P29-D329, F30-D329, S31-D329, L32-D329, Y33-D329, S34-D329, T35-D329, F36-D329, V37-D329, I38-D329, E39-D329, A40-D329, R41-D329, H42-D329, G43-D329, F44-D329, N45-D329, K46-D329, Q47-D329, T48-D329, I49-D329, W50-D329, L51-D329, F52-D329, I53-D329, R54-D329, D55-D329, M56-D329,157-D329, K58-D329, G59-D329, I60-D329, L61-D329, L62-D329, S63-D329, M64-D329, I65-D329, L66-D329, G67-D329, P68-D329, P69-D329, 70-D329, V71-D329, A72-D329, A73-D329, I74-D329, I75-D329, Y76-D329, 77-D329, V78-D329, Q79-D329, 80-D329, G81-D329, G82-D329, P83-D329, Y84-D329, L85-D329, A86-D329, I87-D329, Y88-D329, L89-D329, W90-D329, G91-D329, F92-D329, M93-D329, F94-D329, V95-D329, L96-D329, A97-D329, L98-D329, L99-D329, M100-D329, M101-D329, T102-D329, I103-D329, Y104-D329, P105-D329, I106-D329, V107-D329, I108-D329, A109-D329, P110-D329, L111-D329, F112-D329, N113-D329, K114-D329, F115-D329, T116-D329, P117-D329, L118-D329, P119-D329, E120-D329, G121-D329, V122-D329, L123-D329, R124-D329, E125-D329, K126-D329, I127-D329, E128-D329, K129-D329, L130-D329, A131-D329, A132-D329, S133-D329, L134-D329, K135-D329, F136-D329, P137-D329, L138-D329, K139-D329, K140-D329, L141-D329, F142-D329, V143-D329, V144-D329, D145-D329, G146-D329, S147-D329, T148-D329, R149-D329, S150-D329, S151-D329, H152-D329, S153-D329, N154-D329, A155-D329, Y156-D329, M157-D329, Y158-D329, G159-D329, F160-D329, F161-D329, K162-D329, N163-D329, K164-D329, R165-D329, I166-D329, V167-D329, L168-D329, Y169-D329, D170-D329, T171-D329, L172-D329, I173-D329, Q174-D329, Q175-D329, C176-D329, S177-D329, N178-D329, E179-D329, D180-D329, E181-D329, I182-D329, V183-D329, S184-D329, V185-D329, I186-D329, A187-D329, H188-D329, E189-D329, L190-D329, G191-D329, H192-D329, W193-D329, K194-D329, L195-D329, N196-D329, H197-D329, T198-D329, V199-D329, Y200-D329, S201-D329, F202-D329, V203-D329, A204-D329, V205-D329, Q206-D329, L207-D329, L208-D329, M209-D329, F210-D329, L211-D329, Q212-D329, F213-D329, G214-D329, G215-D329, Y216-D329, T217-D329, L218-D329, V219-D329, R220-D329, S221-D329, S222-D329, K223-D329, D224-D329, L225-D329, F226-D329, G227-D329, S228-D329, F229-D329, G230-D329, F231-D329, K232-D329, D233-D329, Q234-D329, P235-D329, V236-D329, I237-D329, I238-D329, G239-D329, L240-D329, I241-D329, I242-D329, F243-D329, P244-D329, H245-D329, T246-D329, I247-D329, I248-D329, P249-D329, I250-D329, Q251-D329, H252-D329, L253-D329, L254-D329, S255-D329, F256-D329, R257-D329, L258-D329, N259-D329, L260-D329, V261-D329, S262-D329, R263-D329, A264-D329, F265-D329, E266-D329, F267-D329, Q268-D329, A269-D329, D270-D329, A271-D329, F272-D329, A273-D329, K274-D329, N275-D329, L276-D329, G277-D329, Y278-D329, A279-D329, P280-D329, Q281-D329, L282-D329, R283-D329, A284-D329, A285-D329, L286-D329, V287-D329, K288-D329, L289-D329, Q290-D329, E291-D329, E292-D329, N293-D329, L294-D329, S295-D329, A296-D329, M297-D329, N298-D329, T299-D329, D300-D329, P301-D329, W302-D329, Y303-D329, S304-D329, A305-D329, Y306-D329, H307-D329, Y308-D329, S309-D329, H310-D329, P311-D329, P312-D329, L313-D329, V314-D329, E315-D329, R316-D329, L317-D329, Q318-D329, A319-D329, L320-D329, E321-D329, D322-D329, S323-D329, of SEQ ID NO:10. Polynucleotide sequences encoding these polypeptides are also provided.

In preferred embodiments, the following C-terminal deletion mutants are encompassed by the present invention: T1-D329, T1-E328, T1-K327, T1-K326, T1-D325, T1-D324, T1-S323, T1-D322, T1-E321, T1-L320, T1-A319, T1-Q318, T1-L317, T1-R316, T1-E315, T1-V314, T1-L313, T1-P312, T1-P311, T1-H310, T1-S309, T1-Y308, T1-H307, T1-Y306, T1-A305, T1-S304, T1-Y303, T1-W302, T1-P301, T1-D300, T1-T299, T1-N298, T1-M297, T1-A296, T1-S295, T1-L294, T1-N293, T1-E292, T1-E291, T1-Q290, T1-L289, T1-K288, T1-V287, T1-L286, T1-A285, T1-A284, T1-R283, T1-L282, T1-Q281, T1-P280, T1-A279, T1-Y278, T1-G277, T1-L276, T1-N275, T1-K274, T1-A273, T1-F272, T1-A271, T1-D270, T1-A269, T1-Q268, T1-F267, T1-E266, T1-F265, T1-A264, T1-R263, T1-S262, T1-V261, T1-L260, T1-N259, T1-L258, T1-R257, T1-F256, T1-S255, T1-L254, T1-L253, T1-H252, T1-Q251, T1-I250, T1-P249, T1-I248, T1-I247, T1-T246, T1-H245, T1-P244, T1-F243, T1-I242, T1-I241, T1-L240, T1-G239, T1-I238, T1-I237, T1-V236, T1-P235, T1-Q234, T1-D233, T1-K232, T1-F231, T1-G230, T1-F229, T1-S228, T1-G227, T1-F226, T1-L225, T1-D224, T1-K223, T1-S222, T1-S221, T1-R220, T1-V219, T1-L218, T1-T217, T1-Y216, T1-G215, T1-G214, T1-F213, T1-Q212, T1-L211, T1-F210, T1-M209, T1-L208, T1-L207, T1-Q206, T1-V205, T1-A204, T1-V203, T1-F202, T1-S201, T1-Y200, T1-V199, T1-T198, T1-H197, T1-N196, T1-L195, T1-K194, T1-W193, T1-H192, T1-G191, T1-L190, TI-E189, TI-H188, T1-A187, T1-I186, T1-V185, T1-S184, T1-V183, T1-I182, T1-E181, T1-D180, T1-E179, T1-N178, T1-S177, T1-C176, T1-Q175, T1-Q174, T1-I173, T1-L172, T1-T171, T1-D170, T1-Y169, T1-L168, T1-V167, T1-I166, T1-R165, T1-K164, T1-N163, T1-K162, T1-F161, T1-F160, T1-G159, T1-Y158, T1-M157, T1-Y156, T1-A155, T1-N154, T1-S153, T1-H152, T1-S151, T1-S150, T1-R149, T1-T148, T1-S147, T1-G146, T1-D145, T1-V144, T1-V143, T1-F142, T1-L141, T1-K140, T1-K139, T1-L138, T1-P137, T1-F136, T1-K135, T1-L134, T1-S133, T1-A132, T1-A131, T1-L130, T1-K129, T1-E128, T1-I127, T1-K126, T1-E125, T1-R124, T1-L123, T1-V122, T1-G121, T1-E120, T1-P119, T1-L118, T1-P117, T1-T116, T1-F115, T1-K114, T1-N113, T1-F112, T1-L111, T1-P110, T1-A109, T1-I108, T1-V107, T1-I106, T1-P105, T1-Y104, T1-I103, T1-T102, T1-M101, T1-M100, T1-L99, T1-L98, T1-A97, T1-L96, T1-V95, T1-F94, T1-M93, T1-F92, T1-G91, T1-W90, T1-L89, T1-Y88, T1-I87, T1-A86, T1-L85, T1-Y84, T1-P83, T1-G82, T1-G81, T1-I80, T1-Q79, T1-V78, T1-I77, T1-Y76, T1-I75, T1-I74, T1-A73, T1-A72, T1-V71, T1-I70, T1-P69, T1-P68, T1-G67, T1-L66, T1-I65, T1-M64, T1-563, T1-L62, T1-L61, T1-I60, T1-G59, T1-K58, T1-I57, T1-M56, T1-D55, T1-R54, T1-I53, T1-F52, T1-L51, T1-W50, T1-I49, T1-T48, T1-Q47, T1-K46, T1-N45, T1-F44, T1-G43, T1-H42, T1-R41, T1-A40, T1-E39, T1-I38, T1-V37, T1-F36, T1-T35, T1-S34, T1-Y33, T1-L32, T1-S31, T1-F30, T1-P29, T1-L28, T1-D27, T1-T26, T1-I25, T1-Q24, T1-S23, T1-W22, T1-V21, T1-M20, T1-S19, T1-G18, T1-A17, T1-L16, T1-F15, T1-A14, T1-L13, T1-T12, T1-H11, T1-I10, T1-I9, T1-E8, T1-N7, of SEQ ID NO:10. Polynucleotide sequences encoding these polypeptides are also provided.

Many polynucleotide sequences, such as EST sequences, are publicly available and accessible through sequence databases. Some of these sequences are related to SEQ ID NO: 9 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1301 of SEQ ID NO: 9, b is an integer between 15 to 1301, where both a and b correspond to the positions of nucleotide residues shown in SEQ ID NO:9, and where b is greater than or equal to a+14.

**Table I**

| Gene No. | CDNA Clone ID | ATCC Deposit No. Z and Data | Vector | NT SEQ ID. No. X | Total NT Seq of Clone | 5' NT of Start Codon of ORF | 3' NT of ORF | AA Seq ID No. Y | Total AA of ORF |
|---|---|---|---|---|---|---|---|---|---|
| 1. | PpPrPase1 | Xxxxxx | pCR2.1 | 1 | 1398 | 33 | 1214 | 2 | 394 |
| | | Xx/xx/xx | | | | | | | |
| 2. | AtPrPase1 | Xxxxxx | pCR2.1 | 3 | 1275 | 1 | 1272 | 4 | 424 |
| | | Xx/xx/xx | | | | | | | |
| 3. | AtPrPase2 | Xxxxxx | pCR2.1 | 5 | 1275 | 1 | 1272 | 6 | 424 |
| | | Xx/xx/xx | | | | | | | |
| 4. | GmPrPase1 | Xxxxxx | pCR2.1 | 7 | 1434 | 233 | 1432 | 8 | 400 |
| | | Xx/xx/xx | | | | | | | |
| 5. | ZmPrPase1 | Xxxxxx | pCR2.1 | 9 | 1301 | 1 | 987 | 10 | 329 |
| | | Xx/xx/xx | | | | | | | |

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually several overlapping sequences at each nucleotide position), resulting in a final sequence identified as SEQ ID NO:X.

The cDNA Clone ID was deposited on the date and given the corresponding deposit number listed in "ATCC Deposit No:Z and Date." "Vector" refers to the type of vector contained in the cDNA Clone ID. pCR2.1 was obtained from Invitrogen, Inc.

"Total NT Seq. Of Clone" refers to the total number of nucleotides in the clone contig identified by "Gene No." The deposited clone may contain all or most of the sequence of SEQ ID NO:X. The nucleotide position of SEQ ID NO:X of the putative start codon (methionine) is identified as "5' NT of Start Codon of ORF."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The total number of amino acids within the open reading frame of SEQ ID NO:Y is identified as "Total AA of ORF".

SEQ ID NO:X (where X may be any of the polynucleotide sequences disclosed in the sequence listing) and the translated SEQ ID NO:Y (where Y may be any of the polypeptide sequences disclosed in the sequence listing) are sufficiently accurate and otherwise suitable for a variety of uses well known in the art and described further below. For instance, SEQ ID NO:X is useful for designing nucleic acid hybridization probes that will detect nucleic acid equences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used, for example, to generate antibodies which bind specifically to proteins containing the polypeptides and the proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides may cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs, allelic variants, and/or orthologs. The skilled artisan could, using procedures well-known in the art, obtain the polynucleotide sequence corresponding to full-length genes (including, but not limited to the full-length coding region), allelic variants, splice variants, orthologs, and/or species homologs of genes corresponding to SEQ ID NO:X, SEQ ID NO:Y, or a deposited clone, relying on the sequence from the sequences disclosed herein or the clones deposited with the ATCC. For example, allelic variants and/or species homologs may be isolated and identified by making suitable probes or primers which correspond to the 5', 3', or internal regions of the sequences provided herein and screening a suitable nucleic acid source for allelic variants and/or the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the protein, or may be a part of a larger protein, such as a fusion protein (see below). It is often advantageous to include an additional amino acid sequence which contains secretory or leader sequences, pro-sequences, sequences which aid in purification, such as multiple histidine residues, or an additional sequence for stability during recombinant production.

The polypeptides of the present invention are preferably provided in an isolated form, and preferably are substantially purified. A recombinantly produced version of a polypeptide, can be substantially purified using techniques described herein or otherwise known in the art, such as, for example, by the one-step method described in Smith and Johnson, Gene 67:31-40 (1988). Polypeptides of the invention also can be purified from natural, synthetic or recombinant sources using protocols described herein or otherwise known in the art, such as, for example, antibodies of the invention raised against the full-length form of the protein.

The present invention provides a polynucleotide comprising, or alternatively consisting of, the sequence identified as SEQ ID NO:X, and/or a cDNA provided in ATCC Deposit No. Z:. The present invention also provides a polypeptide comprising, or alternatively consisting of, the sequence identified as SEQ ID NO:Y, and/or a polypeptide encoded by the cDNA provided in ATCC Deposit NO:Z. The present invention also provides polynucleotides encoding a polypeptide comprising, or alternatively consisting of the polypeptide sequence of SEQ ID NO:Y, and/or a polypeptide sequence encoded by the cDNA contained in ATCC Deposit No:Z.

Preferably, the present invention is directed to a polynucleotide comprising, or alternatively consisting of, the sequence identified as SEQ ID NO:X, and/or a cDNA provided in ATCC Deposit No.: that is less than, or equal to, a polynucleotide sequence that is 5 mega basepairs, 1 mega basepairs, 0.5 mega basepairs, 0.1 mega basepairs, 50,000 basepairs, 20,000 basepairs, or 10,000 basepairs in length.

The present invention encompasses polynucleotides with sequences complementary to those of the polynucleotides of the present invention disclosed herein. Such sequences may be complementary to the sequence disclosed as SEQ ID NO:X, the sequence contained in a deposit, and/or the nucleic acid sequence encoding the sequence disclosed as SEQ ID NO:Y.

The present invention also encompasses polynucleotides capable of hybridizing, preferably under reduced stringency conditions, more preferably under stringent conditions, and most preferably under highly stingent conditions, to polynucleotides described herein. Examples of stringency conditions are shown in Table 2 below: highly stringent conditions are those that are at least as stringent as, for example, conditions A-F; stringent conditions are at least as stringent as, for example, conditions G-L; and reduced stringency conditions are at least as stringent as, for example, conditions M-R.

**TABLE 2**

| **Stringency Condition** | **Polynucleotide Hybrid±** | **Hybrid Length (bp)‡** | **Hyridization Temperature and Buffer†** | **Wash Temperature and Buffer †** |
|---|---|---|---|---|
| **A** | DNA:DNA | > or equal to 50 | 65°C; 1xSSC -or- | 65°C; |
| | | | 42°C; 1xSSC, 50% formamide | 0.3xSSC |
| **B** | DNA:DNA | < 50 | Tb*; 1xSSC | Tb*; 1xSSC |
| **C** | DNA:RNA | > or equal to 50 | 67°C; 1xSSC | 67°C; |
| | | | -or- 45°C; 1xSSC, 50% formamide | 0.3xSSC |
| **D** | DNA:RNA | < 50 | Td*; 1xSSC | Td*; |
| | | | | 1xSSC |
| **E** | RNA:RNA | > or equal to 50 | 70°C; 1xSSC | 70°C; |
| | | | -or- 50°C; 1xSSC, 50% formamide | 0.3xSSC |
| **F** | RNA:RNA | < 50 | Tf*; 1xSSC | Tf*; 1xSSC |
| **G** | DNA:DNA | > or equal to 50 | 65°C; 4xSSC -or- 45°C; 4xSSC, 50% formamide | 65°C; 1xSSC |
| | | | | |
| **H** | DNA:DNA | < 50 | Th*; 4xSSC | Th*; 4xSSC |
| **I** | DNA:RNA | > or equal to 50 | 67°C; 4xSSC | 67°C; 1xSSC |
| | | | -or- 45°C; 4xSSC, 50% formamide | |
| **J** | DNA:RNA | < 50 | Tj*; 4xSSC | Tj*; 4xSSC |
| **K** | RNA:RNA | > or equal to 50 | 70°C; 4xSSC | 67°C; 1xSSC |
| | | | -or- 40°C; 6xSSC, 50% formamide | |
| **L** | RNA:RNA | < 50 | Tl*; 2xSSC | Tl*; 2xSSC |
| **M** | DNA:DNA | > or equal to 50 | 50°C; 4xSSC | 50°C; 2xSSC |
| | | | -or- 40°C 6xSSC, 50% formamide | |
| **N** | DNA:DNA | < 50 | Tn*; 6xSSC | Tn*; 6xSSC |
| **O** | DNA:RNA | > or equal to 50 | 55°C; 4xSSC | 55°C; 2xSSC |
| | | | -or- 42°C; 6xSSC, 50% formamide | |
| **P** | DNA:RNA | < 50 | Tp*; 6xSSC | Tp*; 6xSSC |
| **Q** | RNA:RNA | > or equal to 50 | 60°C; 4xSSC | 60°C; 2xSSC |
| | | | -or- 45°C; 6xSSC, 50% formamide | |
| **R** | RNA:RNA | < 50 | Tr*; 4xSSC | Tr*; 4xSSC |

| | | | | |
|---|---|---|---|---|
| ‡: The "hybrid length" is the anticipated length for the hybridized region(s) of the hybridizing polynucleotides. When hybridizing a polynucletotide of unknown sequence, the hybrid is assumed to be that of the hybridizing polynucleotide of the present invention. When polynucleotides of known sequence are hybridized, the hybrid length can be determined by aligning the sequences of the polynucleotides and identifying the region or regions of optimal sequence complementarity. Methods of aligning two or more polynucleotide sequences and/or determining the percent identity between two polynucleotide sequences are well known in the art (e.g., MegAlign program of the DNA*Star suite of programs, etc). † : SSPE (1xSSPE is 0.15M NaCl, 10mM NaH2PO4, and 1.25mM EDTA, pH 7.4) can be substituted for SSC (1xSSC is 0.15M NaCl anmd 15mM sodium citrate) in the hybridization and wash buffers; washes are performed for 15 minutes after hybridization is complete. The hydridizations and washes may additionally include 5X Denhardt's reagent, .5-1.0% SDS, 100ug/ml denatured, fragmented salmon sperm DNA, 0.5% sodium pyrophosphate, and up to 50% formamide. *Tb - Tr: The hybridization temperature for hybrids anticipated to be less than 50 base pairs in length should be 5-10°C less than the melting temperature Tm of the hybrids there Tm is determined according to the following equations. For hybrids less than 18 base pairs in length, Tm(°C) = 2(# of A + T bases) + 4(# of G + C bases). For hybrids between 18 and 49 base pairs in length, Tm(°C) = 81.5 +16.6(log₁₀[Na+]) + 0.41(%G+C) - (600/N), where N is the number of bases in the hybrid, and [Na+] is the concentration of sodium ions in the hybridization buffer ([NA+] for 1xSSC = .165 M). ±: The present invention encompasses the substitution of any one, or more DNA or RNA hybrid partners with either a PNA, or a modified polynucleotide. Such modified polynucleotides are known in the art and are more particularly described elsewhere herein. | | | | |

Additional examples of stringency conditions for polynucleotide hybridization are provided, for example, in Sambrook, J., E.F. Fritsch, and T.Maniatis, 1989, Molecular Cloning: A Laboratory Manual, Cold Spring Harbor Laboratory Press, Cold Spring Harbor, NY, chapters 9 and 11, and Current Protocols in Molecular Biology, 1995, F.M., Ausubel et al., eds, John Wiley and Sons, Inc., sections 2.10 and 6.3-6.4, which are hereby incorporated by reference herein.

Preferably, such hybridizating polynucleotides have at least 70% sequence identity (more preferably, at least 80% identity; and most preferably at least 90% or 95% identity) with the polynucleotide of the present invention to which they hybridize, where sequence identity is determined by comparing the sequences of the hybridizing polynucleotides when aligned so as to maximize overlap and identity while minimizing sequence gaps. The determination of identity is well known in the art, and discussed more specifically elsewere herein.

The invention encompasses the application of PCR methodology to the polynucleotide sequences of the present invention, the clone deposited with the ATCC, and/or the cDNA encoding the polypeptides of the present invention. PCR techniques for the amplification of nucleic acids are described in US Patent No. 4, 683, 195 and Saiki et al., Science, 239:487-491 (1988). PCR, for example, may include the following steps, of denaturation of template nucleic acid (if double-stranded), annealing of primer to target, and polymerization. The nucleic acid probed or used as a template in the amplification reaction may be genomic DNA, cDNA, RNA, or a PNA. PCR may be used to amplify specific sequences from genomic DNA, specific RNA sequence, and/or cDNA transcribed from mRNA. References for the general use of PCR techniques, including specific method parameters, include Mullis et al., Cold Spring Harbor Symp. Quant. Biol., 51:263, (1987), Ehrlich (ed), PCR Technology, Stockton Press, NY, 1989; Ehrlich et al., Science, 252:1643-1650, (1991); and "PCR Protocols, A Guide to Methods and Applications", Eds., Innis et al., Academic Press, New York, (1990).

### Signal Sequences

The present invention also encompasses mature forms of the polypeptide comprising, or alternatively consisting of, the polypeptide sequence of SEQ ID NO:Y, the polypeptide encoded by the polynucleotide described as SEQ ID NO:X, and/or the polypeptide sequence encoded by a cDNA in the deposited clone. The present invention also encompasses polynucleotides encoding mature forms of the present invention, such as, for example the polynucleotide sequence of SEQ ID NO:X, and/or the polynucleotide sequence provided in a cDNA of the deposited clone.

According to the signal hypothesis, proteins secreted by eukaryotic cells have a signal or secretary leader sequence which is cleaved from the mature protein once export of the growing protein chain across the rough endoplasmic reticulum has been initiated. Most eukaryotic cells cleave secreted proteins with the same specificity. However, in some cases, cleavage of a secreted protein is not entirely uniform, which results in two or more mature species of the protein. Further, it has long been known that cleavage specificity of a secreted protein is ultimately determined by the primary structure of the complete protein, that is, it is inherent in the amino acid sequence of the polypeptide.

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

The established method for identifying the location of signal sequences, in addition, to their cleavage sites has been the SignalP program (v1.1) developed by Henrik Nielsen et al., Protein Engineering 10:1-6 (1997). The program relies upon the algorithm developed by von Heinje, though provides additional parameters to increase the prediction accuracy.

More recently, a hidden Markov model has been developed (H. Neilson, et al., Ismb 1998;6:122-30), which has been incorporated into the more recent SignalP (v2.0). This new method increases the ability to identify the cleavage site by discriminating between signal peptides and uncleaved signal anchors. The present invention encompasses the application of the method disclosed therein to the prediction of the signal peptide location, including the cleavage site, to any of the polypeptide sequences of the present invention.

As one of ordinary skill would appreciate, however, cleavage sites sometimes vary from organism to organism and cannot be predicted with absolute certainty. Accordingly, the polypeptide of the present invention may contain a signal sequence. Polypeptides of the invention which comprise a signal sequence have an N-terminus beginning within 5 residues (i.e., + or - 5 residues, or preferrably at the -5, -4, -3, -2, -1, +1, +2, +3, +4, or +5 residue) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. Nonetheless, the present invention provides the mature protein produced by expression of the polynucleotide sequence of SEQ ID NO:X and/or the polynucleotide sequence contained in the cDNA of a deposited clone, in a mammalian cell (e.g., COS cells, as desribed below). These polypeptides, and the polynucleotides encoding such polypeptides, are contemplated by the present invention.

### Polynucleotide and Polypeptide Variants

The present invention also encompases variants (e.g., allelic variants, orthologs, etc.) of the polynucleotide sequence disclosed herein in SEQ ID NO:X, the complementary strand thereto, and/or the cDNA sequence contained in the deposited clone.

The present invention also encompasses variants of the polypeptide sequence, and/or fragments therein, disclosed in SEQ ID NO:Y and/or encoded by a deposited clone.

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

The present invention is also directed to polynucleotide sequences which comprise, or alternatively consist of, a polynucleotide sequence which is at least 70%, 80%, 85%, 90%, 91%, 92%, 93%, 94%, 95%, 96%, 97%, 98%, or 99% identical to, the following non-limiting examples, the polynucleotide sequence of the coding region of the sequence in SEQ ID NO:X, the complementary strand of said coding region, the polynucleotide sequence provided in a cDNA of the deposited clone, the complementary strand of said deposited cDNA, a polynucleotide sequence encoding the polypeptide identified as SEQ ID NO:Y, a polynucleotide sequence encoding the polypeptide of a cDNA provided in the deposited clone. The invention also encompasses polynucleotide fragments of any of the polynucleotide sequences provided herein.

The present invention also encompasses polynucleotide sequences which hybridize to any of the provided polynucleotide sequences of the present invention, including the aforementioned polynucleotide fragments, under stringent or lower stringent hydrization conditions. Polypeptides encoded by the polynucleotide sequences which hybridize to the polynucleotide sequences of the present invention are also encompassed by the present invention.

The present invention encompasses polypeptide sequences which comprise, or alternatively consist of, an amino acid sequence which is at least 70%, 80%, 98%, 90%, 91%, 92%, 93%, 94%, 95%, 96%, 97%, 98%, or 99% identical to, the following non-limited examples, the polypeptide sequence identified as SEQ ID NO:Y, the polypeptide sequence encoded by a cDNA provided in the deposited clone, and/or polypeptide fragments of any of the polypeptides provided herein.

By a nucleic acid having a nucleotide sequence at least, for example, 95% "identical" to a reference nucleotide sequence of the present invention, it is intended that the nucleotide sequence of the nucleic acid is identical to the reference sequence except that the nucleotide sequence may include up to five point mutations per each 100 nucleotides of the reference nucleotide sequence encoding the polypeptide. In other words, to obtain a nucleic acid having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence referenced in Table 1, the ORF (open reading frame), or any fragment specified as described herein.

As a practical matter, whether any particular nucleic acid molecule or polypeptide is at least 70%, 80%, 85%, 90%, 91% 92%, 93%, 94%, 95%, 96%, 97%, 98%, or 99% identical to a nucleotide sequence of the present invention can be determined conventionally using known computer programs. A preferred method for determining the best overall match between a query sequence (a sequence of the present invention) and a subject sequence, also referred to as a global sequence alignment, can be determined using the CLUSTALW computer program (Thompson, J.D., et al.,Nucleic Acids Research, 2(22):4673-4680, (1994)), which is based on the algorithm of Higgins, D.G., et al., Computer Applications in the Biosciences (CABIOS), 8(2):189-191, (1992). In a sequence alignment the query and subject sequences are both DNA sequences. An RNA sequence can be compared by converting U's to T's. The result of said global sequence alignment is in percent identity. Preferred parameters used in a CLUSTALW alignment of DNA sequences to calculate percent identity are: Matrix=BLOSUM, k-tuple=1, Number of Top Diagonals=5, Gap Penalty=3, Gap Open Penalty 10, Gap Extension Penalty=0, Scoring Method=Percent, Window Size=5 or the length of the subject nucleotide sequence, whichever is shorter.

If the subject sequence is shorter than the query sequence because of 5' or 3' deletions, not because of internal deletions, a manual correction must be made to the results. This is because the CLUSTALW program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the CLUSTALW sequence alignment. This percentage is then subtracted from the percent identity, calculated by the above CLUSTALW program using the specified parameters, to arrive at a final percent identity score. This corrected score is what may be used for the purposes of the present invention. Only bases outside the 5' and 3' bases of the subject sequence, as displayed by the CLUSTALW alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the CLUSTALW alignment does not show a matched/alignment of the first 10 bases at 5' end. The 10 unpaired bases represent 10% of the sequence (number of bases at the 5' and 3' ends not matched/total number of bases in the query sequence) so 10% is subtracted from the percent identity score calculated by the CLUSTALW program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by CLUSTALW is not manually corrected. Once again, only bases 5' and 3' of the subject sequence which are not matched/aligned with the query sequence are manually corrected for. No other manual corrections are required for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 70%, 80%, 85%, 91%, 92%, 93%, 94%, 95%, 96%, 97%, 98%, or 99% identical to, for instance, an amino acid sequence referenced in Table 1 (SEQ ID NO:Y) or to the amino acid sequence encoded by cDNA contained in a deposited clone, can be determined conventionally using known computer programs. A preferred method for determining the best overall match between a query sequence (a sequence of the present invention) and a subject sequence, also referred to as a global sequence alignment, can be determined using the CLUSTALW computer program (Thompson, J.D., et al.,Nucleic Acids Research, 2(22):4673-4680, (1994)), which is based on the algorithm of Higgins, D.G., et al., Computer Applications in the Biosciences (CABIOS), 8(2):189-191, (1992). In a sequence alignment the query and subject sequences are both DNA sequences. An RNA sequence can be compared by converting U's to T's. The result of said global sequence alignment is in percent identity. Preferred parameters used in a CLUSTALW alignment of DNA sequences to calculate percent identity are: Matrix=BLOSUM, k-tuple=1, Number of Top Diagonals=5, Gap Penalty=3, Gap Open Penalty 10, Gap Extension Penalty=0, Scoring Method=Percent, Window Size=5 or the length of the subject nucleotide sequence, whichever is shorter.

If the subject sequence is shorter than the query sequence due to N- or C-terminal deletions, not because of internal deletions, a manual correction must be made to the results. This is because the CLUSTALW program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the CLUSTALW sequence alignment. This percentage is then subtracted from the percent identity, calculated by the above CLUSTALW program using the specified parameters, to arrive at a final percent identity score. This final percent identity score is what may be used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the CLUSTALW alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the CLUSTALW program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence, which are not matched/aligned with the query. In this case the percent identity calculated by CLUSTALW is not manually corrected. Once again, only residue positions outside the N- and C-terminal ends of the subject sequence, as displayed in the CLUSTALW alignment, which are not matched/aligned with the query sequnce are manually corrected for. No other manual corrections are required for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level and are included in the present invention. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein (Dobeli et al., J. Biotechnology 7:199-216 (1988)).

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the protein will likely be retained when less than the majority of the residues of the protein are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include, but are not limited to, the following: (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as, for example, an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

Moreover, the invention further includes polypeptide variants created through the application of molecular evolution ("DNA Shuffling") methodology to the polynucleotide disclosed as SEQ ID NO:X, the sequence of the clone submitted in a deposit, and/or the cDNA encoding the polypeptide disclosed as SEQ ID NO:Y. Such DNA Shuffling technology is known in the art and more particularly described elsewere herein (e.g., WPC, Stemmer, PNAS, 91:10747, (1994)), and example 20).

A further embodiment of the invention relates to a polypeptide which comprises the amino acid sequence of the present invention having an amino acid sequence which contains at least one amino acid substitution, but not more than 50 amino acid substitutions, even more preferably, not more than 40 amino acid substitutions, still more preferably, not more than 30 amino acid substitutions, and still even more preferably, not more than 20 amino acid substitutions. Of course, in order of ever-increasing preference, it is highly preferable for a peptide or polypeptide to have an amino acid sequence which comprises the amino acid sequence of the present invention, which contains at least one, but not more than 10, 9, 8, 7, 6, 5, 4, 3, 2 or 1 amino acid substitutions. In specific embodiments, the number of additions, substitutions, and/or deletions in the amino acid sequence of the present invention or fragments thereof (e.g., the mature form and/or other fragments described herein), is 1-5, 5-10, 5-25, 5-50, 10-50 or 50-150, conservative amino acid substitutions are preferable.

### Polynucleotide and Polypeptide Fragments

The present invention is directed to polynucleotide fragments of the polynucleotides of the invention, in addition to polypeptides encoded therein by said polynucleotides and/or fragments.

In the present invention, a "polynucleotide fragment" refers to a short polynucleotide having a nucleic acid sequence which: is a portion of that contained in a deposited clone, or encoding the polypeptide encoded by the cDNA in a deposited clone; is a portion of that shown in SEQ ID NO:X or the complementary strand thereto, or is a portion of a polynucleotide sequence encoding the polypeptide of SEQ ID NO:Y. The nucleotide fragments of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A fragment "at least 20 nt in length," for example, is intended to include 20 or more contiguous bases from the cDNA sequence contained in a deposited clone or the nucleotide sequence shown in SEQ ID NO:X. In this context "about" includes the particularly recited value, a value larger or smaller by several (5, 4, 3, 2, or 1) nucleotides, at either terminus, or at both termini. These nucleotide fragments have uses that include, but are not limited to, as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments comprising, or alternatively consisting of, a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X, or the complementary strand thereto, or the cDNA contained in a deposited clone. In this context "about" includes the particularly recited ranges, and ranges larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein. Also encompassed by the present invention are polynucleotides which hybridize to these nucleic acid molecules under stringent hybridization conditions or lower stringency conditions, as are the polypeptides encoded by these polynucleotides.

In the present invention, a "polypeptide fragment" refers to an amino acid sequence which is a portion of that contained in SEQ ID NO:Y or encoded by the cDNA contained in a deposited clone. Protein (polypeptide) fragments may be "free-standing," or comprised within a larger polypeptide of which the fragment forms a part or region, most preferably as a single continuous region. Representative examples of polypeptide fragments of the invention, include, for example, fragments comprising, or alternatively consisting of, from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges or values, and ranges or values larger or smaller by several (5, 4, 3, 2, or 1) amino acids, at either extreme or at both extremes. Polynucleotides encoding these polypeptides are also encompassed by the invention.

Preferred polypeptide fragments include the full-length protein. Further preferred polypeptide fragments include the full-length protein having a continuous series of deleted residues from the amino or the carboxy terminus, or both. For example, any number of amino acids, ranging from 1-60, can be deleted from the amino terminus of the full-length polypeptide. Similarly, any number of amino acids, ranging from 1-30, can be deleted from the carboxy terminus of the full-length protein. Furthermore, any combination of the above amino and carboxy terminus deletions are preferred. Similarly, polynucleotides encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotides encoding these domains are also contemplated.

Other preferred polypeptide fragments are biologically active fragments. Biologically active fragments are those exhibiting activity similar, but not necessarily identical, to an activity of the polypeptide of the present invention. The biological activity of the fragments may include an improved desired activity, or a decreased undesirable activity. Polynucleotides encoding these polypeptide fragments are also encompassed by the invention.

In a preferred embodiment, the functional activity displayed by a polypeptide encoded by a polynucleotide fragment of the invention may be one or more biological activities typically associated with the full-length polypeptide of the invention. Illustrative of these biological activities includes the fragments ability to bind to at least one of the same antibodies which bind to the full-length protein, the fragments ability to interact with at lease one of the same proteins which bind to the full-length, the fragments ability to elicit at least one of the same immune responses as the full-length protein (i.e., to cause the immune system to create antibodies specific to the same epitope, etc.), the fragments ability to bind to at least one of the same polynucleotides as the full-length protein, the fragments ability to bind to a receptor of the full-length protein, the fragments ability to bind to a ligand of the full-length protein, and the fragments ability to multimerize with the full-length protein. However, the skilled artisan would appreciate that some fragments may have biological activities which are desirable and directly inapposite to the biological activity of the full-length protein. The functional activity of polypeptides of the invention, including fragments, variants, derivatives, and analogs thereof can be determined by numerous methods available to the skilled artisan, some of which are described elsewhere herein.

### Epitopes and Antibodies

In the present invention, "epitopes" refer to polypeptide fragments having antigenic and/or immunogenic activity in an organism, preferrably an animal or a plant. A preferred embodiment of the present invention relates to a polypeptide fragment comprising an epitope, as well as the polynucleotide encoding this fragment. A region of a protein molecule to which an antibody can bind is defined as an "antigenic epitope". In contrast, an "immunogenic epitope" is defined as a part of a protein that elicits an antibody reponse (i.e., in animals), or a defensive response (i.e., in plants). (See, for instance, Geyson et al., Proc. Natl. Aced. Sci. USA 81:3998-4002 (1983)).

Epitope fragments may be produced by any conventional means known in the art. Epitopes that are immunogenic may be useful for inducing antibodies according to methods well known in the art. Such epitopes may be presented together with a carrier protein such as an albumin, or may be presented without a carrier if the epitope is of sufficient length (greater than or equal to 25 amino acids). Nonetheless, epitopes have been found to elicit an immune response that comprise as few as 8 to 10 amino acids. Epitopes of the present invention may be linear epitopes derived from a denatured polypeptide (e.g., as found in Western blots).

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J.G., et al., Science 219:660-666 (1983)).

As used herein, the term "antibody" (Ab) or "monoclonal antibody" (Mab) is meant to include intact molecules, as well as, antibody fragments (such as, for example, Fab and F(ab')2 fragments) which are capable of specifically binding to protein. Fab and F(ab')2 fragments lack the Fc fragment of intact antibody, clear more rapidly from the circulation of the animal or plant, and may have less non-specific tissue binding than an intact antibody (Wahl et al., J. Nucl. Med 24:316-325 (1983)). Thus, these fragments are preferred, as well as the products of a FAB or other immunoglobulin expression library. Moreover, antibodies of the present invention include chimeric, single chain, and humanized antibodies.

The present invention is further directed to antibodies specific to polypeptides, polypeptide fragments, and polypeptide variants of the present invention. The antibodies of the present invention may include, but are not limited to, polyclonal, monoclonal, humanized, fully-human, monospecific, bispecific, trispecific, heteroconjugate, chimeric, single chain, variable light chain, variable heavy chain, one or more complementarity-determining regions (CDRs), phage-display derived antibodies (those derived from a Fab expression library, etc.), anti-idiotype antibodies, and those antibodies having enzymatic activity (i.e., catalytic antibodies). The antibodies of the present invention may be comprised of any of the currently known antibody isotypes (e.g., IgD, IgM, IgE, IgG, IgY, and IgA, etc.), the human IgA and IgG subclasses (e.g., IgG1, IgG2, IgG3, IgG4, IgA1, or IgA2), or the mouse IgA and IgG subclasses (e.g., IgG1, IgG2a, IgG2b, IgG3, IgA1, or IgA2.

The antibodies of the present invention may comprise polyclonal antibodies. Methods of preparing polyclonal antibodies are known to the skilled artisan (Harlow, et al., Antibodies: A Laboratory Manual, (Cold spring Harbor Laboratory Press, 2nd ed. (1988), which is hereby incorporated herein by reference in its entirety). Polyclonal antibodies can be raised in a mammal or bird, for example, by one or more injections of an immunizing agent and, if desired, an adjuvant. For the purposes of the invention, "immunizing agent" may be defined as a polypeptide of the invention, including fragments, variants, and/or derivatives thereof, in addition to fusions with heterologous polypeptides and other forms of the polypeptides described herein.

Typically, the immunizing agent and/or adjuvant will be injected in the mammal by multiple subcutaneous or intraperitoneal injections, though they may also be given intramuscularly, and/or through IV). The immunizing agent may include polypeptides of the present invention or a fusion protein or variants thereof. Depending upon the nature of the polypeptides (i.e., percent hydrophobicity, percent hydrophilicity, stability, net charge, isoelectric point etc.), it may be useful to conjugate the immunizing agent to a protein known to be immunogenic in the mammal being immunized. Such conjugation includes either chemical conjugation by derivitizing active chemical functional groups to both the polypeptide of the present invention and the immunogenic protein such that a convalent bond is formed, or through fusion-protein based methodology, or other methods known to the skilled artisan. Examples of such immunogenic proteins include, but are not limited to keyhole limpet hemocyanin, serum albumin, bovine thyroglobulin, and soybean trypsin inhibitor. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum. Additional examples of adjuvants which may be employed includes the MPL-TDM adjuvant (monophosphoryl lipid A, synthetic trehalose dicorynomycolate). The immunization protocol may be selected by one skilled in the art without undue experimentation.

The antibodies of the present invention may comprise monoclonal antibodies. Monoclonal antibodies may be prepared using hybridoma methods, such as those described by Kohler and Milstein, Nature, 256:495 (1975) and U.S. Pat. No. 4,376,110, by Harlow, et al., Antibodies: A Laboratory Manual, (Cold spring Harbor Laboratory Press, 2nd ed. (1988), by Hammerling, et al., Monoclonal Antibodies and T-Cell Hybridomas (Elsevier, N.Y., (1981)), or other methods known to the artisan. Other examples of methods which may be employed for producing monoclonal antibodies includes, but are not limited to, the human B-cell hybridoma technique (Kosbor et al., 1983, Immunology Today 4:72; Cole et al., 1983, Proc. Natl. Acad. Sci. USA 80:2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or in vivo. Production of high titers of mAbs in vivo makes this the presently preferred method of production.

In a hybridoma method, a mouse, a humanized mouse, a mouse with a human immune system, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro.

The immunizing agent will typically include polypeptides of the present invention or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybrizoma cell (Goding, Monoclonal Antibodies: Principles and Practice, Academic Press, (1986), pp. 59-103). Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hyopxanthine guanine phosphoribosyl transferase (HGPRT or HPRT), the culture medioum for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. As inferred throughout the specification, human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, Marcel Dekker, Inc., New York, (1987) pp. 51-63).

The culture medium in which the hybridoma cells are cultured can then be assayed for the presence of monoclonal antibodies directed against the polypeptides of the present invention. Preferably, the binding specificity of monolconal antibodies produced by the hybridoma cells is determined by immunoprecipitation or by an in vitro binding assay, such as radioimmunoassay (RIA) or enzyme-linked immunoabsorbant assay (ELISA). Such techniques are known in the art and within the skill of the artisan. The binding affinity of the monoclonal antibody can, for example, be determined by the Scatchard analysis of Munson and Pollart, Anal. Biochem., 107:220 (1980).

After the desired hybridoma cells are identified, the clones may be subcloned by limiting dilution procedures and grown by standard methods (Goding, supra). Suitable culture media for this purpose include, for example, Dulbecco's Modified Eagle's Medium and RPMI-1640. Alternatively, the hybridoma cells may be grown in vivo as ascites in a mammal.

The monoclonal antibodies secreted by the subclones may be isolated or purified from the culture medium or ascites fluid by conventional immunoglobulin purification procesures such as, for example, protein A-sepharose, hydroxyapatite chromotography, gel exclusion chromotography, gel electrophoresis, dialysis, or affinity chromotography.

The skilled artisan would acknowledge that a variety of methods exist in the art for the production of monoclonal antibodies and thus, the invention is not limited to their sole production in hydridomas. For example, the monoclonal antibodies may be made by recombinant DNA methods, such as those described in US patent No. 4, 816, 567. In this context, the term "monoclonal antibody" refers to an antibody derived from a single eukaryotic, phage, or prokaryotic clone. The DNA encoding the monoclonal antibodies of the invention can be readily isolated and sequenced using conventional procedures (e.g., by using oligonucleotide probes that are capable of binding specifically to genes encoding the heavy and light chains of murine antibodies, or such chains from human, humanized, or other sources). The hydridoma cells of the invention serve as a preferred source of such DNA. Once isolated, the DNA may be placed into expression vectors, which are then transformed into host cells such as Simian COS cells, Chinese hamster ovary (CHO) cells, or myeloma cells that do not otherwise produce immunoglubulin protein, to obtain the synthesis of monoclonal antibodies in the recombinant host cells. The DNA also may be modified, for example, by substituting the coding sequence for human heavy and light chain constant domains in place of the homologous murine sequences (US Patent No. 4, 816, 567; Morrison et al, supra) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to creat a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

In vitro methods are also suitable for preparing monovalent antibodies. Digestion of antibodies to produce fragments thereof, particularly, Fab fragments, can be accomplished using routine techniques known in the art.

The antibodies of the present invention may further comprise humanized antibodies or fully-human (human) antibodies. Humanized forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat, or rabbit having the desired specificity, affinity and capacity. In some instances, Fv framework residues of the human immunoglubulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin (Jones et al., Nature, 321:522-525 (1986); Riechmann et al., Nature 332:323-329 (1988)1 and Presta, Curr. Op. Struct. Biol., 2:593-596 (1992).

Methods for humanizing non-human antibodies are well known in the art. Generally, a humanized antibody has one or more amino acid residues introduced into it from a source that is non-human. These non-human amino acid residues are often referred to as "import" residues, which are typically taken from an "import" variable domain. Humanization can be essentially performed following the methods of Winter and co-workers (Jones et al., Nature, 321:522-525 (1986); Reichmann et al., Nature, 332:323-327 (1988); Verhoeyen et al., Science, 239:1534-1536 (1988), by substituting rodent CDRs or CDR sequences for the corresponding sequences of a human antibody. Accordingly, such "humanized" antibodies are chimeric antibodies (US Patent No. 4, 816, 567), wherein substantially less than an intact human variable domain has been substituted by the corresponding sequence from a non-human species. In practice, humanized antibodies are typically human antibodies in which some CDR residues and possible some FR residues are substituted from analagous sites in rodent antibodies.

Human antibodies can also be produced using various techniques known in the art, including phage display libraries (Hoogenboom and Winter, J. Mol. Biol., 227:381 (1991); Marks et al., J. Mol. Biol., 222:581 (1991)). The techniques of cole et al., and Boerder et al., are also available for the preparation of human monoclonal antibodies (cole et al., Monoclonal Antibodies and Cancer Therapy, Alan R. Riss, (1985); and Boerner et al., J. Immunol., 147(1):86-95, (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and creation of an antibody repertoire. This approach is described, for example, in US patent Nos. 5,545,807; 5,545,806; 5,569,825; 5,625,126; 5,633,425; 5,661,106, and in the following scientific publications: Marks et al., Biotechnol., 10:779-783 (1992); Lonberg et al., Nature 368:856-859 (1994); Fishwild et al., Nature Biotechnol., 14:845-51 (1996); Neuberger, Nature Biotechnol., 14:826 (1996); Lonberg and Huszer, Intern. Rev. Immunol., 13:65-93 (1995).

In addition, techniques developed for the production of "chimeric antibodies" (Morrison et al., 1984, Proc. Natl. Acad. Sci., 81:6851-6855; Neuberger et al., 1984, Nature, 312:604-608; Takeda et al., 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. (See, e.g., Cabilly et al., U.S. Pat. No. 4,816,567; and Boss et al., U.S. Pat. No. 4,816,397, both of which are incorporated herein by reference in their entirety.)

The antibodies of the present invention may be bispecific antibodies. Bispecific antibodies are monoclonal, preferrably human or humanized, antibodies that have binding specificities for at least two different antigens. In the present invention, one of the binding specificities may be directed towards a polypeptide of the present invention, the other may be for any other antigen, and preferably for a cell-surface protein, receptor, receptor subunit, tissue-specific antigen, virally derived protein, virally encoded envelope protein, bacterially derived protein, or bacterial surface protein, etc.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromotography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglubulin heavy-chain fusions and, if desired, the immunoglobulin light chain, are inserted into separate expression vectors, and are co-transformed into a suitable host organism. For further details of generating bispecific antibodies see, for example Suresh et al., Meth. In Enzym, 121:210 (1986).

Heteroconjugate antibodies are also contemplated by the present invention. Heteroconjugate antibodies are composed of two covalently joined antibodies. Such antibodies have, for example, been proposed to target immune system cells to unwanted cells (US Patent No. 4, 676, 980), and for the treatment of HIV infection (WO 91/00360; WO 92/20373; and EP03089). It is contemplated that the antibodies may be prepared in vitro using known methods in synthetic protein chemistry, including those involving crosslinking agents. For example, immunotoxins may be constructed using a disulfide exchange reaction or by forming a thioester bond. Examples of suitable reagents for this purpose include iminothiolate and methyl-4-mercaptobutyrimidate and those disclosed, for example, in US Patent No. 4,676,980.

### Uses for Antibodies directed against polypeptides of the invention

The antibodies of the present invention have various utilities. For example, such antibodies may be used in diagnostic assays to detect the presence or quantification of the polypeptides of the invention in a sample. Such a diagnostic assay may be comprised of at least two steps. The first, subjecting a sample with the antibody, wherein the sample is a tissue (e.g., animal, plant, etc.), biological fluid (e.g., blood, urine, phloem fluid, xylem fluid, plant secretion, etc.), biological extract (e.g., tissue or cellular homogenate, etc.), a protein microchip (e.g., See Arenkov P, et al., Anal Biochem., 278(2):123-131 (2000)), or a chromatography column, etc. And a second step involving the quantification of antibody bound to the substrate. Alternatively, the method may additionally involve a first step of attaching the antibody, either covalently, electrostatically, or reversably, to a solid support, and a second step of subjecting the bound antibody to the sample, as defined above and elsewhere herein.

Various diagnostic assay techniques are known in the art, such as competitive binding assays, direct or indirect sandwich assays and immunoprecipitation assays conducted in either heterogenous or homogenous phases (Zola, Monoclonal Antibodies: A Manual of Techniques, CRC Press, Inc., (1987), pp147-158). The antibodies used in the diagnostic assays can be labeled with a detectable moiety. The detectable moiety should be capable of producing, either directly or indirectly, a detectable signal. For example, the detectable moiety may be a radioisotope, such as 2H, 14C, 32P, or 125I, a florescent or chemiluminescent compound, such as fluorescein isothiocyanate, rhodamine, or luciferin, or an enzyme, such as alkaline phoshatase, beta-galactosidase, green flourescent protein, or horseradish peroxidase. Any method known in the art for conjugating the antibody to the detectable moiety may be employed, including those methods described by Hunter et al., Nature, 144:945 (1962); Dafvid et al., Biochem., 13:1014 (1974); Pain et al., J. Immunol. Metho., 40:219(1981); and Nygren, J. Histochem. And Cytochem., 30:407 (1982).

Antibodies directed against the polypeptides of the present invention are useful for the affinity purification of such polypeptides from recombinant cell culture or natural sources. In this process, the antibodies against a particular polypeptide are immobilized on a suitable support, such as a Sephadex resin or filter paper, using methods well known in the art. The immobilized antibody then is contacted with a sample containing the polypeptides to be purified, and thereafter the support is washed with a suitable solvent that will remove substantially all the material in the sample except for the desired polypeptides, which are bound to the immobilized antibody. Finally, the support is washed with another suitable solvent that will release the desired polypeptide from the antibody.

Antibodies directed against polypeptides of the present invention are useful for inhibiting allergic reactions in animals. For example, by administering a therapeutically acceptable dose of an antibody, or antibodies, of the present invention, or a cocktail of the present antibodies, or in combination with other antibodies of varying sources, the animal may not elicit an allergic response upon ingestion of certain antigens, particularly plant antigens. In an exemplary example, the tomato LEA lectin, from Lycospersicum esculentum has been shown to elicit a strong immune response when administered both orally and i.n. in mice. Thus, the administration to the mouse of antibodies directed against the LEA lectin may decrease or altogether eliminate an immune response to the LEA lectin as the LEA lectin could effectively be eliminated from circulation prior to eliciting an immune response (for example, See Lavelle EC, et al., Immunology. 99(1):30-7, (2000)). Likewise, one could envision cloning the gene encoding an antibody directed against a polypeptide of the present invention, said polypeptide having the potential to elicit an allergic and/or immune response in an organism, and transforming the organism with said antibody gene such that it is expressed (e.g., constitutitively, inducibly, etc.) in the organism. Thus, the organism would effectively become resistant to an allergic response resulting from the ingestion or presence of such an immune/allergic reactive polypeptide. Detailed descriptions of therapeutic and/or gene therapy applications of the present invention are provided elsewhere herein.

Alternatively, antibodies of the present invention could be produced in a plant (e.g., cloning the gene of the antibody directed against a polypeptide of the present invention, and transforming a plant with a suitable vector comprising said gene for constitutive expression of the antibody within the plant), and the plant subsequently ingested by an animal, thereby conferring temporary immunity to the animal for the specific antigen the antibody is directed towards (See, for example, US Patent Nos. 5,914,123 and 6,034,298).

In another embodiment, antibodies of the present invention, preferably polyclonal antibodies, more preferably monoclonal antibodies, and most preferably single-chain antibodies, can be used as a means of inhibiting gene expression of a particular gene, or genes, in a plant or organism. See, for example, International Publication Number WO 00/05391, published 2/3/00, to Dow Agrosciences LLC. The application of such methods for the antibodies of the present invention are known in the art, and are more particularly described elsewere herein (See, Examples 14 and 15).

In yet another embodiment, antibodies of the present invention may be useful for multimerizing the polypeptides of the present invention. For example, certain proteins may confer enhanced biological activity when present in a multimeric state (i.e., such enhanced activity may be due to the increased effective concentration of such proteins whereby more protein is available in a localized location).

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because certain proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Peptide moieties may be added to the polypeptide to facilitate purification. Such regions may be removed prior to final preparation of the polypeptide. Similarly, peptide cleavage sites can be introduced inbetween such peptide moieties, which could additionally be subjected to protease activity to remove said peptide(s) from the protein of the present invention. The addition of peptide moieties, including peptide cleavage sites, to facilitate handling of polypeptides are familiar and routine techniques in the art.

Moreover, polypeptides of the present invention, including fragments, and specifically epitopes, can be combined with parts of the constant domain of immunoglobulins (IgA, IgE, IgG, IgM) or portions thereof (CH1, CH2, CH3, and any combination thereof, including both entire domains and portions thereof), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of the constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences (also referred to as "tags"). Due to the availability of antibodies specific to such "tags", purification of the fused polypeptide of the invention, and/or its identification is significantly facilitated since antibodies specific to the polypeptides of the invention are not required. Such purification may be in the form of an affinity purification whereby an anti-tag antibody or another type of affinity matrix (e.g., anti-tag antibody attached to the matrix of a flow-thru column) that binds to the epitope tag is present. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984)).

The skilled artisan would acknowledge the existance of other "tags" which could be readily substituted for the tags referred to supra for purification and/or identification of polypeptides of the present invention (Jones C., et al., J Chromatogr A. 707(1):3-22 (1995)). For example, the c-myc tag and the 8F9, 3C7, 6E10, G4m B7 and 9E10 antibodies thereto (Evan et al., Molecular and Cellular Biology 5:3610-3616 (1985)); the Herpes Simplex virus glycoprotein D (gD) tag and its antibody (Paborsky et al., Protein Engineering, 3(6):547-553 (1990), the Flag-peptide - i.e., the octapeptide sequence DYKDDDDK (SEQ ID NO:17), (Hopp et al., Biotech. 6:1204-1210 (1988); the KT3 epitope peptide (Martin et al., Science, 255:192-194 (1992)); a-tubulin epitope peptide (Skinner et al., J. Biol. Chem., 266:15136-15166, (1991)); the T7 gene 10 protein peptide tag (Lutz-Freyermuth et al., Proc. Natl. Sci. USA, 87:6363-6397 (1990)), the FITC epitope (Zymed, Inc.), the GFP epitope (Zymed, Inc.), and the Rhodamine epitope (Zymed, Inc.).

The present invention also encompasses the attachment of the coding region of a repeating series of up to nine argininine amino acids to a polynucleotide of the present invention. The invention also encompasses chemically derivitizing a polypeptide of the present invention with a repeating series of up to nine arginine amino acids. Such a tag, when attached to a polypeptide, has recently been shown to serve as a universal pass, allowing compounds access to the interior of cells without additional derivitization or manipulation (Wender, P., et al., unpublished data).

Protein fusions involving polypeptides of the present invention, including fragments and/or variants thereof, can be used for the following, non-limiting examples, subcellular localization of proteins, determination of protein-protein interactions via immunoprecipitation, purification of proteins via affinity chromatography, functional and/or structural characterization of protein. The present invention also encompasses the application of hapten specific antibodies for any of the uses referenced above for epitope fusion proteins. For example, the polypeptides of the present invention could be chemically derivitized to attach hapten molecules (e.g., DNP, (Zymed, Inc.)). Due to the availability of monoclonal antibodies specific to such haptens, the protein could be readily purified using immunoprecipation, for example.

Polypeptides of the present invention, including fragments and/or variants thereof, in addition to, antibodies directed against such polypeptides, fragments, and/or variants, may be fused to any of a number of known, and yet to be determined, toxins, such as ricin, saporin (Mashiba H, et al., Ann N Y Acad Sci. 1999;886:233-5), HC toxin (Tonukari NJ, et al., Plant Cell. 2000 Feb;12(2):237-248), BT endotoxin, or pseudomonias endotoxin. Such fusions could be used to deliver the toxins to desired tissues for which a ligand or a protein capable of binding to the polypeptides of the invention exists.

The invention encompasses the fusion of antibodies directed against polypeptides of the present invention, including variants and fragments thereof, to said toxins for delivering the toxin to specific locations in a cell, to specific plant tissues, and/or to specific plant species. Such bifunctional antibodies are known in the art, though a review describing additional advantageous fusions, including citations for methods of production, can be found in P.J. Hudson, Curr. Opp. In Imm. 11:548-557, (1999); this publication, in addition to the references cited therein, are hereby incorporated by reference in their entirety herein. In this context, the term "toxin" may be expanded to include any heterologous protein, a small molecule, radionucleotides, cytotoxic drugs, liposomes, adhesion molecules, glycoproteins, ligands, cell or tissue-specific ligands, enzymes, of bioactive agents, biological response modifiers, anti-fungal agents, hormones, steroids, vitamins, peptides, peptide analogs, anti-allergenic agents, anti-tubercular agents, anti-viral agents, antibiotics, anti-protozoan agents, chelates, radioactive particles, radioactive ions, X-ray contrast agents, monoclonal antibodies, polyclonal antibodies and genetic material. In view of the present disclosure, one skilled in the art could determine whether any particular "toxin could be used in the compounds of the present invention. Examples of suitable "toxins" listed above are exemplary only and are not intended to limit the "toxins" that may be used in the present invention.

Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert of the present invention should be operatively linked to an appropriate promoter, such as the 35S promoter, the 34S promoter, CMV promoter, phage lambda PL promoter, the E. coli lac, trp, phoA and tac promoters, the SV40 early and late promoters and promoters of retroviral LTRs, to name a few. In addition, it may be desirable, or required, in some instances to have tissue-specific or cell type-specific promoters operably linked to a polynucleotide of the present invention. Examples of suitable plant-expressible promoters selectively expressed in particular tissues or cell types are well known in the art and include, but are not limited to, seed specific promoters (e.g., WO 89/03887), organ-primordia specific promoters (An et al., Plant Cell, 8:15-30, (1996)), stem-specific promoters (Keller et al., EMBO J., 7:3625-3633, (1988)), leaf specific promoters (Hudspeth et al., Plant. Mol. Biol., 12:579-589, (1989)), mesophyl-specific promoters (such as the light inducible Rubisco promoters), root-specific promoters (Keller et al., Genes Devel., 3:1639-1646, (1989)), tuber-specific promoters (Keil et al., EMBO J., 8:1323-1330, (1989)), vascular tissue specific promoters (Peleman et al., Gene, 84:359-369, (1989)), meristem specific promoters (such as the promoter of the SHOOTMERISTEMLESS (STM) gene, Long, et al., Nature, 379:66-69, (1996)), primodia specific promoter (such as the Antirrhinum CycD3a gene promoter, Doonan et al., in "Plant Cell Division" (Francis, Duditz, and Inze, Eds), Portland Press, London, (1998)), anther specific promoters (WO 89/10396, WO 92/13956, and WO 92/13957), stigma-specific promoters (WO 91/02068), degiscence-zone specific promoters (WO 97/13865), seed-specific promoters (WO 89/03887), etc.

Additional promoters that may be operably linked to a polynucleotide of the present invention may be found in McElroy and Brettel, Tibtech, Vol. 12, February, 1994. Moreover, a number of promoters are currently being used for transformation of dicotyledonous plants. These promoters come from a variety of different sources. One group of commonly used promoters were isolated from Agrobacterium tumefaciens, where they function to drive the expression of opine synthase genes carried on the T-DNA segment that is integrated into the plant genome during infection. These promoters include the octopine synthase (ocs) promoter (L. Comai et al., 1985; C. Waldron et al., 1985), the mannopine synthase (mas) promoter (L. Comai et al., 1985; K. E. McBride and K. R. Summerfelt, 1990) and the nopaline synthase (nos) promoter (M. W. Bevan et al., 1983; L. Herrera-Estrella et al., 1983, R. T. Fraley et al., 1983, M. De Block et al., 1984;, R. Hain et al., 1985). These promoters are active in a wide variety of plant tissue.

In addition, the promoters disclosed in the following publications may also be operably linked to a polynucleotide of the present invention: US Patent Nos. 5,623,067; 5,712,112; 5,723,751; 5,723,754; 5,723,757; 5,744,334; 5,750,385; 5,750,399; 5,767,363; 5,783,393; 5,789,214; 5,792,922; 5,792,933; 5,801,027; 5,804,694; 5,814,618; 5,824,857; 5,824,863; 5,824,865; 5,824,866; 5,824,872; and 5,929,302; and International Publication Nos. WO 97/49727, WO 98/00533, WO 98/03655, WO 98/07846, WO 98/08961, WO 98/08962, WO 98/10734, WO 98/16634, WO 98/22593,WO 98/38295, and WO 98/44097; and European Patent Application No. EP 0 846 770.

Several viral promoters are also used to, drive heterologous gene expression in dicots (J. C. Kridl and R. M. Goodman, 1986) and may be operably linked to a polynucleotide of the present invention. The Cauliflower Mosaic Virus 35S promoter is one of the promoters used most often for dicot transformation because it confers high levels of gene expression in almost all tissues (J. Odell et al., 1985; D. W. Ow et al., 1986; D. M. Shah et al., 1986). Modifications of this promoter are also used, including a configuration with two tandem 35S promoters (R. Kay et al., 1987) and the mas-35S promoter (L. Comai et al., 1990), which consists of the mannopine synthase promoter in tandem with the 35S promoter. Both of these promoters drive even higher levels of gene expression than a single copy of the 35S promoter. Other viral promoters that have been used include the Cauliflower Mosaic Virus 19S promoter (J. Paszkowski et al., 1984; E. Balazs et al.) and the 34S promoter from the figwort mosaic virus (M. Sanger et al., 1990).

Alternatively, the polynucleotide insert of the present invention could be operatively linked to any of a number of inducible promoters known in the art, which include, but are not limited to: tetracycline inducible promoters, small-molecule inducible promoters, light inducible promoters, chemical compounds (e.g., safeners, herbicides, glucocorticoids, etc.), abiotic stress inducible promoters (e.g., wounding, heavy metals, cold-sensitive promoters, heat-sensitive promoters, salt sensitive promoters, drought sensitive promoters, hypoxia indicuble (such as those disclosed in EP1012317), etc.), biotic stress promoters (e.g., pathogen or pest infection including infection by fungi, viruses, bacteria, insects, nematodes, mycoplasms, and mycoplasma-like organisms, etc.). Examples of plant-expressible inducible promoters suitable for the invention are: nematode inducible promoters (such as those disclosed in WO 92/21757 and/or EP1007709), fungus inducible promoters (WO 93/19188, WO 96/28561), chemically inducible Arabidopsis PR-1 promoter (WO 98/03536), the inducible promoters disclosed in WO 98/45445, the inducible promoters disclosed in US Patent No. 5,804,693, the tomato soft fruit inducible promoter disclosed in US Patent No. 5, 821, 398, promoters inducible after application of glucocorticoids such as dexamethasone, or promoters repressed or activated after application of tetracyclin (Gatz et al., PNAS USA, 85:1394-1397, (1988)). Other suitable inducible promoters will be known to the skilled artisan.

In addition, the polynucleotide insert of the present invention could be operatively linked to "artificial" or chimeric promoters and transcription factors. Specifically, the artificial promoter could comprise, or alternatively consist, of any combination of cis-acting DNA sequence elements that are recognized by trans-acting transcription factors. Preferably, the cis acting DNA sequence elements and trans-acting transcription factors are operable in plants. Further, the trans-acting transcription factors of such "artificial" promoters could also be "artificial" or chimeric in design themselves and could act as activators or repressors to said "artificial" promoter. For example, a chimeric promoter of the invention could comprise one or more, upstream activating sequences from the Octopine Synthase gene (OCS), matrix attachment regions (MAR), etc.

The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

The expression constructs may additionally comprise 5' leader sequences in the expression constructs. Such leader sequences can act to enhance translation. Translation leaders are known in the art and include: picornavirus leaders, for example, EMCV leader (Encephalomyocarditis 5' noncoding region) (Elroy-Stein, 0., Fuerst, T.R., and Moss, B. (1989) PNAS USA, 86:6126-6130); potyvirus leaders, for example, TEV leader (Tobacco Etch Virus) (Allison et al. (1986)); MDMV leader (Maize Dwarf Mosaic Virus) Virology, 154:9-20); and human immunoglobulin heavy-chain binding protein (BiP), (Macejak, D.G., and Sarnow, P. (1991) Nature, 353:90-94; untranslated leader from the coat protein mRNA of alfalfa mosaic virus (AMV RNA 4), (Jobling, S.A., and Gehrke, L., (1987) Nature, 325:622-625); tobacco mosaic virus leader (TW), (Gallie, D.R. et al. (1989) Molecular Biology of RNA, pages 237-256); and maize chlorotic mottle virus leader (MCNW) (Lommel, S.A. et al. (1991) Virology, 81:382-385). See also, Della-Cioppa et al. (1987) Plant Physiology, 8196506& Other methods known to enhance translation can also be utilized, for example, introns, and the like.

As indicated, the expression vectors will preferably include at least one selectable marker. Such markers include, but are not limited to, dihydrofolate reductase, G418 or neomycin resistance, kanamycin resistance, hygromycin resistance, bialaphos resistance, sulfonoamide resistance, stretomycin resistance, spectinomycin resistance, chlorosulfuron resistance, glyphosphate resistance, and methotrexate resistance, for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells (e.g., Saccharomyces cerevisiae or Pichia pastoris (ATCC Accession No. 201178)); insect cells such as Drosophila S2 and Spodoptera Sf9 cells; animal cells such as CHO, COS, 293, and Bowes melanoma cells; plant cells, and specifically plant cells and/or tissues derived from any of the plants listed in Table 3. Appropriate culture mediums and conditions for the above-described host cells are known in the art.

The polynucleotides and polypeptides of the present invention can be targeted to the chloroplast or amyloplast for expression. In this manner, the expression construct will additionally contain a polynucleotide sequence encoding a transit peptide operably linked to a polynucleotide of the present invention to direct the polynucleotide of the present invention to the chloroplasts. Such transit peptides are known in the art. See, for example, Von Heijne et al. (1991) Plant Mol. Biol. Rep. 9:104-126; Clark et al. (1989) J Biol. Chem. 264:17544-17550; della-Cioppa et al. (1987) Plant Physiol. 84065060 R.omer et al. (1993) Biochem. Biophys. Res Commun. 196:1414-1421; and Shah et al. (1986) Science 233:478-481.

The expression construct may also comprise any other necessary regulators such as nuclear localization signals (Kalderon et al. (1984) Cell 39:499-509; and Lassner et al. (1991) Plant Molecular Biology 17:229-234); plant translational consensus sequences (Joshi, C.P. (1987) Nucleic Acids Research 15:6643 6653), introns (Luehrsen and Walbot (1991) Mol. Gen. Genet. 225:81-93) and the like, operably linked to a polynucleotide of the present invention.

The polynucleotide sequences encoding the proteins or polypeptides of the present invention may be particularly useful in the genetic manipulation of plants. In this manner, the polynucleotides of the invention are provided in expression cassettes for expression in the plant of interest. Where appropriate, the gene(s) may be optimized for increased expression in the transformed plant. That is, the polynucleotides can be synthesized using plant preferred codons for improved expression specific to a particular species. Methods are available in the art for synthesizing plant preferred genes. See, for example, U.S. Patent Nos. 5,380,831, 5,436, 391, and Murray et al. (1989) Nucleic Acids Res. 17:477-498, herein incorporated by reference.

Depending upon the species in which the DNA sequence of interest is to be expressed, it may be desirable to synthesize the sequence with plant preferred codons, or alternatively with chloroplast preferred codons. The plant preferred codons may be determined from the codons of highest frequency in the proteins expressed in the largest amount in the particular plant species of interest. See, EPA 0359472; EPA 0385962; WO 91/16432; Perlak et al. (1991) Proc. Natl. Acad Sci. USA 88:3324-3328; and Murray et al. (1989) Nucleic Acids Research. In this manner, the polynucleotide sequences can be optimized for expression in any plant. It is recognized that all or any part of the gene sequence may be optimized or synthetic. That is, synthetic or partially optimized sequences may also be used.

Additionally, it may be desirable to selectively express a polypeptide of the present invention in a specific target cell or tissue of a plant by synthesizing the encoding polynucoleotide sequence to contain codons optimized for high translational efficiency within the particular target cell or tissue. Such methods are known in the art and are specifically provided in PCT International Publication No. WO 00/42190 (which is hereby incorporated herein by reference).

Additional sequence modifications are known to enhance gene expression in a cellular host. These include elimination of sequences encoding spurious polyadenylation signals, exon-intron splice site signals, transposon like repeats, and other such well-characterized sequences that may be deleterious to gene expression. The G-C content of the sequence may be adjusted to levels average for a given cellular host, as calculated by reference to known genes expressed in the host cell. When possible, the sequence may be modified to avoid predicted hairpin secondary mRNA structures.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Preferred expression vectors for use in yeast systems include, but are not limited to pYES2, pYD1, pTEFl/Zeo, pYES2/GS, pPICZ,pGAPZ, pGAPZalph, pPIC9, pPIC3.5, pHIL-D2, pHIL-S1, pPIC3.5K, pPIC9K, and PAO815 (all available from Invitrogen, Carlbad, CA).

Preferred expression vectors in plant systems include, but are not limited to, Bin 19 (ATCC Deposit No: 37327), GA437 (ATCC Deposit No: 37350), pAK1003 (ATCC Deposit No: 37425), pAS2022 (ATCC Deposit No: 37426), pAS2023 (ATCC Deposit No: 37427), pAP2034 (ATCC Deposit No: 37428), pC22 (ATCC Deposit No: 37493), pHS24 (ATCC Deposit No: 37841), pHS85 (ATCC Deposit No: 37842), pPM1 (ATCC Deposit No: 40172), pGV311 SE (ATCC Deposit No: 53213), pCGN978 (ATCC Deposit No: 67064), pFL61 (ATCC Deposit No: 77215), pGPTV-KAN (ATCC Deposit No: 77388), pGPTV-HPT (ATCC Deposit No: 77389), pGPTV-DHFR (ATCC Deposit No: 77390), pGPTV-BAR (ATCC Deposit No: 77391), pGPTV-BLEO (ATCC Deposit No: 77392), and/or pPE1000 (ATCC Deposit No: 87573). The skilled artisan would appreciate that any of the above vectors could easily be modified to either include or delete specific elements as may be required for operability. Other suitable vectors will be readily apparent to the skilled artisan.

Introduction of the construct into the host cell can be effected by biolistic transformation (Klein et al., Nature, 327:70-73 (1987)), PEG-mediated transfection (Paskowski, et al., EMBO J., 3:2717, (1984)), calcium phosphate transfection, DEAE-dextran mediated transfection, cationic lipid-mediated transfection, electroporation (Fromm, et al., PNAS, USA, 82:5824 (1985)), transduction, infection, Agrobacterium tumefaciens -directed infection, or other methods. Such methods are described in many standard laboratory manuals, such as Davis et al., Basic Methods in Molecular Biology (1986).

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells.

Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

In addition to encompassing host cells containing the vector constructs discussed herein, the invention also encompasses primary, secondary, and immortalized host cells that have been engineered to delete or replace endogenous genetic material (e.g., coding sequence), and/or to include genetic material (e.g., heterologous polynucleotide sequences) that is operably associated with the polynucleotides of the invention, and which activates, alters, and/or amplifies endogenous polynucleotides. For example, techniques known in the art may be used to operably associate heterologous control regions (e.g., promoter and/or enhancer) and endogenous polynucleotide sequences via homologous recombination, resulting in the formation of a new transcription unit (see, e.g., U.S. Patent No. 5,641,670, issued June 24, 1997; U.S. Patent No. 5,733,761, issued March 31, 1998; International Publication No. WO 96/29411, published September 26, 1996; International Publication No. WO 94/12650, published August 4, 1994; Koller et al., Proc. Natl. Acad. Sci. USA 86:8932-8935 (1989); and Zijlstra et al., Nature 342:435-438 (1989), the disclosures of each of which are incorporated by reference in their entireties).

In addition, polypeptides, analogs, derivatives, and/or fragments of the invention can be chemically synthesized. (See, e.g., Merrifield, 1963, J. Amer. Chem. Soc. 85:2149-2156). For example, polypeptides can be synthesized by solid phase techniques, cleaved from the resin, and purified by preparative high performance liquid chromatography (e.g., see Creighton, 1983, Proteins, Structures and Molecular Principles, W.H. Freeman and Co., N.Y., pp. 50-60). The polypeptides can also be synthesized by use of a peptide synthesizer. The composition of the synthetic peptides may be confirmed by amino acid analysis or sequencing (e.g., the Edman degradation procedure; see Creighton, 1983, Proteins, Structures and Molecular Principles, W.H. Freeman and Co., N.Y., pp. 34-49; Mass Spectroscopy peptide sequencing, etc). Furthermore, it desired, nonclassical amino acids or chemical amino acid analogs can be introduced as a substitution or addition into the polypeptide of the invention. Non-classical amino acids include but are not limited to the D-isomers of the common amino acids, 2,4-diaminobutyric acid, alpha.-amino isobutyric acid, 4-aminobutyric acid, Abu, 2-amino butyric acid, .gamma.-Abu, .epsilon.-Ahx, 6-amino hexanoic acid, Aib, 2-amino isobutyric acid, 3-amino propionic acid, ornithine, norleucine, norvaline, hydroxyproline, sarcosine, citrulline, cysteic acid, t-butylglycine, t-butylalanine, phenylglycine, cyclohexylalanine, beta.-alanine, fluoro-amino acids, designer amino acids such as .beta.-methyl amino acids, C.alpha.-methyl amino acids, N.alpha.-methyl amino acids, and amino acid analogs in general. Furthermore, the amino acid can be D (dextrorotary) or L (levorotary).

Manipulations of the polypeptide sequences of the invention may be made at the protein level. Included within the scope of the invention are polypeptides of the invention, fragments thereof, derivatives or analogs which are differentially modified during or after translation, e.g., by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including, but not limited to, specific chemical cleavage by cyanogen bromide, trypsin, chymotrypsin, papain, V8 protease, NaBH4, acetylation, formulation, oxidation, reduction, metabolic synthesis in the presence of tunicamycin, etc.

Moreover, the invention encompasses additional post-translational modifications which include, for example, the addition of N-linked or O-linked carbohydrate moieties or chains, the addition of detectable labels which may be flourescent, radioisotopic, enzymatic, in nature, the addition of epitope tagged peptide fragments (e.g., FLAG, HA, GST, thioredoxin, maltose binding protein, etc.), attachment of affinity tags such as biotin and/or streptavidin, the covalent attachment of chemical moieties to the amino acid backbone, N- or C-terminal processing of the polypeptides ends (e.g., proteolytic processing), deletion of the N-terminal methioine residue, etc.

Furthermore, the invention encompasses chemical derivitization of the polypeptides of the present invention, preferably where the chemical is a hydrophilic polymer residue. Exemplary hydrophilic polymers, including derivitives, may be those that include polymers in which the repeating units contain one or more hydroxy groups (polyhydroxy polymers), including, for example, poly(vinyl alcohol); polymers in which the repeating units contain one or more amino groups (polyamine polymers), including, for example, peptides, polypeptides, proteins and lipoproteins, such as albumin and natural lipoproteins; polymers in which the repeating units contain one or more carboxy groups (polycarboxy polymers), including, for example, carboxymethylcellulose, alginic acid and salts thereof, such as sodium and calcium alginate, glycosaminoglycans and salts thereof, including salts of hyaluronic acid, phosphorylated and sulfonated derivatives of carbohydrates, genetic material, such as interleukin-2 and interferon, and phosphorothioate oligomers; and polymers in which the repeating units contain one or more saccharide moieties (polysaccharide polymers), including, for example, carbohydrates.

The molecular weight of the hydrophilic polymers may vary, and is generally about 50 to about 5,000,000, with polymers having a molecular weight of about 100 to about 50,000 being preferred. More preferred polymers have a molecular weight of about 150 to about 10,000, with molecular weights of 200 to about 8,000 being even more preferred.

Additional preferred polymers which may be used to derivitize polypeptides of the invention, include, for example, poly(ethylene glycol) (PEG), poly(vinylpyrrolidine), polyoxomers, polysorbate and poly(vinyl alcohol), with PEG polymers being particularly preferred. Preferred among the PEG polymers are PEG polymers having a molecular weight of from about 100 to about 10,000. More preferably, the PEG polymers have a molecular weight of from about 200 to about 8,000, with PEG 2,000, PEG 5,000 and PEG 8,000, which have molecular weights of 2,000, 5,000 and 8,000, respectively, being even more preferred. Other suitable hydrophilic polymers, in addition to those exemplified above, will be readily apparent to one skilled in the art based on the present disclosure. Generally, the polymers used may include polymers that can be attached to the polypeptides of the invention via alkylation or acylation reactions.

As with the various polymers exemplified above, it is contemplated that the polymeric residues may contain functional groups in addition, for example, to those typically involved in linking the polymeric residues to the polypeptides of the present invention. Such functionalities include, for example, carboxyl, amine, hydroxy and thiol groups. These functional groups on the polymeric residues can be further reacted, if desired, with materials that are generally reactive with such functional groups and which can assist in targeting specific tissues in the body including, for example, diseased tissue. Exemplary materials which can be reacted with the additional functional groups include, for example, proteins, including antibodies, carbohydrates, peptides, glycopeptides, glycolipids, lectins, and nucleosides.

In addition to residues of hydrophilic polymers, the chemical used to derivitize the polypeptides of the present invention can be a saccharide residue. Exemplary saccharides which can be derived include, for example, monosaccharides or sugar alcohols, such as erythrose, threose, ribose, arabinose, xylose, lyxose, fructose, sorbitol, mannitol and sedoheptulose, with preferred monosaccharides being fructose, mannose, xylose, arabinose, mannitol and sorbitol; and disaccharides, such as lactose, sucrose, maltose and cellobiose. Other saccharides include, for example, inositol and ganglioside head groups. Other suitable saccharides, in addition to those exemplified above, will be readily apparent to one skilled in the art based on the present disclosure. Generally, saccharides which may be used for derivitization include saccharides that can be attached to the polypeptides of the invention via alkylation or acylation reactions.

Moreover, the invention also encompasses derivitization of the polypeptides of the present invention, for example, with lipids (including cationic, anionic, polymerized, charged, synthetic, saturated, unsaturated, and any combination of the above, etc.). stabilizing agents.

The invention encompasses derivitization of the polypeptides of the present invention, for example, with compounds that may serve a stabilizing function (e.g., to increase the polypeptides half-life in solution, to make the polypeptides more water soluble, to increase the polypeptides hydrophilic or hydrophobic character, etc.). Polymers useful as stabilizing materials may be of natural, semi-synthetic (modified natural) or synthetic origin. Exemplary natural polymers include naturally occurring polysaccharides, such as, for example, arabinans, fructans, fucans, galactans, galacturonans, glucans, mannans, xylans (such as, for example, inulin), levan, fucoidan, carrageenan, galatocarolose, pectic acid, pectins, including amylose, pullulan, glycogen, amylopectin, cellulose, dextran, dextrin, dextrose, glucose, polyglucose, polydextrose, pustulan, chitin, agarose, keratin, chondroitin, dermatan, hyaluronic acid, alginic acid, xanthin gum, starch and various other natural homopolymer or heteropolymers, such as those containing one or more of the following aldoses, ketoses, acids or amines: erythose, threose, ribose, arabinose, xylose, lyxose, allose, altrose, glucose, dextrose, mannose, gulose, idose, galactose, talose, erythrulose, ribulose, xylulose, psicose, fructose, sorbose, tagatose, mannitol, sorbitol, lactose, sucrose, trehalose, maltose, cellobiose, glycine, serine, threonine, cysteine, tyrosine, asparagine, glutamine, aspartic acid, glutamic acid, lysine, arginine, histidine, glucuronic acid, gluconic acid, glucaric acid, galacturonic acid, mannuronic acid, glucosamine, galactosamine, and neuraminic acid, and naturally occurring derivatives thereof Accordingly, suitable polymers include, for example, proteins, such as albumin, polyalginates, and polylactide-coglycolide polymers. Exemplary semi-synthetic polymers include carboxymethylcellulose, hydroxymethylcellulose, hydroxypropylmethylcellulose, methylcellulose, and methoxycellulose. Exemplary synthetic polymers include polyphosphazenes, hydroxyapatites, fluoroapatite polymers, polyethylenes (such as, for example, polyethylene glycol (including for example, the class of compounds referred to as Pluronics.RTM., commercially available from BASF, Parsippany, N.J.), polyoxyethylene, and polyethylene terephthlate), polypropylenes (such as, for example, polypropylene glycol), polyurethanes (such as, for example, polyvinyl alcohol (PVA), polyvinyl chloride and polyvinylpyrrolidone), polyamides including nylon, polystyrene, polylactic acids, fluorinated hydrocarbon polymers, fluorinated carbon polymers (such as, for example, polytetrafluoroethylene), acrylate, methacrylate, and polymethylmethacrylate, and derivatives thereof. Methods for the preparation of derivitized polypeptides of the invention which employ polymers as stabilizing compounds will be readily apparent to one skilled in the art, in view of the present disclosure, when coupled with information known in the art, such as that described and referred to in Unger, U.S. Pat. No. 5,205,290, the disclosure of which is hereby incorporated by reference herein in its entirety.

Moreover, the invention encompasses additional modifications of the polypeptides of the present invention. Such additional modifications are known in the art, and are specifically provided, in addition to methods of derivitization, etc., in US Patent No. 6,028,066, which is hereby incorporated in its entirety herein.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. Disease mapping data are known in the art. Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected organisms can be examined. First, visible structural alterations in the chromosomes, such as deletions or translocations, are examined in chromosome spreads or by PCR. If no structural alterations exist, the presence of point mutations are ascertained. Mutations observed in some or all affected organisms, but not in normal organisms, indicates that the mutation may cause the disease. However, complete sequencing of the polypeptide and the corresponding gene from several normal organisms is required to distinguish the mutation from a polymorphism. If a new polymorphism is identified, this polymorphic polypeptide can be used for further linkage analysis.

Furthermore, increased or decreased expression of the gene in affected organisms as compared to unaffected organisms can be assessed using polynucleotides of the present invention. Any of these alterations (altered expression, chromosomal rearrangement, or mutation) can be used as a diagnostic or prognostic marker.

Thus, the invention also provides a diagnostic method useful during diagnosis of a disorder, involving measuring the expression level of polynucleotides of the present invention in cells or body fluid from an organism and comparing the measured gene expression level with a standard level of polynucleotide expression level, whereby an increase or decrease in the gene expression level compared to the standard is indicative of a disorder.

By "measuring the expression level of a polynucleotide of the present invention" is intended qualitatively or quantitatively measuring or estimating the level of the polypeptide of the present invention or the level of the mRNA encoding the polypeptide in a first biological sample either directly (e.g., by determining or estimating absolute protein level or mRNA level) or relatively (e.g., by comparing to the polypeptide level or mRNA level in a second biological sample). Preferably, the polypeptide level or mRNA level in the first biological sample is measured or estimated and compared to a standard polypeptide level or mRNA level, the standard being taken from a second biological sample obtained from an individual not having the disorder or being determined by averaging levels from a population of organisms not having a disorder. As will be appreciated in the art, once a standard polypeptide level or mRNA level is known, it can be used repeatedly as a standard for comparison.

By "biological sample" is intended any biological sample obtained from an organism, body fluids, cell line, tissue culture, or other source which contains the polypeptide of the present invention or mRNA. As indicated, biological samples include body fluids (such as the following non-limiting examples, phloem, xylym, secreted fluids, nectar, nodule fluid, appressorium fluid, canker fluid, gall fluid, fruit juice, trichome fluid, vacuole fluid, plastid fluid, cytosolic fluid, root exudates, interstitial fluid, etc.) which contain the polypeptide of the present invention, and other tissue sources found to express the polypeptide of the present invention (such as the following non-limiting examples root, stem, apical meristem, leaves, flowers, pedals, etc.). Methods for obtaining tissue biopsies and body fluids from plants are well known in the art. For example, methods for isolating interstitial fluid of plant cells and tissues can be found, for example, in International Publication No. WO 00/09725. Where the biological sample is to include mRNA, a tissue biopsy is the preferred source.

The method(s) provided above may preferrably be applied in a diagnostic method and/or kits in which polynucleotides and/or polypeptides are attached to a solid support. In one exemplary method, the support may be a "gene chip" or a "biological chip" as described in US Patents 5,837,832, 5,874,219, and 5,856,174. Further, such a gene chip with polynucleotides of the present invention attached may be used to identify polymorphisms between the polynucleotide sequences, with polynucleotides isolated from a test subject. The knowledge of such polymorphisms (i.e. their location, as well as, their existence) would be beneficial in identifying disease loci for many disorders, including proliferative diseases and conditions. Such a method is described in US Patents 5,858,659 and 5,856,104. The US Patents referenced supra are hereby incorporated by reference in their entirety herein.

The present invention encompasses polynucleotides of the present invention that are chemically synthesized, or reproduced as peptide nucleic acids (PNA), or according to other methods known in the art. The use of PNAs would serve as the preferred form if the polynucleotides are incorporated onto a solid support, or gene chip. For the purposes of the present invention, a peptide nucleic acid (PNA) is a polyamide type of DNA analog and the monomeric units for adenine, guanine, thymine and cytosine are available commercially (Perceptive Biosystems). Certain components of DNA, such as phosphorus, phosphorus oxides, or deoxyribose derivatives, are not present in PNAs. As disclosed by P. E. Nielsen, M. Egholm, R. H. Berg and O. Buchardt, Science 254, 1497 (1991); and M. Egholm, O. Buchardt, L.Christensen, C. Behrens, S. M. Freier, D. A. Driver, R. H. Berg, S. K. Kim, B. Norden, and P. E. Nielsen, Nature 365, 666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the stronger binding characteristics of PNA:DNA hybrids. In addition, it is more likely that single base mismatches can be determined with PNA/DNA hybridization because a single mismatch in a PNA/DNA 15-mer lowers the melting point (T.sub.m) by 8°-20° C, vs. 4°-16° C for the DNA/DNA 15-mer duplex. Also, the absence of charge groups in PNA means that hybridization can be done at low ionic strengths and reduce possible interference by salt during the analysis.

In addition to the foregoing, a polynucleotide can be used to control gene expression through triple helix formation or antisense DNA or RNA. Antisense techniques are discussed, for example, in Okano, J. Neurochem. 56: 560 (1991); "Oligodeoxynucleotides as Antisense Inhibitors of Gene Expression,CRCPress, Boca Raton, FL (1988). Triple helix formation is discussed in, for instance Lee et al., Nucleic Acids Research 6: 3073 (1979); Cooney et al., Science 241: 456 (1988); and Dervan et al., Science 251: 1360 (1991). Both methods rely on binding of the polynucleotide to a complementary DNA or RNA. For these techniques, preferred polynucleotides are usually oligonucleotides 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360 (1991)) or to the mRNA itself (antisense - Okano, J. Neurochem. 56:560 (1991); Oligodeoxynucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat or prevent disease.

The present invention encompasses the addition of a nuclear localization signal, operably linked to the 5' end, 3' end, or any location therein, to any of the oligonucleotides, antisense oligonucleotides, triple helix oligonucleotides, ribozymes, PNA oligonucleotides, and/or polynucleotides, of the present invention. See, for example, G. Cutrona, et al., Nat. Biotech., 18:300-303, (2000); which is hereby incorporated herein by reference.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell. In one example, polynucleotide sequences of the present invention may be used to construct chimeric RNA/DNA oligonucleotides corresponding to said sequences, specifically designed to induce host cell mismatch repair mechanisms in a plant upon systemic injection, for example (Bartlett, R.J., et al., Nat. Biotech, 18:615-622 (2000), which is hereby incorporated by reference herein in its entirety). Such RNA/DNA oligonucleotides could be designed to correct genetic defects in certain host strains, and/or to introduce desired traits in the plant host (e.g., introduction of a specific polymorphism within an endogenous gene corresponding to a polynucleotide of the present invention that may confer resistence to certain herbicides, pesticides, fungicides, etc.). Alternatively, the polynucleotide sequence of the present invention may be used to construct duplex oligonucleotides corresponding to said sequence, specifically designed to correct genetic defects in certain host strains, and/or to introduce desired traits in the plant host (e.g., introduction of a specific polymorphism within an endogenous gene corresponding to a polynucleotide of the present invention that may confer resistence to certain herbicides, pesticides, fungicides, etc.). Such methods of using duplex oligonucleotides are known in the art and are encompassed by the present invention (see EP1007712, which is hereby incorporated by reference herein in its entirety).

The polynucleotides are also useful for identifying organisms from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an organisms genome. These sequences can be used to prepare PCR primers for amplifying and isolating such selected DNA, which can then be sequenced. Using this technique, organisms can be identified because each organism will have a unique set of DNA sequences. Once an unique ID database is established for an organism, positive identification of that organism, living or dead, can be made from extremely small tissue samples. Similarly, polynucleotides of the present invention can be used as polymorphic markers, in addition to, the identification of transformed or non-transformed plant cells and/or tissues.

There is also a need for reagents capable of identifying the source of a particular tissue. Such need arises, for example, when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination. Moreover, as mentioned above, such reagents can be used to screen and/or identify transformed and non-transformed plant cells and/or tissues.

Moreover, the polynucleotides of the present invention, the polynucleotides encoding the polypeptides of the present invention, the cDNA contained in the deposit, including variants and/or fragments therein, may be useful for modulating, inhibiting, increasing, decreasing, or introducing the following, non-limiting traits in a plant: drought tolerance, UV tolerance, flower development, terpene synthesis, abiotic stresse tolerance, heat stress tolerance, cold stress tolerance, nutritional stress tolerance, xenobiotic stress tolerance, protein storage capability, oil storage capability, amino acid content, amino acid composition, carbohydrate storage capability, oil content, oil composition, carbohydrate content, carbohydrate composition, fiber content, fiber composition, metabolite content, metaboliter composition, vitamin content, and/or vitamin composition. The polynucleotides of the invention, may also be useful in modulating plant yield, plant development, plant differentiation, root growth, root morphology, plant color, plant aroma, plant flavor, palatability of plant tissue, plant organoleptic properties, may be useful in phytoremediation, and/or plant defense. Moreover, the polypeptides of the invention may also be useful in modulating the plants ability to serve as a plant neutriceutical, pharmaceutical, or phytoceutical. Alternatively, polypeptides of the invention may also be useful in modulating the plants ability to produce plant neutriceuticals, pharmaceuticals, or phytoceuticals of either endogenous or exogenous origin (e.g., from another plant species, a human, a mammal, an animal, or other organism). In these contexts, the term "plant" may be applied to mean any plant cell, plant tissue, plant fluid, or plant feature, and includes plant infection structures, which may include, but are not limited to an appressorium, a gall, a canker, and/or nodules. In these contexts, the term "modulate" may be applied to mean the qualitative or quantitative increase, decrease, introduction of, inhibition of, complete loss of, or over-expression of a specific trait or characteristic as described above and elsewere herein.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell. Biol. 105:3087-3096 (1987)). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technecium (99mTc), and fluorescent labels, such as fluorescein and rhodamine, and biotin.

In addition to assaying protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the organism. It will be understood in the art that the size of the subject and the imaging system used will determine the quantity of imaging moiety needed to produce diagnostic images. In the case of a radioisotope moiety, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982)). Such methods are equally applicable to plants.

Thus, the invention provides a diagnostic method of a disorder, which involves (a) assaying the expression of a polypeptide of the present invention in cells or body fluid of an organism; (b) comparing the level of gene expression with a standard gene expression level, whereby an increase or decrease in the assayed polypeptide gene expression level compared to the standard expression level is indicative of a disorder.

Moreover, polypeptides of the present invention can be used for the treatment, detection, and/or prevention of a disease, or disease state. For example, an organism can be administered a polypeptide of the present invention in an effort to replace absent or decreased levels of the polypeptide, to supplement absent or decreased levels of a different polypeptide, to inhibit the activity of a polypeptide, either directly or indirectly, to activate the activity of a polypeptide, either directly or indirectly, (e.g., by binding to a receptor), to reduce the activity of a membrane bound receptor by competing with it for free ligand, or to bring about a desired response (e.g., induction of differentiation, growth, senescence, germination, etc,).

Similarly, antibodies directed to a polypeptide of the present invention can also be used to treat, prevent, and/or diagnose disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Transgenic Methods

Another aspect of the present invention is to gene therapy methods for treating or preventing disorders, diseases and conditions. The gene therapy methods relate to the introduction of nucleic acid (DNA, RNA, and antisense DNA or RNA) sequences into an organism, preferably a plant, to achieve expression of a polypeptide of the present invention. This method requires a polynucleotide which codes for a polypeptide of the invention operatively linked to a promoter and any other genetic elements necessary for the expression of the polypeptide by the target tissue. Such transgenic and delivery techniques are known in the art, see, for example, WO90/11092, which is herein incorporated by reference.

Thus, for example, cells from a plant may be engineered with a polynucleotide (DNA or RNA) comprising a promoter operably linked to a polynucleotide of the invention ex vivo, with the engineered cells then being introduced back into the plant to "treat" the deficiency. Such methods are well-known in the art and are equally applicable to plants. For example, see Belldegrun et al., J. Natl. Cancer Inst., 85:207-216 (1993); Ferrantini et al., Cancer Research, 53:107-1112 (1993); Ferrantini et al., J. Immunology 153: 4604-4615 (1994); Kaido, T., et al., Int. J. Cancer 60: 221-229 (1995); Ogura et al., Cancer Research 50: 5102-5106 (1990); Santodonato, et al., Human Gene Therapy 7:1-10 (1996); Santodonato, et al., Gene Therapy 4:1246-1255 (1997); and Zhang, et al., Cancer Gene Therapy 3: 31-38 (1996)), which are herein incorporated by reference.

As discussed in more detail below, the polynucleotide constructs can be delivered by any method that delivers materials to the cells of an organism, such as, biolistic injection into the plant tissues (apical meristem, root, flower, stem, and the like). The polynucleotide constructs may be delivered in an acceptable liquid or aqueous carrier.

In one embodiment, the polynucleotide of the invention is delivered as a naked polynucleotide. The term "naked" polynucleotide, DNA or RNA refers to sequences that are free from any delivery vehicle that acts to assist, promote or facilitate entry into the cell, including viral sequences, viral particles, liposome formulations, lipofectin or precipitating agents and the like. However, the polynucleotides of the invention can also be delivered in liposome formulations and lipofectin formulations and the like can be prepared by methods well known to those skilled in the art. Such methods are described, for example, in U.S. Patent Nos. 5,593,972, 5,589,466, and 5,580,859, which are herein incorporated by reference.

The polynucleotide vector constructs of the invention used in the gene therapy method are preferably constructs that will not integrate into the host genome nor will they contain sequences that allow for replication. Alternatively, the polynucleotide vector construcuts of the invention may integrate into the host genome and may replicate. Appropriate vectors include pWLNEO, pSV2CAT, pOG44, pXT1 and pSG available from Stratagene; pSVK3, pBPV, pMSG and pSVL available from Pharmacia; and pEF1/V5, pcDNA3.1, and pRc/CMV2 available from Invitrogen. Other suitable vectors will be readily apparent to the skilled artisan.

Any strong promoter known to those skilled in the art can be used for driving the expression of polynucleotide sequence of the invention. Suitable promoters include the 35S, 34S, and actin promoters, in addition to any other promoter known in the art and/or described elsewhere herein. The promoter also may be the native promoter for the polynucleotides of the invention.

Unlike other gene therapy techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The preferred route of administration is by the parenteral route of injection into the interstitial space of tissues. However, other parenteral routes may also be used. In addition, naked DNA constructs can be delivered to a plants circulatory system by direct injection.

The naked polynucleotides are delivered by any method known in the art, including, but not limited to, direct needle injection at the delivery site, topical administration, and so-called "gene guns". These delivery methods are known in the art.

The constructs may also be delivered with delivery vehicles such as viral sequences, viral particles, liposome formulations, lipofectin, precipitating agents, etc. Such methods of delivery are known in the art.

In certain embodiments, the polynucleotide constructs of the invention are complexed in a liposome preparation. Liposomal preparations for use in the instant invention include cationic (positively charged), anionic (negatively charged) and neutral preparations. However, cationic liposomes are particularly preferred because a tight charge complex can be formed between the cationic liposome and the polyanionic nucleic acid. Cationic liposomes have been shown to mediate intracellular delivery of plasmid DNA (Felgner et al., Proc. Natl. Acad. Sci. USA , 84:7413-7416 (1987), which is herein incorporated by reference); mRNA (Malone et al., Proc. Natl. Acad. Sci. USA , 86:6077-6081 (1989), which is herein incorporated by reference); and purified transcription factors (Debs et al., J. Biol. Chem., 265:10189-10192 (1990), which is herein incorporated by reference), in functional form.

Cationic liposomes are readily available. For example, N[1-2,3-dioleyloxy)propyl]-N,N,N-triethylammonium (DOTMA) liposomes are particularly useful and are available under the trademark Lipofectin, from GIBCO BRL, Grand Island, N.Y. (See, also, Felgner et al., Proc. Natl Acad. Sci. USA , 84:7413-7416 (1987), which is herein incorporated by reference). Other commercially available liposomes include transfectace (DDAB/DOPE) and DOTAP/DOPE (Boehringer).

Other cationic liposomes can be prepared from readily available materials using techniques well known in the art. See, e.g. PCT Publication NO: WO 90/11092 (which is herein incorporated by reference) for a description of the synthesis of DOTAP (1,2-bis(oleoyloxy)-3-(trimethylammonio)propane) liposomes. Preparation of DOTMA liposomes is explained in the literature, see, e.g., Felgner et al., Proc. Natl. Acad. Sci. USA, 84:7413-7417, which is herein incorporated by reference. Similar methods can be used to prepare liposomes from other cationic lipid materials.

Similarly, anionic and neutral liposomes are readily available, such as from Avanti Polar Lipids (Birmingham, Ala.), or can be easily prepared using readily available materials. Such materials include phosphatidyl, choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios.

Methods for making liposomes using these materials are well known in the art. For example, commercially dioleoylphosphatidyl choline (DOPC), dioleoylphosphatidyl glycerol (DOPG), and dioleoylphosphatidyl ethanolamine (DOPE) can be used in various combinations to make conventional liposomes, with or without the addition of cholesterol. Thus, for example, DOPG/DOPC vesicles can be prepared by drying 50 mg each of DOPG and DOPC under a stream of nitrogen gas into a sonication vial. The sample is placed under a vacuum pump overnight and is hydrated the following day with deionized water. The sample is then sonicated for 2 hours in a capped vial, using a Heat Systems model 350 sonicator equipped with an inverted cup (bath type) probe at the maximum setting while the bath is circulated at 15EC. Alternatively, negatively charged vesicles can be prepared without sonication to produce multilamellar vesicles or by extrusion through nucleopore membranes to produce unilamellar vesicles of discrete size. Other methods are known and available to those of skill in the art.

The liposomes can comprise multilamellar vesicles (MLVs), small unilamellar vesicles (SUVs), or large unilamellar vesicles (LUVs), with SUVs being preferred. The various liposome-nucleic acid complexes are prepared using methods well known in the art. See, e.g., Straubinger et al., Methods of Immunology , 101:512-527 (1983), which is herein incorporated by reference. For example, MLVs containing nucleic acid can be prepared by depositing a thin film of phospholipid on the walls of a glass tube and subsequently hydrating with a solution of the material to be encapsulated. SUVs are prepared by extended sonication of MLVs to produce a homogeneous population of unilamellar liposomes. The material to be entrapped is added to a suspension of preformed MLVs and then sonicated. When using liposomes containing cationic lipids, the dried lipid film is resuspended in an appropriate solution such as sterile water or an isotonic buffer solution such as 10 mM Tris/NaCl, sonicated, and then the preformed liposomes are mixed directly with the DNA. The liposome and DNA form a very stable complex due to binding of the positively charged liposomes to the cationic DNA. SUVs find use with small nucleic acid fragments. LUVs are prepared by a number of methods, well known in the art. Commonly used methods include Ca2+-EDTA chelation (Papahadjopoulos et al., Biochim. Biophys. Acta, 394:483 (1975); Wilson et al., Cell, 17:77 (1979)); ether injection (Deamer et al., Biochim. Biophys. Acta, 443:629 (1976); Ostro et al., Biochem. Biophys. Res. Commun., 76:836 (1977); Fraley et al., Proc. Natl. Acad. Sci. USA, 76:3348 (1979)); detergent dialysis (Enoch et al., Proc. Natl. Acad. Sci. USA , 76:145 (1979)); and reverse-phase evaporation (REV) (Fraley et al., J. Biol. Chem., 255:10431 (1980); Szoka et al., Proc. Natl. Acad. Sci. USA , 75:145 (1978); Schaefer-Ridder et al., Science, 215:166 (1982)), which are herein incorporated by reference.

Generally, the ratio of DNA to liposomes will be from about 10:1 to about 1:10. Preferably, the ration will be from about 5:1 to about 1:5. More preferably, the ration will be about 3:1 to about 1:3. Still more preferably, the ratio will be about 1:1. U.S. Patent NO: 5,676,954 (which is herein incorporated by reference) reports on the injection of genetic material, complexed with cationic liposomes carriers, into mice. U.S. Patent Nos. 4,897,355, 4,946,787, 5,049,386, 5,459,127, 5,589,466, 5,693,622, 5,580,859, 5,703,055, and international publication NO: WO 94/9469 (which are herein incorporated by reference) provide cationic lipids for use in transfecting DNA into cells and mammals. U.S. Patent Nos. 5,589,466, 5,693,622, 5,580,859, 5,703,055, and international publication NO: WO 94/9469 (which are herein incorporated by reference) provide methods for delivering DNA-cationic lipid complexes to mammals. Such methods are equally applicable to plants and is within the skill of the artisan.

In certain embodiments, cells are engineered, ex vivo or in vivo, using a retroviral particle containing RNA that comprises a sequence encoding polypeptides of the invention. Retroviral integration has been detected to occur in plants based upon the identification of a pararetrovirus sequences within the tabacco genome. Since such integration was determined to occur at very limited integration sites, such a pararetrovirus may represent a desirable genetic transformation vehicle for the polynucleotides of the present invention (Jakowitsch, J., et al., PNAS 96(23):13241-6 (1999).

The retroviral plasmid vector is employed to transduce packaging cell lines to form producer cell lines. Examples of packaging cells which may be transfected include, but are not limited to, the PE501, PA317, R-2, R-AM, PA12, T19-14X, VT-19-17-H2, RCRE, RCRIP, GP+E-86, GP+envAm12, and DAN cell lines as described in Miller, Human Gene Therapy , 1:5-14 (1990), which is incorporated herein by reference in its entirety. The vector may transduce the packaging cells through any means known in the art. Such means include, but are not limited to, electroporation, the use of liposomes, and CaPO4 precipitation. In one alternative, the retroviral plasmid vector may be encapsulated into a liposome, or coupled to a lipid, and then administered to a host.

The producer cell line generates infectious retroviral vector particles that include polynucleotides encoding polypeptides of the invention. Such retroviral vector particles then may be employed, to transduce eukaryotic cells, either in vitro or in vivo. The transduced eukaryotic cells will express polypeptides of the invention.

The present invention also encompasses the application of retrotransposons to the genetic transformation of plants. The retrotransposons would preferably represent retrotransposons with known plant host range and would comprise polynucleotides encoding polypeptides of the present invention. Many retrotransposons are known in the art, some of which are described by Bennetzen JL, Trends Microbiol., 4(9):347-53 (1996) which is hereby incorporated herein by reference.

Another method of gene therapy involves operably associating heterologous control regions and endogenous polynucleotide sequences (e.g. encoding the polypeptide sequence of interest) via homologous recombination (see, e.g., U.S. Patent NO: 5,641,670, issued June 24, 1997; International Publication NO: WO 96/29411, published September 26, 1996; International Publication NO: WO 94/12650, published August 4, 1994; Koller et al., Proc. Natl. Acad. Sci. USA, 86:8932-8935 (1989); and Zijlstra et al., Nature, 342:435-438 (1989). This method involves the activation of a gene which is present in the target cells, but which is not normally expressed in the cells, or is expressed at a lower level than desired.

Polynucleotide constructs are made, using standard techniques known in the art, which contain the promoter with targeting sequences flanking the promoter. Suitable promoters are described herein. The targeting sequence is sufficiently complementary to an endogenous sequence to permit homologous recombination of the promoter-targeting sequence with the endogenous sequence. The targeting sequence will be sufficiently near the 5' end of the desired endogenous polynucleotide sequence so the promoter will be operably linked to the endogenous sequence upon homologous recombination.

The promoter and the targeting sequences can be amplified using PCR. Preferably, the amplified promoter contains distinct restriction enzyme sites on the 5' and 3' ends. Preferably, the 3' end of the first targeting sequence contains the same restriction enzyme site as the 5' end of the amplified promoter and the 5' end of the second targeting sequence contains the same restriction site as the 3' end of the amplified promoter. The amplified promoter and targeting sequences are digested and ligated together.

The promoter-targeting sequence construct is delivered to the cells, either as naked polynucleotide, or in conjunction with transformation-facilitating agents, such as liposomes, viral sequences, viral particles, whole viruses, lipofection, precipitating agents, etc., described in more detail above. The P promoter-targeting sequence can be delivered by any method, included direct needle injection, intravenous injection, topical administration, infusion, particle accelerators, etc. The methods are described in more detail below.

The promoter-targeting sequence construct is taken up by cells. Homologous recombination between the construct and the endogenous sequence takes place, such that an endogenous sequence is placed under the control of the promoter. The promoter then drives the expression of the endogenous sequence.

Preferably, the polynucleotide encoding a polypeptide of the invention contains a secretory signal sequence that facilitates secretion of the protein. Typically, the signal sequence is positioned in the coding region of the polynucleotide to be expressed towards or at the 5' end of the coding region. The signal sequence may be homologous or heterologous to the polynucleotide of interest and may be homologous or heterologous to the cells to be transformed. Additionally, the signal sequence may be chemically synthesized using methods known in the art.

Any mode of administration of any of the above-described polynucleotides constructs can be used so long as the mode results in the expression of one or more molecules in an amount sufficient to provide a therapeutic effect. This includes direct needle injection, systemic injection, infusion, biolistic injectors, particle accelerators (i.e., "gene guns"), gelfoam sponge depots, other commercially available depot materials, osmotic pumps (e.g., Alza minipumps), and decanting or topical application. For example, direct injection of naked calcium phosphate-precipitated plasmid into rat liver and rat spleen or a protein-coated plasmid into the portal vein has resulted in gene expression of the foreign gene in the rat livers. (Kaneda et al., Science, 243:375 (1989)). Moreover, direct injection of naked DNA has been reported in plants and is encompassed by the present invention (Davey MR, et al., Plant Mol Biol, 13(3):273-85 (1989), and Potrykus I, Ciba Found Symp, 154:198-212 (1990)).

A preferred method of local administration is by direct injection. Preferably, a recombinant molecule of the present invention complexed with a delivery vehicle is administered by direct injection into or locally within the area of the organisms circulatory system (e.g., phloem, xylem, etc). Administration of a composition locally within the area of the organisms circulatory system refers to injecting the composition centimeters and preferably, millimeters within the organisms circulatory system.

Another method of local administration is to contact a polynucleotide construct of the present invention in or around a surgical wound or grafting. For example, the polynucleotide construct can be coated on the surface of tissue inside the wound or the construct can be injected into areas of tissue inside the wound.

Therapeutic compositions useful in systemic administration, include recombinant molecules of the present invention complexed to a targeted delivery vehicle of the present invention. Suitable delivery vehicles for use with systemic administration comprise liposomes comprising ligands for targeting the vehicle to a particular site.

Preferred methods of systemic administration, include injection, aerosol, percutaneous (topical) delivery. Injections can be performed using methods standard in the art. Aerosol delivery can also be performed using methods standard in the art (see, for example, Stribling et al., Proc. Natl. Acad. Sci. USA , 189:11277-11281 (1992), which is incorporated herein by reference). Topical delivery can be performed by mixing a polynucleotide construct of the present invention with a lipophilic reagent (e.g., DMSO) that is capable of passing into the skin.

Determining an effective amount of substance to be delivered can depend upon a number of factors including, for example, the chemical structure and biological activity of the substance, the age and weight of the plant or animal, the precise condition requiring treatment and its severity, and the route of administration. The frequency of treatments depends upon a number of factors, such as the amount of polynucleotide constructs administered per application, as well as the half-life of the polynucleotide and polypeptides (i.e., the effective period of application). The precise amount, number of applications and timing of applications will be determined per desired application.

Therapeutic compositions of the present invention can be administered to any organism, preferably to plants. Preferred plants, may include the following non-limiting examples, including barley, oats, rye, sorghum, pea, sunflower, tobacco, cotton, petunia, tomato, broccoli, lettuce, apple, plum, orange, and lemon, and more preferrably rice, maize, conola, wheat, sugerbeet, sugercane, and soybean, in addition to other plants known in the art and referenced more particularly elsewhere herein (e.g., Table 3).

Moreover, the present invention encompasses transgenic cells, including, but not limited to seeds, organisms, and plants into which genes encoding polypeptides of the present invention have been introduced. Non-limiting examples of suitable recipient plants for introducing polynucleotides of the invention, polynucleotides encoding the polypeptides of the invention, the cDNA contained in a deposit, and/or fragments, and varients therein, are listed in Table 3 below:

**TABLE 3**

| **RECIPIENT PLANTS** | | |
|---|---|---|
| **COMMON NAME** | **FAMILY** | **LATIN NAME** |
| Maize | Gramineae | Zea mays |
| Maize, Dent | Gramineae | Zea mays dentiformis |
| Maize, Flint | Gramineae | Zea mays vulgaris |
| Maize, Pop | Gramineae | Zea mays microsperma |
| Maize, Soft | Gramineae | Zea mays amylacea |
| Maize, Sweet | Gramineae | Zea mays amyleasaccharata |
| Maize, Sweet | Gramineae | Zea mays saccharate |
| Maize, Waxy | Gramineae | Zea mays ceratina |
| Wheat, Dinkel | Pooideae | Triticum spelta |
| Wheat, Durum | Pooideae | Triticum durum |
| Wheat, English | Pooideae | Triticum turgidum |
| Wheat, Large Spelt | Pooideae | Triticum spelta |
| Wheat, Polish | Pooideae | Triticum polonium |
| Wheat, Poulard | Pooideae | Triticum turgidum |
| Wheat, Singlegrained | Pooideae | Triticum monococcum |
| Wheat, Small Spelt | Pooideae | Triticum monococcum |
| Wheat, Soft | Pooideae | Triticum aestivum |
| Rice | Gramineae | Oryza sativa |
| Rice, American Wild | Gramineae | Zizania aquatica |
| Rice, Australian | Gramineae | Oryza australiensis |
| Rice, Indian | Gramineae | Zizania aquatica |
| Rice, Red | Gramineae | Oryza glaberrima |
| Rice, Tuscarora | Gramineae | Zizania aquatica |
| Rice, West African | Gramineae | Oryza glaberrima |
| Barley | Pooideae | Hordeum vulgare |
| Barley, Abyssinian Intermediate, also Irregular | Pooideae | Hordeum irregulare |
| Barley, Ancestral Tworow | Pooideae | Hordeum spontaneum |
| Barley. Beardless | Pooideae | Hordeum trifurcatum |
| Barley, Egyptian | Pooideae | Hordeum trifurcatum |
| Barley, fourrowed | Pooideae | Hordeum vulgare polystichon |
| Barley, sixrowed | Pooideae | Hordeum vulgare hexastichon |
| Barley, Tworowed | Pooideae | Hordeum distichon |
| Cotton, Abroma | Dicotyledoneae | Abroma augusta |
| Cotton, American Upland | Malvaceae | Gossypium hirsutum |
| Cotton, Asiatic Tree, also Indian Tree | Malvaceae | Gossypium arboreum |
| Cotton, Brazilian, also, Kidney, and, Pernambuco | Malvaceae | Gossypium barbadense brasiliense |
| Cotton, Levant | Malvaceae | Gossypium herbaceum |
| Cotton, Long Silk, also Long Staple, Sea Island | Malvaceae | Gossypium barbadense |
| Cotton, Mexican, also Short Staple | Malvaceae | Gossypium hirsutum |
| Soybean, Soya | Leguminosae | Glycine max |
| Sugar beet | Chenopodiaceae | Beta vulgaris altissima |
| Sugar cane | Woody-plant | Arenga pinnata |
| Tomato | Solanaceae | Lycopersicon esculentum |
| Tomato, Cherry | Solanaceae | Lycopersicon esculentum cerasiforme |
| Tomato, Common | Solanaceae | Lycopersicon esculentum commune |
| Tomato, Currant | Solanaceae | Lycopersicon pimpinellifolium |
| Tomato, Husk | Solanaceae | Physalis ixocarpa |
| Tomato, Hyenas | Solanaceae | Solanum incanum |
| Tomato, Pear | Solanaceae | Lycopersicon esculentum pyriforme |
| Tomato, Tree | Solanaceae | Cyphomandra betacea |
| Potato | Solanaceae | Solanum tuberosum |
| Potato, Spanish, Sweet potato | Convolvulaceae | Ipomoea batatas |
| Rye, Common | Pooideae | Secale cereale |
| Rye, Mountain | Pooideae | Secale montanum |
| Pepper, Bell | Solanaceae | Capsicum annuum grossum |
| Pepper, Bird, also Cayenne, Guinea | Solanaceae | Capsicum annuum minimum |
| Pepper, Bonnet | Solanaceae | Capsicum sinense |
| Pepper, Bullnose, also Sweet | Solanaceae | Capsicum annuum grossum |
| Pepper, Cherry | Solanaceae | Capsicum annuum cerasiforme |
| Pepper, Cluster, also Red Cluster | Solanaceae | Capsicum annuum fasciculatum |
| Pepper, Cone | Solanaceae | Capsicum annuum conoides |
| Pepper, Goat, also Spur | Solanaceae | Capsicum frutescens |
| Pepper, Long | Solanaceae | Capsicum frutescens longum |
| Pepper, Oranamental Red, also Wrinkled | Solanaceae | Capsicum annuum abbreviatum |
| Pepper, Tabasco Red | Solanaceae | Capsicum annuum conoides |
| Lettuce, Garden | Compositae | Lactuca sativa |
| Lettuce, Asparagus, also Celery | Compositae | Lactuca sativa asparagina |
| Lettuce, Blue | Compositae | Lactuca perennis |
| Lettuce, Blue, also Chicory | Compositae | Lactuca pulchella |
| Lettuce, Cabbage, also Head | Compositae | Lactuca sativa capitata |
| Lettuce, Cos, also Longleaf, Romaine | Compositae | Lactuca sativa longifolia |
| Lettuce, Crinkle, also Curled, Cutting, Leaf | Compositae | Lactuca sativa crispa |
| Celery | Umbelliferae | Apium graveolens dulce |
| Celery, Blanching, also Garden | Umbelliferae | Apium graveolens dulce |
| Celery, Root, also Tumiprooted | Umbelliferae | Apium graveolens rapaceum |
| Eggplant, Garden | Solanaceae | Solanum melongena |
| Sorghum | Sorghum | All crop species |
| Alfalfa | Leguminosae | Medicago sativum |
| Carrot | Umbelliferae | Daucus carota sativa |
| Bean, Climbing | Leguminosae | Phaseolus vulgaris vulgaris |
| Bean, Sprouts | Leguminosae | Phaseolus aureus |
| Bean, Brazilian Broad | Leguminosae | Canavalia ensiformis |
| Bean, Broad | Leguminosae | Vicia faba |
| Bean, Common, also French, White, Kidney | Leguminosae | Phaseolus vulgaris |
| Bean, Egyptian | Leguminosae | Dolichos lablab |
| Bean, Long, also Yardlong | Leguminosae | Vigna sesquipedalis |
| Bean, Winged | Leguminosae | Psophocarpus tetragonolobus |
| Oat, also Common, Side, Tree | Avena | Sativa |
| Oat, Black, also Bristle, Lopsided | Avena | Strigosa |
| Oat, Bristle | Avena | |
| Pea, also Garden, Green, Shelling | Leguminosae | Pisum, sativum sativum |
| Pea, Blackeyed | Leguminosae | Vigna sinensis |
| Pea, Edible Podded | Leguminosae | Pisum sativum axiphium |
| Pea, Grey | Leguminosae | Pisum sativum speciosum |
| Pea, Winged | Leguminosae | Tetragonolobus purpureus |
| Pea, Wrinkled | Leguminosae | Pisum sativum medullare |
| Sunflower | Compositae | Helianthus annuus |
| Squash, Autumn, Winter | Dicotyledoneae | Cucurbita maxima |
| Squash, Bush, also Summer | Dicotyledoneae | Cucurbita pepo melopepo |
| Squash, Turban | Dicotyledoneae | Cucurbita maxima turbaniformis |
| Cucumber | Dicotyledoneae | Cucumis sativus |
| Cucumber, African, also Bitter | | Momordica charantia |
| Cucumber, Squirting, also Wild | | Ecballium elaterium |
| Cucumber, Wild | | Cucumis anguria |
| Poplar, California | Woody-Plant | Populus trichocarpa |
| Poplar, European Black | | Populus nigra |
| Poplar, Gray | | Populus canescens |
| Poplar, Lombardy | | Populus italica |
| Poplar, Silverleaf, also White | | Populus alba |
| Poplar, Western Balsam | | Populus trichocarpa |
| | | |
| Tobacco | Solanaceae | Nicotiana |
| | | |
| Arabidopsis Thaliana | Cruciferae | Arabidopsis thaliana |
| | | |
| Turfgrass | Lolium | |
| Turfgrass | Agrostis | |
| | Other families of turfgrass | |
| Clover | Leguminosae | |

### Biological Activities

The polynucleotides or polypeptides, or agonists or antagonists of the present invention can be used in assays to test for one or more biological activities. If these polynucleotides and polypeptides do exhibit activity in a particular assay, it is likely that these molecules may be involved in the diseases associated with the biological activity. Thus, the polynucleotides or polypeptides, or agonists or antagonists could be used to treat the associated disease.

### Hyperproliferative Disorders

Polynucleotides or polypeptides, or agonists or antagonists of the invention can be used to detect, prevent, and/or confer resistence to hyperproliferative diseases, disorders, and/or conditions, which include, but are not limited to, cankers, galls, tumors, appressorium, etc. Polynucleotides or polypeptides, or agonists or antagonists of the present invention may inhibit the proliferation of the disorder through direct or indirect interactions. Alternatively, polynucleotides or polypeptides, or agonists or antagonists of the present invention may proliferate other cells which can inhibit the hyperproliferative disorder.

One preferred embodiment utilizes polynucleotides of the present invention to inhibit aberrant cellular division, by gene therapy using the present invention, and/or protein fusions or fragments thereof.

Thus, the present invention provides a method for treating or preventing cell proliferative diseases, disorders, and/or conditions by inserting into an abnormally proliferating cell a polynucleotide of the present invention, wherein said polynucleotide represses said expression.

Another embodiment of the present invention provides a method of treating or preventing cell-proliferative diseases, disorders, and/or conditions in organisms comprising administration of one or more active gene copies of the present invention to an abnormally proliferating cell or cells. In a preferred embodiment, a polynucleotide of the present invention is a DNA construct comprising a recombinant expression vector effective in expressing a DNA sequence encoding said polynucleotides. In another preferred embodiment of the present invention, the DNA construct encoding the poynucleotides of the present invention is inserted into cells to be treated utilizing a retrovirus vector, or more preferrably a plant retrotransposon-based vector. In a most preferred embodiment, the viral vector is defective and will not transform non-proliferating cells, only proliferating cells. Moreover, in a preferred embodiment, the polynucleotides of the present invention inserted into proliferating cells either alone, or in combination with or fused to other polynucleotides, can then be modulated via an external stimulus (i.e. magnetic, specific small molecule, chemical, or drug administration, etc.), which acts upon the promoter upstream of said polynucleotides to induce expression of the encoded protein product. As such, a beneficial therapeutic affect of the present invention may be expressly modulated (i.e. to increase, decrease, or inhibit expression of the present invention) based upon said external stimulus.

Polynucleotides of the present invention may be useful in repressing expression of proliferating genes or antigens. By "repressing expression of the proliferating genes" is intended the suppression of the transcription of the gene, the degradation of the gene transcript (pre-message RNA), the inhibition of splicing, the destruction of the messenger RNA, the prevention of the post-translational modifications of the protein, the destruction of the protein, or the inhibition of the normal function of the protein.

For local administration to abnormally proliferating cells, polynucleotides of the present invention may be administered by any method known to those of skill in the art including, but not limited to transformation, electroporation, microinjection of cells, or in vehicles such as liposomes, lipofectin, or as naked polynucleotides, or any other method described throughout the specification. The polynucleotide of the present invention may be delivered by known gene delivery systems such as, but not limited to, retroviral vectors (Gilboa, J. Virology 44:845 (1982); Hocke, Nature 320:275 (1986); Wilson, et al., Proc. Natl. Acad. Sci. U.S.A. 85:3014), vaccinia virus system (Chakrabarty et al., Mol. Cell Biol. 5:3403 (1985) or other efficient DNA delivery systems (Yates et al., Nature 313:812 (1985)) known to those skilled in the art. These references are exemplary only and are hereby incorporated by reference. In order to specifically deliver or transform cells which are abnormally proliferating and spare non-dividing cells, it is preferable to utilize a retrovirus, or a plant retrotransposon-based vector (as described in the art and elsewhere herein) delivery system known to those of skill in the art. Since host DNA replication is required for retroviral DNA to integrate and the retrovirus will be unable to self replicate due to the lack of the retrovirus genes needed for its life cycle. Utilizing such a retroviral delivery system for polynucleotides of the present invention will target said gene and constructs to abnormally proliferating cells and will spare the non-dividing normal cells.

The polynucleotides of the present invention may be delivered directly to cell proliferative disorder/disease sites in the plants body by use of imaging devices used to guide an injecting needle directly to the disease site. The polynucleotides of the present invention may also be administered to disease sites at the time of grafting, etc.

By "cell proliferative disease" is meant any disease or disorder, affecting any one or any combination of organs, cavities, or body parts, which is characterized by single or multiple local abnormal proliferations of cells, groups of cells, or tissues.

Any amount of the polynucleotides of the present invention may be administered as long as it has a biologically inhibiting effect on the proliferation of the treated cells. Moreover, it is possible to administer more than one of the polynucleotide of the present invention simultaneously to the same site. By "biologically inhibiting" is meant partial or total growth inhibition as well as decreases in the rate of proliferation or growth of the cells. The biologically inhibitory dose may be determined by assessing the effects of the polynucleotides of the present invention on target or abnormally proliferating cell growth in tissue culture, tumor growth in plants and cell cultures, or any other method known to one of ordinary skill in the art.

The present invention is further directed to antibody-based therapies which involve administering anti-polypeptides and anti-polynucleotide antibodies to a plant, for detecting, preventing, and/or conferring resistence to one or more of the described diseases, disorders, and/or conditions. Methods for producing anti-polypeptides and anti-polynucleotide antibodies polyclonal and monoclonal antibodies are described in detail elsewhere herein. Such antibodies may be provided in pharmaceutically acceptable compositions as known in the art or as described herein.

A summary of the ways in which the antibodies of the present invention may be used therapeutically includes binding polynucleotides or polypeptides of the present invention locally or systemically in the body or by direct cytotoxicity of the antibody, e.g. as mediated by complement (CDC) or by effector cells (ADCC). Some of these approaches are described in more detail below. Armed with the teachings provided herein, one of ordinary skill in the art will know how to use the antibodies of the present invention for diagnostic, monitoring or therapeutic purposes without undue experimentation.

In particular, the antibodies, fragments and derivatives of the present invention are useful for detecting, preventing, and/or conferring resistence to a plant having or developing cell proliferative and/or differentiation diseases, disorders, and/or conditions as described herein. Such treatment comprises administering a single or multiple doses of the antibody, or a fragment, derivative, or a conjugate thereof.

The antibodies of this invention may be advantageously utilized in combination with other monoclonal or chimeric antibodies, or with cytokinins or plant growth factors, for example, which serve to increase the number or activity of effector cells which interact with the antibodies.

It is preferred to use high affinity and/or potent in vivo inhibiting and/or neutralizing antibodies against polypeptides or polynucleotides of the present invention, fragments or regions thereof, for both immunoassays directed to and therapy of diseases, disorders, and/or conditions related to polynucleotides or polypeptides, including fragements thereof, of the present invention. Such antibodies, fragments, or regions, will preferably have an affinity for polynucleotides or polypeptides, including fragements thereof. Preferred binding affinities include those with a dissociation constant or Kd less than 5X10-6M, 10-6M, 5X10-7M, 10-7M, 5X10-8M, 10-8M, 5X10-9M, 10-9M, 5X10-10M, 10-10M, 5X10-11M, 10-11M, 5X10-12M, 10-12M, 5X10-13M, 10-13M, 5X10-14M, 10-14M, 5X10-15M, and 10-15M.

Polypeptides, including protein fusions, of the present invention, or fragments thereof may be useful in inhibiting proliferative cells or tissues through the induction of apoptosis. Said polypeptides may act either directly, or indirectly to induce apoptosis of proliferative cells and tissues. Apoptosis has been described in plants and is thought to be regulated via mechanisms analogous to apoptosis induction in animals (see, for example, LoSchiavo F, et al., Eur J Cell Biol., 79(4):294-8 (2000); and Tian R, et al., FEBS Lett., 474(1):11-15 (2000)).

Moreover, in another preferred embodiment of the present invention, said polypeptides may induce apoptosis through other mechanisms, such as in the activation of other proteins which will activate apoptosis (e.g., caspase 3, poly(ADP-ribose) polymerase (PARP), etc), or through stimulating the expression of said proteins, either alone or in combination with small molecule drugs or adjuviants, such as apoptonin, galectins, thioredoxins, antiinflammatory proteins (See for example, Mutat Res 400(1-2):447-55 (1998), Med Hypotheses.50(5):423-33 (1998), Chem Biol Interact. Apr 24;111-112:23-34 (1998), J Mol Med.76(6):402-12 (1998), Int J Tissue React;20(1):3-15 (1998), which are all hereby incorporated by reference).

In another embodiment, the invention provides a method of delivering compositions containing the polypeptides of the invention (e.g., compositions containing polypeptides or polypeptide antibodies associated with heterologous polypeptides, heterologous nucleic acids, toxins, or prodrugs) to targeted cells expressing the polypeptide of the present invention. Polypeptides or polypeptide antibodies of the invention may be associated with with heterologous polypeptides, heterologous nucleic acids, toxins, or prodrugs via hydrophobic, hydrophilic, ionic and/or covalent interactions.

Polypeptides, protein fusions to, or fragments thereof, of the present invention are useful in enhancing the immunogenicity and/or antigenicity of proliferating cells or tissues, either directly, or indirectly, to said antigens and immunogens.

### Infectious Diseases

Infectious agents may inhibit the plants overall ability to maintain plant homeostasis and/or cellular homeostasis. For example, the infectious agent may inhibit the plants ability to control cellular division, differentiation, and development; absorption of water and minerals from the soil and the translocation of these substances throughout the plant; photosynthesis and translocation of the photosynthetic products to areas of use or storage; metabolism of systhesized compounds; reproduction; and storage of plant food-stuffs for overwintering or reproduction, for example.

Affected cells and tissues of diseased plants are usually weakened or destroyed by disease-causing agents. The ability of such infected cells and tissues to perform normal physiological functions is thus reduced or completely inhibited, causing cell or plant death. The type of tissue affected determines the physiological function affected. For example, infection of the root (e.g., root rot), interferes with absorption of water and nutrients from the soil; infection of the xylem vessels (e.g., vascular wilts, cankers, etc.) interferes with translocation of water and minerals to the crown of the plant; infection of the foliage (e.g., leaf spots, blights, mosaics, etc.) interferes with photosynthesis; infection of the cortex (e.g., cortical canker, viral and mycoplasmal infections of phloem, etc.) interferes with the downward translocation of photosynthetic products; flower infections (e.g., bacterial and fungal blights, viral, mycoplasmal, and fungal infections of flowers, etc.) interfere with reproduction; and infections of fruit (e.g., fruit rot, etc.) interefer with reproduction or storage of reserve food stuffs for the new plant. The above list of infectious traits and/or symptoms is exemplary and should not be construed as limiting the present invention. Additional infectious traits are known in the art, some of which are described elsewere herein (see for example, Agrios, G.N., in "Plant Pathology", 3rd Ed., Academic Press, Inc., (1988); which is hereby incorporated in its entirety herein).

Viruses are one example of an infectious agent that can cause disease or traits that can be detected, prevented, and/or resistence conferred to by a polynucleotide or polypeptide and/or agonist or antagonist of the present invention. Examples of viruses, include, but are not limited to to the following DNA and RNA viruses and viral families: Arbovirus, Adenoviridae, Arenaviridae, Arterivirus, Birnaviridae, Bunyaviridae, Caliciviridae, Circoviridae, Coronaviridae, Dengue, EBV, HIV, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza A, Influenza B, and parainfluenza), Papiloma virus, Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus).. Additional examples of viruses, include, but are not limited to to the following Tobamovirus group (e.g., Tobacco Mosaic), Tobravirus group (e.g., Tobacco rattle virus), Hordeivirus group (e.g., Barlet strip mosaic), Potexvirus group (e.g., Potato virus X), Carlavirus group (e.g., Carnation latent virus), Potyvirus group (e.g., potato virus Y), Closterovirus group (e.g., beet yellows virus), Maize chlorotic dwarf virus, Tobacco necrosis virus, Tymovirus group (e.g., Turnip yellow mosiac virus), Tombusvirus group (e.g., Tomato bushy stunt virus), Sobemovirus group (e.g., Southern bean mosaic virus), Luteovirus group (e.g., Barley yellow dwarf virus), Comovirus group (e.g., Cowpea mosaic virus), Nepovirus group (e.g., Tobacco ringspot virus), Pea enation mosaic virus, Dianthovirus group (e.g., Carnation ringspot virus), Cucumovirus group (e.g., Cucumber mosaic virus), Bromovirus group (e.g., Brome mosaic virus), Ilavirus group (e.g., Tobacco streak virus), Alfalfa mosaic virus, Tomatto spotter wilt virus, Rhabdoviridae (e.g., Lettuce necrotic yellows virus), Rioviridae (e.g., Wound tumor virus), Geminivirus group (e.g., Maize streak virus), and Caulimovirus (e.g., Cauliflower mosaic virus). Additional viruses capable of infecting a plant or animal are known in the art (see, for example, G.N., Agrios, supra, and Jones, T.C., in "Veterinary Pathology", 4^{th} Edition, Lea and Febiger, Philadelphia, (1972).

Viruses falling within these families can cause a variety of diseases or symptoms, generally including, but not limited to: mosaics, ring spots, stunting, dwarfing, leaf roll, yellowing, streaking, pox, enation, tumors, pitting of stem, aspermy, sterility, pitting of fruit, flattening and distortion of the stem; and specifically include, but not limited to tobacco mosaic, bean mosaic, apple mosaic, pear ring pattern mosaic, maize dwarf mosaic, tulip breaking, tobacco ringspot, prunus necrtic ringspot, elm ringspot, chysanthemum ringspot, lilac ringspot, blueberry ringspot, beet yellows, wheat streak mosaic, tobacco etch, vein enation, vein clearing, vein banding, vein necrosis, potato leaf roll, grape fan leaf, tomato shoestring, stunting, banana bunchy top, citrus tristeza, cocao swollen shoot, stem pitting, apple flat limb, pear rough bark, stem necrosis, graft brown line, cherry black canker, elm zonate canker, citrus woody gall, clover wound tumor, apple russet ring, apple scar skin, pear stony pit, spotted wilt, etc. Viruses may also lead to decreased photosynthesis, decreased chorophyll per leaf, decreased photosynthesis, decreased chorophyll per leaf, decreased chlorophyll efficiency, decrease in plant hormone production, decreased growth rate, decreased soluble nitrogen, decreased carbohydrate levels, either an increase or decrease in respiration, aberant plant metabolism, decrease water translocation, decreased nutrient retention, increased transpiration, reduced yields, modulate transcription of the plant, modulate translation of the plant, and aberrant cellular metababolism. Additional symptoms caused by viral infections are known in the art (see, for example, G.N., Agrios, supra; Jones, T.C., in "Veterinary Pathology", 4th Edition, Lea and Febiger, Philadelphia, (1972); and in "Viral and Rickettsial Infections of Animals", eds, Betts, A.O., and York, C.J., Academic Press, NY, (1967)). Polynucleotides or polypeptides, or agonists or antagonists of the invention, can be used to either directly or indirectly, detect, prevent, and/or confer resistence to any of these traits or diseases. For example, a polynucleotide or polypeptide of the present invention may directly inhibit a disorder or infection when transgenically overexpressed in a plant. Alternatively, for example, a polynucleotide or polypeptide of the present invention may indirectly inhibit a disorder or infection by inhibiting the ability of the virus to transmit the infection from one plant to another.

As inferred to above, plant viral infections may be transmitted through a number of mechanisms, which include, but are not limited to the following: transmission through vegetative propagation, mechanical transmission through sap, seed transmission, pollen transmission, insect transmission, mite transmission, nematode transmission, fungal transmission, and dodder transmission. Polynucleotides or polypeptides, or agonists or antagonists of the invention, can be used to either directly or indirectly, detect, prevent, inhibit, and/or confer resistence to any of these mechanisms of viral transmission.

Similarly, bacterial agents that can cause disease or symptoms in plants or animals can be detected, prevented, and/or resistence conferred to the plant by a polynucleotide or polypeptide and/or agonist or antagonist of the present invention. Examples of such bacterial agents include, but not limited to, the following: Gram-Negative and Gram-positive bacteria and bacterial families and fungi: Bacillaceae (e.g., Anthrax, Clostridium), Bacteroidaceae, Blastomycosis, Bordetella, Borrelia (e.g., Borrelia burgdorferi), Brucellosis, Candidiasis, Campylobacter, Coccidioidomycosis, Cryptococcosis, Dermatocycoses, E. coli (e.g., Enterotoxigenic E. coli and Enterohemorrhagic E. coli), Enterobacteriaceae (Klebsiella, Salmonella (e.g., Salmonella typhi, and Salmonella paratyphi), Serratia, Yersinia), Erysipelothrix, Helicobacter, Legionellosis, Leptospirosis, Listeria, Mycoplasmatales, Mycobacterium leprae, Vibrio cholerae, Neisseriaceae (e.g., Acinetobacter, Gonorrhea, Menigococcal), Meisseria meningitidis, Pasteurellacea Infections (e.g., Actinobacillus, Heamophilus (e.g., Heamophilus influenza type B), Pasteurella), Pseudomonas, Rickettsiaceae, Chlamydiaceae, Syphilis, Shigella spp., Staphylococcal, Meningiococcal, Pneumococcal and Streptococcal (e.g., Streptococcus pneumoniae and Group B Streptococcus). Additional examples of bacterial agents include, for example, Agrobacterium, Clavibacter, Erwinia, Pseodomonas, Xanthomonas, Streptomyces, Xylella, Mycoplasm, Acholeplasma, and Spiroplasmas. Additional bacterial agents capable of infecting a plant or animal are known in the art (see, for example, G.N., Agrios, supra, and Jones, T.C., in "Veterinary Pathology", 4th Edition, Lea and Febiger, Philadelphia, (1972)

Bacterial agents falling within any of the aforementioned families can cause a variety of diseases or symptoms, generally including, but not limited to: leaf spots, leaf blights, soft rots (e.g., of fruit, root, and storage organs, etc.), wilts, overgrowths, scabs, cankers, nodules, galls, yellowing, phloem necrosis, X-disease, fasciation, and hairy root; and specifically, for example, crown gall, twig gall, cane gall, potato ring rot, tomato canker and wilt, banana wilt bud blast, cutting rot, black venation, bulb rot, citrus canker, walnut blight, pototo scab, sweet potato soil rot, tobacco wildfire, bean blight, cucumber angular leaf spot, cotton angular leaf spot, cereal blight, grass blight, tomato bacterial spot, pepper bacterial spot, stone fruit bacterial spot, bacterial vascular wilts, cucurbit bacterial wilt, pear fire blight, apple fire blight, pototo ring rot, southern bacterial wilt of solanaceous plants, Moko disease of banana, gummosis of stone fruit trees, Pierce's Disease of grape, almpond leaf scorch, alfalfa dwarf, phony peach, plum leaf scald, ratoon stunting, clover club leaf, aster yellows, big bud, apple proliferation, peach yellows, apple rubbery wood, pear decline, elm phloem necrosis, coconut lethal yellowing, citrus tubborn, decreased photosynthesis, decreased chorophyll per leaf, decreased chlorophyll efficiency, decrease in plant hormone production, decreased growth rate, decreased soluble nitrogen, decreased carbohydrate levels, either an increase or decrease in respiration, aberant plant metabolism, decrease water translocation, decreased nutrient retention, increased transpiration, reduced yields, modulate transcription of the plant, modulate translation of the plant, aberrant cellular metababolism, and corn stunt. Additional symptoms and diseases caused by bacterial agents are known in the art (see, for example, G.N., Agrios, supra, and Jones, T.C., in "Veterinary Pathology", 4th Edition, Lea and Febiger, Philadelphia, (1972). Polynucleotides or polypeptides, agonists or antagonists of the invention, can be used, either directly or indirectly, to detect, prevent, and/or confer resistence to any of these symptoms or diseases.

Similarly, fungal agents that can cause disease or symptoms in plants or animals can be detected, prevented, and/or resistence conferred to the plant by a polynucleotide or polypeptide and/or agonist or antagonist of the present invention. Examples of such fungal agents include, but not limited to, the following: Actinomycetales (e.g., Corynebacterium, Mycobacterium, Norcardia), Cryptococcus neoformans, Aspergillosis, Myxomycota (e.g., Myxomycetes (Fuligo, Muciliago, Physarum, Physarales, etc), and Plasmodiophoromycetes (Plasmodiophora (e.g., P. brassicae), Polymyxa (e.g., P. gramminis, etc.), Spongospora (e.g., S. subteranea, etc.))), Eumycota (e.g., Mastigomycotina, Chrytridiomycetes (e.g., Olpidium brassicae, Physoderma maydia, Synchytrium endobioticum, Urophlyctis alfalfae, etc.), Oomycetes, Saprolegniales (e.g., Aphanomyces, etc.), Peronosporales, Pythiaceae, Pythium (e.g., Phytophthora infestans, etc.), Albuginaceae (e.g., Albugo candida, etc.), Peronosporaceae (e.g., Plasmopara viticola, Peronospora nicotianae, Bremia lactucae, Sclerospora graminicola, and Pseudoperonospora cubensis, etc.), Zygomycotina, Zygomycetes, Mucorales, Rhizopus (e.g., Choanephora cucurbitarum, etc.), Endogonales, Endogone, Ascomycotina, Hemiascomycetes, Endomycetales (e.g., Saccharomyces cerevisiae, etc.), Taphrina, Pytenomycetes, Erysiphales (e.g., Erysiphe, Microsphaera, Podosphaera leucotricha, Spaerotheca pannosa, Uncinula necator etc.), Sphaeriales (e.g., Botryosphaeria obtusa, Ceratocystis, Diaporthe, Endothia parasitica, Eutypa armeniacae, Glomerella cingulata, Gnomonia, Hypoxylon mammatum, Rosellinia, Valsa, Xylaria, etc.), Hypocreales (e.g., Claviceps purpurea, Gibberella, Nectria, etc.), Loculoascomycetes, Myriangiales (e.g., Elsinoe, etc.), Dothideales (e.g., Capnodium, Didymella, Guignardia bidwellii, Microcyclus elei, Plowrightia morbosum, etc.) Pleosporales (e.g., Cochliobolus sativus, Gaeumannomyces graminis, Pyrenophora, Venturia inaequalis, etc.), Discomycetes, Phacidiales (e.g., Rhytisma acerinum), Helotiales (e.g., Diplocarpos rosae, Higginsia hiemalis, Lophodermium, Monilinia fructicola, Pseudopeziza trifolii, Sclerotinia sclerotiorum, etc.), Deuteromycotina, Coelomycetes, Sphaeropsidales (e.g., Ascochyta pisi, Coniothyrium, Cytospora, Diplodia maydis, Phoma lingam, Phomopsis, Phyllosticta, Septoria apii, etc.), Melanconiales (e.g., Celletotrichum, Coryneium beijerincki, Cylindrosporium, Gloeosporium, Marssonina, Melanconium fuligenum, Sphaceloma, etc.), Hyphomycetes, Hyphales (e.g., Alternaria, Asperigillus, Bipolaris, dreschslerea, Excerophilum, Botrytis cinerea, Cercospora, Fulvia fulva, Fusarium, Geotrichum candidum, Graphium ulmi, Peniciuum, Phymatotrichum omnivorum, Pyricularia, Spilocaea, Theilaviopsis basicola, Trichoderma, Verticillum, etc.), Agonomycetes, Agonomycetales (e.g., Rhizoctonia, Sclerotium, etc.), Basidomycotina, Hemibasidiomycetes, Ustilaginales (e.g., Sphaceltheca, Tilletia, Urocystis cepulae, Ustilago, etc.), Uredinales (e.g., Cronartium, Gymnosporangium juniperivirginianae, Melampsora lini, Phragmidium, Puccinia, Uromyces appendiculatus, etc.), Hymenomycetes, Exobasidiales (e.g., Exobasidium, etc.), Aphyllochorales (e.g., Aethalia, Corticium, Heterobasidum, Lenzites, Peniophora, Polyporus, Poria, Schizophyllum, Stereum, etc.), Tulasnellales (e.g., Thanatephorus, Typhula, etc.), Agaricales (e.g., Armillaria mellea, Marasmius, Pholiota, Pleurotus, etc.), and particularly Ascomycota, Basidiomycota, Zygomycota, Oomycota, Plasmodiophoromycota, Puccinia recondita, Rhizo puschinensis, Plasmo paraviticola, Plasmodiophora brassicae, Erwinia amylovora, Elsinoe fawcettii, Phaeosphaeria nodorum, Mycosphaerella arachidis, Mycosphaerella berkeleyi, Mycosphaerella fijiensis, Mycosphaerella graminicola, Pyrenophora teres, Pyremophora tritici-repentis, Venturia carpophila, Alternaria brassicae, Alternaria kikuchiana, Alternaria mali, Alternaria solani, Cercospora beticola, Cladosporium cucumerinum, Septoria lycopersici, Blumaria graminis, Erysiphe cichoracearum, Podosphaera leucotricha, Sphaerotheca fuliginea, Uncinila necator, Emericella nidulans, Penicillium digitatum, Penicillium italicum, Gibberella fujikuroi, Nectria haematococca, Fusarium culmorum, Fusarium oxysporum, Fusarium roseum, Gliocladium virens, Botryotinia fuckeliana, Monilinia fructigena, Sclerotinia homoeocarpa, Sclerotinia sclerotiorum, Mollisia yallundae, Glomerella lagenaria, Saccaromyces cerevisiae, Neurospora crassa, Gaemannomyces graminis, Magneporthe grisea, Monographella nivalis, Rhyncosporium secalis, Athelia rolfsii, Typhula incarnata, Thanatephorus cucumeris, Rhizoctonia solani, Hemileia vastatrix, Puccinia hordei, and Uromyces appendiculatus,. Additional fungal agents are known in the art (see, for example, G.N., Agrios, supra, G.C. Ainsworth, in "Fungal Diseases of Animals", Commonwealth Agricultural Bureaux, Farmham Royal Bucks, England, (1959), and Jones, T.C., in "Veterinary Pathology", 4th Edition, Lea and Febiger, Philadelphia, (1972)).

Fungal agents falling within any of the aforementioned divisions, subdivisions, classes, orders, genus's, or species can cause a variety of diseases or symptoms, generally including, but not limited to: necrosis, plant death, cell death, hypotrophy, hypoplasmia, stunting, hyperplasia (e.g., clubroot, galls, warts, witches brooms, leaf curls, etc.), tumors, leaf spots, blight, cankers, dieback, root rot, damping off, basal stem rot, soft rots, dry rots, anthracnose, scab, decline, wilt, rust, mildew, and smut; and specifically, fructifications, powdery scab of potato, clubroot of crucifers, black wort of potato, crown wart of alfalfa, brown spot of corn, seed rot, seedling damping off, root and stem rot, blight, tuber rot, white rust, upper side, downy mildews, oospores on soybean seed, rhizopus soft rots, rhizopus fruit rot, choanephora squash rot, bread mold, bud rot, stem rot, collar rot, crown rot, trunk rot, black pod disease, late blight of potatoes, Anthracnose diseases, Colletotrichum diseases, onion smudge, ergot, Botrytis diseases, vascular wilts, Dutch Elm disease, Gibberella diseases, Sclerotinia Diseases, Rhizoctonia Diseases, Sclerotium Diseases, postharvest decay of fruits and vegetables, decreased photosynthesis, decreased chorophyll per leaf, decreased chlorophyll efficiency, decrease in plant hormone production, decreased growth rate, decreased soluble nitrogen, decreased carbohydrate levels, either an increase or decrease in respiration, aberant plant metabolism, decrease water translocation, decreased nutrient retention, increased transpiration, reduced yields, modulate transcription of the plant, modulate translation of the plant, and aberrant cellular metababolism; and particularly citrus scab, grape black rot, wheat septoria nodorum blotch, early leaf spot of peanut, banana black sigatoka, wheat septoria tritici blotch, barley net blotch, cereal tan spot, apple scab, peach feckle, cabbage blackspot, apple leaf blotch, tomato and potato early blight, cercospora leaf spot of beet, cucumber scab, tomato seporial leaf spot, wheat powdery mildew, barlet powdery mildew, cucumber powdery mildew, apple powdery mildew, cucumber powdery mildew, grape powdery mildew, saporphyte, green mold of citrus, blue mold of citrus, rice bakanae disease, cereal headscab, cucumber fusarium wilt, tomato fusarium wilt, radish yellow fusarium, rice damping, pepper botrytis blight, botrytis blights of bean, botrytis blights of cuke, botrytis blights of grape, botrytis blights of marigold, brown rot of stone fruits, turf dollar spot, wheat eye spot, cucumber anthracnose, pestalotial leaf spot, rice blast, turf snow mold, barley scald, typhula blight, rhizoctonia damping, coffee rust, wheat lead rust, barley leaf rust, and bean rust. Polynucleotides or polypeptides, agonists or antagonists of the invention, can be used, either directly or indirectly, to detect, prevent, and/or confer resistence to any of these symptoms or diseases. Additional fungal agents capable of infecting a plant or animal are known in the art (see, for example, G.N., Agrios, supra, G.C. Ainsworth, in "Fungal Diseases of Animals", Commonwealth Agricultural Bureaux, Farmham Royal Bucks, England, (1959), and Jones, T.C., in "Veterinary Pathology", 4th Edition, Lea and Febiger, Philadelphia, (1972)).

Moreover, parasitic agents causing disease or symptoms that can be detected, prevented, and/or conferred resistence to by a polynucleotide or polypeptide and/or agonist or antagonist of the present invention include, but not limited to, the following families or class: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas and Sporozoans (e.g., Plasmodium virax, Plasmodium falciparium, Plasmodium malariae, and Plasmodium ovale). Additional examples of parasitic agents include, for example, Cuscutaceae (e.g., Cuscuta, dodder, etc.), Viscaceae (e.g., Arceuthobium (dwarf mistletoe of conifers), Phoradendron (American true mistletoes of broadleaved trees), and Viscum (European true mistletoes)), Orobanchaceae (e.g., Orobanche (broomrapes of tabacco)), and Scrophulariaceae (e.g., Striga (witchweeds of monocot plants)). These parasites can cause a variety of diseases or symptoms, including, but not limited to: decreased water storage, decreased mineral availability, decreased carbohydrate storage, decreased plant defense, and increased susceptability to fungal, bacterial, or viral infections. Additional parasitic diseases are known in the art (see, for example, G.N., Agrios, supra, and Jones, T.C., in "Veterinary Pathology", 4th Edition, Lea and Febiger, Philadelphia, (1972). Polynucleotides or polypeptides, or agonists or antagonists of the invention, can be used to detect, prevent, and/or confer resistence to any of these symptoms or diseases.

Methods of treatment or prevention using a polypeptide or polynucleotide and/or agonist or antagonist of the present invention are well known in the art, though may include administering an effective amount of a polypeptide to the plant, seed, tissue, or cells. Alternatively, treatment or prevention could be conferred by transforming the plant, seed, tissue, or cells with a polynucleotide of the present invention, or cells could be removed from the plant, transformed, and then returning the engineered cells to the plant (ex vivo therapy). Moreover, the polypeptide or polynucleotide of the present invention can be used as an antigen to create antibodies that inhibit the pathogenicity of a particular infectious disease (e.g., inhibiting the expression of a plant-specific gene or protein critical for the pathogenicity of an infectious organism). In addition, any method of detecting, preventing, conferring resistence to, or inhibiting an infectious agent using a polypeptide or polynucleotide and/or agonist or antagonist of the present invention may be applied to detecting, preventing, conferring resistence to, or inhibiting an infectious agent in a human, animal, mammal, or other organism, with or without additional modification.

### Pest Tolerance

Nematodes are one example of pests capable of causing disease or traits that can be detected, prevented, and/or resistence conferred to by a polynucleotide or polypeptide and/or agonist or antagonist of the present invention. Examples of nematodes include, but are not limited to, the following: Tylenchida, Tylenchina, Tylenchoidia, Tylenchidea, Tylenchidae, Anguina, Ditylenchus, Tylenchorhynchidae, Tylenchorhynchus, Pratylenchidae, Pratylenchus, Radopholus, Hoplolaimidae, Hoplolaimus, Rotylenchus, Helicotylenchus, Belonolaimidae, Belonolaimus, Heteroderoidea, Heterderidae, Globodera, Heterodera, Meloidogyne, Nacobbidae, Nacobbus, Rotylenchulus, Criconematoidea, Criconematidae, Criconemella, Hemicycliophora, Paratylenchidae, Paratylenchus, Tylenchuidae, Tylenchulus, Aphelenchina, Aphelenchoidea, Aphelenchoididae, Aphlenenchoides, Bursaphelenchus, Rhadinaphelenchus, Dorylaimida, Longidoridae, Longidorus, Xiphinema, Trichodoridae, Paratrichodorus, And Trichodorus. Other members of the nematode phylum, orders, suborders, superfamilies, families, genuses, and species are known in the art.

Nematodes falling within any of the aforementioned, orders, suborders, superfamilies, families, genuses, and/or species can cause a variety of diseases or symptoms in plants, generally including, but not limited to: root knots, root galls, reduced plant growth, plant nutrient deficiencies, yellowing, wilting, reduced yields, poor product quality, plant galls, necrotic lesions, rots, twisting or distortion of leaves and stems, abnormal floral development; hypertrophy, hypotrophy, cysts, and chlorosis. Polynucleotides or polypeptides, agonists or antagonists of the invention, can be used, either directly or indirectly, to detect, prevent, and/or confer resistence to any of these symptoms or diseases. Additional diseases and/or disorders caused by nematodes are known in the art.

Insects are another example of pests capable of causing disease or traits that can be detected, prevented, and/or resistence conferred to by a polynucleotide or polypeptide and/or agonist or antagonist of the present invention. Examples of insects include, but are not limited to, the following: insects selected from the orders Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera, etc., particularly Coleoptera.

Polynucleotides or polypeptides, agonists or antagonists of the invention, can be used, either directly or indirectly, to detect, prevent, and/or confer resistence to any of the following, non-limiting, symptoms or diseases caused by insect pests of major crops: Maize: Ostrinia nubilalis, European corn borer; Agrotis ipsilon, black cutworm; Helicoverpa zea, corn earworm; Spodoptera ftugiperda, fall armyworm; Diatraea grandiosella, southwestern corn borer; Elasmopalpus lignosellus, lesser cornstalk borer; Diatraea saccharalis, surgarcane borer; Diabrotica virgifera, western corn rootworm; Diabrotica barberi, northern corn rootworm; Diabrotica undecimpunctata howardi, spotted cucumber beetle, Melanotus spp., wireworms; Cyclocephala borealis, northern masked chafer (white grub); Cyclocephala immaculata, southern masked chafer (white grub); Popilliajaponica, Japanese beetle; Chaetocnema pulicaria, corn flea beetle; Sphenophorus maidis, maize billbug; Rhopalosiphum maidis, corn leaf aphid; Anuraphis maidiradicis, corn root aphid; Blissus leucopterus, chinch bug; Melanoplus femurrubrum, redlegged grasshopper; Melanoplus sanguinipes, migratory grasshopper; Delia platura, seedcorn maggot; Agromyza parvicornis, corn blotch leafminer; Anaphothrips obscrurus, grass thrips; Solenopsis milesta, thief ant; Tetranychus urticae, twospotted spider mite; Busseola Jusca, African Maize Stem Borer (AMB); Sesamia calamistis, African Pink Borer (AP13); Eldana sacchharina, African Sugarcane Borer (ASB); Chilo partellus, Sorghum Stem Borer (SSB); Ostrinia furnacalis, Oriental Corn Borer (OCB); Sesamia nonagrioides, Corn borer in Europe/N. Africa; Syrahum: Clilo partellus, sorghum borer; Spodoptera ftugiperda, fall armyworm; Relicoverpa zea, corn earworm; Elasmopalpus lignosellus, lesser cornstalk borer; Agrotis subterranea, granulate cutworm; Phyllophaga crinita, white grub; Eleodes, Conoderus, and Aeolus spp., wireworms; Oulema melanopus, cereal leaf beetle; Chaetocnema pulicaria, corn flea beetle; Sphenophorus maidis, maize billbug; Rhopalosiphum maidis; corn leaf aphid; Siphaflava, yellow sugarcane aphid; Blissus leucopterus, chinch bug; Contarinia sorghicola, sorghum midge; Tetranychus cinnabarinus, carmine spider mite; Tetranychus urticae, twospotted spider mite; Schizaphis graminum, Greenbug (aphid); Wheat: Pseudaletia unipunctata, army worm; Spodoptera ftugiperda, fall armyworm; Elasmopalpus - lignosellus, lesser cornstalk borer; Agrotis orthogonia, plae western cutworm; Oulema melanopus, cereal leaf beetle; Hypera punctata, clover leaf weevil; Diabrotica undecimpunctata howardi, spotted cucumber beetle; Russian wheat aphid; Schizaphis graminum, greenbug; Sitobion avenae, English grain aphid; Melanoplus femurrubrum, redlegged grasshopper; Melanoplus differentialis, differential grasshopper; Melanoplus sanguinipes, migratory grasshopper; Mayetiola destructor, Hessian fly; Sitodiplosis niosellana, wheat midge; Meromyza americana, wheat stem maggot; Hylemya coarctata, wheat bulb fly; Frankliniella jusca, tobacco thrips; Cephus cinctus, wheat stem sawfly; Eriophyes tulipae, wheat curl mite; Sunflower. Suleima helianthana, sunflower bud moth; Homeosoma ellectellum, sunflower head moth; Zygoramma exclamationis, sunflower beetle; Bothyrus gibbosus, carrot beetle; Neolasioptera mur∼feldtiana, sunflower seed midge; Cochylis hospes, banded sunflower moth; Rachiplusia nu, agentina looper; Smicronyx julvus, red sunflower seed weevil; Cylindrocopturus adspersus, spotted sunflower stem weevil; Cotton: Heliothis virescens, tobacco budworm; Helicoverpa zea, bollworm; Spodoptera exigua, beet armyworm; Pectinophora gossypiella, pink bollworm; Anthonomus grandis, boll weevil; Aphis gossypii, cotton aphid; Pseudatomoscelis seriatus, cotton fleahopper; Trialeurodes abutilonea, bandedwinged whitefly; Lygus lineolaris, tarnished plant bug; Melanoplus femurrubrum, redlegged grasshopper; Melanoplus differentialis, differential grasshopper; Thrips tabaci, onion thrips; Franklinkiella jusca, tobacco thrips; Tetranychus cinnabarinus, carmine spider mite; Tetranychus urticae, twospotted spider mite; Rice: Diatraea saccharalis, sugarcane borer; Spodoptera ftugiperda, fall armyworm; Helicoverpa zea, corn earworm; Colaspis brunnea, grape colaspis; Lissorhoptrus oryzophilus, rice water weevil; Sitophilus oryzae, rice weevil; Nephotettix nigropictus, rice leafhoper; Blissus leucopterus, chinch bug; Acrosternum hilare, green stink bug; Pseudoplusia includens, soybean looper; Anticarsia gemmatalis, velvetbean caterpillar; Plathypena scabra, green cloverworm; Ostrinia nubilalis, European corn borer; Agrotis ipsilon, black cutworm; Spodoptera exigua, beet armyworm; Heliothis virescens, cotton boll worm; Helicoverpa zea, cotton bollworm; Epilachna varivestis, Mexican bean beetle; Myzus persicae, green peach aphid; Empoasca jabae, potato leafhopper; Acrosternum hilare, green stink bug; Melanoplus femurrubrum, redlegged grasshopper; Melanoplus dififerentialis, differential grasshopper; Delia platura, seedcorn maggot; Sericothrips variabilis, soybean thrips; Thrips tabaci, onion thrips; Tetranychus turkestani, strawberry spider mite; Tetranychus urticae, twospotted spider mite; Barley. Ostrinia nubilalis, European corn borer; Agrotis ipsilon, black cutworm; Schizaphis graminum, greenbug; Blissus leucopterus, chinch bug; Acrosternum hilare, green stink bug; Euschistus servus, brown stink bug; Delia platura, seedcorn maggot; Mayetiola destructor, Hessian fly; Petrobia latens, brown wheat mite; Oil Seed Rgpe; Brevicoryne brassicae, cabbage aphid; Flea beetle, Phyllotreta spp.; Bertha Armyworm; Mamestra configurata; Diamondback Moth; Plutella xylostella; Alfalfa: alfalfa looper, Autographa californica; alfalfa snout beetle, Otiorhynchus ligusticii; alfalfa caterpillar, Colias eurytheme; alfalfa blotch leafrunner, Agronyza frontella; Egyptian alfalfa weevil, hypera brunneipeonis; meadow spittlebug, Philaerius spumarius; spotted alfalfa aphid, Theriophis meculata; clover leaf weevil, Hypera punctata; pea aphid, Acyrthosiphon pisum; blue alfalfa aphid, Acyrthosiphor kondoi; green cloverworm, Plathypena scabia; clover root curculio, Sitona hispidulus; alfalfa seed chalcid, Brachophagus roddi; tarnished plantbug, Lygus lineolaris; Say stink bug, Chlorochroa sayi; velvetbean caterpillar, Anticarsia ftiegiperda, alfalfa weevil, Hypera postica; fall annyworm, Spodoptera; potato leafhopper, Empoasca jabae; soybean looper, Psuedolusia includens; Three cornered alfalfa hopper, Spissistilus jestinus; etc. Additional insect pests are known in the art. See, for example, Manya B. Stoetzel (1989) Common Names of Insects & Related Organisms, Entomological Society of America, herein incorporated by reference.

In addition, polynucleotides or polypeptides, agonists or antagonists of the invention may be useful in detecting, preventing, and/or confering resistence to other plant pests - including, but not limited to, insects, herbacious species, fungi, bacteria, viruses, and other pests disclosed herein. For example, polynucleotides or polypeptides, agonists or antagonists of the invention may, either directly, or indirectly, modulate (preferably decrease) the biosynthesis, assimulation, and/or concentration of a nutrient essential for pest survival. Alternatively, polynucleotides or polypeptides, agonists or antagonists of the invention may modulate (preferably increase) the biosynthesis of a metabolite and/or protein capable of decreasing the biosynthesis, assimulation, and/or concentration of a nutrient essential for pest survival. One example concerns the observation that plants harboring the cholesteral oxidase gene were resistent to boll weevil (Anthonomus grandis grandis Boheman) larvae (Purcell, JP., Biochem Biophys Res Commun, 196(3):1406-13 (1993)). Since cholesterol is an essential nutrient for most organisms, including insects, polynucleotides or polypeptides, agonists or antagonists of the invention capable of modulating levels and/or activity of cholesteral degrading enzymes, including cholesteral oxidase, would be useful in confering resistence to plant pests. In another example, plants expressing certain latherogens have been shown to confer resitance to some pathogens. Such latherogens have been identified as being able to interfere with collagen synthesis and/or aggregation.

In another embodiment, the polynucleotides or polypeptides, agonists or antagonists of the invention, can be used, either directly or indirectly, to detect, prevent, and/or confer resistence to any of the pests and/or diseases described herein through direct topical application to the plants for which protection is desired. For example, several protein- and/or peptide-based commercial pesticides have recently been introduced into the marketplace that provide broad pesiticide resistance to pests when applied directly to the plant - Messenger, from Eden Bioscience, and Actigard, from Novartis Crop Protection (Nat. Biotech., 18:595 (2000)). Messenger is based upon the *Erwinia amylovara* harpin gene (Wei, Z., Science, 257:85-88 (1992)). Such protein/peptide-based pesticides have been shown to activate the systemic acquired resistance (SAR) pathway in plants. The invention encompasses additional modes of action for confering resistance to plant pests, though direct or indirect activation of the SAR pathway by the polynucleotides or polypeptides, agonists or antagonists of the invention, is preferred. The method of topical application, including any formulation requirements, will vary based the unique characterisitics of each polynucleotide or polypeptide, agonist or antagonist of the invention. In addition, polynucleotides or polypeptides, agonists or antagonists of the invention that provides plant pathogen resistance may also have other beneficial uses, such as, for example, enhancing plant growth.

### Plant Defense

A polypeptide or polynucleotide and/or agonist or antagonist of the present invention can be used to increase a plants defense mechanisms against either environmental or pathogenic stresses (e.g., viral, fungal, mycoplasma, bacterial, nematode, herbicidal, insecticidal, acid rain, drought, chemical, etc.). Such defense mechanisms may be a combination of structural characteristics (i.e., to serve as a physical barrier to inhibit a pathogen, for example, from entering or spreading throughout the plant), and biochemical reactions either on the scale of the whole plant or of individual cells (e.g., producing substances that are either toxic to the pathogen, or create an environment that is non-permissive for pathogen survival, etc.).

Structurally, a polypeptide or polynucleotide and/or agonist or antagonist of the present invention, may be useful for increasing the number of trichomes, increasing the thickness and/or composition of wax secretions or the waxy layer, increasing the thickness and/or composition of the cuticle, altering the structure of the epidermal cell wall, altering the size, shape, and/or location of the stomata and lenticels, inducing the plant to create or increase a layer of thick-walled cells (e.g., cork cell layer, etc.), increasing the thickness and/or composition of the outer epidermal cell wall, inducing the formation of an abscission layer, induce the formation of tyloses, induce the production and/or deposition of gums, inducing the thickening of the outer parenchyma cell layer of the cell wall, inducing the thickening of the cell wall, inducing the deposition of callose papillae in the inner layer of the cell wall, inducing a necrotic or hypersensitive defense reaction in cells and/or tissues (i.e., cell death), inducing the polymerization of oxidized phenolic compounds into lignin-like substances to structurally interefer with pathogen development, and/or inducing a cytoplasmic defense reaction.

Biochemically, a polypeptide or polynucleotide and/or agonist or antagonist of the present invention, may be useful for releasing pathogenic inhibitors into the plants environment, releasing fungitoxic exudates, and/or releasing phenolic compounds (e.g., protocatechioc acid, catechol, etc.). Alternatively, a polypeptide or polynucleotide and/or agonist or antagonist of the present invention, may be useful for increasing the synthesis of phenolic compounds (e.g., chlorogenic acids, caffeic acids, scopoletin, oxidation products of phenolic compounds, phytoalexins (see, Bell, et al., Ann. Rev. Plant Physiol, 32, 1981, for specific examples of phytoalexins), phaseolin, rishitin, kievitone, pisatin, glyceollin, gossypol, capsidiol, etc.), tannins, and/or saponins (e.g., tomatine, avenacin, etc.) within the cells and tissues of the plant. Alternatively, a polypeptide or polynucleotide and/or agonist or antagonist of the present invention, may be useful for increasing the expression of plant hydrolytic enzymes (e.g., glucanases, chitinases, etc.) that may cause degradation of the pathogen cell wall, etc.

In another embodiment, a polypeptide or polynucleotide and/or agonist or antagonist of the present invention, may be useful for inhibiting the expression of recognition factors essential for host-pathogen interaction (e.g., specific oligosaccarides, carbohydrate moieties, receptors, ligands, proteins, glycoproteins, lectins, etc.). For example, a polypeptide or polynucleotide and/or agonist or antagonist of the present invention, may be useful for inhibiting the expression of a protein that serves as a target for a pathogenic toxin, thus rendering the host in-sensitive to the toxin.

In another embodiment, a polypeptide or polynucleotide and/or agonist or antagonist of the present invention, may be useful in inhibiting the ability of the plants metabolic machinary to complete essential steps required for a competent pathogenic response (e.g., inhibiting the ability of plant ribosomes to recognize the pathogens nucleic acid, such as a viral nucleic acid; and/or inhibiting the ability of the plants DNA polymerase machninary to recognize and/or synthesize pathogenic DNA; or inhibiting the plants ability to catalyze a specific enzymatic step essential to eliciting a pathogenic response, etc.)

In yet another embodiment, a polypeptide or polynucleotide and/or agonist or antagonist of the present invention, may be useful for inhibiting either the production or transport or retention of essential nutrients required for a permissive pathogenic infection (e.g., inhibiting the transport of non-essential minerals or vitamins required for a pathogenic response, etc.).

In one embodiment of the invention, a polypeptide or polynucleotide and/or agonist or antagonist of the present invention, may be useful for increasing the expression or activity of phenol oxidizing enzymes (e.g., polyphenoloxidases, peroxidase, etc.), increasing the expression or activity of phenylalanine ammonia lyase, increasing the activity or expression of proteins capable of forming pectin salts or pectin complexes, etc.

In a further embodiment, a polypeptide or polynucleotide and/or agonist or antagonist of the present invention, may be useful for either directly or indirectly inhibiting the activity of a pathogenic protein essential to eliciting an infection (e.g., inhibiting the enzymatic activity of the protein, such as for a hydrolytic enzyme, for example, inhibiting the proteins ability to bind to a receptor or ligand, inhibiting protein-protein or protein-DNA interactions of the pathogenic protein, etc.). Specifically, a polypeptide or polynucleotide and/or agonist or antagonist of the present invention, may be useful for either directly or indirectly inhibiting wildfire toxin, chlorosis-inducing toxins, tabtoxin, phaseoloyoxin, rhizobitoxine, wilt-inducing bacterial polysaccarides, amylovorin, glycopeptide toxins, peptide toxins, syringomycin, tagetitoxin, helminthosporoside, victorin, helminthospoium maydis T-toxin, helminthospoium carbonum toxin, periconia circinata toxin, phyllosticta maydis toxin, alternaria toxins, fusarial wilt toxins, ophiobolin, helminthosporal, terpinoid toxins, fusicoccin, pyricularin, colletotin, alternaric acid, tentoxin, phytotoxins, zinniol, tentoxin, ascochitine, diaporthin, skyrin, Didymella applanata toxin, Myrothecium roridum toxin, Leptosphaerulina briosiana toxin, Alternaria tenuis phenolic toxins, Cercospora beticola toxin, Verticillium albo-atrum toxin, Phytophthora nicotianae var. parasitica toxin, Phytophthora megasperma var. sojae toxin, Ceratocystis ulmi toxins, peptidorhamnomannan, Stemphylium botryosum toxins, stemphylin, stemphyloxin, Pyrenophora teres toxins, N-(2-amino-2-carboxyethyl) aspartic acid, aspergillomarasmine A, and Rhynchosporosides toxins, for example.

In another embodiment, a polypeptide or polynucleotide and/or agonist or antagonist of the present invention, may be useful for either increasing or inducing the production of cyanogenic glycosides or esters, increasing the activity or expression of hydrolytic enzymes capable of hydrolyzing cyanogenic glycosides or esters, increasing the activity or expression of enzymes capable of releasing cyanide into plant cells and tissues, increasing the activity or expression of enzymes capable of detoxifying cyanide (e.g., formamide hydro-lyase, etc.) and/or increasing the expression of b-proteins, etc.

In yet a further embodiment, a polypeptide or polynucleotide and/or agonist or antagonist of the present invention, in addition to the non-coding 5' or 3' region of any of the polynucleotides of the present invention, and/or fragments thereof, may be useful for producing viral coat proteins, bacterial or fungal proteins, lipoproteins, polysaccarides, yeast RNA, polyanions, polyacrylic acid, salicyic acid, and/or 2-chloroethylphosphonic acid, in the plant, for example. Such proteins and/or compounds have been shown to elicite a local resistence response to plant pathogens when applied systemically or topically to the plant.

In another embodiment, a polypeptide or polynucleotide and/or agonist or antagonist of the present invention, may be useful for either increasing or inducing the production of secondary metabolites, which include, but are not limited to the following: acetyl salicylic acid, aconitine, atropine, cytisine, germinine, cardiac glycosides (e.g., calotropin, oleandrin, etc.), linarine, quinine, atropine, taxine, cicutoxin, hyoscyamine, pyrethrin, rotenone, camphor, etc.

In another embodiment, a polypeptide or polynucleotide and/or agonist or antagonist of the present invention, may be useful for either increasing or inducing the production of non-protein amino acids, which include, but are not limited to the following: b-cyanoalanine, azetidine 2-carboxylic acid, canavanine, 3,4-dihyroxyphenylalanine, etc.

In another embodiment, a polypeptide or polynucleotide and/or agonist or antagonist of the present invention, may be useful for either increasing or inducing the production of terpenes, which include, but are not limited to the following: 1.8 cineole, camphor, a-pinene, b-pinene, camphene, thujone, etc.

In yet another embodiment, a polypeptide or polynucleotide and/or agonist or antagonist of the present invention may either directly or indirectly inhibit the infectious agent, without necessarily increasing the plants defense mechanisms.

The present invention encompasses the application of one, two, three, four, or more, including any combination thereof, of any of the methods of increasing plant defense mechanisms against either an environmental or infectious agent described above and elsewere herein. Additional methods of increasing a plants defense mechanisms are known in the art. Additionally, a list of compounds and/or proteins that could serve as targets for increased production or expression by the use of a polynucleotide or polypeptide of the present invention to increase a plants defense mechanisms are known in the art (see, for example, Agrious, N.C., supra; Goodman, R.N., in "The Biochemistry and Physiology of Plant Disease", University of Missouri Press, Columbia, 1986; and Lambers, H., et al., in "Plant Physiological Ecology", Spinger-Verlag, New York, (1998); which are hereby incorporated herein by reference in their entirety).

### Plant Hormones

A polynucleotide or polypeptide and/or agonist or antagonist of the present invention can be used to modulate the hormone levels within a plant (including any of its cells, tissues, and/or organs, etc.). Examples of hormones that may be modulated by the present invention, either directly or indirectly, generally include, but are not limited to, the following: auxins, indoleacetic acid, gibberellins, cytokinins, ethylene, abscisic acid, polyamines, jasmonates, tuberonic acid, salicylic acid, systemin, brassinolides, zeatin; and specifically, indole-3-acetic acid, indole-3-butyric acid, 4-chloroindole-3-acetic acid, indole-3-acetyl-1-O-B-D-glucose, indole-3-acetyl-myo-inositol, jasmonic acid, methyl jasmonate, kinetin, including any known derivatives of the hormones described above, etc. In this context, modulate should be applied to mean a quantitative, or qualitative increase, decrease, induction, or termination, of the expression levels of any of the aforementioned hormones. Additional examples of plant hormones are known in the art (see, for example, Davies, P.J., in "Plant Hormones: Physiology, Biochemistry, and Molecular Biology", Kluwer Academic Publishers, Boston, 1995; which is hereby incorporated by reference in its entirety herein).

A polynucleotide or polypeptide and/or agonist or antagonist of the present invention capable of modulating plant auxin levels, would necessarily be capable of the following, non-limiting, effects on a plant: stimulating cell enlargement, stimulating stem growth, stimulating cell division in the cambium, stimulating differentiation of phloem and xylem, stimulating root initiation on stem cuttings, stimulating the development of branch roots, stimulating the differentiation of roots, mediating the bending (tropistic) response of shoots and roots to gravity and light, repression of lateral buds, delay of leaf senscence, inhibition or promotion of leaf and fruit abscission (via ethylene), induction of fruit setting and growth, enhancement of assimilate transport via phloem, delay of fruit ripening, promotion of flowering in Bromeliads, stimulating flower growth, promotion of femaleness in dioecious flowers, and stimulating the production of ethylene, for example.

A polynucleotide or polypeptide and/or agonist or antagonist of the present invention capable of modulating plant gibberellin levels, would necessarily be capable of the following, non-limiting, effects on a plant: stimulating cell division and cell elongation, inducing hyperelongation, inducing bolting, inducing stem elongation in response to long days, inducing germination, inducing germination in seeds in the absence of stratification or hardening, stimulating production of a-amylase, inducing fruit setting and growth, and inducing maleness in dioecious flowers, for example.

A polynucleotide or polypeptide and/or agonist or antagonist of the present invention capable of modulating plant cytokinin levels, would necessarily be capable of the following, non-limiting, effects on a plant: inducing cell division in the presence of auxin, inducing cell division in crown gall tumors, inducing cell division in apical meristem, inducing cell division in rapidly dividing cells, promoting shoot initiation, inducing bud formation, inducing growth of lateral buds, releasing lateral bud growth from apical dominance, inducing cell enlargement, inducing leaf expansion, enhancing stomatal opening, stimulating the accumulation of chlorophyll, inducing the conversion of etioplasts to chloroplasts, and delaying leaf senescence, for example.

A polynucleotide or polypeptide and/or agonist or antagonist of the present invention capable of modulating plant ethylene levels, would necessarily be capable of the following, non-limiting, effects on a plant: releasing the plant from dormancy, inducing shoot and root growth and differentiation, inducing adventitious root formation, inducing leaf and fruit abscission, inducing flowering, inducing femaleness in dioecious flowers, inducing flower opening, inducing flower and leaf senescence, and inducing fruit ripening, for example.

A polynucleotide or polypeptide and/or agonist or antagonist of the present invention capable of modulating plant abscisic acid levels, would necessarily be capable of the following, non-limiting, effects on a plant: inducing stomatal closure, inhibition of shoot growth, inducing storage protein synthesis in seeds, inhibition of a-amylase production in germinating cereal grains, induction of some aspects of dormancy, and induction of proteinase inhibitor synthesis, for example.

A polynucleotide or polypeptide and/or agonist or antagonist of the present invention capable of modulating plant polamine levels, would necessarily be capable of the following, non-limiting, effects on a plant: regulation of growth and development of plant cells and tissues, modulating the synthesis of macromolecules, modulating the activity of macromolecules, stabilizing cellular plasma membrane, decreasing leakage of betacyanin from wounded tissue, preservation of thylakoid strucuture in excised barley leaves, counteraction of hormone-induced affects on the cell membrane, binding to nucleic acids, protection of nucleic acids from alkylating agents, controlling chromosome condensation, controling nuclear membrane dissolution during pre-prophase, and modulating the structure and function of tRNA's, for example.

A polynucleotide or polypeptide and/or agonist or antagonist of the present invention capable of modulating plant jasmonate levels, would necessarily be capable of the following, non-limiting, effects on a plant: inhibition of plant growth, inhibition of seed germination, promotion of senescence, promotion of abscision, promotion of tuber formation, promotion of fruit ripening, promotion of pigment formation, promotion of tendril coiling, induction of proteinase inhibitors, and inhibit insect infestation, for example.

A polynucleotide or polypeptide and/or agonist or antagonist of the present invention capable of modulating plant salicylic acid levels, would necessarily be capable of the following, non-limiting, effects on a plant: induction of thermogenesis, providing resistence to pathogens via induction of pathogenesis related proteins, enhancement of flower longevity, inhibition of ethylene biosynthesis, inhibition of seed germination, inhibiting the wound response, couteracting the plants response to abscisic acid, for example.

A polynucleotide or polypeptide and/or agonist or antagonist of the present invention capable of modulating plant brassinosteroid levels, would necessarily be capable of the following, non-limiting, effects on a plant: promotion of stem elongation, inhibition of root growth, inhibition of root development, promotion of ethylene biosynthesis, and promotion of epinasty, for example.

The polynucleotides or polypeptide and/or agonist or antagonist of the present invention may modulate one, two, three, or more, or any combination of the above, hormones in a plant. Additional effects of hormones on a plant, including its cells, tissues, and organs are known in the art and the aforementioned plant hormone effects should not be construed as limiting the utility of any of the polynucleotide or polypeptides of the present invention.

### Regeneration

A polynucleotide or polypeptide and/or agonist or antagonist of the present invention can be used to differentiate, proliferate, and/or attract cells, leading to the regeneration of tissues. (See, Science 276:59-87 (1997).) The regeneration of tissues could be used to repair, replace, and/or protect tissue damaged by environmental insults (e.g., herbicidal, photobleaching, acid rain, drought, nematodes, insects, chemical, etc.), disease (e.g., fungal, viral, bacterial, mycoplasmal, etc.), necrosis, hypersensitive reaction, and/or cytokine damage.

Tissues that could be regenerated using polynucleotides or polypeptides of the present invention include tissues (e.g., apical meristem, lateral shoot, lateral bud, leaf, pith, vascular cambium, stem, phloem, xylem, cortex, epidermis, lateral root, root meristem, cuticle, etc.), in addition to cellular organelles and constituents (vacuole, mitochondrion, chloroplast, plastid, lysosomes, peroxisomes, glyoxysomes, cytoplasm, endoplasmic reticulum, ribosomes, vacuolar membrane, nucleus, nuclear membrane, plasmodesmata, scherosomes, microbodies, primary cell wall, etc.).

### Nutrients

A polynucleotide or polypeptide and/or agonist or antagonist of the present invention can be used to modulate the plants nutritional status through a number of different mechanisms. For example, A polynucleotide or polypeptide and/or agonist or antagonist of the present invention can be used to modulate the plants ability to retain a particular nutrient, to modulate the plants ability to synthesize a particular nutrient, to modulate the plants ability to assimilate a nutrient, to modulate the plants ability to absorb or uptake a particular nutrient, to modulate the plants ability to transport a particular nutrient, to modulate the plants ability to store a particular nutrient, to modulate the plants ability to survive under nutrient deficiencies, and to prevent, detect, and/or provide resistence to nutrient defiency symptoms and traits.

Specific examples of nutrients that may be modulated in a plant by a polynucleotide or polypeptide and/or agonist or antagonist of the present invention include the following, non-limiting, nutrients: carbon, hydrogen, oxygen, nitrogen, phosphorus, sulfur, potassium, calcium, magnesium, boron, chlorine, copper, iron, manganese, zinc, molybdenum, cobalt, selenium, silicon, sodium, nickel, water, carbon dioxide, in addition to metabolic by-products, etc. Additional nutrients essential to maintaining plant homeostasis are known in the art.

A polynucleotide or polypeptide and/or agonist or antagonist of the present invention capable of modulating plant boron levels, may be capable of preventing, detecting, alleviating, and/or confering resistence to the following, non-limiting, symptoms of plant boron deficiency: terminal leaf necrosis, premature leaf abscission layer formation, terminal shoot internode shortening, blackening and/or death of apical meristem tissue, shortening of root shoots, plant dwarfing, plant stunting, impairment of flower development, impairment of seed development, etc.

A polynucleotide or polypeptide and/or agonist or antagonist of the present invention capable of modulating plant calcium levels, may be capable of preventing, detecting, alleviating, and/or confering resistence to the following, non-limiting, symptoms of plant calcium deficiency: chlorotic leaves, leaf curling, leaf rolling, degradation of meristematic tissues in stems and roots, meristematic tissue death, decreased root development, decreased root fiber content, decreased fruit development, etc.

A polynucleotide or polypeptide and/or agonist or antagonist of the present invention capable of modulating plant chlorine levels, may be capable of preventing, detecting, alleviating, and/or confering resistence to the following, non-limiting, symptoms of plant chlorine deficiency: leaf tip wilting, leaf chlorosis, leaf bronzing, basipetal leaf necrosis proximal to areas of wilting, etc.

A polynucleotide or polypeptide and/or agonist or antagonist of the present invention capable of modulating plant copper levels, may be capable of preventing, detecting, alleviating, and/or confering resistence to the following, non-limiting, symptoms of plant copper deficiency: terminal shoot wilting, terminal shoot death, fading of leaf color, reduction of carotene in plant cells and tissues, reduction of other pigments in plant cells and tissues, etc.

A polynucleotide or polypeptide and/or agonist or antagonist of the present invention capable of modulating plant iron levels, may be capable of preventing, detecting, alleviating, and/or confering resistence to the following, non-limiting, symptoms of iron deficiency: interveinal white chlorosis of young leaves first, chlorisis of aerial tissues, aerial tissue necrosis, bleaching of leaves, scorching of leave margins and tips, etc.

A polynucleotide or polypeptide and/or agonist or antagonist of the present invention capable of modulating plant magnesium levels, may be capable of preventing, detecting, alleviating, and/or confering resistence to the following, non-limiting, symptoms of magnesium deficiency: mottling chlorosis with green veins and leaf web tissue yellow or white on old leaves first, wilting of leaves, formation of leaf ascission layer in the absence of the wilting stage, necrosis of plant cells and tissues, etc.

A polynucleotide or polypeptide and/or agonist or antagonist of the present invention capable of modulating plant manganese levels, may be capable of preventing, detecting, alleviating, and/or confering resistence to the following, non-limiting, symptoms of manganese deficiency: mottling chlorosis with green veins and leaf web tissue yellow or white on young leaves first, then spreading to old leaves, yellowish green stem, hardening and/or wooding of stem, reduction of carotene, etc.

A polynucleotide or polypeptide and/or agonist or antagonist of the present invention capable of modulating plant molybdenum levels, may be capable of preventing, detecting, alleviating, and/or confering resistence to the following, non-limiting, symptoms of molybdenum deficiency: light yellow chlorosis of leaves, failure of leaf blade expansion, etc.

A polynucleotide or polypeptide and/or agonist or antagonist of the present invention capable of modulating plant nitrogen levels, may be capable of preventing, detecting, alleviating, and/or confering resistence to the following, non-limiting, symptoms of nitrogen deficiency: stunting plant growth of young plants, yellowish green leaves in young plants, light green leaves in older leaves followed by yellowing and drying or shedding, increased accumulation of anthocyanins in veins, thin stem, spindely appearance of plant, reduced flowering, etc.

A polynucleotide or polypeptide and/or agonist or antagonist of the present invention capable of modulating plant phosphorus levels, may be capable of preventing, detecting, alleviating, and/or confering resistence to the following, non-limiting, symptoms of phosphorus deficiency: stunting of young plants, dark blue-green leaves with purplish undertones, slender stems, increased accumulation of anthocyanin in leaves, necrosis of leaves, cessation of meristematic growth, decreased rate of fruit ripening, plant dwarfing at maturity, etc.

A polynucleotide or polypeptide and/or agonist or antagonist of the present invention capable of modulating plant potassium levels, may be capable of preventing, detecting, alleviating, and/or confering resistence to the following, non-limiting, symptoms of potassium deficiency: dark green leaves, pale green monocotyledon leaves, yellowing streaking of monocoytledon leaves, marginal chlorosis of leaves, necrosis of leaves appearing first on old leaves, wrinkling of veins, currugating of veins, crinkling of veins, etc.

A polynucleotide or polypeptide and/or agonist or antagonist of the present invention capable of modulating plant sulfur levels, may be capable of preventing, detecting, alleviating, and/or confering resistence to the following, non-limiting, symptoms of sulfur deficiency: light green to yellow leaves appearing first along veins of young leaves, slender stems, etc.

A polynucleotide or polypeptide and/or agonist or antagonist of the present invention capable of modulating plant zinc levels, may be capable of preventing, detecting, alleviating, and/or confering resistence to the following, non-limiting, symptoms of zinc deficiency: chlorosis of leaves and/or necrosis of leaves affecting young leaves first, rosetting, premature formation of ascission layer of leaves, whitish chlorotic streaks between veins in older laves, whiting of upper leaves in monocotyledons, chlorosis of lower leaves in dicotyledons, etc.

Additional symptoms of plant nutrient deficiencies are known in the art (see for example, Noggle, G.R., and Fritz, G.J., in "Introductory Plant Physiology", 2nd edition, Prentice-Hall, Inc., Englewood Cliffs, 1983).

In a specific embodiment, a polynucleotide or polypeptide and/or agonist or antagonist of the present invention may be able to modulate plant nutrient levels either directly or indirectly by increasing the activity, kinetics, and/or expression of transport proteins, ion channels, and/or ion carrier proteins.

In another embodiment, a polynucleotide or polypeptide and/or agonist or antagonist of the present invention may be able to modulate plant nutrient levels by either directly or indirectly increasing or inducing the secretion of mineral solubilizing or mineral stabilizing compounds or chelating compounds (e.g., citric acid, malic acid, pisidic acid, etc.). Alternatively, the secreted compound may be an organic chelating compound (e.g., phytometallophore, see for example, Cakmak et al., Plant Soil, 180:183-189, (1996)). Alternatively, the secreted compound is a root exudate, such as an organic acid (e.g., lactic, acetic, formic, pyruvic, succinic, tartaric, oxalic, citric, isocitric, aconitic, etc.), carbohydrate, amino acid, or polysaccaride capable of assimilating carbon (see, for example, Paul, E.A., and Clark, F.E., in "Soil microbiology and biochemistry", Academic Press, San Diego, (1989)).

In another embodiment, a polynucleotide or polypeptide and/or agonist or antagonist of the present invention may be able to modulate plant nutrient levels by modulating, either directly or indirectly, the activity, kinetics, and/or expression of phosphatase enzymes, nitrate reductase enzymes, citrate synthesis enzymes, etc.

In another embodiment, a polynucleotide or polypeptide and/or agonist or antagonist of the present invention may be able to modulate plant nutrient levels by modulating the active transport and/or passive transport mechanisms of the plant. Alternatively, a polynucleotide or polypeptide and/or agonist or antagonist of the present invention may be able to modulate plant nutrient levels by modulating the inter- and intra-tissue and/or cellular transport of nutrients in the plant (e.g., transport through the phloem, xylem, desmosomes, etc.). Additional mechanisms of modulating plant nutrient transport are known in the art (see, for example, Lambers, H., et al., in "Plant Physiological Ecology", Spinger-Verlag, New York, (1998); which is hereby incorporated herein by reference in its entirety).

### Biotic Associations

A polynucleotide or polypeptide and/or agonist or antagonist of the present invention may increase the plants ability, either directly or indirectly, to initiate and/or maintain biotic associations with other organisms. Such associations may be symbiotic, nonsymbiotic, endosymbiotic, macrosymbiotic, and/or microsymbiotic in nature. In general, a polynucleotide or polypeptide and/or agonist or antagonist of the present invention may increase the plants ability to form biotic associations with any member of the fungal, bacterial, lichen, mycorrhizal, cyanobacterial, dinoflaggellate, and/or algal, kingdom, phylums, families, classes, genuses, and/or species.

Specific, non-limiting, examples of organisms known to form biotic associations with a plant are: ectomycorrhizas (e.g., members of the Diperocarpaceae, Pinaceae, Fagaceae, Myrtaceae, Salicaceae, Betulaceae, Fabaceae, etc.), endomycorrhizas, vesicular arbuscular mycorrhiza (e.g., members of the Glomales), nonmycorrhizal (e.g., members of tge Brassicaceae, Caryophyllaceae, Chenopodiaceae, Lecythideceae, Proeaceae, Restionaceae, Sapotaceae, Urticaceae, Zygophyllaceae, etc.), symbiotic N2 fixing organisms (e.g., members of the Rhizobium, Bradyrhizobium, Sinorhizobium, Mesorhizobium, Azorhizobium, etc.), nonsybiotic N2 fixing organisms (e.g., Azospirillum, etc.), endosymbiontic organisms (e.g., members of the Clavicipitaceae, Ascomycetes, etc.), etc. Addional organisms capable of forming biotic associations with plants are known in the art and are encompassed by the invention (see, for example, Lambers, H., et al., in "Plant Physiological Ecology", Spinger-Verlag, New York, (1998); Raven, P.H., et al., in "Biology of Plants", 5th Edition, Worth Publishers, New York, (1992); which are hereby incorporated herein by reference in its entirety

The mechanism by which a polynucleotide or polypeptide and/or agonist or antagonist of the present invention may increase the plants ability, either directly or indirectly, to initiate and/or maintain biotic associations is variable, though may include, modulated secretions of organic acids, phenolic compounds, nutrients, or the increased expression of a protein required for host-biotic organisms interactions (e.g., a receptor, ligand, etc.). Additional mechanisms are known in the art and are encompassed by the invention.

In an alternative embodiment, a polynucleotide or polypeptide and/or agonist or antagonist of the present invention may decrease a plants ability to form biotic associations with a plant. Such a decrease may be due to increasing the plants ability to utilize, obtain, store, and/or synthesize essential nutrients that the biotic organism supplied to the plant through the association.

Mechanisms by which a plant could inhibit colonization of biotic associating organisms are known in the art (see, for example, Smith, S.E., and Read, D.J., in "Mycorrhizal Symbiosis", Academic Press, London, 1997, etc.). For example, in low phosphorus containing soils, a plant may form beneficial symbiosis colonization with a species capable of providing phosphorus. However, in soils of high phosphorus content, such colonization could be inhibited by the plant. Thus, a polynucleotide or polypeptide of the invention could increase the plants ability to live in phosphorus depleted soils by enabling the plant to assimilate phosphorus through a mechanism previously not endogenous to the plant, for example.

### Pheromones

In another embodiment, a polynucleotide or polypeptide and/or agonist or antagonist of the present invention may increase the plants ability to synthesize and/or release a pheromone. Such a pheromone may attract predatory organisms to the plant that may feed on plant pests or infectious agents. For example, recent studies of the aphid Acyrthosiphon pisum have shown that feeding by the aphid alters the composition of volatiles released by the plant and that these compounds act as synomones for the aphids foraging parasitoid, Aphidius ervi. Additional studies have shown that other pheromones released by the plant in response to aphid foraging, namely(E)-beta-farnesene, may also attract other aphid predators, such as the lacewing Chrysoperla carnea and the seven-spot ladybird, Coccinella septempunctata.

A polynucleotide or polypeptide and/or agonist or antagonist of the present invention may modulate the biosynthesis and/or release of pheromones, volatiles having pheromone-like effects, volatiles having kairomone effects, volatiles having synomones effects, and/or may releases (E)-beta-famesene specifically. Alternatively, a polynucleotide or polypeptide and/or agonist or antagonist of the present invention may modulate the release of pheromones, indirectly, through the induction of the following, non-limiting, compounds, compounds from the octadecanoid signalling pathway, structurally non-related amino acid conjugates such as the bacterial phytotoxin coronatine, the synthetic indanoyl-isoleucine, or amino acid conjugates of linolenic, and/or volatile terpenoids, such as members of the sesqui- and diterpenoids. Preferably, any of the pheromones, and/or volatiles released from the plant, or induced, by a polynucleotide or polypeptide and/or agonist or antagonist of the invention have behavioral effects on predators and/or pests of plants. Other examples of known pheromones or volatiles released by a plant having behavioral modulating effects on plant pest predators and/or plant pests are known in the art (see, Boland W., et al., Novartis Found Symp, 223:110-26 (1999); Wadhams LJ., Novartis Found Symp, 223:60-7 (1999); Tumlinson JH, et al., Novartis Found Symp, 223:95-105 (1999)).

In yet another embodiment, a polynucleotide or polypeptide and/or agonists or antagonists of the present invention may modulate the biosynthesis of furanones. Furonones are naturally occuring compounds found in a variety of plants that have been shown to have pheromone-like effects, antibacterial effects, anti-viral effects, etc., on a variety of organisms. For example, 5-Methyl-4-hydroxy-3(2H)-furanone is a male pheromone in the cockroach Eurycolis florionda (Walker) and the 2,5-dimethyl derivative deters fungal growth on strawberries and is an important component of the attractive aroma of the fruit. The red seaweed Delisea pulchra (Greville) Montagne produces a range of brominated furanones which prevent colonisation of the plant by bacteria by interfering with the acylated homoserine lactone (AHL) signalling system used by the bacteria for quorum sensing. In addition, furonones have been shown to have mutagenic properties in bacteria and viruses, and thus could serve as anti-bacterials and anti-virals (Colin, Slaughter J, Biol Rev Camb Philos Soc, 74(3):259-76 (1999).

### Chemotaxis

A polynucleotide or polypeptide and/or agonist or antagonist of the present invention may have chemotaxis activity. A chemotaxic molecule attracts or mobilizes cells to a particular site in the plant or animal body, such as inflammation, infection, or site of hyperproliferation. The mobilized cells can then fight off and/or heal the particular trauma or abnormality.

A polynucleotide or polypeptide and/or agonist or antagonist of the present invention may increase chemotaxic activity of particular cells. These chemotactic molecules can then be used to detect, prevent, and/or alleviate inflammation, infection, hyperproliferative diseases, disorders, and/or conditions, or any plant disorder by increasing the number of cells targeted to a particular location in the plants body. For example, chemotaxic molecules can be used to treat, prevent, and/or diagnose wounds and other trauma to tissues by attracting cells to the injured location.

It is also contemplated that a polynucleotide or polypeptide and/or agonist or antagonist of the present invention may inhibit chemotactic activity. These molecules could also be used to detect, prevent, and/or alleviate diseases, disorders, and/or conditions. Thus, a polynucleotide or polypeptide and/or agonist or antagonist of the present invention could be used as an inhibitor of chemotaxis.

In an alternative embodiment, a polynucleotide or polypeptide and/or agonist or antagonist of the present invention may modulate the ability of a plant to sense the presence of plant neighbors (i.e., chemoperception). Such chemoperceptive modulation may come in the form of sensing the presence of plant hormones (e.g., jasmonate, etc.), heat exchange differential, and/or allelochemical detection (see, for example, Boller, Ann. Rev. Plant Physiol. Plant Mol. Biol., 46:189-214, (1995)).

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells that express the polypeptide, either as a secreted protein or on the cell membrane. Preferred cells include cells from plants, yeast, or E. coli. Cells expressing the polypeptide (or cell membrane containing the expressed polypeptide) are then preferably contacted with a test compound potentially containing the molecule to observe binding, stimulation, or inhibition of activity of either the polypeptide or the molecule. Moreover, the identification of such molecules may be obtained through the application of the yeast 2 or 3 hybrid system (see for example, Wallach D, et al., Curr Opin Immunol., 10(2):131-6, (1998); Young KH., Biol Reprod., 58(2):302-11, (1998); and Fernandes, PB., Curr Opin Chem Biol., 2(5):597-603 (1998); which are hereby incorporated by reference in their entirety herein, including the methods disclosed and the references cited therein). Further, the identification of such molecules may be obtained through the application of additional screening technologies which include, but are not limited to, the following issued US Patents: 5,284,746; 5,576,210; 5,691,188; 5,846,819; and International Publication No. WO 95/34646; which are hereby incorporated by reference in their entirety herein, including the methods disclosed and references cited therein.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

As an alternative approach for receptor identification, the labeled polypeptides can be photoaffinity linked with cell membrane or extract preparations that express the receptor molecule. Cross-linked material is resolved by PAGE analysis and exposed to X-ray film. The labeled complex containing the receptors of the polypeptides can be excised, resolved into peptide fragments, and subjected to protein microsequencing. The amino acid sequence obtained from microsequencing would be used to design a set of degenerate oligonucleotide probes to screen a cDNA library to identify the genes encoding the putative receptors.

Moreover, the techniques of gene-shuffling, motif-shuffling, exon-shuffling, and/or codon-shuffling (collectively referred to as "DNA shuffling") may be employed to modulate the activities of polypeptides of the invention thereby effectively generating agonists and antagonists of polypeptides of the invention. See generally, U.S. Patent Nos. 5,605,793, 5,811,238, 5,830,721, 5,834,252, and 5,837,458, and Patten, P. A., et al., Curr. Opinion Biotechnol. 8:724-33 (1997); Harayama, S. Trends Biotechnol. 16(2):76-82 (1998); Hansson, L. O., et al., J. Mol. Biol. 287:265-76 (1999); and Lorenzo, M. M. and Blasco, R. Biotechniques 24(2):308-13 (1998) (each of these patents and publications are hereby incorporated by reference). The techniques of DNA shuffling are known in the art and more particularly described elsewere herein.

All of the assays referenced above, and elsewere herein, can be used as diagnostic or prognostic markers. The molecules discovered using these assays may be used to detect, prevent, and/or confer resistence to a disease or to bring about a particular result in an organism (e.g., vessel growth, etc.) by activating or inhibiting the polypeptide/molecule. Moreover, the assays can discover agents which may inhibit or enhance the production of the polypeptides of the invention from suitably manipulated cells or tissues.

Therefore, the invention includes a method of identifying compounds which bind to the polypeptides of the invention comprising the steps of: (a) incubating a candidate binding compound with the polypeptide; and (b) determining if binding has occurred. Moreover, the invention includes a method of identifying agonists/antagonists comprising the steps of: (a) incubating a candidate compound with the polypeptide, (b) assaying a biological activity , and (b) determining if a biological activity of the polypeptide has been altered.

### Antisense and Ribozyme (Antagonists)

In preferred embodiments, the invention encompasses antagonists which correspond to the polynucleotide sequences shown in SEQ ID NO:X, the complimentary strand thereof, and/or to the polynucleotide sequences contained within the deposited clone. Antisense technology results in modulation (i.e., complete or partial inhibition), of the expression of a particular protein through direct inhibition of the proteins mRNA. Antisense nucleic acids may be in the form of DNA, RNA, PNA, triple helix, quad helix, a chimeric mixture of any of these aforementioned types (e.g., DNA:RNA, PNA:RNA, PNA:DNA, etc.), and may be single or double stranded. Antisense nucleic acids modulate gene expression by binding to the RNA of the gene of interest, effectively inhibiting translation. Such intereactions may rely follow typical Watson-Crick base pair recognition, or the case of a triple or quad helix, may rely upon Hoogsteen basepair recognition.

The antisense nucleic acids may be transiently generated within the organism (e.g., sequence contained within an inducible or constitutively expressed vector introduced into the cells of an organism), stably generated within the organism (e.g., sequence contained within an inducible or constitutively expressed vector introduced into the cells of an organism using transgenic methods, including viral integration, etc.) or may be exogenously administered. For a nucleic acid to serve an antisense role, it is only necessary that it has sequence homology to the sense RNA product of the gene of interest. A number of methods of administering antisense nucleic acids, their compositions, and designs are known in the art and encompassed by the invention (see for example, Agrawal S, et al., Mol Med Today. 2000 Feb;6(2):72-81; Yacyshyn BR, et al, Can J Gastroenterol. 1999 Nov;13(9):745-51; Mrsny RJ., J Drug Target. 1999;7(1):1-10; Toulme JJ, et al, Nucleic Acids Symp Ser. 1997;(36):39-41.), Okano, Neurochem., 56:560 (1991); Oligodeoxynucleotides as Antisense Inhibitors of Gene Expression, CRC Press, Boca Raton, FL (1988); and Cooper SR, et al., Pharmacol Ther. 1999 May-Jun;82(2-3):427-35). Likewise, a number of methods have been developed regarding the application of triple helix antisense technology to modulating gene expression (see, for example, Gowers DM, et al, Nucleic Acids Res. 1999 Apr 1;27(7):1569-77; and Chan PP, et al., J Mol Med. 1997 Apr;75(4):267-82).

Antisense technology has wide-ranging applications in plants. For example, antisense RNA has been shown to effectively downregulate a variety of plant genes as described by Shimada, et al., Theor. Appl. Genet., 86:665-672, (1993); Kull, et al., J. Genet. Breed., 49:67-76, (1995)., Slabas and Elborough, WO 97/07222; Knutzon et al., Proc. Natl. Acad. Sci. USA, 89:2624-2628, (1992), and Baulcombe DC., Plant Mol Biol. 1996 Oct;32(1-2):79-88)..

The antisense nucleic acids of the invention comprise a sequence complementary to at least a portion of an RNA transcript of a gene of interest. However, absolute complementarity, although preferred, is not required. A sequence "complementary to at least a portion of an RNA," referred to herein, means a sequence having sufficient complementarity to be able to hybridize with the RNA, forming a stable duplex; in the case of double stranded antisense nucleic acids of the invention, a single strand of the duplex DNA may thus be tested, or triplex formation may be assayed. The ability to hybridize will depend on both the degree of complementarity and the length of the antisense nucleic acid Generally, the larger the hybridizing nucleic acid, the more base mismatches with a RNA sequence of the invention it may contain and still form a stable duplex (or triplex as the case may be). One skilled in the art can ascertain a tolerable degree of mismatch by use of standard procedures to determine the melting point of the hybridized complex.

Antisense oligonucleotides that are complementary to the 5' end of the message, e.g., the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. See generally, Wagner, R., Nature, 372:333-335 (1994). Thus, oligonucleotides complementary to either the 5' - or 3' - non-translated, non-coding regions of a polynucleotide sequence of the invention could be used in an antisense approach to inhibit translation of endogenous mRNA. Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Whether designed to hybridize to the 5' -, 3' - or coding region of mRNA, antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

The antisense oligonucleotide may be modified at the base moiety, sugar moiety, or phosphate backbone. The oligonucleotide may include other appending groups such as peptides, or agents facilitating transport across the cell membrane (see, e.g., Letsinger et al., 1989, Proc. Natl. Acad. Sci. USA 86:6553-6556; Lemaitre et al., 1987, Proc. Natl. Acad. Sci.84:648-652; PCT Publication No. WO 88/09810, published Dec. 15, 1988) or blood-brain barrier (see, e.g., PCT Publication No. WO 89/10134, published Apr. 25, 1988), hybridization-triggered cleavage agents (see, e.g., Krol et al., 1988, BioTechniques 6:958-976) or intercalating agents (see, e.g., Zon, 1988, Pharm. Res. 5:539-549).

The oligonucleotide can also be a peptido-nucleic acid ("PNA") based on a linked N-(2-aminoethyl)glycine backbone to which normal DNA bases have been attached (Egholm et al., 1993, Nature 365:566-67). This PNA obeys specific Watson-Crick base pairing, but with greater free energy of binding and correspondingly higher melting temperatures. Suitable oligomers may be constructed entirely from PNAs or from mixed PNA and DNA and/or RNA oligomers. In fact, PNA:DNA chimeras have increased solubility characteristics, as compared to DNA:DNA or DNA:RNA chimeras of the same sequence. Most notably, PNAs have the unique ability to displace one strand of a DNA double-helix thus making them highly suitable in antisense applications (Uhlmann E., Biol Chem. 1998 Aug-Sep;379(8-9):1045-52).

In a preferred embodiment, the oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In another embodiment, the anti-sense oligonucleotide of the invention may comprise at least one modified base moiety which is selected from the group including, but not limited to, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, 3-(3-amino-3-N-2-carboxypropyl) uracil, and 2,6-diaminopurine.

In another embodiment, the anti-sense oligonucleotide comprises at least one modified sugar moiety selected from the group including, but not limited to, arabinose, 2-fluoroarabinose, xylulose, and hexose.

In yet another embodiment, the anti-sense oligonucleotide may be conjugated to another molecule, e.g., a peptide, hybridization triggered cross-linking agent, transport agent, hybridization-triggered cleavage agent, etc.

The anti-sense oligonucleotides of the invention may be synthesized by standard methods known in the art, e.g., by use of an automated DNA synthesizer (such as are commercially available from Biosearch, Applied Biosystems, etc.). As examples, phosphorothioate oligos may be synthesized by the method of Stein et al. (1988, Nucl. Acids Res. 16:3209), methylphosphonate oligos can be prepared by use of controlled pore glass polymer supports (Sarin et al., 1988, Proc.Natl. Acad. Sci. USA 85:7448-7451), etc.

In a specific embodiment, the oligonucleotide comprises catalytic RNA, or a ribozyme (see, e.g., PCT International Publication WO 90/11364, published Oct. 4, 1990; Sarver et al., 1990, Science 247:1222-1225; Hasselhoff, et al., Nature 342:76-79 (1988)). Ribozymes have been used to downregulate gene expression, and more recently in the downregulation of plant proteins (seem e.g., PCT International Publication WO 97/10328). In another embodiment, the oligonucleotide is a 2'-0-methylribonucleotide (Inoue et al., 1987, Nucl. Acids Res. 15:6131-6148), or a chimeric RNA-DNA analogue (Inoue et al., 1987, FEBS Lett. 215:327-330).

### Other Activities

In another embodiment, the polypeptides, polynucleotides encoding these polypeptides, variants, and/or fragments thereof, may be used to inhibit gene expression using co-suppression methodology. The mechanism of co-suppression is not known, though its application to inhibiting plant gene expression has been documented and described (e.g., Seymour, et al., Plant. Mol. Biol., 23:1-9, (1993), Brusslan, et al., Plant Cell, 5:667-677, (1993),; Vaucheret, et al., Mol. Gen. Genet., 248:311-317, (1995); and Jorgensen, et al., Plant Mol. Biol., 31:957-973, (1996)). Co-suppression involves creating a consititutively expressed vector construct comprising, for example, a CaMV 35S promoter, the 5' coding region of a first gene (R) for which inhibited expression is desired, in-frame and upstream from, the entire coding region of a second gene (S) for which inhibited expression is desired, and a terminator. Upon positive transformation of plants with this vector (i.e., transgenic plants), no detectable mRNA expression will be detected for either R nor S (see Seymour, supra).

The polypeptide or polynucleotides and/or agonist or antagonists of the present invention may also increase or decrease the differentiation or proliferation of protoplast cells, emyloblast cells, etc.

The polypeptide or polynucleotides and/or agonist or antagonists of the present invention may be useful in modulating programmed cell death in plants, plant cells, and eukaryotic cells and organisms, in general. Such modulation would either be through direct or indirect interaction between a polypeptide or polynucleotides and/or agonist or antagonists of the present invention with the gene or protein critical in modulating programmed cell death in the organism. Specific targets of interaction for program cell death, particularly in a plant, are provided in International Publication Number WO 00/04173 (e.g., poly-ADP-ribose polymerase (PARP) genes, specifically PARP genes of the ZAP class, etc.).

The polypeptide or polynucleotides and/or agonist or antagonists of the present invention may also be used to modulate plant characteristics, such as stem height, pigmentation, stress tolerance, etc. Similarly, polypeptides or polynucleotides and/or agonist or antagonists of the present invention may be used to modulate plant metabolism affecting catabolism, anabolism, processing, utilization, and storage of energy.

Polypeptide or polynucleotides and/or agonist or antagonists of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the "5' NT of Clone" and ending with the nucleotide at about the position of the "3' NT of Clone" as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the "5' NT of Start Codon of Clone ORF" and ending with the nucleotide at about the position of the "3' NT of Clone ORF" as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the "5' NT of the First AA of the Signal Peptide" and ending with the nucleotide at about the position of the "3' NT of Clone ORF" as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the "5' NT of Clone ORF" and ending with the nucleotide at about the position of the "3' NT of Clone ORF" as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said cDNA clone.

Also preferred is an isolated nucleic acid molecule comprising a nucleotide sequence which is at least 95% identical to sequence of at least 150 contiguous nucleotides in the nucleotide sequence encoded by said cDNA clone.

A further preferred embodiment is an isolated nucleic acid molecule comprising a nucleotide sequence which is at least 95% identical to sequence of at least 500 contiguous nucleotides in the nucleotide sequence encoded by said cDNA clone.

A further preferred embodiment is an isolated nucleic acid molecule comprising a nucleotide sequence which is at least 95% identical to the complete nucleotide sequence encoded by said cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions "Total AA of the Open Reading Frame (ORF)" as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a protein encoded by a cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of the protein encoded by a cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the protein encoded by a cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the protein encoded by a cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the protein encoded by a cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing a pathological condition associated with an organism with abnormal structure or expression of a gene encoding a protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a protein encoded by a cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule(s) into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a protein comprising an amino acid sequence selected from the group consisting of: an amino acid sequence of SEQ ID NO:Y wherein Y is an integer set forth in Table 1 and said position of the "Total AA of ORF" of SEQ ID NO:Y is defined in Table 1; and an amino acid sequence of a protein encoded by a cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a protein activity, which method comprises administering to such an individual a pharmaceutical composition comprising an amount of an isolated polypeptide, polynucleotide, or antibody of the claimed invention effective to increase the level of said protein activity in said individual.

The above-recited applications have uses in a wide variety of hosts. Such hosts include, but are not limited to, barley, oats, rye, sorghum, pea, sunflower, tobacco, cotton, petunia, tomato, broccoli, lettuce, apple, plum, orange, and lemon, and more preferrably rice, maize, conola, wheat, sugerbeet, sugercane, and soybean, in addition to other hosts referenced elsewhere herein.

Having generally described the invention, the same will be more readily understood by reference to the following examples, which are provided by way of illustration and are not intended as limiting.

### Examples

### Description of the Preferred Embodiments

### Example 1 - Growth of Physcomitrella patens cultures

For this study, plants of the species *Physcomitrella patens* (Hedw.) B.S.G. from the collection of the genetic studies section of the University of Hamburg were used. They originate from the strain 16/14 collected by H.L.K. Whitehouse in Gransden Wood, Huntingdonshire (England), which was subcultured from a spore by Engel (1968, Am J Bot 55, 438-446). Proliferation of the plants was carried out by means of spores and by means of regeneration of the gametophytes. The protonema developed from the haploid spore as a chloroplast-rich chloronema and chloroplast-low caulonema, on which buds formed after approximately 12 days. These grew to give gametophores bearing antheridia and archegonia. After fertilization, the diploid sporophyte with a short seta and the spore capsule resulted, in which the meiospores mature.

Culturing was carried out in a climatic chamber at an air temperature of 25°C and light intensity of 55 micromols^{-1m2} (white light; Philips TL 65W/25 fluorescent tube) and a light/dark change of 16/8 hours. The moss was either modified in liquid culture using Knop medium according to Reski and Abel (1985, Planta 165, 354-358) or cultured on Knop solid medium using 1% oxoid agar (Unipath, Basingstoke, England).

The protonemas used for RNA and DNA isolation were cultured in aerated liquid cultures. The protonemas were comminuted every 9 days and transferred to fresh culture medium.

### Example 2 - Isolation of total RNA and poly-(A)+ RNA and cDNA library construction from Physcomitrella patens

For the investigation of transcripts, both total RNA and poly-(A)⁺ RNA were isolated. The total RNA was obtained from wild-type 9d old protonemata following the GTC-method (Reski et al. 1994, Mol. Gen. Genet., 244:352-359).

The Poly(A)+ RNA was isolated using Dyna Beads^{R} (Dynal, Oslo, Norway) following the instructions of the manufacturers protocol. After determination of the concentration of the RNA or of the poly(A)+ RNA, the RNA was precipitated by addition of 1/10 volumes of 3 M sodium acetate pH 4.6 and 2 volumes of ehanol and stored at -70 degree C.

**RNA preparation from *Arabidiopsis* seeds - "hot" extraction:**
1. **Buffers, enzymes and solutions**
   - 2M KCI
   - Proteinase K
   - Phenol (for RNA)
   - Chloroform:Isoamylalcohol
      (Phenol:choloroform 1:1; pH adjusted for RNA)
   - 4 M LiCl, DEPC-treated
   - DEPC-treated water
   - 3M NaOAc, pH 5, DEPC-treated
   - Isopropanol
   - 70% ethanol (made up with DEPC-treated water)
   - Resuspension buffer: 0.5% SDS, 10 mM Tris pH 7.5, 1 mM EDTA made up with DEPC-treated water as this solution can not be DEPC-treated
   - Extraction Buffer:

   0.2M Na Borate
   30 mM EDTA
   30 mM EGTA
   1% SDS * (250µl of 10% SDS-solution for 2.5ml buffer)
   1% Deoxycholate (25mg for 2,5ml buffer)
   2% PVPP (unsoluable - 50mg for 2.5ml buffer)
   2% PVP 40K (50mg for 2.5ml buffer)
   10 mM DTT *
   100 mM □-Mercaptoethanol * (fresh, handle under fume hood - use 35µl of 14.3M solution for 5ml buffer)
**2. Extraction**
   Heat extraction buffer up to 80°C. Grind tissue in liquid nitrogen-cooled mortar, transfer tissue powder to 1,5ml tube. Tissue should kept frozen until buffer is added so transfer the sample with precooled spatula and keep the tube in liquid nitrogen all time. Add 350µl preheated extraction buffer (here for 100mg tissue. Buffer volume can be as much as 500µl for bigger samples) to tube, vortex and heat tube to 80°C for ∼1 min. Keep then on ice.Vortex sample, grind additionally with electric mortar.
**3. Digestion**
   Add Proteinase K (0.15mg/100mg tissue), vortex and keep at 37°C for one hour.
**4. First Purification**
   Add 27µl 2M KCI. Chill on ice for 10 min. Centrifuge at 12.000 rpm for 10 minutes at room temperature. Transfer supernatant to fresh, RNAase-free tube and do one phenol extraction, followed by a choloroform:isoamylalcohol extraction. Add 1 vol. isopropanol to supernatant and chill on ice for 10 min. Pellet RNA by centrifugation (7000 rpm for 10 min at RT). Resolve pellet in 1ml 4M LiCl by 10 to 15min vortexing. Pellet RNA by 5min centrifugation.
**4. Second Purification**
   Resuspend pellet in 500µl Resuspension buffer. Add 500µl phenol and vortex. Add 250µl chloroform:isoamylalcohol and vortex. Spin for 5 min. and transfer supernatant to fresh tube. Repeat choloform:isoamylalcohol extraction until interface is clear. Transfer supernatant to fresh tube and add 1/10 vol 3M NaOAc, pH 5 and 600µl isopropanol. Keep at -20 for 20 min or longer. Pellet RNA by 10 min centrifugation. Wash pellet once with 70% ethanol. Remove all remaining alcohol before resolving pellet with 15 to 20µl DEPC-water. Determine quantity and quality by measuring the absorbance of a 1:200 dilution at 260 and 280nm. 40µg RNA/ml = 1OD260 For cDNA library construction first strand synthesis was achieved using Murine Leukemia Virus reverse transcriptase (Roche, Mannheim, Germany) and oligo-d(T)-primers, second strand synthesis by incubation with DNA polymerase I, Klenow enzyme and RNAseH digestion at 12 degree C (2h), 16 degree C (1h) and 22 degree C (1h). The reaction was stopped by incubation at 65 degree C (10 min) and subsequently transferred to ice. Double stranded DNA molecules were blunted by T4-DNA-polymerase (Roche, Mannheim) at 37 degree C (30 min). Nucleotides were removed by phenol/chloroform extraction and Sephadex G50 spin columns. EcoRI adapters (Pharmacia, Freiburg, Germany) were ligated to the cDNA ends by T4-DNA-ligase (Roche, 12 degree C, overnight) and phosphorylated by incubation with polynucleotide kinase (Roche, 37 degree C, 30 min). This mixture was subjected to separation on a low melting agarose gel. DNA molecules larger than 300 basepairs were eluted from the gel, phenol extracted, concentrated on Elutip-D-columns (Schleicher and Schuell, Dassel, Germany) and were ligated to vector arms and packed into lambda ZAPII phages or lambda ZAP-Express phages using the Gigapack Gold Kit (Stratagene, Amsterdam, Netherlands) using material and following the instructions of the manufacturer.

### Example 3 - Sequencing and function annotation of Physcomitrella patens ESTs

cDNA libraries as described in Example 2 were used for DNA sequencing according to standard methods, in particular by the chain termination method using the ABI PRISM Big Dye Terminator Cycle Sequencing Ready Reaction Kit (Perkin-Elmer, Weiterstadt, Germany). Random Sequencing was carried out subsequent to preparative plasmid recovery from cDNA libraries via in vivo mass excision, retransformation, and subsequent plating of DH10B on agar plates (material and protocol details from Stratagene, Amsterdam, Netherlands. Plasmid DNA was prepared from overnight grown E. coli cultures grown in Luria-Broth medium containing ampicillin (see Sambrook et al. (1989) (Cold Spring Harbor Laboratory Press: ISBN 0-87969-309-6)) on a Qiagene DNA preparation robot (Qiagen, Hilden) according to the manufacturers protocols. Sequencing primers with the following nucleotide sequences were used:
Qiagen1: 5'-CAGGAAACAGCTATGACC-3' (SEQ ID NO:12)
Qiagen2: 5'-CTAAAGGGAACAAAAGCTG-3' (SEQ ID NO:13)
Qiagen3: 5'-TGTAAAACGACGGCCAGT-3' (SEQ ID NO:14)
Sequences were processed and annotated using the software package EST-MAX commercially provided by Bio-Max (Munich, Germany). The program incorporates practically all bioinformatics methods important for functional and structural characterization of protein sequences. For reference see *http:llpedant.mips.biochem.mpg.de.*

The most important algorythms incorporated in EST-MAX are:
FASTA: Very sensitive sequence database searches with estimates of statistical significance; Pearson W.R. (1990) Rapid and sensitive sequence comparison with FASTP and FASTA. Methods Enzymol. 183:63-98.
BLAST: Very sensitive sequence database searches with estimates of statistical significance. Altschul S.F., Gish W., Miller W., Myers E.W., and Lipman D.J. Basic local alignment search tool. Journal of Molecular Biology 215:403-10.
PREDATOR: High-accuracy secondary structure prediction from single and multiple sequences. Frishman, D. and Argos, P. (1997) 75% accuracy in protein secondary structure prediction. Proteins, 27:329-335.
CLUSTALW: Multiple sequence alignment. Thompson, J.D., Higgins, D.G. and Gibson, T.J. (1994)
CLUSTAL W: improving the sensitivity of progressive multiple sequence alignment through sequence weighting, positions-specific gap penalties and weight matrix choice. Nucleic Acids Research, 22:4673-4680.
TMAP: Transmembrane region prediction from multiply aligned sequences. Persson, B. and Argos, P. (1994) Prediction of transmembrane segments in proteins utilising multiple sequence alignments. J. Mol. Biol. 237:182-192.
ALOM2: Transmembrane region prediction from single sequences. Klein, P., Kanehisa, M., and DeLisi, C. Prediction of protein function from sequence properties: A discriminant analysis of a database. Biochim. Biophys. Acta 787:221-226 (1984). Version 2 by Dr. K. Nakai.
PROSEARCH: Detection of PROSITE protein sequence patterns. Kolakowski L.F. Jr., Leunissen J.A.M., Smith J.E. (1992) ProSearch: fast searching of protein sequences with regular expression patterns related to protein structure and function. Biotechniques 13, 919-921.
BLIMPS: Similarity searches against a database of ungapped blocks. J.C. Wallace and Henikoff S., (1992) PATMAT: A searching and extraction program for sequence, pattern and block queries and databases, CABIOS 8:249-254. Written by Bill Alford.

### Example 4 - Identification of Arabidopsis, soybean and corn ORFs corresponding to PrPase

The *Physcomitrella patens,* PpPrPase (SEQ ID NO:1), was identified in EST-MAX through BLAST analysis. The top hit of the BLAST is an Arabidopsis unknown ORF. The second and the third hits are human and yeast caax prenyl protease. Further analysis of the unknown arabidopsis ORF revealed that it is a predicted ORF from computer analysis, using the program Genefinder (P.Green and L.Hillier, www.ncbi.nlm.nih.gov). The ORF is located on the complementary strand of the BAC clone AF007269 (GenBank accession number, gene= "A_IG002N01.21) from 24979 to 28076.

Using this computer predicted arabidopsis AtPrPase cDNA as a query, BLAST search in various maize and soybean databases has identified one corn ZmPPase EST (SEQ ID NO:9) and one soybean GmPPase EST (SEQ ID NO:7).

### Example 5 - Cloning of Arabidopsis cDNAs encoding for PrPase

### Total RNA isolation from Arabidopsis thaliana

The total RNA was obtained from wild-type 14 day old *Arabidopsis thaliana* following the Van Slogteren (1983 Plant Mol. Biol. 2: 321-333.) method with slight modifications. Tissue (200mg) was frozen with liquid nitrogen and ground to a fine powder with a mortar and pestle. The powder was placed in a microfuge tube and the RNA was extracted with 500ul of extraction buffer (phenyl: 0.1M LiCl, 100mM Tris-HCl [pH8.0], 10mM EDTA, 1% SDS (w/v) [1:1] ) preheated to 90°C. The mixture was heated further for 1 min at 90°C and then vortexed for 5 mins. Proteins were extracted by adding 250 ul of chloroform: isoamyl alcohol (24:1) and the mixture was vortexed for 5 mins and centrifuged for 15 mins at 13,000 rpm in an Eppendorf centrifuge 5414 at 4°C. The aqueous layer was removed and the protein extraction was repeated twice more. One vol of 4mM LiCl was added and the RNA was allowed to precipitate overnight at 4°C. To collect the RNA, the mixture was centrifuged for 15 min at 4°C at 13,000 rpm in an Eppendorf centrifuge 5414. The pellet was resuspended in 250 ul sterile, deionized water. To precipitate the RNA, 0.1 vols of 3M sodium acetate (pH5.2) and 2 vols 100% ethanol were added. An aliquote was taken and centrifuged for 20 mins at 4°C at 13,000 rpm in an Eppendorf centrifuge 5414. The pellet was washed with 70% ethanol to remove salts from the pellet and dried using a speed vac. The pellet was resuspended in 25 ul DEPC H₂O and analyzed for integrity via electrophoresis. The RNA was stored at -70°C.

### RT-PCR and cloning of Arabidopsis AtPrPase

The synthetic oligonucleotide primers (MWG-Biotech) were designed based on the BAC clone sequence (GenBank accession number AF007269, gene= "A_IG002N01.21", complement 24979...28076).
APP forward: 5' CCGTTAACAGCCATGGCGATTCCTTTCATGGAA 3' (SEQ ID NO:15)
APP reverse: 5' GTCCCGGGACTTAATCTGTCTTCTTGTCTT 3' (SEQ ID NO:16)
The primers designed contained a HpaI site in the 5' region and an XmaI site in the 3' region for cloning purpose.

The synthesis of the first strand cDNA was achieved using AMV Reverse Transcriptase (Roche, Mannheim, Germany). The resulting single-stranded cDNA was amplified via Polymerase Chain Reaction (PCR) utilizing the two gene specific primers. The conditions for the reaction were standard conditions with Expand High Fidelity PCR system (Roche). The parameters for the reaction were: five minutes at 94°C followed by five cycles of 40 seconds at 94°C, 40 seconds at 50°C and 1.5 minutes at 72°C. This was followed by thirty cycles of 40 seconds at 94°C, 40 seconds at 65°C and 1.5 minutes at 72°C. The fragment generated under these RT-PCR conditions was 1.3 kilobase long.

The fragment was extracted from agarose gel with a QIAquick Gel Extraction Kit (Qiagen) and ligated into the TOPO pCR 2.1 vector (Invitrogen) following manufacture's instructions. Recombinant vectors were transformed into Top10 cells (Invitrogen) using standard conditions. Transformed cells were selected for on LB agar containing 100µg/ml carbenicillin, 0.8mg X-gal (5-bromo-4-chloro-3-indolyl-β-D-galactoside) and 0.8mg IPTG (isopropylthio-β-D-galactoside) grown overnight at 37°C. White colonies were selected and used to inoculate 3ml of liquid LB containing 100µg/ml ampicillin and grown overnight at 37°C. Plasmid DNA was extracted using the QIAprep Spin Miniprep Kit (Qiagen) following manufacture's instructions.

The RT-PCR cloned Arabidopsis AtPrPase1-2 were sequenced to obtain its complete cDNA sequence (SEQ ID NO:3 and SEQ ID NO:5).

### Example 6 - In vivo complementation of the SM3614 yeast (PrPase) mutant

The fragment containing the arabidopsis AtPrPasel cDNA was excised from the recombinant PCR2.1 TOPO vector by digestion with EcoRI (Roche) according to manufacture's instructions. The subsequent fragment was excised from agarose gel with a QIAquick Gel Extraction Kit (QIAgen) according to manufacture's instructions and ligated into the yeast expression vector pYES2 (Invitrogen), also cleaved with EcoRI and dephosphorylated prior to ligation.

The recombinant expression pYES2 vector containing the Arabidopsis AtPrPasel cDNA in the sense orientation under the yeast GAL1 promoter was transformed into the yeast mutant SM3614 (MATa rcel Δ::TRP1 ste24D::LEU2) (Tam et al. 1998) following Invitrogen's protocol. The transformed cells were selected for on Complete Supplement Mixture (CSM) minus Uracil 0.8% agar (Bio 101, Inc.) grown at 30°C for two days. The transformed colonies were selected to make master plates containing patches of the transformed SM3614 on CSM plates minus Uracil supplemented with 2% galactose for induction of the expression of the arabidopsis AtPrPase1. The plates were grown at 30°C for two days. The master plates were replica plated onto a lawn of wild type yeast SM 1068 (MATα lysl) (Tam et al. 1998, The Journal of Cell Biology, 142, 635-649) on SD plates supplemented with 2% galactose under various mating conditions and incubated at 30°C for two days.

### Example 7 - Cloning of soybeans and corn cDNAs encoding for PrPase

### Construction of cDNA libraries of soybeans and corn

To isolate the clones encoding PrPase from soybeans and corn the cDNA libraries were created with SMART RACE cDNA Amplification kit (Clontech Laboratories) following manufacture's instructions. Total RNA created as described in Example 5 was used as the template. Three-week old corn leaves and stems and soybean leaves were used for total RNA preparation respectively.

### Cloning of soybeans and corn cDNAs encoding for PrPase

The EST sequences for ZmPrPase and GmPrPase identified from the database search as described in Example 4 were used to design oligos for RACE. The extended partial sequences for ZmPrPase amd GmPrPase were by obtained from performing Rapid Amplification of cDNA Ends polymerase chain reaction (RACE PCR) using the Advantage 2 PCR kit (Clontech Laboratories) and the SMART RACE cDNA amplification kit (Clontech Laboratories) using a Biometra T3 Thermocycler following the manufacture's instructions. The gene specific synthetic oligonucleotide primer (MWG-Biotech) used were:
For ZmPrPase:
   5' RACE oligo: 5' AGCAGCCACGATTGGTGGCCCCAAT 3' (SEQ ID NO:21)
   3' RACE oligo: 5' GGGCCACCAATCGTGGCTGCTATCA 3' (SEQ ID NO:22)
For GmPrPase:
   5' RACE oligo: 5' CGCAGCCAGTCCTCATTGGGCTCATC 3' (SEQ ID NO:23)
   3' RACE oligo: 5' CGGATAGTTGAGGGAGGAAGCAAG 3' (SEQ ID NO:24)

The sequences obtained from the RACE reactions were compiled to give the nucleotide sequences for the partial GmPrPase (SEQ ID NO:7) and the partial ZmPrPase (SEQ ID NO:9).

### Example 8 - Engineering drought-tolerant Arabidopsis plants by reducing the activity of the endogenous PrPase gene

### Binary vector construction: pGMSG any pGMGG

The pLMNC53 (Mankin, 2000, PHD thesis) vector was digested with HindIII (Roche) and blunt-end filled with Klenow enzyme and 0.1mM dNTPs (Roche) according to manufacture's instructions. This fragment was extracted from agarose gel with a QIAquick Gel Extraction Kit (Qiagen) according to manufacture's instructions. The purified fragment was then digested with EcoRI_(Roche) according to manufacture's instructions. This fragment was extracted from agarose gel with a QIAquick Gel Extraction Kit (Qiagen) according to manufacture's instructions. The resulting 1.4kilobase fragment, the gentamycin casstte, included the nos promoter, aacCI gene and the g7 terminator.

The vector pBlueScript was digested with EcoRI and SmaI (Roche) according to manufacture's instructions. The resulting fragment was extracted from agarose gel with a QIAquick Gel Extraction Kit (Qiagen) according to manufacture's instructions. The digested pBlueScript vector and the gentamycin cassette fragments were ligated with T4 DNA Ligase (Roche) according to manufacture's instructions, joining the two respective EcoRI sites and joining the blunt-ended HindIII site with the SmaI site.

The recombinant vector (pGMBS) was transformed into Top10 cells (Invitrogen) using standard conditions. Transformed cells were selected for on LB agar containing 100µg/ml carbenicillin, 0.8mg X-gal (5-bromo-4-chloro-3-indolyl-β-D-galactoside) and 0.8mg IPTG (isopropylthio-β-D-galactoside), grown overnight at 37°C. White colonies were selected and used to inoculate 3ml of liquid LB containing 100µg/ml ampicillin and grown overnight at 37°C. Plasmid DNA was extracted using the QIAprep Spin Miniprep Kit (Qiagen) following manufacture's instructions. Analyses of subsquent clones and restriction mapping was performed according to standard molecular biology techniques (Sambrook et al. 1989).

Both the pGMBS vector and p1bxSuperGUS vector were digested with XbaI and KpnI (Roche) according to manufacture's instructions, excising the gentamycin casstte from pGMBS and producing the backbone from the p1bxSuperGUS vector. The resulting fragments were extracted from agarose gel with a QIAquick Gel Extraction Kit (Qiagen) according to manufacture's instructions. These two fragments were ligated with T4 DNA ligase (Roche) according to manufacture's instructions.

The resulting recombinant vector (pGMSG) was transformed into Top10 cells (Invitrogen) using standard conditions. Transformed cells were selected for on LB agar containing 100µg/ml carbenicillin, 0.8mg X-gal (5-bromo-4-chloro-3-indolyl-β-D-galactoside) and 0.8mg IPTG (isopropylthio-β-D-galactoside), grown overnight at 37°C. White colonies were selected and used to inoculate 3ml of liquid LB containing 100µg/ml ampicillin and grown overnight at 37°C. Plasmid DNA was extracted using the QIAprep Spin Miniprep Kit (Qiagen) following manufacture's instructions. Analyses of subsquent clones and restriction mapping was performed according to standard molecular biology techniques (Sambrook et al. 1989).

Both the pBinK vector containing the guard cell-specific promoter KST1 (Bernd Muller-Rober, 1999) and pGMSG vector were digested with XbaI and SmaI according to manufacture's instructions, excising KST1 from pBinK and producing the backbone from the pGMSG vector. The resulting fragments were extracted from agarose gel with QIAquick Gel Extraction Kit (Qiagen) according to manufacture's instructions. These two fragments were ligated with T4 DNA ligase (Roche) according to manufacture's instructions.

The resulting recombinant vector (pGMGG) was transformed into Top10 cells (Invitrogen) using standard conditions. Transformed cells were selected for on LB agar containing 100µg/ml carbenicillin, 0.8mg X-gal (5-bromo-4-chloro-3-indolyl-β-D-galactoside) and 0.8mg IPTG (isopropylthio-β-D-galactoside), grown overnight at 37°C. White colonies were selected and used to inoculate 3ml of liquid LB containing 100µg/ml ampicillin and grown overnight at 37°C. Plasmid DNA was extracted using the QIAprep Spin Miniprep Kit (Qiagen) following manufacture's instructions. Analyses of subsequent clones and restriction mapping were performed according to standard molecular biology techniques (Sambrook et al. 1989).

A further example for a plant binary vector is the pBPS-GB1 vector into which the LMP gene candidates are cloned. The binary vector contains a kanamycin resistance gene driven under the control of the AtAct2-I promoter and a USP seed specific promoter in front of the candidate gene with the NOSpA terminator. Partial or full-length LMP cDNA are cloned into the multiple cloning site of the plant binary vector in sense or antisense orientation behind the USP seed-specific promoter. The recombinant vector containing the gene of interest is transformed into Top10 cells (Invitrogen) using standard conditions. Transformed cells are selected for on LB agar containing 50 µg/ml kanamycin grown overnight at 37°C. Plasmid DNA is extracted using the QIAprep Spin Miniprep Kit (Qiagen) following manufacturer's instructions. Analysis of subsequent clones and restriction mapping is performed according to standard molecular biology techniques (Sambrook et al. 1989, Molecular Cloning, A Laboratory Manual. 2nd Edition. Cold Spring Harbor Laboratory Press. Cold Spring Harbor, NY).

### Subcloning of the Arabidopsis AtPrPasel into the binary vectors

The fragment containing the arabidopsis PPase cDNA was excised from the recombinant PCR2.1 TOPO vector by digestion with HpaI and XmaI (Roche) according to manufacture's instructions. The subsequence fragment was excised from agarose gel with a QIAquick Gel Extraction Kit (QIAgen) according to manufacture's instructions and ligated into the binary vectors pGMSG and pGMGG, cleaved with XmaI and Ecl136II and dephosphorylated prior to ligation and ligated into binary vector with USP promoter pBPSGB01, cleaved with AsCI and PacI prior to ligation, respectively. The resulting recombinant pGMSG, and pGMGG vectors contained the Arabidopsis prenyl protease in the antisense orientation under the constitutive super promoter and the guard cell specific promoter KST1. The pBPSGB01 vector contained the Arabidopsis PPase cDNA in sense and antisense orientation under the control of the seed-specific USP promoter.

### Agrobacterium Transformaiton

The recombinant vectors were transformed into Agrobacterium tumefaciens C58C1 and PMP90 according to standard conditions (Hoefgen and Willmitzer, 1990).

### Plant Transformation

Arabidopsis thaliana ecotypes C24 and Col-2 were grown and transformed according to standard conditions (Bechtold 1993, Acad.Sci.Paris. 316:1194-1199, Bent et al. 1994, Science 265:1856-1860).

### Screening of Transformed Plants

Seeds were sterilized according to standard protocols (Xiong et al. 1999, Plant Molecular Biology Reporter 17: 159-170). Seeds were plated on ½ MS 0.6% agar supplemented with 1-3% sucrose and 50-150µg/ml gentamycin. Seeds on plates were vernalized for two days at 4°C. The seeds were germinated in a climatic chamber at an air temperature of 22°C and light intensity of 55 micromols^{-1m2} (white light; Philips TL 65W/25 fluorescent tube) and 24 hour light. Transformed seedlings were selected after 7-14 and transferred to ½ MS 0.6% agar plates supplemented with 1% sucrose and allowed to recover for 1-5 days.

### Drought Tolerance Screening

The transgenic plants are screened for their improved drought tolerance according to the screening method described (seedlings are transferred to dry, sterile filter paper and allowed to desiccate for 4 hours. Seedlings are then removed and placed on ½ MS 0.6% agar plates and scored after two days.

### Salt Tolerance Screening

The transgenic plants are screened for the improved salt tolerance according to the screening method described (seedlings are transferred to ½ MS liquid supplemented with 600mM NaCl and allowed to incubate for 2-4 hours. Seedlings are then removed and placed on ½ MS 0.6% agar plates and scored for surviving seedlings after two days).

### Seed Storage Compound Screening

T2 and T3 seeds of the transgenic plants are screened for increased amounts of seed-storage compounds (oil, sugars, protein) according to the screening method described in Example 34 (see below).

### Example 9 - Engineering drought-tolerant soybean plants by reducing the activity of the endogenous GmPrPase gene

The clone GmPrPase (SEQ ID NO:7) was cloned into the vectors pGMSG and pGMGG in antisense orientation. These constructs were used to transform soybean as described below.

Seeds of soybean are surface sterilized with 70 % ethanol for 4 minutes at room temperature with continuous shaking, followed by 20% (v/v) Clorox supplemented with 0.05 % (v/v) Tween for 20 minutes with continuous shaking. Then, the seeds are rinsed 4 times with distilled water and placed on moistened sterile filter paper in a Petri dish at room temperature for 6 to 39 hours. The seed coats are peeled off, and cotyledons are detached from the embryo axis. The embryo axis is examined to make sure that the meristematic region is not damaged. The excised embryo axes are collected in a half-open sterile Petri dish and air dried to a moisture content less than 20% (fresh weight) in a sealed Petri dish until further use.

*Agrobacterium tumefaciens* culture is prepared from a single colony in LB solid medium plus appropriate antibiotics (e.g. 100 mg/l streptomycin, 50 mg/l kanamycin) followed by growth of the single colony in liquid LB medium to an optical density at 600 nm of 0.8. Then, the bacteria culture is pelleted at 7000 rpm for 7 minutes at RT, and resuspended in MS (Murashige and Skoog, 1962) medium supplemented with 100 µM acetosyringone. Bacteria cultures are incubated in this pre-induction medium for 2 hours at RT before use. The axis of soybean zygotic seed embryos at approximately 15% moisture content are imbibed for 2 hours at RT with the pre-induced *Agrobacterium* suspension culture. The embryos are removed from the imbibition culture and are transferred to Petri dishes containing solid MS medium supplemented with 2% sucrose and incubated for 2 days, in the dark at RT. Alternatively, the embryos are placed on top of moistened (liquid MS medium) sterile filter paper in a Petri dish and incubated under the same conditions described above. After this period, the embryos are transferred to either solid or liquid MS medium supplemented with 500 mg/L carbenicillin or 300 mg/L cefotaxime to kill the agrobacteria. The liquid medium is used to moisten the sterile filter paper. The embryos are incubated during 4 weeks at 25 °C, under 150 µmol m⁻²sec⁻¹ and 12 hours photoperiod. Once the seedlings have produced roots, they are transferred to sterile metromix soil. The medium of the *in vitro* plants is washed off before transferring the plants to soil. The plants are kept under a plastic cover for 1 week to favor the acclimatization process. Then the plants are transferred to a growth room where they are incubated at 25 °C, under 150 µmol m⁻²sec⁻¹ light intensity and 12 hours photoperiod for about 80 days.

The transgenic plants are screened for their improved drought tolerance according to the screening method described in Example 7 demonstrating that transgene expression confers drought tolerance.

### Example 10 - Engineering drought-tolerant Rapeseed plants by reducing the activity of the endogenous PrPase gene with the AtPrPase1 clone

The constructs pBPSRC003 and pBPSRC005 were used to transform rapeseed as described below.

The method of plant transformation described in Example 8 is also applicable to Brassica and other crops. Seeds of canola are surface sterilized with 70% ethanol for 4 minutes at room temperature with continuous shaking, followed by 20% (v/v) Clorox supplemented with 0.05 % (v/v) Tween for 20 minutes, at RT with continuous shaking. Then, the seeds are rinsed 4 times with distilled water and placed on moistened sterile filter paper in a Petri dish at room temperature for 18 hours. Then the seed coats are removed and the seeds are air dried overnight in a half-open sterile Petri dish. During this period the seeds loses approx. 85% of its water content. The seeds are then stored at room temperature in a sealed Petri dish until further use. DNA constructs and embryo imbibitions are as described in Example 8. Samples of the primary transgenic plants (T0) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

The transgenic plants are screened for their improved drought tolerance according to the screening method described in Example 7 demonstrating that transgene expression confers drought tolerance.

### Example 11 - Engineering drought-tolerant corn plants by reducing the activity of the endogenous ZmPrPase gene

The clone GmPrPase (SEQ ID NO:9) was cloned into the vectors pGMSG and pGMGG in antisense orientation. These constructs were used to transform corn as described below.

The imbibition of dry embryos with a culture of *Agrobacterium* is also applicable to maize embryo axes. The experimental protocol is the same as described in Example 8 but using maize seeds as the source of embryos.

The transgenic plants are screened for their improved drought tolerance according to the screening method described in Example 7 demonstrating that transgene expression confers drought tolerance.

### Example 12 - Guard-cell specific expression of the AtPrPase1 promoter

The promoter region of AtPrPase (SEQ ID NO11) was cloned into pGMSG in place of the superpromoter, driving the reporter gene GUS (Jefferson et al., 1987). The resulting construct pBPSRC006 was transformed into Arabidopsis plants as described in Example 7.

The transgenic plants are screened for their guard-cell specific staining demonstrating that the transgene expression confers guard-cell specific promoter activity.

### Example 13 - Over-expression of PrPase in plants leads to increased stress tolerance and plant growth.

The clones AtPrPasel (SEQ ID NO:3), AtPrPase2 (SEQ ID NO: 5) were cloned into the vectors pGMSG in sense orientation. These constructs were used to transform Arabidopsis, soybeans, rapeseed, and corn as described in Example 7, 8, 9, and 10, respectively.

The transgenic plants are screened for their improved stress tolerance according to the screening method described in Example 7 demonstrating that transgene expression confers stress tolerance.

The transgenic plants are further screened for their growth rate demonstrating that transgene expression confers increased growth rates.

### Example 14 - Isolation of a Specific Clone from the Deposited Sample.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 1-10 plasmid DNAs, each containing a different cDNA clone and/or partial cDNA clone; but such a deposit sample may include plasmids for more or less than 2 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with 32P-(-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 ul of reaction mixture with 0.5 ug of the above cDNA template. A convenient reaction mixture is 1.5-5 mM MgCl2, 0.01% (w/v) gelatin, 20 uM each of dATP, dCTP, dGTP, dTTP, 25 pmol of each primer and 0.25 Unit of Taq polymerase. Thirty five cycles of PCR (denaturation at 94 degree C for 1 min; annealing at 55 degree C for 1 min; elongation at 72 degree C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding and/or coding portions of a gene which may not be present in the deposited clone. These methods include but are not limited to, filter probing, clone enrichment using specific probes, and protocols similar or identical to 5' and 3' "RACE" protocols that are well known in the art. For instance, a method similar to 5' RACE is available for generating the missing 5' end of a desired full-length transcript. (Fromont-Racine et al., Nucleic Acids Res. 21(7):1683-1684 (1993)).

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full-length gene.

This above method starts with total RNA isolated from the desired source, although poly-A+ RNA can be used. The RNA preparation can then be treated with phosphatase if necessary to eliminate 5' phosphate groups on degraded or damaged RNA that may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene. Moreover, it may be advantageous to optimize the RACE prototol to increase the probability of isolating additional 5' or 3' coding or non-coding sequences. Various methods of optimizing a RACE protocol are known in the art, though a detailed description summarizing these methods can be found in B.C. Schaefer, Anal. Biochem., 227:255-273, (1995).

### Example 15 - Tissue Distribution of Polypeptide.

Tissue distribution of mRNA expression of polynucleotides of the present invention is determined using protocols for Northern blot analysis, described by, among others, Sambrook et al. For example, a cDNA probe produced by the method described in Example 1 is labeled with p32 using the rediprimetm DNA labeling system (Amersham Life Scinece), according to manufacturer's instructions. After labeling, the probe is purified using CHROMA SPIN0-100 column (Clontech Laboratories, Inc.) according to manufacturer's protocol number PT1200-1. The purified labeled probe is then used to examine various tissues for mRNA expression.

Tissue Northern blots containing the bound mRNA of various tissues are examined with the labeled probe using ExpressHybtm hybridization solution (Clonetech according to manufacturers protocol number PT1190-1. Northern blots can be produced using various protocols well known in the art (e.g., Sambrook et al). Following hybridization and washing, the blots are mounted and exposed to film at - 70C overnight, and the films developed according to standard procedures.

### Example 16 - Chromosomal Mapping of the Polynucleotides.

An oligonucleotide primer set is designed according to the sequence at the 5' end of SEQ ID NO:X. This primer preferably spans about 100 nucleotides. This primer set is then used in a polymerase chain reaction under the following set of conditions: 30 seconds,95 degree C; 1 minute, 56 degree C; 1 minute, 70 degree C. This cycle is repeated 32 times followed by one 5 minute cycle at 70 degree C. Plant DNA is used as template in addition to a somatic cell hybrid panel containing individual chromosomes or chromosome fragments (Bios, Inc). The reactions are analyzed on either 8% polyacrylamide gels or 3.5 % agarose gels. Chromosome mapping is determined by the presence of an approximately 100 bp PCR fragment in the particular somatic cell hybrid.

### Example 17 - Bacterial Expression of a Polypeptide.

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Ampr), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, that expresses the lacI repressor and also confers kanamycin resistance (Kanr). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.600) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCI by stirring for 3-4 hours at 4 degree C. The cell debris is removed by centrifugation, and the supernatant containing the polypeptide is loaded onto a nickel-nitrilo-tri-acetic acid ("Ni-NTA") affinity resin column (available from QIAGEN, Inc., supra). Proteins with a 6 x His tag bind to the Ni-NTA resin with high affinity and can be purified in a simple one-step procedure (for details see: The QIAexpressionist (1995) QIAGEN, Inc., supra).

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4 degree C or frozen at -80 degree C.

### Example 18 - Purification of a Polypeptide from an Inclusion Body.

The following alternative method can be used to purify a polypeptide expressed in E coli when it is present in the form of inclusion bodies. Unless otherwise specified, all of the following steps are conducted at 4-10 degree C.

Upon completion of the production phase of the E. coli fermentation, the cell culture is cooled to 4-10 degree C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4 degree C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4 degree C without mixing for 12 hours prior to further purification steps.

To clarify the refolded polypeptide solution, a previously prepared tangential filtration unit equipped with 0.16 um membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A280 monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 ug of purified protein is loaded. The purified protein can also be tested for endotoxin/LPS contamination, and typically the LPS content is less than 0.1 ng/ml according to LAL assays.

### Example 19 - Cloning and Expression of a Polypeptide in a Baculovirus Expression System.

In this example, the plasmid shuttle vector pAc373 is used to insert a polynucleotide into a baculovirus to express a polypeptide. A typical baculovirus expression vector contains the strong polyhedrin promoter of the Autographa californica nuclear polyhedrosis virus (AcMNPV) followed by convenient restriction sites, which may include, for example BamHI, Xba I and Asp718. The polyadenylation site of the simian virus 40 ("SV40") is often used for efficient polyadenylation. For easy selection of recombinant virus, the plasmid contains the beta-galactosidase gene from E. coli under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pVL941 and pAcIMl, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites at the 5' end of the primers in order to clone the amplified product into the expression vector. Specifically, the cDNA sequence contained in the deposited clone, including the AUG initiation codon and the naturally associated leader sequence identified elsewhere herein (if applicable), is amplified using the PCR protocol described in Example 1. If the naturally occurring signal sequence is used to produce the protein, the vector used does not need a second signal peptide. Alternatively, the vector can be modified to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.).

The fragment and the dephosphorylated plasmid are ligated together with T4 DNA ligase. E. coli HB101 or other suitable E. coli hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five ug of a plasmid containing the polynucleotide is co-transformed with 1.0 ug of a commercially available linearized bacuolvirus DNA ("BaculoGoldtm baculovirus DNA", Pharmingen, San Diego, CA), using the lipofection method described by Felgner et al., Proc. Natl. Acad. Sci. USA 84:7413-7417 (1987). One ug of BaculoGoldtm virus DNA and 5ug of the plasmid are mixed in a sterile well of a microtiter plate containing 50ul of serum-free Grace's medium (Life Technologoes Inc., Gaithersburg, MD). Afterwards, 10 ul Lipofectin plus 90 ul Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27 degrees C. The transfection solution is then removed from the plate and 1 ml of Grace's insect medium supplemented with 10% fetal calf serum is added. Cultivation is then continued at 27 degrees C for four days.

After four days the supernatant is collected and a plaque assay is performed, as described by Summers and Smith, supra. An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 ul of Grace's medium and the suspension containing the recombinant baculovirus is used to infect Sf9 cells seeded in 35 mm dishes. Four days later the supernatants of these culture dishes are harvested and then they are stored at 4 degree C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 uCi of 35S-methionine and 5 uCi 35S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 20 - Expression of a Polypeptide in Mammalian Cells.

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transformation with a selectable marker such as dhfr, gpt, neomycin, hygromycin allows the identification and isolation of the transformed cells.

The transformed gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

A polynucleotide of the present invention is amplified according to the protocol outlined in Example 1. If the naturally occurring signal sequence is used to produce the protein, the vector does not need a second signal peptide. Alternatively, if the naturally occurring signal sequence is not used, the vector can be modified to include a heterologous signal sequence. (See, e.g., WO 96/34891.) The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. E. coli HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transformation. Five µg of an expression plasmid is cotransformed with 0.5 ug of the plasmid pSVneo using lipofectin (Felgner et al., supra). The plasmid pSV2-neo contains a dominant selectable marker, the neo gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 uM, 2 uM, 5 uM, 10 mM, 20 mM). The same procedure is repeated until clones are obtained which grow at a concentration of 100 - 200 uM. Expression of the desired gene product is analyzed, for instance, by SDS-PAGE and Western blot or by reversed phase HPLC analysis.

In addition to the method provided above, other methods of expressing polypeptides, preferably polypeptides of the present invention are known in the art. For example, US Patent No. 6,066,781 (which is hereby incorporated by reference herein in its entirety) describes a chimeric gene consisting of the encoding polynucleotides for the N-terminal moiety corresponding to a portion of the rice alpha-amylase signal sequence peptide (MKNTSSLCLLLLVVLCSLTCNSGQA (SEQ ID NO:20)), operatively linked, to the encoding polynucleotide sequence of the protein of interest - in this case, a polypeptide of the present invention. This signal sequence peptide may be operatively substituted for the native signal sequence of a polypeptide of the present invention as a heterologous signal sequence. Such a method of producing mature forms of the inventive polypeptides is encompassed by the present invention and may be used either alone or in conjunction with other methods known in the art and/or disclosed herein.

Moreover, enhanced protein production of recombinant proteins in higher plants has recently been obtained by N-terminal fusion of a ubiquitin or a cucumber mosaic virus coat protein peptide (see International Publication No. WO 00/36129). Such methods may be applied to increase the expression of a polypeptide of the present invention in a suitable plant host.

### Example 21 - Protein Fusions.

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example described herein; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector. Note that the polynucleotide is cloned without a stop codon, otherwise a fusion protein will not be produced.

The naturally occurring signal sequence may be used to produce the protein (if applicable). Alternatively, if the naturally occurring signal sequence is not used, the vector can be modified to include a heterologous signal sequence. (See, e.g., WO 96/34891 and/or US Patent No. 6,066,781, supra.)

### Human IgG Fc region:

GGGATCCGGAGCCCAAATCTTCTGACAAAACTCACACATGCCCACCGTGCCCAGCACCTGAA
TTCGAGGGTGCACCGTCAGTCTTCCTCTTCCCCCCAAAACCCAAGGACACCCTCATGATCTCC
CGGACTCCTGAGGTCACATGCGTGGTGGTGGACGTAAGCCACGAAGACCCTGAGGTCAAGT
TCAACTGGTACGTGGACGGCGTGGAGGTGCATAATGCCAAGACAAAGCCGCGGGAGGAGCA
GTACAACAGCACGTACCGTGTGGTCAGCGTCCTCACCGTCCTGCACCAGGACTGGCTGAATG
GCAAGGAGTACAAGTGCAAGGTCTCCAACAAAGCCCTCCCAACCCCCATCGAGAAAACCAT
CTCCAAAGCCAAAGGGCAGCCCCGAGAACCACAGGTGTACACCCTGCCCCCATCCCGGGAT
GAGCTGACCAAGAACCAGGTCAGCCTGACCTGCCTGGTCAAAGGCTTCTATCCAAGCGACAT
CGCCGTGGAGTGGGAGAGCAATGGGCAGCCGGAGAACAACTACAAGACCACGCCTCCCGTG
CTGGACTCCGACGGCTCCTTCTTCCTCTACAGCAAGCTCACCGTGGACAAGAGCAGGTGGCA
GCAGGGGAACGTCTTCTCATGCTCCGTGATGCATGAGGCTCTGCACAACCACTACACGCAGA
AGAGCCTCTCCCTGTCTCCGGGTAAATGAGTGCGACGGCCGCGACTCTAGAGGAT (SEQ ID
NO:18)

### Example 22 - Production of an Antibody from a Polypeptide.

The antibodies of the present invention can be prepared by a variety of methods. (See, Current Protocols, Chapter 2.) As one example of such methods, cells expressing a polypeptide of the present invention are administered to an animal to induce the production of sera containing polyclonal antibodies. In a preferred method, a preparation of the protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a polypeptide-expressing cell. Such cells may be cultured in any suitable tissue culture medium; however, it is preferable to culture cells in Earle's modified Eagle's medium supplemented with 10% fetal bovine serum (inactivated at about 56 degrees C), and supplemented with about 10 g/l of nonessential amino acids, about 1,000 U/ml of penicillin, and about 100 ug/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody that binds to a second antibody. In accordance with this method, protein specific antibodies are used to immunize an animal, preferably a mouse. The splenocytes of such an animal are then used to produce hybridoma cells, and the hybridoma cells are screened to identify clones that produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

Moreover, in a more preferred method, the antibodies directed against the polypeptides of the present invention may be produced in plants. Specific methods are disclosed in US Patent Nos. 5,959,177, and 6,080,560, which are hereby incorporated in their entirety herein. The methods not only describe methods of expressing antibodies, but also the means of assembling foreign multimeric proteins in plants (i.e., antibodies, etc,), and the subsequent secretion of such antibodies from the plant.

### Example 23 - Antibody Mediated Down-Regulation of Plant Proteins.

The process of genetically modifying a plant to modulate specific characteristics, to introduce novel traits, or to inhibit endogenous traits represents a signficant area of research in the agricultural field. Recently, a new method of modulating endogenous gene expression using antibodies has been elucited (see, International Publication Number WO 00/05391, which is hereby incorporated in its entirety herein). In this example, the researchers were able to achieve 40-70% inhibition of an endogenous plant protein through the use of a single-chain antibody construct directed towards the plant protein.

The method is directed towards the production of monoclonal antibodies, specifically, single chain antibodies, specific to endogenous transit peptides in a plant in an effort to decrease steady state levels of such transit peptides within the plant. The method is comprised of the following steps: I) generating monoclonal antibodies to a specific plant, II) cloning the gene for said monoclonal antibody, III) creating an expression vector comprising a fusion of the heavy-chain and light chain gene sequences of said monoclonal antibody gene downstream of the p67 leader peptide, and under the control of a consititutive plant promotor, IV) optimizing the codons of said heavy-chain and light chain fusion vector for efficient expression of the gene encoded thereof in a plant, and V), transforming a plant with said heavy-chain and light chain fusion expression vector.

The skilled artisan would appreciate the methods described therein (WO 00/05391), and would have the ability to apply such methods to inhibiting the steady-state expression levels of any of the polypeptides of the present invention, including variants, and fragments, thereof. The skilled artisan would appreciate that any leader peptide (i.e., signal sequence) from a plant protein could be used in creating the heavy-chain and light chain fusion vector. The skilled artisan would also appreciate that different plant species may have different codon usage requirements, and thus, the decision to optimize the codons of the heavy-chain and light chain fusion vector would be affected according to the codons required for that particular plant species.

The method could not only be applied to transit peptides, but also to secreted proteins, membrane proteins, receptors, and ligands. The method could also be applied in combination with other antibody production methods in plants. For example, antibodies directed towards polypeptides of the present invention may inhibit specific traits in a plant which could increase the plants defense mechanisms to pathogens. Thus, where such an antbody was expressed, another antibody could be expressed in combination with the first, to inhibiting the pathogenicity of a plant pathogen by directing the expression of antibodies directed towards pathogenic proteins (e.g., those proteins critical to the initiating events of infection, such as the BUF1 gene from M.grisea, stage two juvenile salivary gland proteins which include, svp30, scp31a, scp31b, scp32, scp32, scp39, and scp49 from G.rostochiensis (WO 96/22372), etc.). Such a combination would also be of value where the second "anti-pathogenic" antibody is an antibody directed towards a pathogen and fused to a toxic protein wherein such a toxin could be chitinase, glucanase, lysozyme, BT, or colicin F, for example (see WO 96/09398), etc.).

As described eleswere herein, the method could also be used as a means of inhibiting allergic reactions to plant antigens in humans, mammals, animals, etc., by directing the production of a single chain antibody construct specific towards said plant antigen in the plant (via transgenic methodology). In the latter example, the plant would not be limited to edible plants, as inhibiting the production of such a plant antigen would provide benefit to a human by removing the antigen from the humans environment, for example, irrespective of whether the plant was ingested.

Of particular interest to this example, is the fact that secretion of functional antibody through the plasma membrane of plant cells has been reported for protoplasts isolated from transgenic plants and for callus cells adapted to suspension culture (Hein et al., Biotechnol. Prog. 7:455-561, 1991). However, the levels of secreted antibody detected in both culture systems were extremely low. In other studies, cultured tobacco cells were transformed with a gene encoding a synthetic antibody derivative expressed as a single chain consisting of both the heavy- and light-chain variable domains of the intact immunoglobulin joined together by a flexible peptide linker (Pluckthun, Immunol. Rev. 130:151-188, 1991; and Bird et al., Science 242:423-426, 1988). This synthetic single-chain antibody retained the full antigen-binding potential of the intact immunoglobulin but accumulated in the extracellular apoplastic space of the transformed cells (Firek et al., Plant Molecular Biology 23:861-870, 1993), indicating that the antibody was being transported through the plasma membrane but not through the cell wall to the external environment. Moreover, recent studies have shown that increased antibody production in a plant, and heterologous protein expression, in general, could be increased by including in the plant culture medium a protein stabilizing agent (e.g., polyvinylpyrrolidone), see US Patent No. 6,020,169, which is hereby incorporated by reference in its entirety herein.

### Example 24 - Regulation of Protein Secretion Via Controlled Aggregation in the Endoplasmic Reticulum.

As described more particularly herein, proteins regulate diverse cellular processes in higher organisms, ranging from rapid metabolic changes to growth and differentiation. Increased production of specific proteins could be used to prevent certain diseases and/or disease states. By analogy, it would be expected that proteins also play a primary role in plants defense mechanisms during environmental, pathogenic, herbicidal, and insecticidal injury. Thus, the ability to modulate the expression of specific proteins in an organism would provide significant benefits.

Numerous methods have been developed to date for introducing foreign genes, either under the control of an inducible, constituitively active, or endogenous promoter, into organisms. Of particular interest are the inducible promoters (see, M. Gossen, et al., Proc. Natl. Acad. Sci. USA., 89:5547 (1992); Y. Wang, et al., Proc. Natl. Acad. Sci. USA, 91:8180 (1994), D. No., et al., Proc. Natl. Acad. Sci. USA, 93:3346 (1996); and V.M. Rivera, et al., Nature Med, 2:1028 (1996); in addition to additional examples disclosed elsewere herein). In one example, the gene for erthropoietin (Epo) was transferred into mice and primates under the control of a small molecule inducer for expression (e.g., tetracycline or rapamycin) (see, D. Bohl, et.al., Blood, 92:1512, (1998); K.G. Rendahl, et al., Nat. Biotech, 16:757, (1998); V.M. Rivera, et al., Proc. Natl. Acad. Sci. USA, 96:8657 (1999); and X.Ye et al., Science, 283:88 (1999). Although such systems enable efficient induction of the gene of interest in the organism upon addition of the inducing agent (i.e., tetracycline, rapamycin, etc,.), the levels of expression tend to peak at 24 hours and trail off to backgrond levels after 4 to 14 days. Thus, controlled transient expression is virtually impossible using these systems, though such control would be desirable.

A new alternative method of controlling gene expression levels of a protein from a transgene (i.e., includes stable and transient transformants) has recently been elucidated (V.M. Rivera., et al., Science, 287:826-830, (2000)). This method does not control gene expression at the level of the mRNA like the aforementioned systems. Rather, the system controls the level of protein in an active secreted form. In the absence of the inducing agent, the protein aggregates in the ER and is not secreted. However, addition of the inducing agent results in dis-aggregation of the protein and the subsequent secretion from the ER. Such a system affords low basal secretion, rapid, high level secretion in the presence of the inducing agent, and rapid cessation of secretion upon removal of the inducing agent. In fact, protein secretion reached a maximum level within 30 minutes of induction, and a rapid cessation of secretion within 1 hour of removing the inducing agent. The method is also applicable for controlling the level of production for membrane proteins.

Detailed methods are presented in V.M. Rivera., et al., Science, 287:826-830, (2000)), briefly:

Fusion protein contructs are created using polynucleotide sequences of the present invention with one or more copies (preferably at least 2, 3, 4, or more) of a conditional aggregation domain (CAD) a domain that interacts with itself in a ligand-reversible manner (i.e., in the presence of an inducing agent) using molecular biology methods known in the art and discussed elsewere herein. The CAD domain may be the mutant domain isolated from the human FKBP12 (Phe³⁶ to Met) protein (as disclosed in V.M. Rivera., et al., Science, 287:826-830, (2000), or alternatively other proteins having domains with similar ligand-reversible, self-aggregation properties. As a principle of design the fusion protein vector would contain a furin cleavage sequence operably linked between the polynucleotides of the present invention and the CAD domains. Such a cleavage site would enable the proteolytic cleavage of the CAD domains from the polypeptide of the present invention subsequent to secretion from the ER and upon entry into the trans-Golgi (J.B. Denault, et al., FEBS Lett., 379:113, (1996)). Alternatively, the skilled artisan would recognize that any proteolytic cleavage sequence could be substituted for the furin sequence provided the substituted sequence is cleavable either endogenously (e.g., the furin sequence) or exogenously (e.g., post secretion, post purification, post production, etc.). The preferred sequence of each feature of the fusion protein construct, from the 5' to 3' direction with each feature being operably linked to the other, would be a promoter, signal sequence, "X" number of (CAD)x domains, the furin sequence (or other proteolytic sequence), and the coding sequence of the polypeptide of the present invention. The artisan would appreciate that the promotor and signal sequence, independent from the other, could be either the endogenous promotor or signal sequence of a polypeptide of the present invention, or alternatively, could be a heterologous signal sequence and promotor.

The specific methods described herein for controlling protein secretion levels through controlled ER aggregation are not meant to be limiting are would be generally applicable to any of the polynucleotides and polypeptides of the present invention, including variants, homologues, orthologs, and fragements therein.

### EXAMPLE 25 - Alteration of Protein Glycosylation Sites to Enhance Secretion Characteristics of Polypeptides of the Invention.

Many eukaryotic cell surface and proteins are post-translationally processed to incorporate N-linked and O-linked carbohydrates (Kornfeld and Kornfeld (1985) Annu. Rev. Biochem. 54:631-64; Rademacher et al., (1988) Annu. Rev. Biochem. 57:785-838). Protein glycosylation is thought to serve a variety of functions including: augmentation of protein folding, inhibition of protein aggregation, regulation of intracellular trafficking to organelles, increasing resistance to proteolysis, modulation of protein antigenicity, and mediation of intercellular adhesion (Fieldler and Simons (1995) Cell, 81:309-312; Helenius (1994) Mol. Biol. Of the Cell 5:253-265; Olden et al., (1978) Cell, 13:461-473; Caton et al., (1982) Cell, 37:417-427; Alexamnder and Elder (1984), Science, 226:1328-1330; and Flack et al., (1994), J. Biol. Chem., 269:14015-14020). In higher organisms, the nature and extent of glycosylation can markedly affect the circulating half-life and bio-availability of proteins by mechanisms involving receptor mediated uptake and clearance (Ashwell and Morrell, (1974), Adv. Enzymol., 41:99-128; Ashwell and Harford (1982), Ann. Rev. Biochem., 51:531-54). Receptor systems have been identified that are thought to play a major role in the clearance of serum proteins through recongition of various carbohydrate strucutures on the glycoproteins (Stockert (1995), Physiol. Rev., 75:591-609; Kery et al., (1992), Arch. Biochem. Biophys., 298:49-55). Thus, production strategies resulting in incomplete attachment of terminal sialic acid residues might provide a means of shortening the bioavailability and half-life of glycoproteins. Conversely, expression strategies resulting in saturation of terminal sialic acid attachment sites might lengthen protein bioavailability and half-life.

In the development of recombinant glycoproteins for use as pharmaceutical products, for example, it has been speculated that the pharmacodynamics of recombinant proteins can be modulated by the addition or deletion of glycosylation sites from a glycoproteins primary strucuture (Berman and Lasky (1985a) Trends in Biotechnol., 3:51-53). However, studies have reported that the deletion of N-linked glycosylation sites often impairs intracellular transport and results in the intraceullular accumulation of glycosylation site variants (Machamer and Rose (1988), J. Biol Chem., 263:5955-5960; Gallagher et al., (1992), J. Viology., 66:7136-7145; Collier et al., (1993), Biochem., 32:7818-7823; Claffey et al., (1995) Biochemica et Biophysica Acta, 1246:1-9; Dube et al., (1988), J. Biol. Chem. 263:17516-17521). While glycosylation site variants of proteins can be expressed intracellularly, it has proved difficult to recover useful quantities from growth conditioned cell culture medium.

Moreover, it is unclear to what extent a glycosyolation site in one species will be recognized by another species glycosylation machinary. Due to the importance of glycosylation in protein metabolism, particularly the secretion and/or expression of the protein, whether a glycosylation signal is recognized may profoundly determine a proteins ability to be expressed, either endogenously or recombinately, in another organism (i.e., expressing a maize protein in E.coli, or an E.coli protein in maize, etc.). Thus, it may be desirable to add, delete, or modify a glycosylation site, and possibly add a glycosylation site of one species to a protein of another species to improve the proteins functional, bioprocess purification, and/or structural characteristics (e.g., a polypeptide of the present invention).

A number of methods may be employed to identify the location of glycosylation sites within a protein. One preferred method is to run the translated protein sequence through the PROSITE computer program (Swiss Institute of Bioinformatics). Once identified, the sites could be systematically deleted, or impaired, at the level of the DNA using mutagenesis methodology known in the art and available to the skilled artisan, preferrably using PCR-directed mutagenesis (See Maniatis, Molecular Cloning: A Laboratory Manual, Cold Spring Harbor Press, Cold Spring, NY (1982)). Similarly, glycosylation sites could be added, or modified at the level of the DNA using similar methods, preferably PCR methods (See, Maniatis, supra). The results of modifying the glycosylation sites for a particular protein (e.g., solubility, secretion potential, activity, aggregation, proteolytic resistence, etc.) could then be analyzed using methods know in the art.

### EXAMPLE 26 - Transformation of Dicotyledons.

The polynucleotides of the present invention, including the polynucleotides encoding the polypeptides of the present invention, in addition to the polynucleotides encoding antibodies directed against the polypeptides of the present invention may be used to transform monocotyledons in an effort to confer specific traits into the plant. Such polynucleotides may be either the full-length polynucleotide, fragments, the complementary strand, or variants thereof, and may be either by themselves or operably fused to heterologous polynucleotides as described in more detail elsewhere herein.

Transformation techniques for dicotyledons are well known in the art and include Agrobacterium-based techniques and techniques which do not require Agrobacterium. Non-Agrobacterium techniques involve the uptake of exogenous genetic material directly by protoplasts or cells. This can be accomplished by PEG or electroporation mediated uptake, particle bombardment-mediated delivery, or microinjection. Examples of these techniques are described by Paszkowski et al., EMBO J 3: 2717-2722 (1984), Potrykus et al., Mol. Gen. Genet. 199: 169-177 (1985), Reich et al., Biotechnology 4: 1001-1004 (1986), and Klein et al., Nature 327: 70-73 (1987). In each case the transformed cells are regenerated to whole plants using standard techniques known in the art.

Agrobacterium-mediated transformation is a preferred technique for transformation of dicotyledons because of its high efficiency of transformation and its broad utility with many different species. The many crop species which are routinely transformable by Agrobacterium include tobacco, tomato, sunflower, cotton, oilseed rape, potato, soybean, alfalfa and poplar (EP 0 317 511 (cotton), EP 0 249 432 (tomato, to Calgene), WO 87/07299 (Brassica, to Calgene), U.S. Pat. No. 4,795,855 (poplar)). Agrobacterium transformation typically involves the transfer of the binary vector carrying the foreign DNA of interest (e.g. pCIB200 or pCIB2001) to an appropriate Agrobacterium strain which may depend of the complement of vir genes carried by the host Agrobacterium strain either on a co-resident Ti plasmid or chromosomally (e.g. strain CIB542 for pCIB200 and pCIB2001 (Uknes et al. Plant Cell 5: 159-169 (1993)). The transfer of the recombinant binary vector to Agrobacterium is accomplished by a triparental mating procedure using E. coli carrying the recombinant binary vector, a helper E. coli strain which carries a plasmid such as pRK2013 and which is able to mobilize the recombinant binary vector to the target Agrobacterium strain. Alternatively, the recombinant binary vector can be transferred to Agrobacterium by DNA transformation (Hofgen & Willmitzer, Nucl. Acids Res. 16: 9877(1988)).

Transformation of the target plant species by recombinant Agrobacterium usually involves cocultivation of the Agrobacterium with explants from the plant and follows protocols well known in the art. Transformed tissue is regenerated on selectable medium carrying the antibiotic or herbicide resistance marker present between the binary plasmid T-DNA borders.

Other methods for the transformation of dicotyledons are known in the art. Thus, this example should be not construed as limiting the scope of the invention to only those examples illustrated above or elsewhere herein.

### EXAMPLE 27 - Transformation of Monocotyledons.

The polynucleotides of the present invention, including the polynucleotides encoding the polypeptides of the present invention, in addition to the polynucleotides encoding antibodies directed against the polypeptides of the present invention may be used to transform monocotyledons in an effort to confer specific traits into the plant. Such polynucleotides may be either the full-length polynucleotide, fragments, the complementary strand, or variants thereof, and may be either by themselves or operably fused to heterologous polynucleotides as described in more detail elsewhere herein.

Transformation of most monocotyledon species has now also become routine. Preferred techniques include direct gene transfer into protoplasts using PEG or electroporation techniques, and particle bombardment into callus tissue. Transformations can be undertaken with a single DNA species or multiple DNA species (i.e. co-transformation) and both these techniques are suitable for use with this invention. Co-transformation may have the advantage of avoiding complex vector construction and of generating transgenic plants with unlinked loci for the gene of interest and the selectable marker, enabling the removal of the selectable marker in subsequent generations, should this be regarded desirable. However, a disadvantage of the use of co-transformation is the less than 100% frequency with which separate DNA species are integrated into the genome (Schocher et al. Biotechnology 4: 1093-1096 (1986)).

Patent Applications EP 0 292 435 (to Ciba-Geigy), EP 0 392 225 (to Ciba-Geigy) and WO 93/07278 (to Ciba-Geigy) describe techniques for the preparation of callus and protoplasts from an elite inbred line of maize, transformation of protoplasts using PEG or electroporation, and the regeneration of maize plants from transformed protoplasts. Gordon-Kamm et al., Plant Cell 2: 603-618 (1990)) and Fromm et al., Biotechnology 8: 833-839 (1990)) have published techniques for transformation of A188-derived maize line using particle bombardment. Furthermore, application WO 93/07278 (to Ciba-Geigy) and Koziel et al., Biotechnology 11: 194-200 (1993)) describe techniques for the transformation of elite inbred lines of maize by particle bombardment. This technique utilizes immature maize embryos of 1.5-2.5 mm length excised from a maize ear 14-15 days after pollination and a PDS-1000He Biolistics device for bombardment.

Transformation of rice can also be undertaken by direct gene transfer techniques utilizing protoplasts or particle bombardment. Protoplast-mediated transformation has been described for Japonica-types and Indica-types (Zhang et al., Plant Cell Rep 7: 379-384 (1988); Shimamoto et al. Nature 338: 274-277 (1989); Datta et al. Biotechnology 8: 736-740 (1990)). Both types are also routinely transformable using particle bombardment (Christou et al. Biotechnology 9: 957-962 (1991)).

Patent Application EP 0 332 581 (to Ciba-Geigy) describes techniques for the generation, transformation and regeneration of Pooideae protoplasts. These techniques allow the transformation of Dactylis and wheat. Furthermore, wheat transformation was been described by Vasil et al., Biotechnology 10: 667-674 (1992)) using particle bombardment into cells of type C long-term regenerable callus, and also by Vasil et al., Biotechnology 11: 1553-1558 (1993)) and Weeks et al., Plant Physiol. 102: 1077-1084 (1993) using particle bombardment of immature embryos and immature embryo-derived callus. A preferred technique for wheat transformation, however, involves the transformation of wheat by particle bombardment of immature embryos and includes either a high sucrose or a high maltose step prior to gene delivery. Prior to bombardment, any number of embryos (0.75-1 mm in length) are plated onto MS medium with 3% sucrose (Murashige & Skoog, Physiologia Plantarum 15: 473-497 (1962)) and 3 mg/l 2,4-D for induction of somatic embryos which is allowed to proceed in the dark. On the chosen day of bombardment, embryos are removed from the induction medium and placed onto the osmoticum (i.e. induction medium with sucrose or maltose added at the desired concentration, typically 15%). The embryos are allowed to plasmolyze for 2-3 h and are then bombarded. Twenty embryos per target plate is typical, although not critical. An appropriate gene-carrying plasmid (such as pCIB3064 or pSG35) is precipitated onto micrometer size gold particles using standard procedures. Each plate of embryos is shot with the DuPont Biolistics, helium device using a burst pressure of .about.1000 psi using a standard 80 mesh screen. After bombardment, the embryos are placed back into the dark to recover for about 24 h (still on osmoticum). After 24 hrs, the embryos are removed from the osmoticum and placed back onto induction medium where they stay for about a month before regeneration. Approximately one month later the embryo explants with developing embryogenic callus are transferred to regeneration medium (MS+1 mg/liter NAA, 5 mg/liter GA), further containing the appropriate selection agent (10 mg/l basta in the case of pCIB3064 and 2 mg/l methotrexate in the case of pSOG35). After approximately one month, developed shoots are transferred to larger sterile containers known as "GA7s" which contained half-strength MS, 2% sucrose, and the same concentration of selection agent.

An additional method of transforming monocots is found in International Publication No. WO 00/12734, and describes the application of the Ac-Ds transposon system to the insertion of transgenes into plants.

Other methods for the transformation of monocotyledons are known in the art. Thus, this example should be not construed as limiting the scope of the invention to only those examples illustrated above or elsewhere herein.

### Example 28 - Method of Enhancing the Biological Activity/Functional Characteristics of Invention through Molecular Evolution.

Although many of the most biologically active proteins known are highly effective for their specified function in an organism, they often possess characteristics that make them undesirable for transgenic, therapeutic, agricultural, and/or industrial applications. Among these traits, a short physiological half-life is the most prominent problem, and is present either at the level of the protein, or the level of the proteins mRNA. The ability to extend the half-life, for example, would be particularly important for a proteins use in gene therapy, transgenic plant or animal production, the bioprocess production and purification of the protein, and use of the protein as a chemical modulator (e.g., herbicide, insectide, etc.), among others. Therefore, there is a need to identify novel variants of isolated proteins possessing characteristics which enhance their application as a therapeutic for treating diseases of both plant and animal origin, in addition to the proteins applicability to common industrial and agricultural applications.

Thus, one aspect of the present invention relates to the ability to enhance specific characteristics of invention through directed molecular evolution. Such an enhancement may, in a non-limiting example, benefit the inventions utility as an essential component in a kit, the inventions physical attributes such as its solubility, structure, or codon optimization, the inventions specific biological activity, including any associated enzymatic activity, the proteins enzyme kinetics, the proteins Ki, Kcat, Km, Vmax, Kd, protein-protein activity, protein-DNA binding activity, antagonist/inhibitory activity (including direct or indirect interaction), agonist activity (including direct or indirect interaction), the proteins antigenicity (e.g., where it would be desirable to either increase or decrease the antigenic potential of the protein), the immunogenicity of the protein, the ability of the protein to form dimers, trimers, or multimers with either itself or other proteins, the antigenic efficacy of the invention, including its subsequent use a preventative treatment for disease or disease states, or as an effector for targeting diseased genes. Moreover, the ability to enhance specific characteristics of a protein may also be applicable to changing the characterized activity of an enzyme to an activity completely unrelated to its initially characterized activity. Other desirable enhancements of the invention would be specific to each individual protein, and would thus be well known in the art and contemplated by the present invention.

Directed evolution is comprised of several steps. The first step is to establish a library of variants for the gene or protein of interest. The most important step is to then select for those variants that entail the activity you wish to identify. The design of the screen is essential since your screen should be selective enough to eliminate non-useful variants, but not so stringent as to eliminate all variants. The last step is then to repeat the above steps using the best variant from the previous screen. Each successive cycle, can then be tailored as necessary, such as increasing the stringency of the screen, for example.

Over the years, there have been a number of methods developed to introduce mutations into macromolecules. Some of these methods include, random mutagenesis, "error-prone" PCR, chemical mutagenesis, site-directed mutagenesis, and other methods well known in the art (for a comprehensive listing of current mutagenesis methods, see Maniatis, Molecular Cloning: A Laboratory Manual, Cold Spring Harbor Press, Cold Spring, NY (1982)). Typically, such methods have been used, for example, as tools for identifying the core functional region(s) of a protein or the function of specific domains of a protein (if a multi-domain protein). However, such methods have more recently been applied to the identification of macromolecule variants with specific or enhanced characteristics.

Random mutagenesis has been the most widely recognized method to date. Typically, this has been carried out either through the use of "error-prone" PCR (as described in Moore, J., et al, Nature Biotechnology 14:458, (1996), or through the application of randomized synthetic oligonucleotides corresponding to specific regions of interest (as descibed by Derbyshire, K.M. et al, Gene, 46:145-152, (1986), and Hill, DE, et al, Methods Enzymol., 55:559-568, (1987). Both approaches have limits to the level of mutagenesis that can be obtained. However, either approach enables the investigator to effectively control the rate of mutagenesis. This is particularly important considering the fact that mutations beneficial to the activity of the enzyme are fairly rare. In fact, using too high a level of mutagenesis may counter or inhibit the desired benefit of a useful mutation.

While both of the aforementioned methods are effective for creating randomized pools of macromolecule variants, a third method, termed "DNA Shuffling", or "sexual PCR" (WPC, Stemmer, PNAS, 91:10747, (1994)) has recently been elucidated. DNA shuffling has also been referred to as "directed molecular evolution", "exon-shuffling", "directed enzyme evolution", "in vitro evolution", and "artificial evolution". Such reference terms are known in the art and are encompassed by the invention. This new, preferred, method apparently overcomes the limitations of the previous methods in that it not only propagates positive traits, but simultaneously eliminates negative traits in the resulting progeny.

DNA shuffling accomplishes this task by combining the principal of in vitro recombination, along with the method of "error-prone" PCR. In effect, you begin with a randomly digested pool of small fragments of your gene, created by Dnase I digestion, and then introduce said random fragments into an "error-prone" PCR assembly reaction. During the PCR reaction, the randomly sized DNA fragments not only hybridize to their cognate strand, but also may hybridize to other DNA fragments corresponding to different regions of the polynucleotide of interest - regions not typically accessible via hybridization of the entire polynucleotide. Moreover, since the PCR assembly reaction utilizes "error-prone" PCR reaction conditions, random mutations are introduced during the DNA synthesis step of the PCR reaction for all of the fragments -further diversifying the potential hybridation sites during the annealing step of the reaction.

A variety of reaction conditions could be utilized to carry-out the DNA shuffling reaction. However, specific reaction conditions for DNA shuffling are provided, for example, in PNAS, 91:10747, (1994). Briefly:

Prepare the DNA substrate to be subjected to the DNA shuffling reaction. Preparation may be in the form of simply purifying the DNA from contaminating cellular material, chemicals, buffers, oligonucleotide primers, deoxynucleotides, RNAs, etc., and may entail the use of DNA purification kits as those provided by Qiagen, Inc., or by the Promega, Corp., for example.

Once the DNA substrate has been purified, it would be subjected to Dnase I digestion. About 2-4ug of the DNA substrate(s) would be digested with .0015 units of Dnase I (Sigma) per ul in 100ul of 50mM Tris-HCL, pH 7.4/1mM MgCl2 for 10-20 min. at room temperature. The resulting fragments of 10-50bp could then be purified by running them through a 2% low-melting point agarose gel by electrophoresis onto DE81 ion-exchange paper (Whatman) or could be purified using Microcon concentrators (Amicon) of the appropriate molecular weight cuttoff, or could use oligonucleotide purification columns (Qiagen), in addition to other methods known in the art. If using DE81 ion-exchange paper, the 10-50bp fragments could be eluted from said paper using 1M NaCL, followed by ethanol precipitation.

The resulting purified fragments would then be subjected to a PCR assembly reaction by resuspension in a PCR mixture containing: 2mM of each dNTP, 2.2mM MgCl2, 50 mM KCl, 10mM Tris•HCL, pH 9.0, and 0.1% Triton X-100, at a final fragment concentration of 10-30ng/ul. No primers are added at this point. *Taq* DNA polymerase (Promega) would be used at 2.5 units per 100ul of reaction mixture. A PCR program of 94 C for 60s; 94 C for 30s, 50-55 C for 30s, and 72 C for 30s using 30-45 cycles, followed by 72 C for 5min using an MJ Research (Cambridge, MA) PTC-150 thermocycler. After the assembly reaction is completed, a 1:40 dilution of the resulting primerless product would then be introduced into a PCR mixture (using the same buffer mixture used for the assembly reaction) containing 0.8um of each primer and subjecting this mixture to 15 cycles of PCR (using 94 C for 30s, 50 C for 30s, and 72 C for 30s). The referred primers would be primers corresponding to the nucleic acid sequences of the polynucleotide(s) utilized in the shuffling reaction. Said primers could consist of modified nucleic acid base pairs using methods known in the art and referred to else where herein, or could contain additional sequences (i.e., for adding restriction sites, mutating specific base-pairs, etc.).

The resulting shuffled, assembled, and amplified product can be purified using methods well known in the art (e.g., Qiagen PCR purification kits) and then subsequently cloned using appropriate restriction enzymes.

Although a number of variations of DNA shuffling have been published to date, such variations would be obvious to the skilled artisan and are encompassed by the invention. The DNA shuffling method can also be tailered to the desired level of mutagenesis using the methods described by Zhao, et al. (Nucl Acid Res., 25(6):1307-1308, (1997).

As described above, once the randomized pool has been created, it can then be subjected to a specific screen to identify the variant possessing the desired characteristic(s). Once the variant has been identified, DNA corresponding to the variant could then be used as the DNA substrate for initiating another round of DNA shuffling. This cycle of shuffling, selecting the optimized variant of interest, and then re-shuffling, can be repeated until the ultimate variant is obtained. Examples of model screens applied to identify variants created using DNA shuffling technology may be found in the following publications: J. C., Moore, et al., J. Mol. Biol., 272:336-347, (1997), F.R., Cross, et al., Mol. Cell. Biol., 18:2923-2931, (1998), and A. Crameri., et al., Nat. Biotech., 15:436-438, (1997).

DNA shuffling has several advantages. First, it makes use of beneficial mutations. When combined with screening, DNA shuffling allows the discovery of the best mutational combinations and does not assume that the best combination contains all the mutations in a population. Secondly, recombination occurs simultaneously with point mutagenesis. An effect of forcing DNA polymerase to synthesize full-length genes from the small fragment DNA pool is a background mutagenesis rate. In combination with a stringent selection method, enzymatic activity has been evolved up to 16000 fold increase over the wild-type form of the enzyme. In essence, the background mutagenesis yielded the genetic variability on which recombination acted to enhance the activity.

A third feature of recombination is that it can be used to remove deleterious mutations. As discussed above, during the process of the randomization, for every one beneficial mutation, there may be at least one or more neutral or inhibitory mutations. Such mutations can be removed by including in the assembly reaction an excess of the wild-type random-size fragments, in addition to the random-size fragments of the selected mutant from the previous selection. During the next selection, some of the most active variants of the polynucleotide/polypeptide/enzyme, should have lost the inhibitory mutations.

Finally, recombination enables parallel processing. This represents a significant advantage since there are likely multiple characteristics that would make a protein more desirable (e.g. solubility, activity, etc.). Since it is increasingly difficult to screen for more than one desirable trait at a time, other methods of molecular evolution tend to be inhibitory. However, using recombination, it would be possible to combine the randomized fragments of the best representative variants for the various traits, and then select for multiple properties at once.

DNA shuffling can also be applied to the polynucleotides and polypeptides of the present invention to decrease their immunogenicity in a specified host. For example, a particular varient of the present invention may be created and isolated using DNA shuffling technology. Such a variant may have all of the desired characteristics, though may be highly immunogenic in a host due to its novel intrinsic structure. Specifically, the desired characteristic may cause the polypeptide to have a non-native strucuture which could no longer be recognized as a "self' molecule, but rather as a "foreign", and thus activate a host immune response directed against the novel variant. Such a limitation can be overcome, for example, by including a copy of the gene sequence for a xenobiotic ortholog of the native protein in with the gene sequence of the novel variant gene in one or more cycles of DNA shuffling. The molar ratio of the ortholog and novel variant DNAs could be varied accordingly. Ideally, the resulting hybrid variant identified would contain at least some of the coding sequence which enabled the xenobiotic protein to evade the host immune system, and additionally, the coding sequence of the original novel varient that provided the desired characteristics.

Likewise, the invention encompasses the application of DNA shuffling technology to the evolution of polynucletotides and polypeptides of the invention, wherein one or more cycles of DNA shuffling include, in addition to the gene template DNA, oligonucleotides coding for known allelic sequences, optimized codon sequences, known variant sequences, known polynucleotide polymorphism sequences, known ortholog sequences, known homolog sequences, additional homologous sequences, additional non-homologous sequences, sequences from another species, and any number and combination of the above.

In addition to the described methods above, there are a number of related methods that may also be applicable, or desirable in certain cases. Representative among these are the methods discussed in PCT applications WO 98/31700, and WO 98/32845, which are hereby incorporated by reference. Furthermore, related methods can also be applied to the polynucleotide sequences of the present invention in order to evolve invention for creating ideal variants for use in gene therapy, protein engineering, evolution of whole cells containing the variant, or in the evolution of entire enzyme pathways containing polynucleotides of the invention as described in PCT applications WO 98/13485, WO 98/13487, WO 98/27230, WO 98/31837, and Crameri, A., et al., Nat. Biotech., 15:436-438, (1997), respectively.

Additional methods of applying "DNA Shuffling" technology to the polynucleotides and polypeptides of the present invention, including their proposed applications, may be found in US Patent No. 5,605,793; PCT Application No. WO 95/22625; PCT Application No. WO 97/20078; PCT Application No. WO 97/35966; and PCT Application No. WO 98/42832; PCT Application No. WO 00/09727 specifically provides methods for applying DNA shuffling to the identification of herbicide selective crops which could be applied to the polynucleotides and polypeptides of the present invention; additionally, PCT Application No. WO 00/12680 provides methods and compositions for generating, modifying, adapting, and optimizing polynucleotide sequences that confer detectable phenotypic properties on plant species; each of the above are hereby incorporated in their entirety herein for all purposes.

### Example 29 - Functional Determination of Proteins Using Microarray.

### Preparation of DNA Microarrays.

Clones in YTK12 yeast cells are grown on CMD-W plates at 30°C. PCR in 96 wells plates is used to amplify the inserts as follow: per clone, a 100 ul reaction mix consisting of 80ul sterile water, 10ul 10X RedTaq Buffer (Sigma), 2ul 10mM dNTP Mix (Boehringer Manheim), 2ul of a forward primer corresponding to a polynucleotide of the present invention, 100ng/ul, 2ul T7 Primer 100ng/ul, 4ul RedTaq DNA Polymerase (Sigma) and an aliquot of the yeast colony are mixed and subjected to 95°C for 2 minutes (once) then 94°C for 30 seconds, followed by 50°C for 30 seconds, and 72°C for 30 seconds (35 to 40 cycles) and finally 72°C step for 7 minutes. The PCR products are purified using gel filtration cartridges (Edge BioSytems) twice to eliminate completely excess primers. All PCR products are evaluated by agarose gel electrophoresis. Using a robot (TECAN), the PCR products are transferred to 384 well plates and samples are then dried under vacuum and resuspended in 20ul of 50% DMSO.

The DNA samples are arrayed onto amino silinated coated glass microscopic slides using a microarray GEN III spotter from Molecular Dynamics/Amersham Pharmacia Biotech. After the samples are air dried, UV crosslinking of the samples to the slide can be done by exposing the slides to 50 mJ pulse of light of wavelength 254nm.

### RNA isolation and preparation of labeled mRNA by in vitro transcription of cDNA.

Total RNA is isolated from various tissue (leaf, root, flower, stem, meristems, cell cultures, treated or untreated with chemicals or submitted or not to biotic or abiotic stresses) using a RNeasy kit from Qiagen. Total RNA is converted to cDNA as described below. First strand cDNA synthesis can be performed as follows: 3-5 ug of total RNA is mixed with 1 ul of T7-oligo(dT)₂₄ primer 5'-GGCCAGTGAATTGTAATACGCTCACTATAGGGAGGCGGTTTTTTTTTTTTTTTTTTTTTTTT-3' (SEQ ID NO:19) and the final volume is brought to 12 ul with DEPC-H₂O After heating the tube at 70°C for 5 minutes, 4ul of 5 X first Strand Buffer (Life technology), 1 ul of 0.1 M DTT and 1 ul of 10 mM dNTP are added. After 2 minutes incubation at 37°C, 3 ul of reverse transcriptase (SSII RT, 200-unit/ul, Life Technology) is added and incubation at 37°C for 1 hour followed.

The second strand synthesis is performed as follows: to the first strand reaction mixture 89 ul of DEPC-H₂O, 3 ul of 10mM dNTP, 1 ul of 10 U/ul *E.coli* DNA ligase, 4 ul of 10 U/ul *E.coli* DNA polymerase, and 2 ul of 2 U/ul *E.coli* RNase H are added and incubated at 16 °C for 2 hour. Then 2 ul of 5 U/ul T4 DNA polymerase are added and incubated at 16 °C for 5 min. The reaction is stopped by one phenol/chloroform extraction and the supernatant precipitated in the presence of 2.5 volume of 100% ethanol and 1/10 volume of 5M NH₄Ac. After mixing the sample, it is centrifuged immediately at 12 000g for 5 minutes at room temperature. The pellet is washed twice with 80% ethanol and air dried before being dissolved in 2 ul of RNase free water. In vitro transcription with Cy dye labeling is performed as follow:
per reaction, 1ul T7 10 X reaction buffer (Ambion), 3 ul of 25 mM ATP, CTP, GTP mix, 1 ul of 20 mM UTP (Ambion), 1 ul of cDNA, 3 ul of 5 mM of Cy3 or Cy5 (Amersham), 0.5 ul of the internal control PCR product (i.e. human CCR5), 1 ul of enzyme mix (Ambion, MEGAscript ^{™}) and 1 ul of T7 RNA polymerase (USB, 100 units/ul) are combined and incubated 37 °C for 4 - 6 hours in a water bath in the dark. The template cDNA is then removed by incubating at 37 °C for 15 minutes with 1 ul of RNase-free DNaseI. The RNA probe is then purified using the RNeasy mini protocol for RNA probe purification (Qiagen). Each probe is store protected from light at - 70 °C.

### Microarray hybridization and data analysis.

The two mRNA pools to be compared are mixed and applied to a microarray slide in 36 ul of a hybridization mixture containing 50% formamide, 5X SSC, 0.1% SDS. Hybridization can take place under a glass coverslip in a humidified plastic tube with a lid, in a 42°C oven for 14-18 hours. The slides are then washed twice for 5 minutes at 42°C in 0.5X SSC, 0.1% SDS, once for 10 minutes at 42°C in 0.25X SSC, 0.1% SDS, once in 0.25X SSC, 0.1% SDS at 55°C for 30 minutes, before being dried under a gentle stream of N₂.

Scanning of the slides is performed with a Generation III Array Scanner (Molecular Dynamic) at a PMT of 665V for Cy3 labeled probes and a PTM of 685V for Cy5 labeled probes. Capture of the data and critical analysis (background removal, normalization, and mean values...) is done using the software ArrayVision^{™} (Imaging Research, Inc.). The Cy5/Cy3 fluorescence ratio and the log₁₀-transformed ratio are calculated from the normalized values. The software SpotFire™ (Spotfire, Inc.) and GeneSpring™ (Silicon Genetics, Inc.) are used for data visualization and clustering analysis.

### Example 30 - Method of Transforming Plants Using Vacuum Infiltration

Transformation of plants may serve as a vital tool in assessing the biological function of a particular polynucleotide or polypeptide. For example, a plant may be transformed with a vector capable of downregulating a particular gene via anti-sense regulation (i.e., the vector may express a transcript of the gene of interest in the anti-sense direction), or the vector may simply be capable of overexpressing a particular polypeptide, for example. By observing the resulting phenotypes of the transformant, one may derive protein function using techniques known in the art and described elsewhere herein. Other uses for transformation methods are described elsewhere herein.

The following method of transforming plant material may be applicable to any plant species, though is particularly suited for use in Arabidopsis.

Grow Arabidopsis at 20°C, 8 hr light, 18°C 16 dark until needed for transformation. Fertilize once a week from below. Thin plants to∼1 per square inch. Use immediately upon bolting. Short days allow stronger vegetative plant growth and increase seed yield.

Transfer plants to 20°C, 16 hr light, 18 °C 8 hr dark. The plants should bolt quickly, and they are ready to infiltrate when the primary inflorescences are 10-15 cm tall and the secondary inflorecences are appearing at the rosette.

In the meantime, transform your constructs into Agrobacterium tumefaciens strain EHA105 (Koncz and Schell, 1986) (see Direct *Agrobacterium* Transformation:Freeze-Thaw Method below). When the plants are ready to transform use 1 ml of an overnight culture to innoculate a 500 ml culture of YEB medium (2 L flask) containing the appropriate antibiotic for your construct and 50 ug/mL rifampicin (C58 Agro_and or 25 mg/mL gentamycin (pMP90). Grow cultures 2 days at 28 °C, ∼275 rmp. YEB media is described below.

When OD₆₀₀ is greater than 2.0, centrifuge the culture 30 min, 3500 rpm and resuspend it in 0.5-1.0 ml of infiltration medium described below.

Place resuspended culture in a container with a large bell jar, and invert pots containing plants to be infiltrated into the infiltration medium so that the entire plant is covered (including the rosette, but not too much soil). Remove any large air bubbles under the plants. Draw a vacuum (∼700 Hg). Close the suction, and allow the plants to sit under vacuum for 5 min. Quickly release the vacuum pressure. Briefly drain the pots.

Grow infiltrated plants as before in 20°C, 16 hr light, 18 °C 8 hr dark. Stake plants as the bolts grow. When the plants are finished flowering, harvest the To seeds.

Sterilize seeds and screen for transformants on the selective medium descrived below. Transfer dark green (resistant) plants to secondary selection plate a week after germination, then to soil after 6-10 days. Keep new transplants covered for several days.

| Plant media: | | |
|---|---|---|
| For 1 L | **acuum Infiltration Medium** | **election Medium** |
| MS Salts | .2 g | 4.3 g |
| B5Vitamins, 1000X | .0 mL | 1.0 mL |
| Sucrose | 0 g | 10g |
| MES, 200mg/mL pH 5.7 with KOH | .5 mL | 2.5 mL |
| Benzylamonipurine (BAP, 1mg/mL) | 4 µL | - |
| Silweet L-77 | 00 µL | - |
| Phytagar | - | 8 g |

| Bacterial Media | YEP |
|---|---|
| Yeast extract | 1.0 g |
| Beef Extract | 5.0 g |
| Peptone | 5.0 g |
| Sucrose | 5.0 g |
| MgSO₄ | 0.5 g |

The skilled artisan would appreciate that the above transformation method could be modified to apply to other species of plants. Such modification may include the addition of new steps, the deletion of any of the steps described, and/or subsititution of reagents.

### Direct Agrobacterium Transformation:Freeze-Thaw Method.

Grow an *Agrobacterium* strain containing the appropriate helper Ti plasmid in 5 mL of YEP medium (YEP medium is described elsewhere herein) overnight at 28 °C. Add 2 ml of the overnight culture to 50 ml YEP medium in a 259 mL flask and shake vigorously (250 rpm) at 28 °C until the culture grows to an OD₆₀₀ of 0.5 to 1.0. Chill the culture on ice. Centrifuge the cell suspention at 3000 g for 5 min at 4 °C.

Discard the supernatant solution. Resuspend the cells in 1 mL of 20 mM CaCl₂ solution (ice-cold). Dispense 0.1 mL aliquots into prechilled Eppendorf tubes. Add about 1 ug of plasmid DNA to the cells.

Freeze the cells in liquid nitrogen Thaw the cells by incubating the test tube in a 37 °C water bath for 5 min. Add 1 mL of YEP medium to the tube and incubate at 28 °C for 2-4 hr with gentle rocking. This period allows the bacteria to express the antibiotic resistance genes. Centrifuge the tubes for 30 s in an Eppendorf centrifuge. Discard the supernatant solution. Resuspend the cells in 0.1 mL YEP medium

Spread the cells onto a YEP agar plate containing 3-5 ug/mL tetracycline and 10-25 ug/mL kanamycin. Incubate the plate at 28 °C. Transformed colonies should appear in 2-3 days.

The skilled artisan would appreciate that the above transformation method could be modified to apply to other species of plants. Such modification may include the addition of new steps, the deletion of any of the steps described, and/or subsititution of reagents.

### Example 31 - Functional Determination of Proteins Using Metabolite Profiling.

The present invention encompasses the application of metabolite profiling to the identification of gene function for the polypeptides of the present invention. In one example, transgenic plants could be produced which are either incapable of expressing a protein of the present invention, or that have decreased expression levels of a protein of the present invention. Such transgenic plants could be produced by creating knockout constructs to inactivate or delete the endogenous gene, for example, using methods known in the art and described elsewere herein. Alternatively, the transgenic plants could be produced by inserting into the plant a construct that expresses antagonists of a protein of the present invention (e.g., antisense oligonucleotides, antisense genes, antibodies, etc.). Other examples of methods of producing transgenic plants, including specific strategies, are known in the art, some of which are described elsewere herein.

Once a protein of the present invention is inactivated, or its expression inhibited, the resulting metabolite profile of the plant can be ascertained, and the function of the protein assigned. Some of the anticipated metabolic profiles of inhibiting or inactivating the expression of a protein of the present invention in a plant may resemble known nutritional deficiencies, pathogenic diseases, biotic stresses, or abiotic stresses, for example, many of which are disclosed elsewere herein. In addition, the metabolic profile of a transgenic plant of the present invention may be useful in identifying the specific pathways the polypeptide of the present invention is a member of, in addition, to identifying the potential downstream and/or upstream effectors or affectors, respectively. In addition, it may be possible to identify the mode of action of a polypeptide of the present invention.

A number of methods are known in the art for identifying the metabolic profile of a plant. A non-limiting example is provided by Sauter, H., et al., in "Metabolic Profiling of Plants: A New Diagnostic Technique", Synthesis and Chemistry of Agrochemicals II, Baker, D.R., Fenyes, J.G., and Moberg, W.K., eds, ACS Symposium Series, 433, Chapter 24, pp. 288-299, (1991). Briefly, transgenic plants of the invention, or plants in which the expression of a polypeptide of the present invention is inhibited or inactivated, are grown in growth chambers. The shoots are harvested and immediately deep frozen until further treatment. The frozen plant samples are weighed and a threefold amount (W:W) of ethanol is added. The mixture is then macerated in a mixer and the resulting suspension is left for 2 hours for extraction. The next steps are filtration, evaporation, and silylation with N-Methyl-N-(trimethylsilyl)triflouroacetamide (MSTFA). Internal standard alkanes are also added, thus allowing for the calculation of retention coefficients, as well as, quantification. The crude mixture is then subjected to gas chromotography on a methyl silicon gum fused silica capillary column (30 m DB-1. Injection temperature 230°C. Oven temperature 100°-320°C, 4°C/min; 15 min 320°C). Retention coefficients are then calculated relative to internal standards (n-C10H22=1000, n-C28H58=2800).

The above protocol can be applied to numerous test plants, in addition, to controls. The data from the resulting profiles are then grouped together (i.e., one group for the test plants, another group for the controls) to arrive at an average profile for each group. In the latter step, the corresponding peaks (i.e., those peaks with equal retention coefficients) are grouped together and the peak heights are subjected to statistical analysis.

The differences in metabolic profiles between the test and control plants are determined by calculating the "difference profile" between the two groups. The difference profile is calculated by dividing the peal heights. This difference profile provides a semiquantitative estimate of the change in magnitude of one metabolic with respect to the other.

Once the above is completed, the peaks are then associated with particular metabolites (i.e., the metabolite identify of each peak is determined). By comparing the metabolite profile of proteins known to modulate specific pathways in a plant, to those of the present invention, clues for and/or identification of the function of a polypeptide of the present invention may be determined. Other methods are known in the art, and any one or more steps, may be equally substituted with such methods.

### Example 32 - Functional Determination of Proteins Using Morphological Phenotyping.

The present invention encompasses the application of morphological phenotyping to the identification of gene function for the polypeptides of the present invention. In one example, transgenic plants could be produced which are either incapable of expressing a protein of the present invention, or that have decreased expression levels of a protein of the present invention. Such transgenic plants could be produced by creating knockout constructs to inactivate or delete the endogenous gene, for example, using methods known in the art and described elsewere herein. Alternatively, the transgenic plants could be produced by inserting into the plant a construct that expresses antagonists of a protein of the present invention (e.g., antisense oligonucleotides, antisense genes, antibodies, etc.). Other examples of methods of producing transgenic plants, including specific strategies, are known in the art, some of which are described elsewere herein.

Once a protein of the present invention is inactivated, or its expression inhibited, the resulting morphological phenotype of the plant can be ascertained, and the function of the protein assigned. Some of the anticipated phenotypes of inhibiting or inactivating the expression of a protein of the present invention in a plant may resemble known nutritional deficiencies, pathogenic diseases, biotic stresses, or abiotic stresses, for example, many of which are disclosed elsewere herein.

### Example 33 - Functional Determination of Proteins Using TransGenomic Technology (TGT).

The present invention encompasses the application of TransGenomic Technology (TGT) to the identification of gene function for the polypeptides of the present invention (Cambia Biosystems, Bellevue, WA). TGT combines both genomics and functional genomics, enabling the association of gene sequences (e.g., the polynucleotide sequences of the present invention) with specific phenotypes or traits. The technology is based upon the random integration of reporter gene [e.g. beta-Glucuronidase (GUS), BOGUS, GFP] transposons throughout a plant's genome (specifically rice and Arabidopsis).

The transposon may comprise an enhancer trap vector containing a transcriptional activator and a reporter gene capable of responding to the transactivator. Since the integration is random, it is expected that the transposon may integrate near a gene regulatory element - effectively turning on the transactivator gene, the activity of said gene promoting activation of the reporter gene. Thus, by repeating the process above, a large population of cell lines with unique expression patterns can be generated. These "pattern lines" are genetically characterized in an effort to select lines with desired expression patterns. The pattern lines can be used for reverse genetics studies to identify lines with mutations in genes of interest and may also be used as a genetic background to obtain gain-of-function (GOF) and loss-of-function (LOF) mutations. Moreover, novel expression (or lack of expression) patterns may uncover novel phenotypes and traits. In addition, the pattern lines showing desirable expression patterns could serve as a genetic foundation to direct the expression of genes in target tissues or at desired developmental stages.

The TGT technology described above can be used to identify the function of the polynucleotides and/or polypeptides of the present invention. For example, the polynucleotide sequences of the present invention can be used to generate PCR primers suitable for the screening and identification of cell lines positively tagged with the TGT technology upstream of a polynucleotide of the present invention. Once identified, the function of the polynucleotide sequence may be ascertained by linking the genes of the present invention tagged with the TGT technology with specific phenotypes or traits observed in a particular cell line, or pattern line. Another possible application of TGT to the polynucleotides of the present invention would be in targeting gene expression of selected polynucleotides of the present invention. For example, polynucleotide sequences of the present invention flanked by an upstream regulatory sequence may be introduced into pattern lines showing desired expression patterns using techniques known in the art of molecular biology and described elsewhere herein.

A number of variations of the above technology are known in the art and would be appreciated by the skilled artisan. For example, the skilled artisan would appreciate that other transposons, reporter genes, and activating sequences, etc., could be substituted or used in conjunction with the TGT technology. Specific methods of applying TGT technology can be found, for example, in Wilson KJ, et al., Microbiol, 141:1691-1705, (1995), and in US Patent Nos. 5,879,906, 5,599,670, and 5,432,081; which are hereby incorporated by reference in their entirety herein.

### Example 34 - Analysis of the Impact of Recombinant Proteins on the Production of a Desired Seed Storage Compound

The effect of the genetic modification in plants on a desired seed storage compound (such as sugars, lipids and fatty acids) can be assessed by growing the modified plant under suitable conditions and analyzing the seeds or any other plant organ for increased production of the desired product (i.e., a lipid or a fatty acid). Such analysis techniques are well known to one skilled in the art, and include spectroscopy, thin layer chromatography, staining methods of various kinds, enzymatic and microbiological methods, and analytical chromatography such as high performance liquid chromatography (see, for example, Ullman 1985, Encyclopedia of Industrial Chemistry, vol. A2, pp. 89-90 and 443-613, VCH: Weinheim; Fallon, A. et al. 1987, Applications of HPLC in Biochemistry in: Laboratory Techniques in Biochemistry and Molecular Biology, vol. 17; Rehm et al., 1993 Product recovery and purification, Biotechnology, vol. 3, Chapter III, pp. 469-714, VCH: Weinheim; Belter, P.A. et al., 1988 Bioseparations: downstream processing for biotechnology, John Wiley & Sons; Kennedy J.F. & Cabral J.M.S. 1992, Recovery processes for biological materials, John Wiley and Sons; Shaeiwitz J.A. & Henry J.D. 1988, Biochemical separations in: Ulmann's Encyclopedia of Industrial Chemistry, Separation and purification techniques in biotechnology, vol. B3, Chapter 11, pp. 1-27, VCH: Weinheim; and Dechow F.J. 1989).

Besides the above-mentioned methods, plant lipids are extracted from plant material as described by Cahoon et al. (1999, Proc. Natl. Acad. Sci. USA 96, 22: 12935-12940) and Browse et al. (1986, Anal. Biochemistry 442: 141-145). Qualitative and quantitative lipid or fatty acid analysis is described in Christie, William W., Advances in Lipid Methodology. Ayr/Scotland : Oily Press. - (Oily Press Lipid Library ; 2); Christie, William W., Gas Chromatography and Lipids. A Practical Guide - Ayr, Scotland : Oily Press, 1989 Repr. 1992. - IX,307 S. - (Oily Press Lipid Library ; 1); "Progress in Lipid Research, Oxford : Pergamon Press, 1 (1952) - 16 (1977) Progress in the Chemistry of Fats and Other Lipids CODEN.

Unequivocal proof for the presence of fatty acid products can be obtained by the analysis of transgenic plants following standard analytical procedures: GC, GC-MS or TLC as variously described by Christie and references therein (1997 in: Advances on Lipid Methodology 4th ed.: Christie, Oily Press, Dundee, pp. 119-169; 1998). Detailed methods are described for leaves by Lemieux et al. (1990, Theor. Appl. Genet. 80: 234-240) and for seeds by Focks & Benning (1998, Plant Physiol. 118: 91-101)

Positional analysis of the fatty acid composition at the C-1, C-2 or C-3 positions of the glycerol backbone is determined by lipase digestion (see, e.g., Siebertz & Heinz 1977, Z. Naturforsch. 32c: 193-205, and Christie 1987, Lipid Analysis 2nd Edition, Pergamon Press, Exeter, ISBN 0-08-023791-6).

A typical way to gather information of the influence of increased or decreased protein activities on lipid and sugar biosynthetic pathways is for example via analyzing the carbon fluxes by labeling studies with leaves or seeds using ¹⁴C-acetate or ¹⁴C-pyruvate (see, e.g. Focks & Benning 1998, Plant Physiol. 118: 91-101; Eccleston & Ohlrogge 1998, Plant Cell 10: 613-621). The distribution of carbon-14 into lipids and aqueous soluble components can be determined by liquid scintillation counting after the respective separation (for example on TLC plates) including standards like ¹⁴C-sucrose and ¹⁴C-malate (Eccleston & Ohlrogge 1998, Plant Cell 10: 613-621).

Material to be analyzed can be disintegrated via sonification, glass milling, liquid nitrogen and grinding or via other applicable methods. The material has to be centrifuged after disintegration. The sediment is re-suspended in distilled water, heated for 10 minutes at 100°C, cooled on ice and centrifuged again followed by extraction in 0.5 M sulfuric acid in methanol containing 2% dimethoxypropane for 1 hour at 90°C leading to hydrolyzed oil and lipid compounds resulting in transmethylated lipids. These fatty acid methyl esters are extracted in petrolether and finally subjected to GC analysis using a capillary column (Chrompack, WCOT Fused Silica, CP-Wax-52 CB, 25 m, 0.32 mm) at a temperature gradient between 170°C and 240°C for 20 minutes and 5 min. at 240°C. The identity of the resulting fatty acid methylesters is defined by the use of standards available form commercial sources (i.e., Sigma).

In case of fatty acids where standards are not available, molecule identity is shown via derivatization and subsequent GC-MS analysis. For example, the localization of triple bond fatty acids is shown via GC-MS after derivatization via 4,4-Dimethoxy-oxazolin-Derivaten (Christie, Oily Press, Dundee, 1998).

A common standard method for analyzing sugars, especially starch, is published by Stitt M., Lilley R.Mc.C., Gerhardt R. and Heldt M.W. (1989, "Determination of metabolite levels in specific cells and subcellular compartments of plant leaves" Methods Enzymol. 174: 518-552; for other methods see also Härtel et al. 1998, Plant Physiol. Biochem. 36: 407-417 and Focks & Benning 1998, Plant Physiol. 118: 91-101).

For the extraction of soluble sugars and starch, 50 seeds are homogenized in 500 µl of 80% (v/v) ethanol in a 1.5-ml polypropylene test tube and incubated at 70°C for 90 min. Following centrifugation at 16,000 g for 5 min, the supernatant is transferred to a new test tube. The pellet is extracted twice with 500 µl of 80% ethanol. The solvent of the combined supernatants is evaporated at room temperature under a vacum. The residue is dissolved in 50 µl of water, representing the soluble carbohydrate fraction. The pellet left from the ethanol extraction, which contains the insoluble carbohydrates including starch, is homogenized in 200 µl of 0.2 N KOH, and the suspension is incubated at 95°C for 1 h to dissolve the starch. Following the addition of 35 µl of 1 N acetic acid and centrifugation for 5 min at 16,000 g, the supernatant is used for starch quantification.

To quantify soluble sugars, 10 µl of the sugar extract is added to 990 µl of reaction buffer containing 100 mM imidazole, pH 6.9, 5 mM MgCl₂, 2 mM NADP, 1 mM ATP, and 2 units 2 ml⁻¹ of Glucose-6-P-dehydrogenase. For enymatic determination of glucose, fructose and sucrose, 4.5 units of hexokinase, 1 unit of phosphoglucoisomerase, and 2 µl of a saturated fructosidase solution are added in succession. The production of NADPH is photometrically monitored at a wavelength of 340 nm. Similarly, starch is assayed in 30 µl of the insoluble carbohydrate fraction with a kit from Boehringer Mannheim.

An example for analyzing the protein content in leaves and seeds can be found by Bradford M.M. (1976, "A rapid and sensitive method for the quantification of microgram quantities of protein using the principle of protein dye binding" Anal. Biochem.72: 248-254). For quantification of total seed protein, 15-20 seeds are homogenized in 250 µl of acetone in a 1.5-ml polypropylene test tube. Following centrifugation at 16,000 g, the supernatant is discarded and the vacuum-dried pellet is resuspended in 250 µl of extraction buffer containing 50 mM Tris-HCl, pH 8.0, 250 mM NaCl, 1 mM EDTA, and 1% (w/v) SDS. Following incubation for 2 h at 25°C, the homogenate is centrifuged at 16,000 g for 5 min and 200 ml of the supernatant will be used for protein measurements. In the assay γ-globulin is used for calibration. For protein measurements Lowry DC protein assay (Bio-Rad) or Bradford-assay (Bio-Rad) are used.

Enzymatic assays of hexokinase and fructokinase are performed spectrophotometrically according to Renz et al. (1993, Planta 190: 156-165), of phosphoglucoisomerase, ATP-dependent 6-phosphofructokinase, pyrophosphate-dependent 6-phosphofructokinase, Fructose-1,6-bisphosphate aldolase, triose phosphate isomerase, glyceral-3-P dehydrogenase, phosphoglycerate kinase, phosphoglycerate mutase, enolase and pyruvate kinase are performed according to Burrell et al. (1994, Planta 194: 95-101) and of UDP-Glucose-pyrophosphorylase according to Zrenner et al. (1995, Plant J. 7: 97-107).

Intermediates of the carbohydrate metabolism, like Glucose-1-phosphate, Glucose-6-phosphate, Fructose-6-phosphate, Phosphoenolpyruvate, Pyruvate, and ATP are measured as described in Härtel et al. (1998, Plant Physiol. Biochem. 36: 407-417) and metabolites are measured as described in Jelitto et al. (1992, Planta 188: 238-244).

In addition to the measurement of the final seed storage compound (i.e., lipid, starch or storage protein) it is also possible to analyze other components of the metabolic pathways utilized for the production of a desired seed storage compound, such as intermediates and side-products, to determine the overall efficiency of production of the compound (Fiehn et al. 2000, Nature Biotech. 18: 1447-1161).

For example, yeast expression vectors comprising the nucleic acids disclosed herein, or fragments thereof, can be constructed and transformend into *Saccromycetes cerevisie* using standard protocols. The resulting transgenic cells can then be assayed for alterations in sugar, oil, lipid or fatty acid contents.

Similarly, plant expression vectors comprising the nucleic acids disclosed herein, or fragments thereof, can be constructed and transformend into an appropriate plant cell such as Arabidopsis, soybean, rape, maize, wheat, *Medicago truncatula,* etc., using standard protocols. The resulting transgenic cells and/or plants derived therefrom can then be assayed for alterations in sugar, oil, lipid or fatty acid contents.

Additionally, the sequences disclosed herein, or fragments thereof, can be used to generate knockout mutations in the genomes of various organisms, such as bacteria, mammalian cells, yeast cells, and plant cells (Girke at al. 1998, Plant J. 15: 39-48). The resultant knockout cells can then be evaluated for their composition and content in seed storage compounds, and the effect on the phenotype and/or genotype of the mutation. For other methods of gene inactivation include US 6004804 "Non-Chimeric Mutational Vectors" and Puttaraju et al. (1999, "Spliceosome-mediated RNA trans-splicing as a tool for gene therapy" Nature Biotech. 17:246-252).

### Further disclosure:

1. An isolated prenyl protease coding nucleic acid, wherein the nucleic acid comprises a polynucleotide selected from the group consisting of:
   a) a polynucleotide of SEQ ID NO: 1;
   b) a polynucleotide of SEQ ID NO:3;
   c) a polynucleotide of SEQ ID NO:5;
   d) a polynucleotide of SEQ ID NO:7;
   e) a polynucleotide of SEQ ID NO:9;
   f) a polynucleotide encoding a polypeptide of SEQ ID NO:2;
   g) a polynucleotide encoding a polypeptide of SEQ ID NO:4;
   h) a polynucleotide encoding a polypeptide of SEQ ID NO:6;
   i) a polynucleotide encoding a polypeptide of SEQ ID NO:8;
   j) a polynucleotide encoding a polypeptide of SEQ ID NO: 1 0;
   k) a polynucleotide comprising at least 60 consecutive nucleotides of any of a) through j) above;
   l) a polynucleotide having at least 70% sequence identity with the nucleotide sequence of SEQ ID NOs:1, 3, 5, 7, or 9;
   m) a polynucleotide encoding a polypeptide having at least 70% sequence identity with the polypeptide of SEQ ID NOs:2, 4, 6, 8 or 10; and
   n) a polynucleotide complementary to a polynucleotide of any of a) through m).
2. The prenyl protease coding nucleic acid of Claim 1, wherein the nucleic acid comprises a polynucleotide encoding the polypeptide of SEQ ID NO:2.
3. The prenyl protease coding nucleic acid of Claim 1, wherein the nucleic acid comprises a polynucleotide encoding the polypeptide of SEQ ID NO:4.
4. The prenyl protease coding nucleic acid of Claim 1, wherein the nucleic acid comprises a polynucleotide encoding the polypeptide of SEQ ID NO:6.
5. The prenyl protease coding nucleic acid of Claim 1, wherein the nucleic acid comprises a polynucleotide encoding the polypeptide of SEQ ID NO:8.
6. The prenyl protease coding nucleic acid of Claim 1, wherein the nucleic acid comprises a polynucleotide encoding the polypeptide of SEQ ID NO:10.
7. The prenyl protease coding nucleic acid of Claim 1, wherein the nucleic acid comprises a polynucleotide encoding a polypeptide having at least 90% sequence identity to the polypeptide of SEQ ID NOs:2, 4, 6, 8 or 10.
8. The prenyl protease coding nucleic acid of Claim 1, wherein the nucleic acid comprises at least 60 consecutive nucleotides of any of the polynucleotides of a) through j) of Claim 1.
9. The prenyl protease coding nucleic acid of Claim 1, wherein the nucleic acid comprises a polynucleotide having at least 90% sequence identity with the nucleotide sequence of SEQ ID NOs:1, 3, 5, 7 or 9.
10. A vector comprising a nucleic acid of Claim 1.
11. The prenyl protease coding nucleic acid of Claim 1, wherein the nucleic acid encodes a polypeptide that functions as a modulator of an environmental stress response in a plant.
12. The prenyl protease coding nucleic acid of Claim 1, wherein underexpression of the nucleic acid in a plant increases the plant's tolerance to an environmental stress.
13. The prenyl protease coding nucleic acid of Claim 12, wherein the environmental stress is drought.
14. The prenyl protease coding nucleic acid of Claim 1, wherein the nucleic acid encodes a polypeptide that functions in protein farnesylation.
15. A first isolated nucleic acid that hybridizes under stringent conditions to a second nucleic acid selected from the group consisting of:
   a) a second nucleic acid comprising a polynucleotide of SEQ ID NOs:1, 3, 5 7 or 9; and
   b) a second nucleic acid encoding a polypeptide of SEQ ID NOs:2, 4, 6, 8 or 10, wherein the first nucleic acid encodes a polypeptide that functions as a modulator of an environmental stress response in a plant.
16. A transgenic plant cell comprising a prenyl protease coding nucleic acid of Claim 1.
17. A transgenic plant comprising a plant cell of Claim 16.
18. The plant of Claim 17, wherein the plant is a monocot.
19. The plant of Claim 17, wherein the plant is a dicot.
20. The plant of Claim 17, wherein the plant is selected from the group consisting of maize, wheat, rye, oat, triticale, rice, barley, soybean, peanut, cotton, rapeseed, canola, manihot, pepper, sunflower, tagetes, solanaceous plants, potato, tobacco, eggplant, tomato, Vicia species, pea, alfalfa, coffee, cacao, tea, Salix species, oil palm, coconut, perennial grass and a forage crop.
21. A plant seed produced by the plant of Claim 17.
22. The seed of Claim 21, wherein the seed is true breeding for an increased tolerance to environmental stress as compared to a wild type variety of the seed.
23. A method of modulating a plant's tolerance to an environmental stress comprising, modifying the expression of a prenyl protease coding nucleic acid in the plant, wherein the nucleic acid is selected from the group consisting of:
   a) a polynucleotide of SEQ ID NO:1;
   b) a polynucleotide of SEQ ID NO:3;
   c) a polynucleotide of SEQ ID NO:5;
   d) a polynucleotide of SEQ ID NO:7;
   e) a polynucleotide of SEQ ID NO:9;
   f) a polynucleotide encoding a polypeptide of SEQ ID NO:2;
   g) a polynucleotide encoding a polypeptide of SEQ ID NO:4;
   h) a polynucleotide encoding a polypeptide of SEQ ID NO:6;
   i) a polynucleotide encoding a polypeptide of SEQ ID NO:8;
   j) a polynucleotide encoding a polypeptide of SEQ ID NO: 1 0;
   k) a polynucleotide comprising at least 60 consecutive nucleotides of any of a) through j) above;
   l) a polynucleotide having at least 70% sequence identity with the nucleotide sequence of SEQ ID NOs:1, 3, 5, 7, or 9;
   m) a polynucleotide encoding a polypeptide having at least 70% sequence identity with the polypeptide of SEQ ID NOs:2, 4, 6, 8 or 10; and
   n) a polynucleotide complementary to a polynucleotide of any of a) through m).
24. The method of Claim 23, wherein the environmental stress is selected from the group consisting of high salinity, drought and temperature.
25. The method of Claim 23, wherein the nucleic acid comprises a polynucleotide encoding the polypeptide of SEQ ID NO:2.
26. The method of Claim 23, wherein the nucleic acid comprises a polynucleotide encoding the polypeptide of SEQ ID NO:4.
27. The method of Claim 23, wherein the nucleic acid comprises a polynucleotide encoding the polypeptide of SEQ ID NO:6.
28. The method of Claim 23, wherein the nucleic acid comprises a polynucleotide encoding the polypeptide of SEQ ID NO:8.
29. The method of Claim 23, wherein the nucleic acid comprises a polynucleotide encoding the polypeptide of SEQ ID NO:10.
30. The method of Claim 23, wherein the nucleic acid comprises a polynucleotide encoding a polypeptide having at least 90% sequence identity to the polypeptide of SEQ ID NOs:2, 4, 6, 8 or 10.
31. The method of Claim 23, wherein the nucleic acid comprises at least 60 consecutive nucleotides of any of the polynucleotides of a) through j).
32. The method of Claim 23, wherein the nucleic acid comprises a polynucleotide having at least 90% sequence identity with the nucleotide sequence of SEQ ID Nos:1, 3, 5, 7 or 9.
33. The method of Claim 23, wherein the plant's stress tolerance is increased by decreasing expression of the nucleic acid in the plant.
34. The method of Claim 23, wherein the nucleic acid encodes a polypeptide that functions in protein farnesylation.
35. The method of Claim 23, wherein the plant is not transgenic.
36. The method of Claim 23, wherein the plant is transgenic.
37. The method of Claim 36, wherein the plant is transformed with a promoter that directs expression of the nucleic acid.
38. The method of Claim 37, wherein the promoter is tissue specific.
39. The method of Claim 37, wherein the promoter is developmentally regulated.
40. A method of producing a transgenic plant containing a prenyl protease coding nucleic acid wherein the plant has an increased tolerance to an environmental stress as compared to a wild type variety of the plant comprising, transforming a plant cell with an expression vector comprising the nucleic acid and generating from the plant cell the transgenic plant, wherein the nucleic acid is selected from the group consisting of:
   a) a polynucleotide of SEQ ID NO:1;
   b) a polynucleotide of SEQ ID NO:3;
   c) a polynucleotide of SEQ ID NO:5;
   d) a polynucleotide of SEQ ID NO:7;
   e) a polynucleotide of SEQ ID NO:9;
   f) a polynucleotide encoding a polypeptide of SEQ ID NO:2;
   g) a polynucleotide encoding a polypeptide of SEQ ID NO:4;
   h) a polynucleotide encoding a polypeptide of SEQ ID NO:6;
   i) a polynucleotide encoding a polypeptide of SEQ ID NO:8;
   j) a polynucleotide encoding a polypeptide of SEQ ID NO: 1 0;
   k) a polynucleotide comprising at least 60 consecutive nucleotides of any of a) through j) above;
   l) a polynucleotide having at least 70% sequence identity with the nucleotide sequence of SEQ ID NOs:1, 3, 5, 7, or 9;
   m) a polynucleotide encoding a polypeptide having at least 70% sequence identity with the polypeptide of SEQ ID NOs:2, 4, 6, 8 or 10; and
   n) a polynucleotide complementary to a polynucleotide of any of a) through m).
41. The method of Claim 40, wherein the environmental stress is selected from the group consisting of high salinity, drought and low temperature.
42. The method of Claim 40, wherein the plant is a monocot.
43. The method of Claim 40, wherein the plant is a dicot.
44. The method of Claim 40, wherein the plant is selected from the group consisting of maize, wheat, rye, oat, triticale, rice, barley, soybean, peanut, cotton, rapeseed, canola, manihot, pepper, sunflower, tagetes, solanaceous plants, potato, tobacco, eggplant, tomato, Vicia species, pea, alfalfa, coffee, cacao, tea, Salix species, oil palm, coconut, perennial grass and a forage crop.
45. The method of Claim 44, wherein the nucleic acid comprises a polynucleotide encoding the polypeptide of SEQ ID NO:2.
46. The method of Claim 44, wherein the nucleic acid comprises a polynucleotide encoding the polypeptide of SEQ ID NO:4.
47. The method of Claim 44, wherein the nucleic acid comprises a polynucleotide encoding the polypeptide of SEQ ID NO:6.
48. The method of Claim 44, wherein the nucleic acid comprises a polynucleotide encoding the polypeptide of SEQ ID NO:8.
49. The method of Claim 44, wherein the nucleic acid comprises a polynucleotide encoding the polypeptide of SEQ ID NO:10.
50. The method of Claim 44, wherein the nucleic acid comprises a polynucleotide encoding a polypeptide having at least 90% sequence identity to the polypeptide of SEQ ID NOs:2, 4, 6, 8 or 10.
51. The method of Claim 44, wherein the nucleic acid comprises at least 60 consecutive nucleotides of any of the polynucleotides of a) through j).
52. The method of Claim 44, wherein the nucleic acid comprises a polynucleotide having at least 90% sequence identity with the nucleotide sequence of SEQ ID NOs:1, 3, 5, 7 or 9.
53. The method of Claim 44, wherein the plant's stress tolerance is increased by desreasing expression of the nucleic acid in the plant.
54. The method of Claim 44, wherein the nucleic acid encodes a polypeptide that functions in protein farnesylation.
55. The method of Claim 44, wherein the plant is transformed with a promoter that directs expression of the nucleic acid.
56. The method of Claim 55, wherein the promoter is tissue specific.
57. The method of Claim 55, wherein the promoter is developmentally regulated.
58. An isolated promoter coding nucleic acid, wherein the nucleic acid comprises a polynucleotide selected from the group consisting of:
   a) a polynucleotide of SEQ ID NO:11;
   b) a polynucleotide comprising at least 60 consecutive nucleotides of a) above;
   c) a polynucleotide having at least 70% sequence identity with the polynucleotide sequence of SEQ ID NO:11;
   d) a polynucleotide complementary to a polynucleotide of any of a) through c).
59. The promoter coding nucleic acid of Claim 58, wherein the nucleic acid comprises a polynucleotide encoding the polypeptide of SEQ ID NO: 11.
60. The promoter of Claim 58, wherein the nucleic acid comprises at least 60 consecutive nucleotides of any of the polynucleotides of a) through c) of Claim 58.
61. The promoter coding nucleic acid of Claim 1, wherein the nucleic acid comprises a polynucleotide having at least 90% sequence identity with the nucleotide sequence of SEQ ID NO: 11.
62. A vector comprising a nucleic acid of Claim 58.
63. The promoter coding nucleic acid of Claim 58, wherein the nucleic acid functions as a modulator of an environmental stress response in a plant.
64. The promoter coding nucleic acid of Claim 58, wherein underpromotion of the nucleic acid in a plant increases the plant's tolerance to an environmental stress.
65. The promoter coding nucleic acid of Claim 64, wherein the environmental stress is drought.
66. A first isolated nucleic acid that hybridizes under stringent conditions to a second nucleic acid comprising a polynucleotide of SEQ ID NO: 11.
67. A transgenic plant cell comprising a promoter coding nucleic acid of Claim 58.
68. A transgenic plant comprising a plant cell of Claim 67.
69. The plant of Claim 68, wherein the plant is a monocot.
70. The plant of Claim 68, wherein the plant is a dicot.
71. The plant of Claim 68, wherein the plant is selected from the group consisting of maize, wheat, rye, oat, triticale, rice, barley, soybean, peanut, cotton, rapeseed, canola, manihot, pepper, sunflower, tagetes, solanaceous plants, potato, tobacco, eggplant, tomato, Vicia species, pea, alfalfa, coffee, cacao, tea, Salix species, oil palm, coconut, perennial grass and a forage crop.
72. A plant seed produced by the plant of Claim 68.
73. The seed of Claim 72, wherein the seed is true breeding for an increased tolerance to environmental stress as compared to a wild type variety of the seed.
74. A method of modulating a plant's tolerance to an environmental stress comprising, modifying the expression of a promoter coding nucleic acid in the plant, wherein the nucleic acid is selected from the group consisting of:
   a) a polynucleotide of SEQ ID NO:11;
   b) a polynucleotide comprising at least 60 consecutive nucleotides of a) above;
   c) a polynucleotide having at least 70% sequence identity with the polynucleotide sequence of SEQ ID NO:11;
   d) a polynucleotide complementary to a polynucleotide of any of a) through c).
75. The method of Claim 74, wherein the environmental stress is selected from the group consisting of high salinity, drought and temperature.
76. The method of Claim 74, wherein the nucleic acid comprises a polynucleotide of SEQ ID NO:11.
77. The method of Claim 74, wherein the nucleic acid comprises at least 60 consecutive nucleotides of any of the polynucleotides of a) through c).
78. The method of Claim 74, wherein the nucleic acid comprises a polynucleotide having at least 90% sequence identity with the nucleotide sequence of SEQ ID NO: 11.
79. The method of Claim 74, wherein the plant's stress tolerance is increased by decreasing expression of the nucleic acid in the plant.
80. The method of Claim 74, wherein the plant is not transgenic.
81. The method of Claim 74, wherein the plant is transgenic.
82. The method of Claim 74, wherein the promoter is tissue specific.
83. The method of Claim 74, wherein the promoter is developmentally regulated.
84. A method of producing a transgenic plant containing a promoter coding nucleic acid wherein the plant has an increased tolerance to an environmental stress as compared to a wild type variety of the plant comprising, transforming a plant cell with an expression vector comprising the nucleic acid and generating from the plant cell the transgenic plant, wherein the nucleic acid is selected from the group consisting of:
   a) a polynucleotide of SEQ ID NO:11;
   b) a polynucleotide comprising at least 60 consecutive nucleotides of a) above;
   c) a polynucleotide having at least 70% sequence identity with the polynucleotide sequence of SEQ ID NO:11;
   d) a polynucleotide complementary to a polynucleotide of any of a) through c).
85. The method of Claim 84, wherein the environmental stress is selected from the group consisting of high salinity, drought and low temperature.
86. The method of Claim 84, wherein the plant is a monocot.
87. The method of Claim 84, wherein the plant is a dicot.
88. The method of Claim 84, wherein the plant is selected from the group consisting of maize, wheat, rye, oat, triticale, rice, barley, soybean, peanut, cotton, rapeseed, canola, manihot, pepper, sunflower, tagetes, solanaceous plants, potato, tobacco, eggplant, tomato, Vicia species, pea, alfalfa, coffee, cacao, tea, Salix species, oil palm, coconut, perennial grass and a forage crop.
89. The method of Claim 84, wherein the nucleic acid comprises a polynucleotide of SEQ ID NO:11.
90. The method of Claim 84, wherein the nucleic acid comprises at least 60 consecutive nucleotides of any of the polynucleotides of a) through c).
91. The method of Claim 84, wherein the nucleic acid comprises a polynucleotide having at least 90% sequence identity with the nucleotide sequence of SEQ ID NO: 11.
92. The method of Claim 84, wherein the plant's stress tolerance is increased by desreasing expression of the nucleic acid in the plant.
93. The method of Claim 84, wherein the promoter is tissue specific.
94. The method of Claim 84, wherein the promoter is developmentally regulated.

## Claims

**1.** An isolated prenyl protease coding nucleic acid, wherein the nucleic acid comprises a polynucleotide selected from the group consisting of:
a) a polynucleotide of SEQ ID NO: 3;
b) a polynucleotide encoding a polypeptide of SEQ ID NO: 4;
c) a polynucleotide having at least 70% sequence identity with the nucleotide sequence of SEQ ID NO: 3,
d) a polynucleotide encoding a polypeptide having at least 70% sequence identity with the polypeptide of SEQ ID NO: 4 and
e) a polynucleotide complementary to a polynucleotide of any of a) through d).

**2.** The prenyl protease coding nucleic acid of Claim 1, wherein the nucleic acid encodes a polypeptide that functions as a modulator of an environmental stress response in a plant.

**3.** The prenyl protease coding nucleic acid of Claim 1 or 2, wherein reduced expression of said nucleic acid in a plant increases the plant's tolerance to an environmental stress.

**4.** The prenyl protease coding nucleic acid of Claim 3, wherein the environmental stress is drought.

**5.** The prenyl protease coding nucleic acid of any one of Claims 1 to 4, wherein the nucleic acid encodes a polypeptide that functions in protein farnesylation.

**6.** A first isolated nucleic acid that hybridizes under stringent conditions to a second nucleic acid selected from the group consisting of:
a) a second nucleic acid comprising a polynucleotide of SEQ ID NO: 3 and
b) a second nucleic acid encoding a polypeptide of SEQ ID NO: 4, wherein the first nucleic acid encodes a prenyl protease.

**5.** A vector or an expression cassette comprising a nucleic acid of any one of Claims 1 to 6.

**6.** A transgenic plant cell which is transformed with a vector according to Claim 5 or comprising the expression cassette of claim 5.

**7.** A transgenic plant comprising a plant cell of Claim 6.

**8.** The plant of Claim 6 or 7, wherein the plant is a monocot or dicot.

**9.** The plant of any one of Claims 6 to 8, wherein the plant is selected *trom* the group consisting of maize, wheat, rye, oat, triticale, rice, barley, soybean, peanut, cotton, rapeseed, canola, manihot, pepper, sunflower, tagetes, solanaceous plants, potato, tobacco, eggplant, tomato, Vicia species, pea, alfalfa, coffee, cacao, tea, Salix species, oil palm, coconut, perennial grass and a forage crop.

**10.** A plant seed produced by the plant of Claim 9, wherein the seed is true breeding for an increased tolerance to environmental stress as compared to a wild type 3 variety of the seed.

**11.** Use of the plant seed of claim 10, the plant of any one of claims 6 to 9, the vector or expression cassette of claim 6, or the nucleic acid of any one of claims 1 to 5 for the production of a plant that has an increased tolerance to an environmental stress as compared to a wild type variety of the plant.
